# EUROPEAN PATENT APPLICATION

(11) **EP 2 298 731 A1**
(43) Date of publication of application: **23.03.2011**
(21) Application number: 09769906.0
(22) Date of filing: 24.06.2009
(51) Int. Cl.: C07C 233/78, A61K 31/166, A61K 31/167, A61K 31/277, A61K 31/341, A61K 31/343, A61K 31/353, A61K 31/404, A61K 31/416, A61K 31/426, A61K 31/427, A61K 31/437, A61K 31/44, A61K 31/519, A61K 31/538, A61K 31/55, A61P 25/18, A61P 43/00, C07C 235/42, C07C 237/22, C07C 255/29

(54) **AMIDE COMPOUND**

(30) Priority: 25.06.2008 JP 2008166467
(71) Applicant: Takeda Pharmaceutical Company Limited, Osaka-shi, Osaka 541-0045 (JP)
(72) Inventor: SETOH, Masaki, Osaka-shi Osaka 532-8686 (JP); MIYANOHANA, Yuhei, Osaka-shi Osaka 532-8686 (JP); KOUNO, Mitsunori, Osaka-shi Osaka 532-8686 (JP)
(74) Representative: Held, Stephan
(86) International application number: PCT/JP2009/002902
(87) International publication number: WO 2009/157196

(57) **Abstract**

The present invention aims to provide a prophylactic or therapeutic agent for schizophrenia and the like, containing the compound of the present invention having a GPR52 agonist activity. A compound represented by the following formula (I) or a salt thereof: 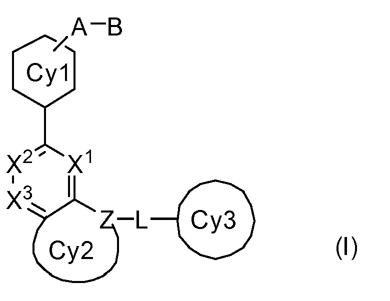 wherein,
A is -CONH or the like,
B is a hydrogen atom or a substituent,
ring Cy1 is a benzene ring or the like,
X¹, X² and X³ are each independently -CH= or -N= or the like, ring Cy2 is a carbon ring or the like,
Z is a carbon atom or a nitrogen atom,
L is a bond or the like,
n is 1 or 2,
R^{b} is a hydrogen atom or a substituent, and
ring Cy3 is a benzene ring or the like.

## Description

### Technical Field

The present invention relates to a novel amide compound and a method for manufacturing the same, and a medicament containing such a novel amide compound. More specifically, the present invention relates to a compound having an agonistic effect on GPR52, which is effective as a medicament for preventing and treating mental disorders, such as schizophrenia, and the like.

### Background Art

Schizophrenia is a disease that occurs in people from adolescence to adulthood and shows characteristic thinking disturbances, disturbances of ego, and behavioral abnormalities associated therewith. The onset of symptoms is allegedly about 1% of the entire population. Most of them are chronic, so that the initiative or interpersonal contact of patients may be decreased, thereby interfering the social lives of the patients. The core symptoms of schizophrenia are broadly classified into (1) positive symptoms such as delusions and hallucination, (2) negative symptoms such as hypesthesia, social withdrawal, diminished motivation, and loss of concentration, and (3) cognitive dysfunction. In these core symptoms, the expression of positive symptoms is intimately involved in over activity of the dopamine nervous system in the mesolimbic system. The expression of the negative symptoms and impaired cognitive function are intimately involved in deterioration of the nervous system such as the glutamic acid nervous system in the cortex of frontal lobe.
In addition, a typical antipsychotic agent having an antagonistic action on a dopamine D2 receptor, such as chlorpromazine, has favorable effects on the positive symptoms. On the other hand, drugs effective to multiple receptors, such as clozapine and olanzapine have certain effects on negative symptoms and cognitive dysfunction. However, it is known that many patients have poor response on these drugs. Also, the typical antipsychotic agent has controversial side effects such as the occurrence of extrapyramidal syndromes, for example, akathisia, dystonia, and Parkinson-like movement disorders and the occurrence of hyperprolactinemia. Furthermore, clozapine may cause agranulocytosis as a grave side effect. An atypical antipsychotic agent such as olanzapine may cause side effects, such as weight gain, lipidosis, excessive sedative effect, and prolonged cardiac QT interval.
Human GPR52 (Sawzdargo et al., Molecular Brain Research, 64: 193-198, 1999) has been known as one of GPCRs. In recent years, because of an increase in cellular cAMP level in nerve cells expressing GPR52 or the like, any of agonists and ligands against GPR52 has been considered to have an effect of improving the positive symptoms of schizophrenia by suppressing the hyperactivation of dopamine pathway in the mesolimbic region, one of the causes of the positive symptoms of schizophrenia. In addition, it has been also found that the agonists and ligands against GPR52 can improve the negative symptoms of schizophrenia and cognitive deficiency by an improvement in decreased function of NMDA receptors in the cerebral cortex, which has been considered as one of the causes of the negative symptoms of schizophrenia and cognitive deficiency (WO 2006/098520).
Therefore, it has been demanded to develop a compound having an agonistic effect on GPR52 and useful as a preventive/therapeutic medicament for mental diseases such as schizophrenia.

On the other hand, as amide compounds, for example, W02007/002433 discloses a protein kinase inhibitor represented by the formula 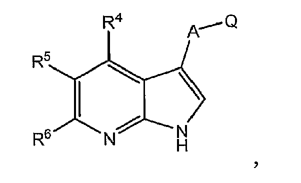 which encompasses amide compounds,
W02006/004984 discloses a protein kinase inhibitor represented by the formula 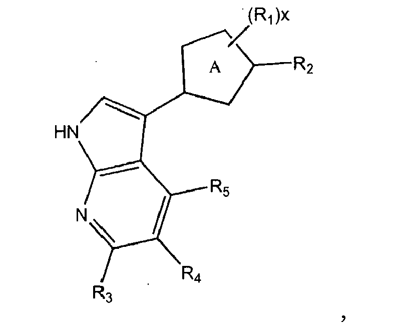 which encompasses amide compounds,
W02005/028475 discloses a protein kinase inhibitor represented by the formula 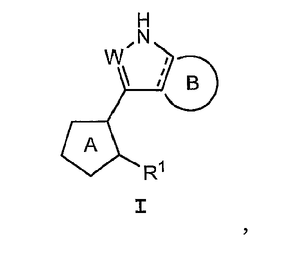 which encompasses amide compounds,
W02005/061519 discloses a kinase inhibitor represented by the formula 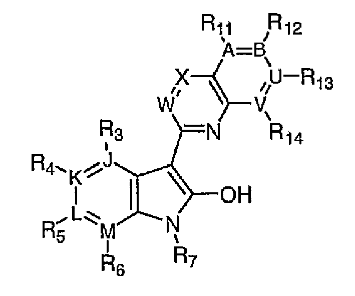 which encompasses amide compounds, and
U.S. patent application publication No. 2007-123519 discloses a phosphodiesterase PDE2 inhibitor represented by the formula 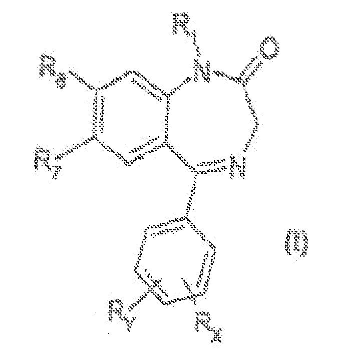 which encompasses amide compounds.

### Citation List

### Patent Literature

[PTL 1]
   WO 2006/098520
[PTL 2]
   WO 2007/002433
[PTL 3]
   WO 2006/004984
[PTL 4]
   WO 2005/028475
[PTL 5]
   WO 2005/061519
[PTL 6]
   U.S. patent application publication No. 2007-123519

### Non Patent Literature

[NPL 1]
   Sawzdargo et al., Molecular Brain Research, 64: pp. 193-198, 1999.

### Summary of Invention

### Technical Problem

An object of the present invention is to provide a compound having an agonistic effect on GPR52 and useful as a preventive/therapeutic medicament for mental diseases such as schizophrenia.

### Solution to Problem

The present inventors have found that compounds represented by the below formula (Iₒ) or salts thereof (herein also referred to as compounds (Iₒ)) have an agonistic effect on GPR52 and finally completed the present invention by further investigations.
Furthermore, among the compounds (Iₒ), compounds represented by the below formula (I) or a salt thereof (herein also referred to as compound (I)) are novel compounds.
The compound (Iₒ) including the compound (I) or prodrugs thereof will be herein also referred to as the compounds of the present invention.

Accordingly, the present invention provides
[1] a compound represented by the formula (I) 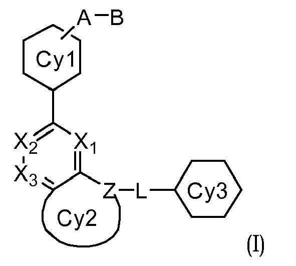 wherein
   A is -CONR^{a}- or -NR^{a}CO-,
   Ra is a hydrogen atom or a substituent,
   B is a hydrogen atom or a substituent,
   ring Cy1 is (1) a benzene ring or (2) a 6-membered nitrogen-containing aromatic heterocycle, each may have substituent(s) in addition to a group represented by -A-B, X¹, X² and X³ are each independently -CRx= or -N=,
   Rx is independently a hydrogen atom, a halogen atom or a lower alkyl group which may be halogenated in each occurrence,
   ring Cy2 is (1) a carbon ring having a carbon number of 5 to 7 or (2) a 5- to 7-membered heterocycle having 1 or 2 heteroatoms selected from a nitrogen atom, an oxygen atom and a sulfur atom, each may have substituent(s) (excluding oxo group, C₆₋₁₄ aryl group and carboxyl group which may be esterified),
   Z is a carbon atom or a nitrogen atom,
   L is a bond, -(CH₂)n-, -L'-, -L'-CH₂- or -CH₂-L'-,
   n is 1 or 2,
   L' is -O-, -NR^{b}- or -S(O)ₘ-,
   R^{b} is a hydrogen atom or a substituent,
   m is an integer of 0 to 2, and
   ring Cy3 is (1) a benzene ring or (2) a 6-membered nitrogen-containing aromatic heterocycle, each may have substituent(s),
   provided that a moiety represented by 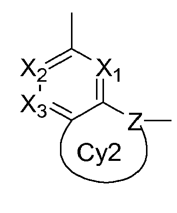 is not 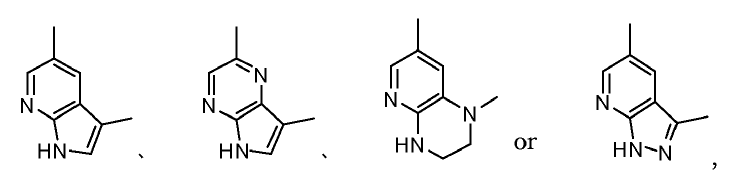 or a salt thereof;
[2] the compound described in the aforementioned [1], wherein the ring Cy1 is a benzene ring or a pyridine ring;
[3] the compound described in the aforementioned [1], wherein X¹ and X² are each independently -CH= or -N=;
[4] the compound described in the aforementioned [1], wherein the ring Cy2 is (1) a carbon ring having a carbon number of 5 or 6 or (2) a 5- or 6-membered heterocycle having 1 or 2 heteroatoms selected from a nitrogen atom, an oxygen atom and a sulfur atom, each may have substituent(s) (excluding oxo group, C₆₋₁₄ aryl group and carboxyl group which may be esterified);
[5] the compound described in the aforementioned [1], wherein the moiety represented by 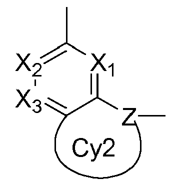 is 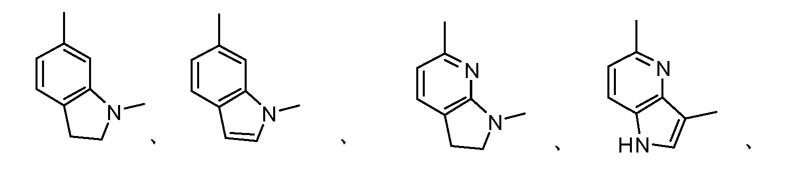 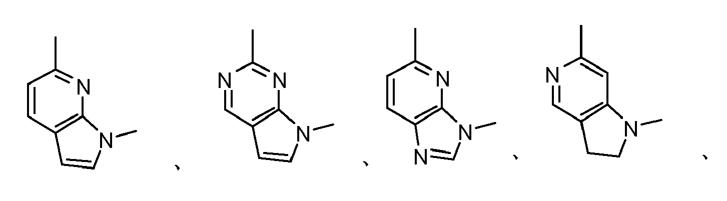 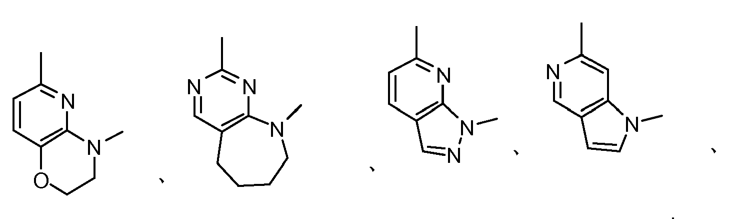 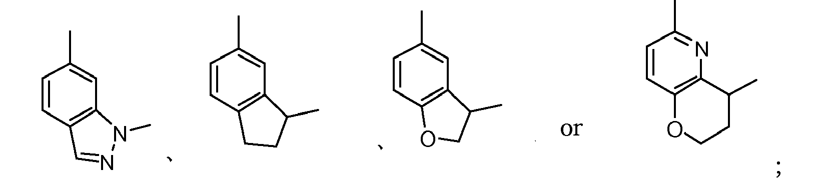
[6] the compound described in the aforementioned [1], wherein the moiety represented by 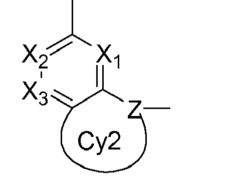 is 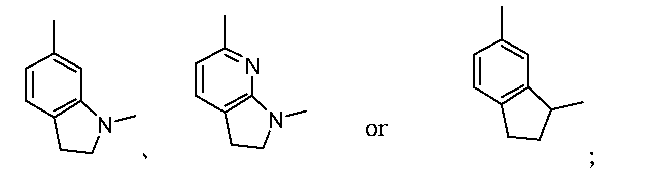
[7] the compound described in the aforementioned [1], wherein L is a bond, -CH₂-, - O-, -NR^{b}- or -S(O)ₘ-;
[8] the compound described in the aforementioned [1], wherein Cy3 is a dichlorobenzene ring;
[9] the compound described in the aforementioned [1], wherein the ring Cy1 is a benzene ring or a pyridine ring,
   X¹ and X² are each independently -CH= or -N=,
   the ring Cy2 is (1) a carbon ring having a carbon number of 5 or 6 or (2) a 5- or 6-membered heterocycle having 1 or 2 heteroatoms selected from a nitrogen atom, an oxygen atom and a sulfur atom, each may have substituent(s) (excluding carboxyl group which may be esterified),
   L is a bond, -CH₂-, -O-, -NR^{b}- or -S(O)ₘ-, and
   Cy3 is a dichlorobenzene ring;
[10] the compound described in the aforementioned [1], wherein A is -CONH- or -CONH-,
   B is
   (1) a C₁₋₆ alkyl group which may have one or more substituents selected from
      (a) a cyano group,
      (b) a hydroxy group,
      (c) C₁₋₆ alkoxy,
      (d) a di-C₁₋₆ alkyl-amino group,
      (e) a carbamoyl group,
      (f) a C₁₋₆ alkyl-sulfanyl group,
      (g) a C₁₋₆ alkyl-sulfinyl group,
      (h) a C₁₋₆ alkyl-sulfonyl group, and
      (i) a 5- to 7-membered heterocyclic group having one or more heteroatoms selected from a nitrogen atom, an oxygen atom and a sulfur atom,
   (2) a C₃₋₁₀ cycloalkyl group, or
   (3) a 5- to 7-membered heterocyclic group,
      the ring Cy1 is (1) a benzene ring or (2) a 6-membered nitrogen-containing heterocycle,
      the moiety represented by 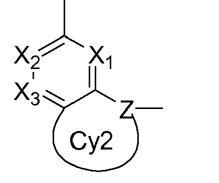 is 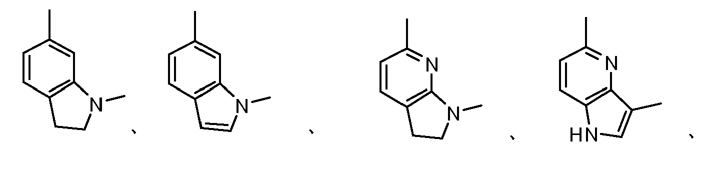 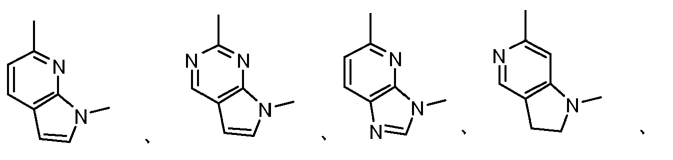 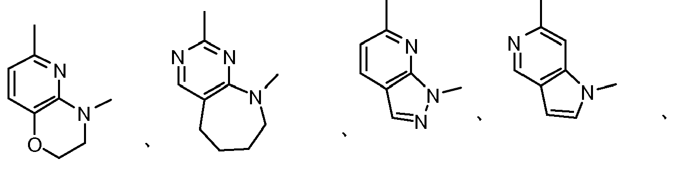 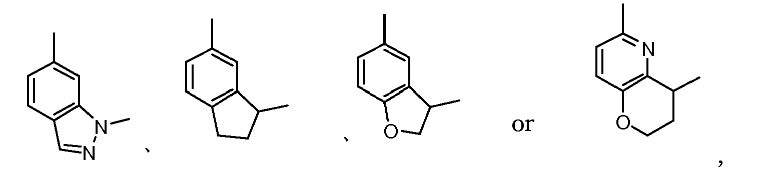 L is a bond, -CH₂-, -NH- or -O-, and
      the ring Cy3 is a benzene ring or a pyridine ring, each may have one or more substituents selected from a halogen atom, a C₁₋₆ alkyl group which may be halogenated and a C₁₋₆ alkoxy group which may be halogenated;
[11] the compound described in the aforementioned [1], which is N-(2-cyanoethyl)-3-[1-(2,4-dichlorophenyl)-2,3-dihydro-1H-indol-6-yl]benzamide or a salt thereof;
[12] the compound described in the aforementioned [1], which is 3-[1-(2,4-dichlorobenzyl)-1H-pyrrolo[2,3-b]pyridin-6-yl]-N-(2-hydroxyethyl)benzamide or a salt thereof;
[13] the compound described in the aforementioned [1], which is 3-[1-(2,4-dichlorobenzyl)-2,3-dihydro-1H-pyrrolo[2,3-b]pyridin-6-yl]-N-(2-hydroxyethyl)benzamide or a salt thereof;
[14] the compound described in the aforementioned [1], which is 3-[3-[2-(3,4-dimethoxyphenyl)ethyl]-3H-imidazo[4,5-b]pyridin-5-yl]-N-(2-pyrrolidin-1-ylethyl)benzamide or a salt thereof;
[15] the compound described in the aforementioned [1], which is N-(2-cyanoethyl)-3-[4-(2,4-dichlorophenyl)-3,4-dihydro-2H-1,4-benzoxazin-6-yl]benzamide or a salt thereof;
[16] the compound described in the aforementioned [1], which is 3-[3-(2,4-dichlorophenyl)-2,3-dihydro-1H-inden-5-yl]-N-[2-(methylsulfinyl)ethyl]benzamide or a salt thereof;
[17] the compound described in the aforementioned [1], which is N-(2-cyanoethyl)-3-[3-(2,4-dichlorophenoxy)-2,3-dihydro-1H-inden-5-yl]benzamide or a salt thereof;
[18] the compound described in the aforementioned [1], which is 3-[3-[(2,4-dichlorophenyl)amino]-2,3-dihydro-1-benzofuran-5-yl]-N-(2-hydroxyethyl)benzamide or a salt thereof;
[19] a prodrug of the compound described in the aforementioned [1];
[20] a medicament comprising the compound described in the aforementioned [1] or the prodrug described in the aforementioned [19];
[21] a GPR52 activating agent comprising a compound represented by the formula (I₀) 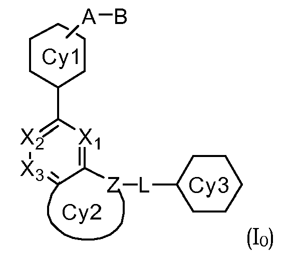 wherein
   A is -CONR^{a}- or -NR^{a}CO-,
   Ra is a hydrogen atom or a substituent,
   B is a hydrogen atom or a substituent,
   ring Cy1 is (1) a benzene ring or (2) a 6-membered nitrogen-containing aromatic heterocycle, each may have substituent(s) in addition to a group represented by -A-B, X¹, X² and X³ are each independently -CR^{x}= or -N=,
   Rx is independently a hydrogen atom, a halogen atom or a lower alkyl group which may be halogenated in each occurrence,
   ring Cy2 is (1) a carbon ring having a carbon number of 5 to 7 or (2) a 5- to 7-membered heterocycle having 1 or 2 heteroatoms selected from a nitrogen atom, an oxygen atom and a sulfur atom, each may have substituent(s) (excluding oxo group),
   Z is a carbon atom or a nitrogen atom,
   L is a bond, -(CH₂)n-, -L'-, -L'-CH₂- or -CH₂-L'-, n is 1 or 2,
   L' is -O-, -NR^{b}- or -S(O)ₘ-,
   R^{b} is a hydrogen atom or a substituent,
   m is an integer of 0 to 2, and
   ring Cy3 is (1) a benzene ring or (2) a 6-membered nitrogen-containing aromatic heterocycle, each may have substituent(s),
   provided that a moiety represented by 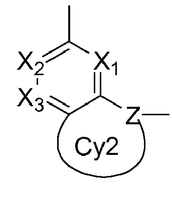 is not 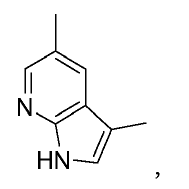 or a salt thereof or a prodrug thereof;
[22] a prophylactic or therapeutic agent for schizophrenia comprising a compound represented by the formula (I₀) 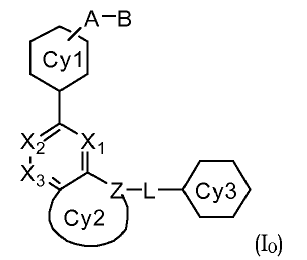 wherein
   A is -CONR^{a}- or -NR^{a}CO-,
   Ra is a hydrogen atom or a substituent,
   B is a hydrogen atom or a substituent,
   ring Cy1 is (1) a benzene ring or (2) a 6-membered nitrogen-containing aromatic heterocycle, each may have substituent(s) in addition to a group represented by -A-B,
   X¹, X² and X³ are each independently -CRx= or -N=,
   Rx is independently a hydrogen atom, a halogen atom or a lower alkyl group which may be halogenated in each occurrence,
   ring Cy2 is (1) a carbon ring having a carbon number of 5 to 7 or (2) a 5- to 7-membered heterocycle having 1 or 2 heteroatoms selected from a nitrogen atom, an oxygen atom and a sulfur atom, each may have substituent(s) (excluding oxo group), Z is a carbon atom or a nitrogen atom,
   L is a bond, -(CH₂)n-, -L'-, -L'-CH₂- or -CH₂-L'-,
   n is 1 or 2,
   L' is -O-, -NR^{b}- or -S(O)ₘ-,
   R^{b} is a hydrogen atom or a substituent,
   m is an integer of 0 to 2, and
   ring Cy3 is (1) a benzene ring or (2) a 6-membered nitrogen-containing aromatic heterocycle, each may have substituent(s),
   provided that a moiety represented by 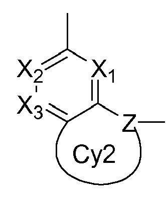 is not 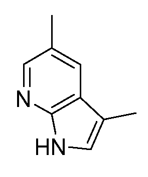 or a salt thereof or a prodrug thereof;

[23] a method of activating GPR52, comprising administering, to a subject, an effective amount of a compound represented by the formula (I₀) 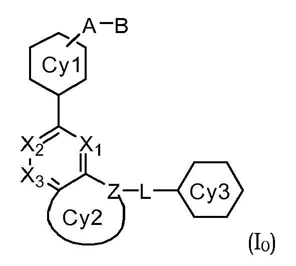 wherein
   A is -CONR^{a}- or -NR^{a}CO-,
   Ra is a hydrogen atom or a substituent,
   B is a hydrogen atom or a substituent,
   ring Cy1 is (1) a benzene ring or (2) a 6-membered nitrogen-containing aromatic heterocycle, each may have substituent(s) in addition to a group represented by -A-B,
   X¹, X² and X³ are each independently -CRx= or -N=,
   Rx is independently a hydrogen atom, a halogen atom or a lower alkyl group which may be halogenated in each occurrence,
   ring Cy2 is (1) a carbon ring having a carbon number of 5 to 7 or (2) a 5- to 7-membered heterocycle having 1 or 2 heteroatoms selected from a nitrogen atom, an oxygen atom and a sulfur atom, each may have substituent(s) (excluding oxo group),
   Z is a carbon atom or a nitrogen atom,
   L is a bond, -(CH₂)n-, -L'-, -L'-CH₂- or -CH₂-L'-,
   n is 1 or 2,
   L' is -O-, -NR^{b}- or -S(O)ₘ-,
   R^{b} is a hydrogen atom or a substituent,
   m is an integer of 0 to 2, and
   ring Cy3 is (1) a benzene ring or (2) a 6-membered nitrogen-containing aromatic heterocycle, each may have substituent(s),
   provided that a moiety represented by 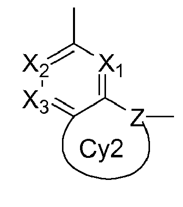 is not 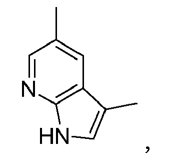 or a salt thereof or a prodrug thereof;
[24] a method of preventing or treating schizophrenia, comprising administering, to a subject, an effective amount of a compound represented by the formula (I₀) 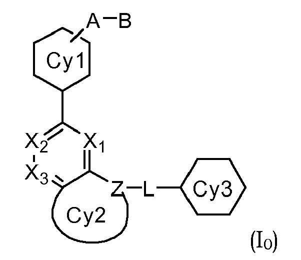 wherein
   A is -CONR^{a}- or -NR^{a}CO-,
   Ra is a hydrogen atom or a substituent,
   B is a hydrogen atom or a substituent, ring Cy1 is (1) a benzene ring or (2) a 6-membered nitrogen-containing aromatic heterocycle, each may have substituent(s) in addition to a group represented by -A-B,
   X¹, X² and X³ are each independently -CRx= or -N=,
   Rx is independently a hydrogen atom, a halogen atom or a lower alkyl group which may be halogenated in each occurrence,
   ring Cy2 is (1) a carbon ring having a carbon number of 5 to 7 or (2) a 5- to 7-membered heterocycle having 1 or 2 heteroatoms selected from a nitrogen atom, an oxygen atom and a sulfur atom, each may have substituent(s) (excluding oxo group), Z is a carbon atom or a nitrogen atom,
   L is a bond, -(CH₂)n-, -L'-, -L'-CH₂- or -CH₂-L'-,
   n is 1 or 2,
   L' is -O-, -NR^{b}- or -S(O)ₘ-,
   R^{b} is a hydrogen atom or a substituent,
   m is an integer of 0 to 2, and
   ring Cy3 is (1) a benzene ring or (2) a 6-membered nitrogen-containing aromatic heterocycle, each may have substituent(s),
   provided that a moiety represented by 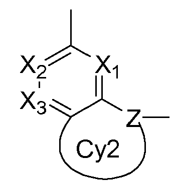 is not 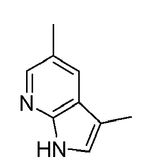 or a salt thereof or a prodrug thereof;

[25] use of a compound represented by the formula (I₀) 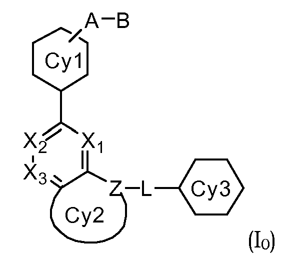 wherein
   A is -CONR^{a}- or -NR^{a}CO-,
   Ra is a hydrogen atom or a substituent,
   B is a hydrogen atom or a substituent,
   ring Cy1 is (1) a benzene ring or (2) a 6-membered nitrogen-containing aromatic heterocycle, each may have substituent(s) in addition to a group represented by -A-B, X¹, X² and X³ are each independently -CRx= or -N=,
   Rx is independently a hydrogen atom, a halogen atom or a lower alkyl group which may be halogenated in each occurrence,
   ring Cy2 is (1) a carbon ring having a carbon number of 5 to 7 or (2) a 5- to 7-membered heterocycle having 1 or 2 heteroatoms selected from a nitrogen atom, an oxygen atom and a sulfur atom, each may have substituent(s) (excluding oxo group),
   Z is a carbon atom or a nitrogen atom,
   L is a bond, -(CH₂)n-, -L'-, -L'-CH₂- or -CH₂-L'-,
   n is 1 or 2,
   L' is -O-, -NR^{b}- or -S(O)ₘ-,
   R^{b} is a hydrogen atom or a substituent,
   m is an integer of 0 to 2, and
   ring Cy3 is (1) a benzene ring or (2) a 6-membered nitrogen-containing aromatic heterocycle, each may have substituent(s),
   provided that a moiety represented by 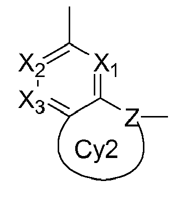 is not 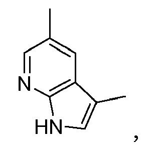 or a salt thereof or a prodrug thereof, for the manufacture of a GPR52 activating agent;
[26] use of a compound represented by the formula (I₀) 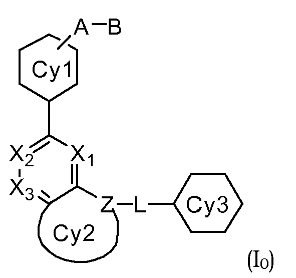 wherein
   A is -CONR^{a}- or -NR^{a}CO-,
   Ra is a hydrogen atom or a substituent,
   B is a hydrogen atom or a substituent,
   ring Cy1 is (1) a benzene ring or (2) a 6-membered nitrogen-containing aromatic heterocycle, each may have substituent(s) in addition to a group represented by -A-B,
   X¹, X² and X³ are each independently -CRx= or -N=,
   Rx is independently a hydrogen atom, a halogen atom or a lower alkyl group which may be halogenated in each occurrence,
   ring Cy2 is (1) a carbon ring having a carbon number of 5 to 7 or (2) a 5- to 7-membered heterocycle having 1 or 2 heteroatoms selected from a nitrogen atom, an oxygen atom and a sulfur atom, each may have substituent(s) (excluding oxo group), Z is a carbon atom or a nitrogen atom,
   L is a bond, -(CH₂)n-, -L'-, -L'-CH₂- or -CH₂-L'-,
   n is 1 or 2,
   L' is -O-, -NR^{b}- or -S(O)ₘ-,
   R^{b} is a hydrogen atom or a substituent,
   m is an integer of 0 to 2, and
   ring Cy3 is (1) a benzene ring or (2) a 6-membered nitrogen-containing aromatic heterocycle, each may have substituent(s),
   provided that a moiety represented by 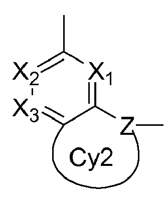 is not 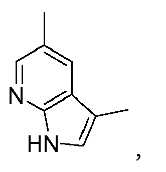 or a salt thereof or a prodrug thereof, for the manufacture of a prophylactic or therapeutic agent for schizophrenia;
   and the like.

### Advantageous Effects of Invention

The compound of the present invention has an agonistic effect on GPR52 and is advantageously used as a preventive/therapeutic medicament for mental diseases such as schizophrenia.

### Description of Embodiments

Hereinafter, the present invention will be described in detail.

Unless otherwise noted, the "halogen atoms" used herein include fluorine, chlorine, bromine, and iodine.
Unless otherwise noted, the expression "which may be halogenated" used herein means that one or more (e.g., one to three) halogen atoms may be provided as substituents.
Unless otherwise noted, the "carboxyl (group) which may be esterified" used herein include carboxyl, lower alkoxy-carbonyl which may be substituted, C₆₋₁₄ aryloxy-carbonyl which may be substituted, C₇₋₁₆ aralkyloxy-carbonyl which may be substituted, and silyloxy-carbonyl which may be substituted (e.g., TMS-O-CO-, TES-O-CO-, TBS-O-CO-, TIPS-O-CO-, and TBDPS-O-CO-).
Unless otherwise noted, for example, the "lower alkoxy-carbonyl (group)" used herein may be any of methoxycarbonyl, ethoxycarbonyl, propoxycarbonyl, and tert-butoxycarbonyl.
Unless otherwise noted, for example, the "C₆₋₁₄ aryloxy-carbonyl (group)" used herein may be phenoxycarbonyl.
Unless otherwise noted, for example, the "C₇₋₁₆ aralkyloxy-carbonyl (group)" used herein may be any of benzyloxycarbonyl and phenethyloxycarbonyl.

Unless otherwise noted, for example, the "lower alkyl (group)" used herein may be C₁₋₆ alkyl (group).
Unless otherwise noted, for example, the "C₁₋₆ alkyl (group)" used herein may be any of methyl, ethyl, isopropyl, butyl, isobutyl, sec-butyl, tert-butyl, pentyl, isopentyl, neopentyl, and hexyl.
Unless otherwise noted, the "C₁₋₆ alkyl (group) which may be halogenated" used herein means C₁₋₆ alkyl (group) which may be substituted with a halogen atom and the example thereof may be trifluoromethyl.

Unless otherwise noted, for example, the "lower alkenyl (group)" used herein may be C₂₋₆ alkenyl (group).
Unless otherwise noted, for example, the "C₂₋₆ alkenyl (group)" used herein may be any of vinyl, 1-propen-1-yl, 2-propen-1-yl, isopropenyl, 2-buten-1-yl, 4-penten-1-yl, and 5-hexen-1-yl.

Unless otherwise noted, the "lower alkynyl (group)", for example, the (C₂₋₆) "lower alkynyl" used herein may be any of ethynyl, 1-propyn-1-yl, 2-propyn-1-yl, 4-pentyn-1-yl, and 5-hexyn-1-yl.

Unless otherwise noted, for example, "C₃₋₈ cycloalkyl (group)" used herein may be any of cyclopropyl, cyclobutyl, cyclopentyl, and cyclohexyl.

Unless otherwise noted, for example, the "C₆₋₁₄ aryl (group)" used herein may be any of phenyl, 1-naphthyl, 2-naphthyl, 2-biphenylyl, 3-biphenylyl, 4-biphenylyl, and 2-anthryl.

Unless otherwise noted, for example, the "C₇₋₁₆ aralkyl (group)" used herein may be any of benzyl, phenethyl, diphenylmethyl, 1-naphthylmethyl, 2-naphthylmethyl, 2,2-diphenylethyl, 3-phenylpropyl, 4-phenylbutyl, 5-phenylpentyl, 2-biphenylylmethyl, 3-biphenylylmethyl, and 4-biphenylylmethyl.

Unless otherwise noted, for example, the "C₆₋₁₄ aryl-C₂₋₆ alkenyl (group)" used herein may be styryl.

Unless otherwise noted, examples of the "heterocyclic group" (and heterocyclic ring portions in the substituents) used herein include: 3- to 14-membered (monocyclic, bicyclic, or tricyclic) heterocyclic groups with one to five of one to three kinds of heteroatoms selected from a nitrogen atom, a sulfur atom, and an oxygen atom in addition to carbon atoms. Examples of such heterocyclic groups include aromatic heterocyclic group such as pyrrolyl (e.g., 1- pyrrolyl, 2-pyrrolyl, 3-pyrrolyl), furyl (e.g., 2-furyl, 3-furyl), thienyl (e.g., 2-thienyl, 3-thienyl), pyrazolyl (e.g., 1-pyrazolyl, 3-pyrazolyl, 4-pyrazolyl), imidazolyl (e.g., 1-imidazolyl, 2-imidazolyl, 4-imidazolyl), isoxazolyl (e.g., 3-isoxazolyl, 4-isoxazolyl, 5-isoxazolyl), oxazolyl (e.g., 2-oxazolyl, 4-oxazolyl, 5-oxazolyl), isothiazolyl (e.g., 3-isothiazolyl, 4-isothiazolyl, 5-isothiazolyl), thiazolyl (e.g., 2-thiazolyl, 4-thiazolyl, 5-thiazolyl), triazolyl (1,2,3-triazol-4-yl, 1,2,4-triazol-3-yl), oxadiazolyl (1,2,4-oxadiazol-3-yl, 1,2,4-oxadiazol-5-yl), thiadiazolyl (1,2,4-thiadiazol-3-yl, 1,2,4-thiadiazol-5-yl), tetrazolyl, pyridyl (e.g., 2-pyridyl, 3-pyridyl, 4-pyridyl), pyridazinyl (e.g., 3-pyridazinyl, 4-pyridazinyl), pyrimidinyl (e.g., 2-pyrimidinyl, 4-pyrimidinyl, 5-pyrimidinyl), pyrazinyl, isoindolyl (e.g., 1-isoindolyl, 2-isoindolyl, 3-isoindolyl, 4-isoindolyl, 5-isoindolyl, 6-isoindolyl, 7-isoindolyl), indolyl (e.g., 1-indolyl, 2-indolyl, 3-indolyl, 4-indolyl, 5-indolyl, 6-indolyl, 7-indolyl), benzo[b]furanyl (e.g., 2-benzo[b]furanyl, 3-benzo[b]furanyl, 4-benzo[b]furanyl, 5-benzo[b]furanyl, 6-benzo[b]furanyl, 7-benzo[b]furanyl), benzo[c]furanyl (e.g., 1-benzo[c]furanyl, 4-benzo[c]furanyl, 5-benzo[c]furanyl), benzo[b]thienyl, (e.g., 2-benzo[b]thienyl, 3-benzo[b]thienyl, 4-benzo[b]thienyl, 5-benzo[b]thienyl, 6-benzo[b]thienyl, 7-benzo[b]thienyl), benzo[c]thienyl (e.g., 1-benzo[c]thienyl, 4-benzo[c]thienyl, 5-benzo[c]thienyl), indazolyl (e.g., 1-indazolyl, 2-indazolyl, 3-indazolyl, 4-indazolyl, 5-indazolyl, 6-indazolyl, 7-indazolyl), benzimidazolyl (e.g., 1-benzimidazolyl, 2-benzimidazolyl, 4-benzimidazolyl, 5-benzimidazolyl), 1,2-benzisoxazolyl (e.g., 1,2-benzisoxazol-3-yl, 1,2-benzisoxazol-4-yl, 1,2-benzisoxazol-5-yl, 1,2-benzisoxazol-6-yl, 1,2-benzisoxazol-7-yl), benzoxazolyl (e.g., 2-benzoxazolyl, 4-benzoxazolyl, 5-benzoxazolyl, 6-benzoxazolyl, 7-benzoxazolyl), 1,2-benzisothiazolyl (e.g., 1,2-benzisothiazol-3-yl, 1,2-benzisothiazol-4-yl, 1,2-benzisothiazol-5-yl, 1,2-benzisothiazol-6-yl, 1,2-benzisothiazol-7-yl), benzothiazolyl (e.g., 2-benzothiazolyl, 4-benzothiazolyl, 5-benzothiazolyl, 6-benzothiazolyl, 7-benzothiazolyl), isoquinolyl (e.g., 1-isoquinolyl, 3-isoquinolyl, 4-isoquinolyl, 5-isoquinolyl), quinolyl (e.g., 2-quinolyl, 3-quinolyl, 4-quinolyl, 5-quinolyl, 8-quinolyl), cinnolinyl (e.g., 3-cinnolinyl, 4-cinnolinyl, 5-cinnolinyl, 6-cinnolinyl, 7-cinnolinyl, 8-cinnolinyl), phthalazinyl (e.g., 1-phthalazinyl, 4-phthalazinyl, 5-phthalazinyl, 6-phthalazinyl, 7-phthalazinyl, 8-phthalazinyl), quinazolinyl (e.g., 2-quinazolinyl, 4-quinazolinyl, 5-quinazolinyl, 6-quinazolinyl, 7-quinazolinyl, 8-quinazolinyl), quinoxalinyl (e.g., 2-quinoxalinyl, 3-quinoxalinyl, 5-quinoxalinyl, 6-quinoxalinyl, 7-quinoxalinyl, 8-quinoxalinyl), pyrazolo[1,5-a]pyridyl (pyrazolo[1,5-a]pyridin-2-yl, pyrazolo[1,5-a]pyridin-3-yl, pyrazolo[1,5-a]pyridin-4-yl, pyrazolo[1,5-a]pyridin-5-yl, pyrazolo[1,5-a]pyridin-6-yl, pyrazolo[1,5-a]pyridin-7-yl), and imidazo[1,2-a]pyridyl (imidazo[1,2-a]pyridin-2-yl, imidazo[1,2-a]pyridin-3-yl, imidazo[1,2-a]pyridin-5-yl, imidazo[1,2-a]pyridin-6-yl, imidazo[1,2-a]pyridin-7-yl, imidazo[1,2-a]pyridin-8-yl); and nonaromatic heterocyclic groups such as oxazolidinyl (e.g., imidazolinyl (e.g., 1-imidazolinyl, 2-imidazolinyl, 4-imidazolinyl), aziridinyl (e.g., 1-aziridinyl, 2-aziridinyl), azetidinyl (e.g., 1-azetidinyl, 2-azetidinyl), pyrrolidinyl (e.g., 1-pyrrolidinyl, 2-pyrrolidinyl, 3-pyrrolidinyl), piperidinyl (e.g., 1-piperidinyl, 2-piperidinyl, 3-piperidinyl), azepanyl (e.g., 1-azepanyl, 2-azepanyl, 3-azepanyl, 4-azepanyl), azocanyl (e.g., 1-azocanyl, 2-azocanyl, 3-azocanyl, 4-azocanyl), piperazinyl (e.g., 1,4-piperazin-1-yl, 1,4-piperazin-2-yl), diazepanyl (e.g., 1,4-diazepan-1-yl, 1,4-diazepan-2-yl, 1,4-diazepan-5-yl, 1,4-diazepan-6-yl), diazocanyl(1,4-diazocan-1-yl, 1,4-diazocan-2-yl, 1,4-diazocan-5-yl, 1,4-diazocan-6-yl, 1,5-diazocan-1-yl, 1,5-diazocan-2-yl, 1,5-diazocan-3-yl), 1-morpholinyl, 4-thiomorpholinyl, and 2-oxazolidinyl;
heterocyclic groups obtained by partially hydrogenating the above aromatic heterocyclic groups (e.g., heterocyclic groups such as indolyl, and dihydroquinolyl); and
heterocyclic groups obtained by partially dehydrogenating the above nonaromatic heterocyclic groups (e.g., dihydrofuranyl).

Unless otherwise noted, for example, the "lower alkoxy (group)" used herein may be C₁₋₆ alkoxy.
Unless otherwise noted, for example, the "C₁₋₆ alkoxy (group)" used herein may be any of methoxy, ethoxy, propoxy, isopropoxy, butoxy, isobutoxy, sec-butoxy, pentyloxy, and hexyloxy.

Unless otherwise noted, for example, the "C₃₋₈ cycloalkoxy (group)" used herein may be any of cyclopropoxy, cyclobutoxy, cyclopentyloxy, and cyclohexyloxy.

Unless otherwise noted, for example, the "C₆₋₁₄ aryloxy (group)" used herein may be any of phenyloxy, 1-naphthyloxy, and 2-naphthyloxy.

Unless otherwise noted, for example, the "C₇₋₁₆ aralkyloxy (group)" may be any of benzyloxy and phenethyloxy.

Unless otherwise noted, for example, the "lower alkyl-carbonyloxy (group)" used herein may be C₁₋₆ alkyl-carbonyloxy.
Unless otherwise noted, for example, the "C₁₋₆ alkyl-carbonyloxy (group)" used herein may be any of acetoxy and propionyloxy.

Unless otherwise noted, for example, the "lower alkoxy-carbonyloxy (group)" used herein may be C₁₋₆ alkoxy-carbonyloxy (group).
Unless otherwise noted, for example, the "C₁₋₆ alkoxy-carbonyloxy (group)" used herein may be any of methoxycarbonyloxy, ethoxycarbonyloxy, propoxycarbonyloxy, and butoxycarbonyloxy.

Unless otherwise noted, for example, the "mono-lower alkyl-carbamoyloxy (group)" used herein may be mono-C₁₋₆ alkyl-carbamoyloxy (group).
Unless otherwise noted, for example, the "mono-C₁₋₆ alkyl-carbamoyloxy (group)" used herein may be any of methylcarbamoyloxy and ethylcarbamoyloxy.

Unless otherwise noted, for example, the "di-lower alkyl-carbamoyloxy (group)" used herein may be di-C₁₋₆ alkyl-carbamoyloxy (group).
Unless otherwise noted, for example, the "di-C₁₋₆ alkyl-carbamoyloxy (group)" used herein may be any of dimethylcarbamoyloxy and diethylcarbamoyloxy.

Unless otherwise noted, for example, the "C₆₋₁₄ aryl-carbonyloxy (group)" used herein may be any of benzoyloxy and naphthylcarbonyloxy.

Unless otherwise noted, for example, the "mono- or di-C₆₋₁₄ aryl-carbamoyloxy (group)" used herein may be phenylcarbamoyloxy and naphthylcarbamoyloxy.

Unless otherwise noted, for example, the heterocyclic moiety of the "heterocyclic oxy (group)" used herein may be the same "heterocyclic group" as any of those described above. Specifically, examples of the "heterocyclic oxy (group)" include 5- to 14-membered heterocyclic-oxy (group) that contains one to five of one to three kinds of heteroatoms selected from a nitrogen atom, a sulfur atom, and an oxygen atom in addition to carbon atoms.

Unless otherwise noted, for example, the aromatic heterocyclic moiety of the "aromatic heterocyclic oxy (group)" used herein may be the same "aromatic heterocyclic group" as one provided as an example of the aforementioned "heterocyclic group". Specifically, examples of the "aromatic heterocyclic oxy (group)" include 3- to 14-membered aromatic heterocyclic-oxy containing one to five of one to three kinds of heteroatoms selected from a nitrogen atom, a sulfur atom, and an oxygen atom in addition to carbon atoms.

Unless otherwise noted, for example, the "lower alkylthio (group)" used herein may be C₁₋₆ alkylthio (group).
Unless otherwise noted, for example, the "C₁₋₆ alkylthio (group)" used herein may be any of methylthio, ethylthio, propylthio, isopropylthio, butylthio, sec-butylthio, and tert-butylthio.

Unless otherwise noted, for example, the "C₃₋₈ cycloalkylthio (group)" used herein may be any of cyclopropylthio, cyclobutylthio, cyclopentylthio, and cyclohexylthio.

Unless otherwise noted, for example, the "C₆₋₁₄ arylthio (group)" used herein may be any of phenylthio, 1-naphthylthio, and 2-naphthylthio.

Unless otherwise noted, for example, the "C₇₋₁₆ aralkylthio (group)" used herein may be any of benzylthio and phenethylthio.

Unless otherwise noted, for example, the heterocyclic moiety of the "heterocyclic thio (group)" may be the same "heterocyclic group" as one described above. Specifically, the "heterocyclic thio (group)" may be 5- to 14-membered heterocyclic-thio (group) containing one to five of one to three kinds of heteroatoms selected from a nitrogen atom, a sulfur atom, and an oxygen atom in addition to carbon atoms.

Unless otherwise noted, for example, the "lower alkyl-carbonyl (group)" used herein may be C₁₋₆ alkyl-carbonyl.
Unless otherwise noted, for example, the "C₁₋₆ alkyl-carbonyl (group)" used herein may be any of acetyl, propionyl, and pivaloyl.

Unless otherwise noted, for example, the "C₃₋₈ cycloalkylcarbonyl (group)" used herein may be any of cyclopropylcarbonyl, cyclopentylcarbonyl, and cyclohexylcarbonyl.

Unless otherwise noted, for example, the "C₆₋₁₄ aryl-carbonyl (group)" used herein may be any of benzoyl, 1-naphthoyl, and 2-naphthoyl.

Unless otherwise noted, for example, the "C₇₋₁₆ aralkyl-carbonyl (group)" used herein may be any of any of phenylacetyl and 3-phenylpropionyl.

Unless otherwise noted, for example, the heterocyclic moiety of the "heterocyclic-carbonyl (group)" may be the same "heterocyclic group" as one described above. Specifically, it may be 3- to 14-membered heterocyclic-carbonyl (group) containing one to five of one to three kinds of heteroatoms selected from a nitrogen atom, a sulfur atom, and an oxygen atom in addition to carbon atoms. More specifically, examples of such heterocyclic-carbonyl (group) include picolinoyl, nicotinoyl, isonicotinoyl, 2-thenoyl, 3-thenoyl, 2- furoyl, 3-furoyl, 1-morpholinylcarbonyl, 4-thiomorpholinylcarbonyl, aziridin-1-ylcarbonyl, aziridin-2-ylcarbonyl, azetidin-1-ylcarbonyl, azetidin-2-ylcarbonyl, pyrrolidin-1-ylcarbonyl, pyrrolidin-2-ylcarbonyl, pyrrolidin-3-ylcarbonyl, piperidin-1-ylcarbonyl, piperidin-2-ylcarbonyl, piperidin-3-ylcarbonyl, azepan-1-ylcarbonyl, azepan-2-ylcarbonyl, azepan-3-ylcarbonyl, azepan-4-ylcarbonyl, azocan-1-ylcarbonyl, azocan-2-ylcarbonyl, azocan-3-ylcarbonyl, azocan-4-ylcarbonyl, 1,4-piperazin-1-ylcarbonyl, 1,4-piperazin-2-ylcarbonyl, 1,4-diazepan-1-ylcarbonyl, 1,4-diazepan-2-ylcarbonyl, 1,4-diazepan-5-ylcarbonyl, 1,4-diazepan-6-ylcarbonyl, 1,4-diazocan-1-ylcarbonyl, 1,4-diazocan-2-ylcarbonyl, 1,4-diazocan-5-ylcarbonyl, 1,4-diazocan-6-ylcarbonyl, 1,5-diazocan-1-ylcarbonyl, 1,5-diazocan-2-ylcarbonyl, and 1,5-diazocan-3-ylcarbonyl.

Unless otherwise noted, for example, the "lower alkylsulfonyl (group)" used herein may be C₁₋₆ alkylsulfonyl (group).
Unless otherwise noted, for example, the "C₁₋₆ alkylsulfonyl (group)" used herein may be any of methylsulfonyl and ethylsulfonyl.

Unless otherwise noted, for example, the "C₃₋₈ cycloalkylsulfonyl (group)" used herein may be any of cyclopropylsulfonyl, cyclobutylsulfonyl, cyclopentylsulfonyl, and cyclohexylsulfonyl.

Unless otherwise noted, for example, the "C₆₋₁₄ arylsulfonyl (group)" used herein may be any of phenylsulfonyl, 1-naphthylsulfonyl, and 2-naphthylsulfonyl.

Unless otherwise noted, for example, the heterocyclic moiety of the "heterocyclic sulfonyl (group)" may be the same "heterocyclic group" as one described above. Specifically, "heterocyclic sulfonyl (group)" may be 5- to 14-membered heterocyclic-sulfonyl (group) containing one to five of one to three kinds of heteroatoms selected from a nitrogen atom, a sulfur atom, and an oxygen atom in addition to carbon atoms.

Unless otherwise noted, for example, the "lower alkylsulfinyl (group)" used herein may be C₁₋₆ alkylsulfinyl (group).
Unless otherwise noted, for example, the "C₁₋₆ alkylsulfinyl (group)" used herein may be any of methylsulfinyl and ethylsulfinyl.

Unless otherwise noted, for example, the "C₃₋₈ cycloalkylsulfinyl (group)" used herein may be any of cyclopropylsulfinyl, cyclobutylsulfinyl, cyclopentylsulfinyl, and cyclohexylsulfinyl.

Unless otherwise noted, for example, the "C₆₋₁₄ arylsulfinyl (group)" used herein may be any of phenylsulfinyl, 1-naphthylsulfinyl, and 2-naphthylsulfinyl.

Unless otherwise noted, for example, the heterocyclic moiety of the "heterocyclic sulfinyl (group)" may be the same "heterocyclic group" as one described above. Specifically, for example, "heterocyclic sulfinyl (group)" may be 5- to 14-membered heterocyclic-sulfinyl (group) containing one to five of one to three kinds of heteroatoms selected from a nitrogen atom, a sulfur atom, and an oxygen atom in addition to carbon atoms.

Unless otherwise noted, for example, the "lower alkyl-carbamoyl (group)" used herein may be C₁₋₆ alkyl-carbamoyl.
Unless otherwise noted, for example, the "C₁₋₆ alkyl-carbamoyl (group)" used herein may be any of methylcarbamoyl, ethylcarbamoyl, and propylcarbamoyl.

Unless otherwise noted, for example, the "mono- or di-lower alkylamino (group)" used herein may be mono- or di-C₁₋₆ alkylamino (group).
Unless otherwise noted, for example, the "mono- or di-C₁₋₆ alkylamino (group)" used herein may be any of methylamino, ethylamino, propylamino, dimethylamino, and diethylamino.

Unless otherwise noted, for example, the "lower alkyl-carbonylamino (group)" used herein may be C₁₋₆ alkyl-carbonylamino.
Unless otherwise noted, for example, the "C₁₋₆ alkyl-carbonylamino (group)" used herein may be any of acetylamino, propionylamino, and pivaloylamino.

Unless otherwise noted, for example, the "heterocyclic group" of the "heterocyclic group-amino (group)" used herein may be the same "heterocyclic group" as one described above. For example, the "heterocyclic group-amino" used herein may be 2-pyridyl-amino.

Unless otherwise noted, for example, the "heterocyclic-carbonyl" of the "heterocyclic-carbonylamino (group)" used herein may be the same "heterocyclic-carbonyl" as one described above. For example, the "heterocyclic-carbonylamino" used herein may be pyridyl-carbonylamino.

Unless otherwise noted, for example, the "heterocyclic group" of the "heterocyclic group-oxycarbonylamino (group)" used herein may be in the same "heterocyclic group" as one described above. For example, the "heterocyclic group-oxycarbonylamino" used herein may be 2-pyridyl-oxycarbonylamino.

Unless otherwise noted, for example, the "heterocyclic group" of the "heterocyclic group-sulfonyl (group)" used herein may be the same "heterocyclic group" as one described above. For example, the "heterocyclic group-sulfonylamino" may be 2-pyridyl-sulfonylamino.

Unless otherwise noted, for example, the "lower alkoxy-carbonylamino (group)" used herein may be C₁₋₆ alkoxy-carbonylamino (group).
Unless otherwise noted, for example, the "C₁₋₆ alkoxy-carbonylamino (group)" used herein may be any of methoxycarbonylamino, ethoxycarbonylamino, propoxycarbonylamino, and butoxycarbonylamino.

Unless otherwise noted, for example, the "lower alkylsulfonylamino (group)" used herein may be C₁₋₆ alkylsulfonylamino (group).
Unless otherwise noted, for example, the "C₁₋₆ alkylsulfonylamino (group)" used herein may be any of methylsulfonylamino and ethylsulfonylamino.

Unless otherwise noted, for example, the "mono- or di-C₃₋₈ cycloalkylamino (group)" used herein may be any of cyclopropylamino, cyclopentylamino, and cyclohexylamino.

Unless otherwise noted, for example, the "C₃₋₈ cycloalkyl-carbonylamino (group)" used herein may be any of cyclopropylcarbonylamino, cyclopentylcarbonylamino, and cyclohexylcarbonylamino.

Unless otherwise noted, for example, the "C₃₋₈ cycloalkoxy-carbonylamino (group)" used herein may be any of cyclopropoxycarbonylamino, cyclopentyloxycarbonylamino, and cyclohexyloxycarbonylamino.

Unless otherwise noted, for example, the "C₃₋₈ cycloalkyl-sulfonylamino (group)" used herein may be any of cyclopropylsulfonylamino, cyclopentylsulfonylamino, and cyclohexylsulfonylamino.

Unless otherwise noted, for example, the "mono- or di-C₆₋₁₄ arylamino (group)" used herein may be any of phenylamino and diphenylamino.

Unless otherwise noted, for example, the "mono- or di-C₇₋₁₆ aralkylamino (group)" used herein may be benzylamino.

Unless otherwise noted, for example, the "C₆₋₁₄ aryl-carbonylamino" used herein may be any of benzoylamino and naphthoylamino.

Unless otherwise noted, for example, the "C₆₋₁₄ arylsulfonylamino" may be any of phenylsulfonylamino, 2-naphthylsulfonylamino, and 1-naphthylsulfonylamino.

Hereinafter, symbols in the above formulae (formula (I₀) and formula (I)) will be described.

In the above formulae, A represents -CONRa- or -NRaCO-.
Ra represents a hydrogen atom or a substituent in each occurrence.
The substituent represented by Ra may be a substituent selected from the following substituents listed in Substituent Group A.

### <Substituent Group A>

(1) Halogen atom;
(2) Nitro;
(3) Cyano;
(4) Carboxyl which may be esterified;

(5) Lower alkyl which may be substituted;
(6) Lower alkenyl which may be substituted;
(7) Lower alkynyl which may be substituted;
(8) C₃₋₈ cycloalkyl which may be substituted;
(9) C₆₋₁₄ aryl which may be substituted;
(10) C₇₋₁₆ aralkyl which may be substituted;
(11) C₆₋₁₄ aryl-C₂₋₆ alkenyl which may be substituted;

(12) Heterocyclic group which may be substituted;

(13) Hydroxy;
(14) Lower alkoxy which may be substituted;
(15) C₃₋₈ cycloalkoxy which may be substituted;
(16) C₆₋₁₄ aryloxy which may be substituted;
(17) C₇₋₁₆ aralkyloxy which may be substituted;
(18) Lower alkyl-carbonyloxy which may be substituted;
(19) Lower alkoxy-carbonyloxy which may be substituted;
(20) Mono-lower alkyl-carbamoyloxy which may be substituted;
(21) Di-lower alkyl-carbamoyloxy which may be substituted;
(22) C₆₋₁₄ aryl-carbonyloxy which may be substituted;
(23) Mono- or di-C₆₋₁₄ aryl-carbamoyloxy which may be substituted;
(24) Heterocyclic oxy which may be substituted (e.g., aromatic heterocyclic oxy which may be substituted);

(25) Mercapto;
(26) Lower alkylthio which may be substituted;
(27) C₃₋₈ cycloalkylthio which may be substituted;
(28) C₆₋₁₄ arylthio which may be substituted;
(29) C₇₋₁₆ aralkylthio which may be substituted;
(30) Heterocyclic thio which may be substituted;

(31) Formyl;
(32) Lower alkyl-carbonyl which may be substituted;
(33) C₃₋₈ cycloalkyl-carbonyl which may be substituted;
(34) C₆₋₁₄ aryl-carbonyl which may be substituted;
(35) C₇₋₁₆ aralkyl-carbonyl which may be substituted;
(36) Heterocyclic-carbonyl which may be substituted;

(37) Lower alkylsulfonyl which may be substituted;
(38) C₃₋₈ cycloalkylsulfonyl which may be substituted;
(39) C₆₋₁₄ arylsulfonyl which may be substituted;
(40) Heterocyclic sulfonyl which may be substituted;
(41) Lower alkylsulfinyl which may be substituted;
(42) C₃₋₈ cycloalkylsulfinyl which may be substituted;
(43) C₆₋₁₄ arylsulfinyl which may be substituted;
(44) Heterocyclic sulfinyl which may be substituted;
(45) Sulfo;
(46) Sulfamoyl;
(47) Sulfinamoyl;
(48) Sulfenamoyl;
(49) Thiocarbamoyl;

(50) Carbamoyl group which may be substituted (e.g., lower alkyl-carbamoyl which may be substituted);

(51) Amino group which may be substituted (e.g., amino, mono- or di-lower alkylamino which may be substituted, mono- or di-C₃₋₈ cycloalkylamino which may be substituted, mono- or di-C₆₋₁₄ arylamino which may be substituted; mono- or di-C₇₋₁₆ aralkylamino which may be substituted, heterocyclic-amino which may be substituted, C₆₋₁₄ aryl-carbonylamino which may be substituted, formylamino, lower alkyl-carbonylamino which may be substituted, C₃₋₈ cycloalkyl-carbonylamino which may be substituted, heterocyclic-carbonylamino which may be substituted, lower alkoxy-carbonylamino which may be substituted, C₃₋₈ cycloalkoxy-carbonylamino which may be substituted, heterocyclic-oxycarbonylamino which may be substituted, carbamoylamino group which may have one or more substituents, lower alkylsulfonylamino which may be substituted, C₃₋₈ cycloalkyl-sulfonylamino which may be substituted, heterocyclic-sulfonylamino which may be substituted, and C₆₋₁₄ arylsulfonylamino which may be substituted).

Any of substitutes used for the aforementioned
"lower alkoxy-carbonyl which may be substituted",
"lower alkyl which may be substituted",
"lower alkenyl which may be substituted",
"lower alkynyl which may be substituted",
"lower alkoxy which may be substituted",
"lower alkyl-carbonyloxy which may be substituted",
"lower alkoxy-carbonyloxy which may be substituted",
"mono-lower alkyl-carbamoyloxy which may be substituted",
"di-lower alkyl-carbamoyloxy which may be substituted",
"lower alkylthio which may be substituted",
"lower alkyl-carbonyl which may be substituted",
"lower alkylsulfonyl which may be substituted",
"lower alkylsulfinyl which may be substituted",
"mono- or di-lower alkylamino which may be substituted",
"lower alkyl-carbonylamino which may be substituted",
"lower alkoxy-carbonylamino which may be substituted", and
"lower alkylsulfonylamino which may be substituted" may be selected from substituents listed in Substituent Group B below. In each case, the number of the substituents may be 1 to a maximum substitutable number, preferably 1 to 3, more preferably 1.

### <Substituent Group B>

Halogen atom;
Hydroxy;
Nitro;
Cyano;

C₆₋₁₄ aryl, which may be substituted with a halogen atom, hydroxy, cyano, amino, C₁-6 alkyl which may be halogenated, mono- or di-C₁₋₆ alkylamino, mono- or di-C₆₋₁₄ arylamino, mono- or di-C₇₋₁₆ aralkylamino, C₃₋₈ cycloalkyl, C₁₋₆ alkoxy, formyl, C₁₋₆ alkyl-carbonyl, C₃₋₈ cycloalkyl-carbonyl, C₆₋₁₄ aryl-carbonyl, C₇₋₁₆ aralkyl-carbonyl, C₁-6 alkoxy-carbonyl, C₆₋₁₄ aryloxy-carbonyl, C₇₋₁₆ aralkyloxy-carbonyl, C₁₋₆ alkylthio, C₁₋₆ alkylsulfinyl, C₁₋₆ alkylsulfonyl, carbamoyl, thiocarbamoyl, mono- or di-C₁₋₆ alkyl-carbamoyl, mono- or di-C₆₋₁₄ aryl-carbamoyl, or the like;

C₆₋₁₄ aryloxy, which may be substituted with a halogen atom, hydroxy, cyano, amino, C₁₋₆ alkyl which may be halogenated, mono- or di-C₁₋₆ alkylamino, mono- or di-C₆₋₁₄ arylamino, mono- or di-C₇₋₁₆ aralkylamino, C₃₋₈ cycloalkyl, C₁₋₆ alkoxy, formyl, C₁-₆ alkyl-carbonyl, C₃₋₈ cycloalkyl-carbonyl, C₆₋₁₄ aryl-carbonyl, C₇₋₁₆ aralkyl-carbonyl, C₁-6 alkoxy-carbonyl, C₆₋₁₄ aryloxy-carbonyl, C₇₋₁₆ aralkyloxy-carbonyl, C₁₋₆ alkylthio, C₁₋₆ alkylsulfinyl, C₁₋₆ alkylsulfonyl, carbamoyl, thiocarbamoyl, mono- or di-C₁₋₆ alkyl-carbamoyl, mono- or di-C₆₋₁₄ aryl-carbamoyl, or the like;

C₇₋₁₆ aralkyloxy, which may be substituted with a halogen atom, hydroxy, cyano, amino, C₁₋₆ alkyl which may be halogenated, mono- or di-C₁₋₆ alkylamino, mono- or di-C₆₋₁₄ arylamino, mono- or di-C₇₋₁₆ aralkylamino, C₃₋₈ cycloalkyl, C₁₋₆ alkoxy, formyl, C₁₋₆ alkyl-carbonyl, C₃₋₈ cycloalkyl-carbonyl, C₆₋₁₄ aryl-carbonyl, C₇₋₁₆ aralkyl-carbonyl, C₁₋₆ alkoxy-carbonyl, C₆₋₁₄ aryloxy-carbonyl, C₇₋₁₆ aralkyloxy-carbonyl, C₁₋₆ alkylthio, C₁₋₆ alkylsulfinyl, C₁₋₆ alkylsulfonyl, carbamoyl, thiocarbamoyl, mono- or di-C₁₋₆ alkyl-carbamoyl, mono- or di-C₆₋₁₄ aryl-carbamoyl, or the like;

Any of 5- to 10-membered mono- or di-heterocyclic groups each containing one to four of one or two kinds of heteroatoms selected from a nitrogen atom, a sulfur atom, and an oxygen atom in addition to carbon atoms (e.g., furyl, pyridyl, thienyl, pyrrolidino, 1-piperidinyl, 4-piperidyl, piperazyl, 1-morpholinyl, 4-thiomorpholinyl, azepan-1-yl, azocan-1-yl, azonan-1-yl, 3,4-dihydroisoquinolin-2-yl, and so on) (the heterocyclic group may be substituted with a halogen atom, hydroxy, cyano, amino, C₁₋₆ alkyl which may be halogenated, mono- or di-C₁₋₆ alkylamino, mono- or di-C₆₋₁₄ arylamino, mono- or di-C₇₋₁₆ aralkylamino, C₃₋₈ cycloalkyl, C₁₋₆ alkoxy, formyl, C₁₋₆ alkyl-carbonyl, C₃₋₈ cycloalkyl-carbonyl, C₆₋₁₄ aryl-carbonyl, C₇₋₁₆ aralkyl-carbonyl, C₁-6 alkoxy-carbonyl, C₆₋₁₄ aryloxy-carbonyl, C₇₋₁₆ aralkyloxy-carbonyl, C₁₋₆ alkylthio, C₁₋₆ alkylsulfinyl, C₁₋₆ alkylsulfonyl, carbamoyl, thiocarbamoyl, mono- or di-C₁₋₆ alkyl-carbamoyl, mono- or di-C₆₋₁₄ aryl-carbamoyl, or the like);

Amino group which may be substituted (e.g., an amino group which may be substituted with one or two substituents selected from a group consisting of C₁₋₆ alkyl, C₂₋₆ alkenyl, C₆₋₁₄ aryl, C₇₋₁₆ aralkyl, a heterocyclic group, and heterocyclic ring-lower alkyl (each of the C₁₋₆ alkyl, C₂₋₆ alkenyl, C₆₋₁₄ aryl, C₇₋₁₆ aralkyl, heterocyclic group, and heterocyclic ring-lower alkyl may be substituted with a halogen atom, hydroxy, cyano, amino, C₁₋₆ alkyl which may be halogenated (but not any substituent of alkyl and alkenyl), mono- or di-C₁₋₆ alkylamino, mono- or di-C₆₋₁₄ arylamino, mono- or di-C₇₋₁₆ aralkylamino, C₃₋₈ cycloalkyl, C₁₋₆ alkoxy, formyl, C₁₋₆ alkyl-carbonyl, C₃₋₈ cycloalkyl-carbonyl, C₆₋₁₄ aryl-carbonyl, C₇₋₁₆ aralkyl-carbonyl, C₁₋₆ alkoxycarbonyl, C₃₋₈ cycloalkoxy-carbonyl, C₆₋₁₄ aryloxy-carbonyl, C₇₋₁₆ aralkyloxy-carbonyl, C₁₋₆ alkylthio, C₃₋₈ cycloalkylthio, C₁₋₆ alkylsulfinyl, C₃₋₈ cycloalkylsulfinyl, C₁₋₆ alkylsulfonyl, C₃₋₈ cycloalkylsulfonyl, carbamoyl, thiocarbamoyl, mono- or di-C₁₋₆ alkyl-carbamoyl, mono- or di-C₆₋₁₄ aryl-carbamoyl, or the like). Here, the "heterocyclic ring" and the "heterocyclic ring" of the "heterocyclic ring-lower alkyl" may be the same "heterocyclic group" as one described above);

C₃₋₈ cycloalkyl;
C₁₋₆ alkoxy, which may be substituted with a halogen atom, hydroxy, amino, mono-or di-C₁₋₆ alkylamino, mono- or di-C₆₋₁₄ arylamino, C₃₋₈ cycloalkyl, C₁₋₆ alkoxy, formyl, C₁₋₆ alkyl-carbonyl, C₃₋₈ cycloalkyl-carbonyl, C₆₋₁₄ aryl-carbonyl, C₇₋₁₆ aralkyl-carbonyl, C₁₋₆ alkoxy-carbonyl, C₆₋₁₄ aryloxy-carbonyl, C₇₋₁₆ aralkyloxy-carbonyl, C₁₋₆ alkylthio, C₁₋₆ alkylsulfinyl, C₁₋₆ alkylsulfonyl, carbamoyl, thiocarbamoyl, mono- or di-C₁₋₆ alkyl-carbamoyl, mono- or di-C₆₋₁₄ aryl-carbamoyl, or the like;

Formyl;
C₁₋₆ alkyl-carbonyl (e.g., acetyl);
C₃₋₈ cycloalkyl-carbonyl;
C₆₋₁₄ aryl-carbonyl;
C₇₋₁₆ aralkyl-carbonyl;
C₁₋₆ alkoxycarbonyl;
C₆₋₁₄ aryloxy-carbonyl;
C₇₋₁₆ aralkyloxy-carbonyl;
C₁₋₆ alkylthio;
C₁₋₆ alkylsulfinyl;
C₁₋₆ alkylsulfonyl;
Carbamoyl;
Thiocarbamoyl;
Mono-C₁₋₆ alkyl-carbamoyl (e.g., methylcarbamoyl or ethylcarbamoyl);
Di-C₁₋₆ alkyl-carbamoyl (e.g., dimethylcarbamoyl, diethylcarbamoyl, or ethylmethylcarbamoyl);

Mono- or di-C₆₋₁₄ aryl-carbamoyl (e.g., phenylcarbamoyl, 1-naphthylcarbamoyl, or 2-naphthylcarbamoyl); and
Mono- or di-5- to 7-membered heterocyclic ring-carbamoyl containing one to four of one or two kinds of heteroatoms selected from a nitrogen atom, a sulfur atom, and an oxygen atom in addition to carbon atoms (e.g., 2-pyridylcarbamoyl, 3-pyridylcarbamoyl, 4-pyridylcarbamoyl, 2-thienylcarbamoyl, or 3-thienylcarbamoyl).

In addition, for example, any of substituents for the aforementioned "C₆₋₁₄ aryloxy-carbonyl which may be substituted",
"C₇₋₁₆ aralkyloxy-carbonyl which may be substituted",
"C₃₋₈ cycloalkyl which may be substituted",
"C₆₋₁₄ aryl which may be substituted",
"C₇₋₁₆ aralkyl which may be substituted",
"C₆₋₁₄ aryl-C₂₋₆ alkenyl which may be substituted",
"heterocyclic group which may be substituted",
"C₃₋₈ cycloalkoxy which may be substituted",
"C₆₋₁₄ aryloxy which may be substituted",
"C₇₋₁₆ aralkyloxy which may be substituted",
"C₆₋₁₄ aryl-carbonyloxy which may be substituted",
"mono- or di-C₆₋₁₄ aryl-carbamoyloxy which may be substituted",
"heterocyclic oxy which may be substituted",
"aromatic heterocyclic oxy which may be substituted",
"C₃₋₈ cycloalkylthio which may be substituted",
"C₆₋₁₄ arylthio which may be substituted",
"C₇₋₁₆ aralkylthio which may be substituted",
"heterocyclic thio which may be substituted",
"C₃₋₈ cycloalkyl-carbonyl which may be substituted",
"C₆₋₁₄ aryl-carbonyl which may be substituted",
"C₇₋₁₆ aralkyl-carbonyl which may be substituted",
"heterocyclic-carbonyl which may be substituted",
"C₃₋₈ cycloalkylsulfonyl which may be substituted",
"C₆₋₁₄ arylsulfonyl which may be substituted",
"heterocyclic sulfonyl which may be substituted",
"C₃₋₈ cycloalkylsulfinyl which may be substituted",
"C₆₋₁₄ arylsulfinyl which may be substituted",
"heterocyclic sulfinyl which may be substituted",
"carbamoyl group which may be substituted", and
"amino group which may be substituted" may be selected from Substituent Group B as listed above and Substituent Group B' as listed below. In each case, the number of the substituents may be 1 to a maximum substitutable number, preferably 1 to 3, more preferably 1.

### <Substituent Group B'>

C₁₋₆ alkyl, which may be substituted with a halogen atom, hydroxy, cyano, amino, mono- or di-C₁₋₆ alkylamino, mono- or di-C₆₋₁₄ arylamino, mono- or di-C₇₋₁₆ aralkylamino, C₃₋₈ cycloalkyl, C₁₋₆ alkoxy, formyl, C₁₋₆ alkyl-carbonyl, C₃₋₈ cycloalkylcarbonyl, C₆₋₁₄ aryl-carbonyl, C₇₋₁₆ aralkyl-carbonyl, C₁₋₆ alkoxy-carbonyl, C₆₋₁₄ aryloxy-carbonyl, C₇₋₁₆ aralkyloxy-carbonyl, C₁₋₆ alkylthio, C₁₋₆ alkylsulfinyl, C₁₋₆ alkylsulfonyl, carbamoyl, thiocarbamoyl, mono- or di-C₁₋₆ alkyl-carbamoyl, mono- or di-C₆₋₁₄ aryl-carbamoyl, or the like;

C₂₋₆ alkenyl, which may be substituted with a halogen atom, hydroxy, cyano, amino, mono- or di-C₁₋₆ alkylamino, mono- or di-C₆₋₁₄ arylamino, mono- or di-C₇₋₁₆ aralkylamino, C₃₋₈ cycloalkyl, C₁₋₆ alkoxy, formyl, C₁₋₆ alkyl-carbonyl, C₃₋₈ cycloalkylcarbonyl, C₆₋₁₄ aryl-carbonyl, C₇₋₁₆ aralkyl-carbonyl, C₁₋₆ alkoxy-carbonyl, C₆₋₁₄ aryloxy-carbonyl, C₇₋₁₆ aralkyloxy-carbonyl, C₁₋₆ alkylthio, C₁₋₆ alkylsulfinyl, C₁₋₆ alkylsulfonyl, carbamoyl, thiocarbamoyl, mono- or di-C₁₋₆ alkyl-carbamoyl, mono- or di-C₆₋₁₄ aryl-carbamoyl, or the like;

C₂₋₆ alkynyl, which may be substituted with a halogen atom, hydroxy, cyano, amino, mono- or di-C₁₋₆ alkylamino, mono- or di-C₆₋₁₄ arylamino, mono- or di-C₇₋₁₆ aralkylamino, C₃₋₈ cycloalkyl, C₁₋₆ alkoxy, formyl, C₁₋₆ alkyl-carbonyl, C₃₋₈ cycloalkylcarbonyl, C₆₋₁₄ aryl-carbonyl, C₇₋₁₆ aralkyl-carbonyl, C₁₋₆ alkoxy-carbonyl, C₆₋₁₄ aryloxy-carbonyl, C₇₋₁₆ aralkyloxy-carbonyl, C₁₋₆ alkylthio, C₁₋₆ alkylsulfinyl, C₁₋₆ alkylsulfonyl, carbamoyl, thiocarbamoyl, mono- or di-C₁₋₆ alkyl-carbamoyl, mono- or di-C₆₋₁₄ aryl-carbamoyl, or the like.

A is preferably -CONH-.

In the above formula, B represents hydrogen or a substituent.
Examples of the substituent represented by B include any substituent selected from Substituent Group A as described above.

B is preferably
(1) a C₁₋₆ alkyl group (e.g., methyl, ethyl, isopropyl) which may have one or more substituents selected from
   (a) a cyano group,
   (b) a hydroxy group,
   (c) C₁₋₆ alkoxy (e.g., methoxy),
   (d) a di- C₁₋₆ alkyl-amino group (e.g., dimethylamino),
   (e) a carbamoyl group,
   (f) a C₁₋₆ alkylsulfanyl group (e.g., methylsulfanyl),
   (g) a C₁₋₆ alkylsulfinyl group (e.g., methylsulfinyl),
   (h) a C₁₋₆ alkylsulfonyl group (e.g., methylsulfonyl), and
   (i) a 5- to 7-membered heterocyclic group having one or more heteroatoms selected from a nitrogen atom, an oxygen atom and a sulfur atom (e.g., pyrrolidinyl, oxolanyl),
(2) a C₃₋₁₀ cycloalkyl group (e.g., cyclopropyl), or
(3) a 5- to 7-membered heterocyclic group (e.g., thiazolyl, pyridyl).

In the above formula, the ring Cy1 represents (1) a benzene ring or (2) a 6-membered nitrogen-containing aromatic heterocycle which may have an additional substituent in addition to a group represented by -A-B.
Examples of the "6-membered nitrogen-containing aromatic heterocycle" represented by the ring Cy1 include a 6-membered nitrogen-containing aromatic heterocycle having at least one (preferably one or two) nitrogen atom as a ring-constituting element in addition to carbon atoms (e.g., pyridine, pyridazine, pyrimidine, or pyrazine).
The "6-membered nitrogen-containing aromatic heterocycle" represented by the ring Cy1 is preferably a pyridine ring.

The substituent of "(1) a benzene ring or (2) a 6-membered nitrogen-containing aromatic heterocycle, each may have substituent(s) in addition to a group represented by -A-B ", which is represented by the ring Cy1, may be a substituent selected from, for example, Substituent Group A as described above.
The "benzene ring" and "6-membered nitrogen-containing aromatic heterocycle" represented by the ring Cy1 may have one or more such substituents (preferably one or two substituents, more preferably one substituent) on a substitutable position.
The ring Cy1 is preferably unsubstituted.

The ring Cy1 is preferably a benzene ring or a 6-membered nitrogen-containing heterocycle.
The ring Cy1 is more preferably a benzene ring or a pyridine ring.

In the above formula, X¹, X² and X³ are each independently -CR^{x}= or -N=.
Here, R^{x} is independently a hydrogen atom, a halogen atom or a lower alkyl group which may be halogenated in each occurrence.

Preferably, X¹ and X² are each independently -CH- or -N=.

In the above formula, the ring Cy2 is (1) a carbon ring having a carbon number of 5 to 7 or (2) a 5- to 7-membered heterocycle having 1 or 2 heteroatoms selected from a nitrogen atom, an oxygen atom and a sulfur atom, each may have substituent(s) (excluding oxo group).
Here, Z in the above-mentioned formula is one of the ring Cy2-constituting atoms, and is a carbon atom or a nitrogen atom.

Examples of the "carbon ring having a carbon number of 5 to 7" represented by the ring Cy2 include C₅₋₇ cycloalkene (e.g., cyclopentene, cyclohexene, cycloheptene), C₅₋₇ cycloalkadiene (e.g., cyclopentadiene, 1,3-cyclohexadiene, cyclohexadiene, cycloheptadiene) and benzene ring. Particularly, a carbon ring having a carbon number of 5 or 6 is preferable.

The "5- to 7-membered heterocycle having 1 or 2 heteroatoms selected from a nitrogen atom, an oxygen atom and a sulfur atom" represented by the ring Cy2 is an aromatic heterocycle or a nonaromatic heterocycle.
Examples of the "aromatic heterocycle" include furan, thiophene, pyridine, pyrimidine, pyridazine, pyrazine, pyrrole, imidazole, pyrazole, isoxazole, isothiazole, oxazole, thiazole, oxadiazole, thiadiazole, triazole, tetrazole, and triazine.
Examples of the "nonaromatic heterocycle" include dihydrofuran, tetrahydrofuran, dihydrothiophene, tetrahydrothiophene, pyrrolidine, pyrroline, pyrazolidine, piperidine, piperazine, morpholine, thiomorpholine, hexamethylenimine, oxazolidine, thiazolidine, imidazolidine, imidazoline, azepane, oxepane and tetrahydropyridine.
Particularly, a 5- or 6-membered heterocycle is preferable.

Examples of the substituent (excluding oxo group) of "(1) a carbon ring having a carbon number of 5 to 7 or (2) a 5- to 7-membered heterocycle having 1 or 2 heteroatoms selected from a nitrogen atom, an oxygen atom and a sulfur atom, each may have substituent(s) (excluding oxo group)" represented by the ring Cy2 include substituents selected from the above-mentioned Substituent Group A.
Preferably, the substituent is not an oxo group, a C₆₋₁₄ aryl group and a carboxyl group which may be esterified.
The ring Cy2 is preferably unsubstituted.

The ring Cy2 is preferably (1) a carbon ring having a carbon number of 5 or 6 or (2) a 5- or 6-membered heterocycle having 1 or 2 heteroatoms selected from a nitrogen atom, an oxygen atom and a sulfur atom, each may have substituent(s) (excluding oxo group, C₆₋₁₄ aryl group and carboxyl group which may be esterified).

However, in the above-mentioned formula, the moiety represented by 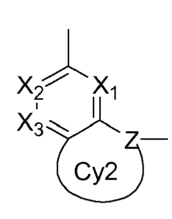 is not

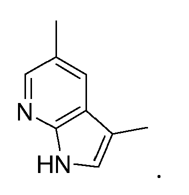

In the above-mentioned formula, the moiety represented by 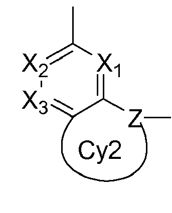 is preferably not

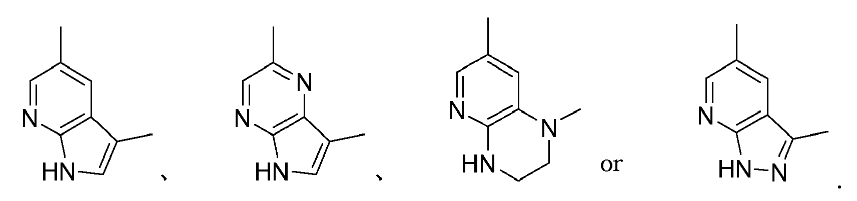

In the above-mentioned formula, the moiety represented by 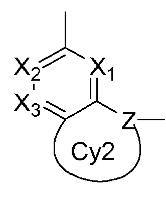 is preferably, for example,

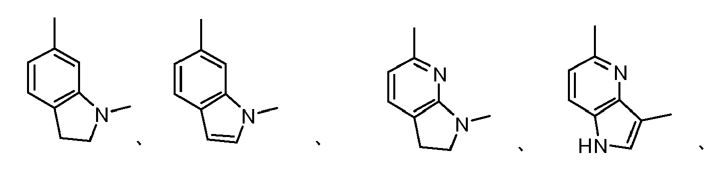

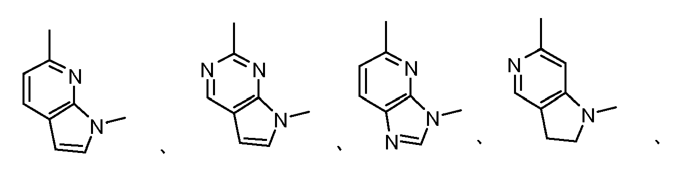

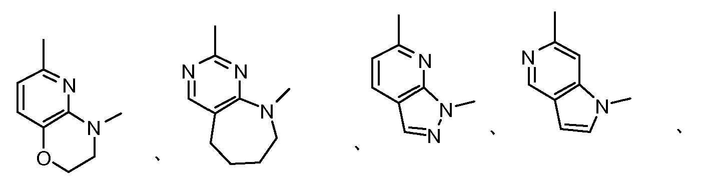

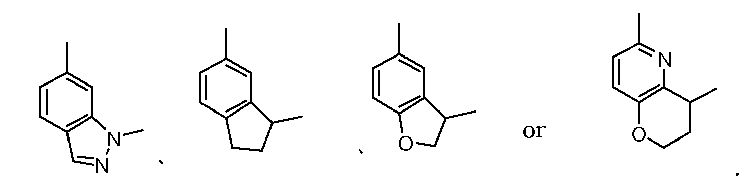

In the above-mentioned formula, the moiety represented by 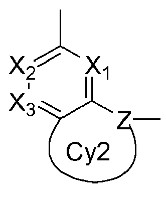 is more preferably, for example,

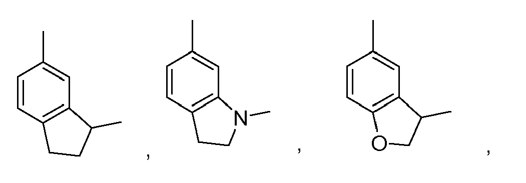

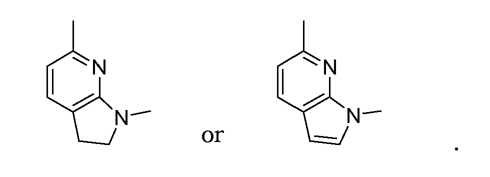

In the above-mentioned formula, the moiety represented by 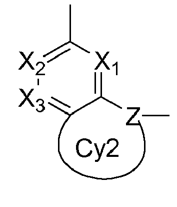 is more preferably, for example,

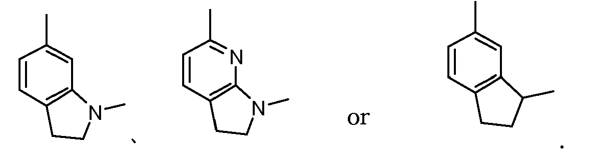

In the above-mentioned formula, L is a bond, -(CH₂)n-, -L'-, -L'-CH₂- or -CH₂-
Here, n is 1 or 2, and L' is -O-, -NR^{b}- or -S(O)ₘ-.
Here, R^{b} is a hydrogen atom or a substituent, and m is an integer of 0 to 2.
Examples of the substituent represented by R^{b} include substituents selected from the above-mentioned Substituent Group A.
R^{b} is preferably a hydrogen atom.

L is preferably a bond, -CH₂-, -O-, -NR^{b}- or -S(O)ₘ-.
L is more preferably a bond, -CH₂-, -NH- or -O-.

In the above-mentioned formula, the ring Cy3 is (1) a benzene ring or (2) a 6-membered nitrogen-containing aromatic heterocycle, each may have substituent(s).
Examples of the "6-membered nitrogen-containing aromatic heterocycle" represented by the ring Cy3 include those similar to the ring of the "6-membered nitrogen-containing aromatic heterocycle" represented by the ring Cy1.

Examples of the substituent of "(1) a benzene ring or (2) a 6-membered nitrogen-containing aromatic heterocycle, each may have substituent(s)" represented by the ring Cy3 include substituents selected from the above-mentioned Substituent Group A.

The ring Cy3 is preferably a benzene ring or a pyridine ring, each may have one or more (preferably, one or two) substituents selected from a halogen atom, a C₁₋₆ alkyl group which may be halogenated and a C₁₋₆ alkoxy group which may be halogenated.
The ring Cy3 is more preferably dichlorobenzene.

These preferable examples are more preferably used in combination.

As compound (I₀), preferred is the following compound.

### [Compound A]

A compound wherein
A is -CONH-,
B is
(1) a C₁₋₆ alkyl group (e.g., methyl, ethyl, isopropyl) which may have one or more substituents selected from
   (a) a cyano group,
   (b) a hydroxy group,
   (c) C₁₋₆ alkoxy (e.g., methoxy)
   (d) a di-C₁₋₆ alkyl-amino group (e.g., dimethylamino),
   (e) a carbamoyl group,
   (f) a C₁₋₆ alkyl-sulfanyl group (e.g., methylsulfanyl),
   (g) a C₁₋₆ alkyl-sulfinyl group (e.g., methylsulfinyl),
   (h) a C₁₋₆ alkyl-sulfonyl group (e.g., methylsulfonyl) and
   (i) a 5- to 7-membered heterocyclic group having one or more heteroatoms selected from a nitrogen atom, an oxygen atom and a sulfur atom (e.g., pyrrolidinyl, oxolanyl),
(2) a C₃₋₁₀ cycloalkyl group (e.g., cyclopropyl), or
(3) a 5- to 7-membered heterocyclic group (e.g., thiazolyl, pyridyl),
   ring Cy1 is (1) a benzene ring or (2) a 6-membered nitrogen-containing heterocycle, X¹, X² and X³ are each independently -CH- or -N=,
   ring Cy2 is (1) a carbon ring having a carbon number of 5 to 7 (e.g., cyclopentene, cyclohexene), or (2) a 5- to 7-membered heterocycle having 1 or 2 heteroatoms selected from a nitrogen atom, an oxygen atom and a sulfur atom (e.g., dihydropyrrole, pyrrole, imidazole, pyrazole, furan, dihydrofuran, tetrahydroazepine, dihydrooxazine),
   Z is a carbon atom or a nitrogen atom,
   L is a bond, -CH₂-, -NH- or -O-, and
   ring Cy3 is a benzene ring or a pyridine ring, each may have one or more substituents selected from a halogen atom, a C₁₋₆ alkyl group which may be halogenated (e.g., trifluoromethane) and a C₁₋₆ alkoxy group which may be halogenated (e.g., methoxy).
However, in compound A, the moiety represented by 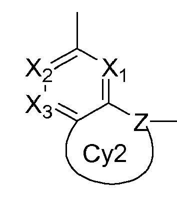 is not

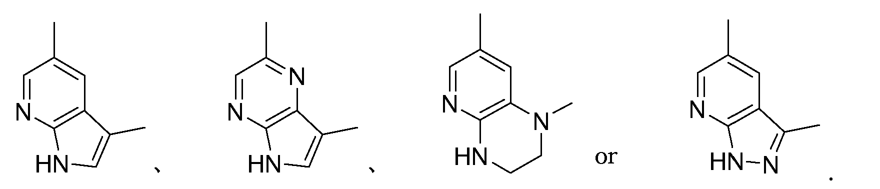

As compound (I₀), preferred is the following compound.

### [Compound B]

A compound wherein
A is -CONH- or -CONH-,
B is
(1) C₁₋₆ alkyl group which may have one or more substituents selected from
   (a) a cyano group,
   (b) a hydroxy group,
   (c) C₁₋₆ alkoxy,
   (d) a di-C₁₋₆ alkyl-amino group,
   (e) a carbamoyl group,
   (f) a C₁₋₆ alkyl-sulfanyl group,
   (g) a C₁₋₆ alkyl-sulfinyl group,
   (h) a C₁₋₆ alkyl-sulfonyl group and
   (i) a 5- to 7-membered heterocyclic group having one or more heteroatoms selected from a nitrogen atom, an oxygen atom and a sulfur atom,
(2) a C₃₋₁₀ cycloalkyl group, or
(3) a 5- to 7-membered heterocyclic group,
   ring Cy1 is (1) a benzene ring or (2) a 6-membered nitrogen-containing heterocycle, the moiety represented by 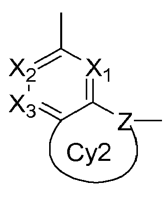 is 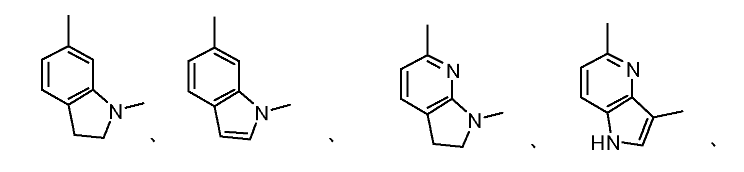 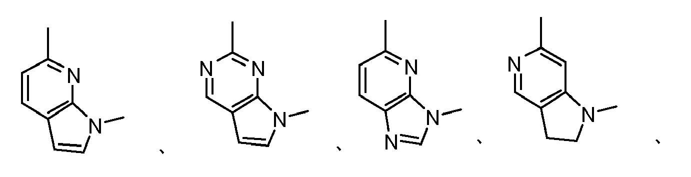 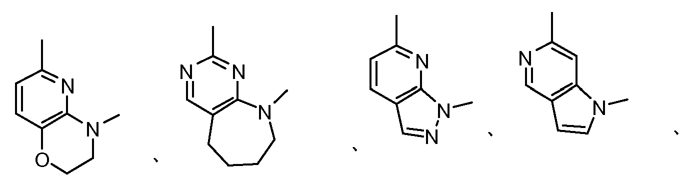 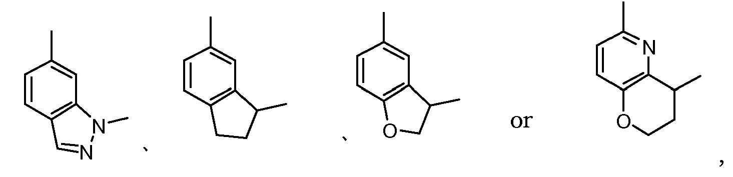 L is a bond, -CH₂-, -NH- or -O- and
   ring Cy3 is a benzene ring or a pyridine ring, each may have one or more substituents selected from a halogen atom, a C₁₋₆ alkyl group which may be halogenated and a C₁₋₆ alkoxy group which may be halogenated.

As compound (I₀), particularly preferred are the following compounds. N-(2-cyanoethyl)-3-[1-(2,4-dichlorophenyl)-2,3-dihydro-1H-indol-6-yl]benzamide or a salt thereof.
3-[1-(2,4-dichlorobenzyl)-1H-pyrrolo[2,3-b]pyridin-6-yl]-N-(2-hydroxyethyl)benzamide or a salt thereof.
3-[1-(2,4-dichlorobenzyl)-2,3-dihydro-1H-pyrrolo[2,3-b]pyridin-6-yl]-N-(2-hydroxyethyl)benzamide or a salt thereof.
3-[3-[2-(3,4-dimethoxyphenyl)ethyl]-3H-imidazo[4,5-b]pyridin-5-yl]-N-(2-pyrrolidin-1-ylethyl)benzamide or a salt thereof.
N-(2-cyanoethyl)-3-[4-(2,4-dichlorophenyl)-3,4-dihydro-2H-1,4-benzoxazin-6-yl]benzamide or a salt thereof.
3-[3-(2,4-dichlorophenyl)-2,3-dihydro-1H-inden-5-yl]-N-[2-(methylsulfinyl)ethyl]benzamide or a salt thereof.
N-(2-cyanoethyl)-3-[3-(2,4-dichlorophenoxy)-2,3-dihydro-1H-inden-5-yl]benzamide or a salt thereof.
3-[3-[(2,4-dichlorophenyl)amino]-2,3-dihydro-1-benzofuran-5-yl]-N-(2-hydroxyethyl)benzamide or a salt thereof.

When the compound (I₀) is a salt, examples of such a salt include metal salt, ammonium salt, salt with organic base, salt with inorganic acid, salt with organic acid, salt with basic or acidic amino salt. Preferable examples of the metal salt include alkaline metal salts such as sodium salt and potassium salt; alkaline earth metal salts such as calcium salt, magnesium salt and barium salt; and aluminum salt. Preferable examples of the salt with organic base include salts with trimethylamine, triethylamine, pyridine, picoline, 2,6-lutidine, ethanolamine, diethanolamine, triethanolamine, cyclohexylamine, dicyclohexylamine and N,N'-dibenzylethylenediamine. Preferable examples of the salt with inorganic acid include salts with hydrochloric acid, hydrobromic acid, nitric acid, sulfuric acid and phosphoric acid. Preferable examples of the salt with organic acid include salts with formic acid, acetic acid, trifluoroacetic acid, phthalic acid, fumaric acid, oxalic acid, tartaric acid, maleic acid, citric acid, succinic acid, malic acid, methanesulfonic acid, benzenesulfonic acid and p-toluenesulfonic acid. Preferable examples of the salt with basic amino acid include salts with arginine, lysine and ornithine. Preferable examples of the salt with acidic amino acid include salt with aspartic acid and glutamic acid. Among them, pharmaceutically acceptable salts are preferable. For example, if the compound has an acidic functional group therein, examples of the salt include inorganic salt such as alkaline metal salt (e.g., sodium salt and potassium salt) and alkaline earth metal salt (e.g., calcium salt, magnesium salt and barium salt); and ammonium salt. If the compound has a basic functional group therein, examples of the salt thereof include salts with inorganic acids such as hydrochloric acid, hydrobromic acid, nitric acid, sulfuric acid and phosphoric acid, or salts with organic acids such as acetic acid, phthalic acid, fumaric acid, oxalic acid, tartaric aid, maleic acid, citric aid, succinic acid, methanesulfonic acid and p-toluenesulfonic acid.

When there is an isomer of the compound (I₀), such as a tautomer, an optical isomer, a stereoisomer, a positional isomer, or a rotational isomer, an isomer may be present alone or in combination and provided as a compound of the present invention. Furthermore, if there is an optical isomer of the compound (I₀), an optical isomer isolated from a racemic mixture is also provided as the compound (I₀) .

The compound (I₀) may be a crystallized compound. Even if the compound (I₀) is in single crystal form or mixed crystal form, it can be provided as the compound (I₀) of the present invention.

The compound (I₀) may be a solvate (e.g., a hydrate) or a nonsolvate. Any of them can be provided as the compound (I₀) of the present invention.

Any of the above compounds may be labeled or substituted with an isotope (e.g., ²H, ³H, ¹¹C, ¹⁴C, ¹⁸F, ³⁵S, or ¹²⁵I) and provided as the compound (I₀) of the present invention.

### <Manufacturing Method>

Hereinafter, a method for manufacturing the compound of the present invention will be described.
For example, the compound (I₀) can be obtained by a process represented by a reaction formula described below or another process based thereof. The symbols for the compounds in the reaction formula are as defined above. Here, the compounds in the formula may also represent those forming salts. Examples of such salts are same as those of the compound (I₀). In addition, compounds obtained in the respective steps may be directly used as a reaction solution or a crude product in the subsequent reaction. Alternatively, it may be isolated from the reaction mixture by a conventional method and can be easily purified by any of well-known separation techniques, such as extraction, concentration, neutralization, filtration, distillation, recrystallization and chromatography. Alternatively, if the compound in the formula is commercially available, a corresponding commercial product may be directly used.

The compound (I₀) can be produced by the process represented by Reaction Formula 1 as follows.

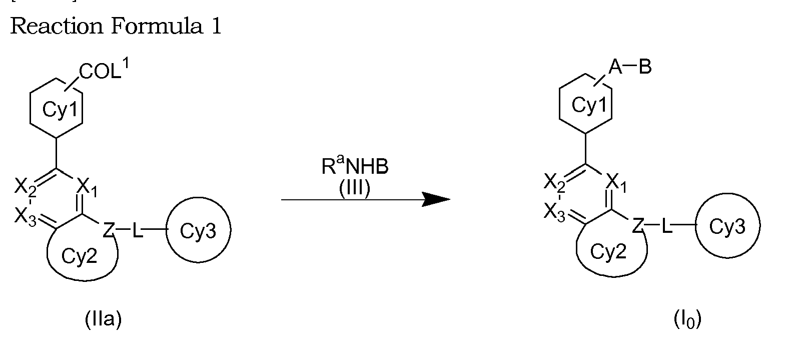

In the reaction formula, L¹ represents a leaving group.

The compound (I₀) can be produced by reaction of the compound (IIa) with the compound (III) in the presence of base or acid if desired.

The compound (III) may be any of commercially available products or may be produced according to a well-known method or another method based thereon.

Examples of the "leaving group" represented by L¹ include a hydroxy group, a halogen atom (e.g. fluorine, chlorine, bromine, iodine), C₁₋₆ alkoxy (e.g., methoxy, ethoxy, propoxy, isopropoxy, butoxy, isobutoxy, sec-butoxy, pentyloxy, or hexyloxy) which may be halogenated, a C₁₋₅ alkylsulfonyloxy group (e.g., methanesulfonyloxy, ethanesulfonyloxy, or trichloromethanesulfonyloxy) which may be halogenated, a C₆-10 arylsulfonyloxy group which may be substituted, a phenyloxy group which may be substituted, or a benzothiazol-2-ylthio group which may be substituted.

Examples of the "C₆₋₁₀ arylsulfonyloxy group which may be substituted" include a C₆₋₁₀ arylsulfonyloxy (e.g., phenylsulfonyloxy, naphthylsulfonyloxy) which may have one to three substituents selected from C₁₋₆ alkyl (e.g., methyl, ethyl, propyl, isopropyl, butyl, isobutyl, sec-butyl, tert-butyl, pentyl or hexyl), C₁₋₆ alkoxy (e.g., methoxy, ethoxy, propoxy, isopropoxy, butoxy, isobutoxy, sec-butoxy, pentyloxy, or hexyloxy) and nitro. Specific examples include benzenesulfonyloxy, m-nitrobenzenesulfonyloxy and p-toluenesulfonyloxy.

Examples of the "phenyloxy group which may be substituted" include a phenyloxy group which may have one to three substituents selected from C₁₋₆ alkyl (e.g., methyl, ethyl, propyl, isopropyl, butyl, isobutyl, sec-butyl, tert-butyl, pentyl, or hexyl), C₁₋₆ alkoxy (e.g., methoxy, ethoxy, propoxy, isopropoxy, butoxy, isobutoxy, sec-butoxy, pentyloxy, or hexyloxy) and nitro. Specific examples include phenyloxy and 4-nitrophenoxy.

Examples the benzothiazol-2-ylthio group which may be substituted include a benzothiazol-2-ylthio group which may have one to three substituents selected from C₁₋₆ alkyl (e.g., methyl, ethyl, propyl, isopropyl, butyl, isobutyl, sec-butyl, tert-butyl, pentyl, or hexyl), C₁₋₆ alkoxy (e.g., methoxy, ethoxy, propoxy, isopropoxy, butoxy, isobutoxy, sec-butoxy, pentyloxy, or hexyloxy) and nitro. Specific examples include benzothiazol-2-ylthio.

The amount of the compound (III) used is about 1 to 10 mol, preferably about 1 to 2 mol, per mol of the compound (IIa).

Examples of the "base" include basic salts such as sodium carbonate, potassium carbonate, cesium carbonate and sodium hydrogen carbonate; aromatic amines such as pyridine and lutidine; tertiary amines such as triethylamine, tripropylamine, tributylamine, cyclohexyldimethylamine, 4-dimethylaminopyridine, N-methylpiperidine, N-methylpyrrolidine and N-methylinorpholine; alkaline metal hydrides such as sodium hydride and potassium hydride; metal amides such as sodium amide, lithium diisopropylamide and lithium hexamethyldisilazide; and metal alkoxides such as sodium methoxide, sodium ethoxide and potassium tert-butoxide.

The amount of the "base" used is generally about 0.1 to 10, preferably 0.8 to 2 equivalents per compound (IIa).

Examples of the "acid" include methanesulfonic acid, p-toluenesulfonic acid and camphorsulfonic acid.

The amount of the "acid" used is generally about 0.1 to 10, preferably 0.8 to 3 equivalents per compound (IIa).

It is advantageous to carry out the present reaction in the absence of a solvent or in the presence of a solvent inactive to the reaction. Preferable examples of such a solvent include, but not specifically limited as long as the reaction proceeds, water; ethers such as diethyl ether, tetrahydrofuran, 1,4-dioxane and 1,2-dimethoxyethane; hydrocarbons such as benzene, toluene, cyclohexane and hexane; amides such as N,N-dimethylformamide and N,N-dimethylacetamide; halogenated hydrocarbons such as dichloromethane, chloroform, carbon tetrachloride and 1,2-dichloroethane; nitriles such as acetonitrile and propionitrile; sulfoxides such as dimethyl sulfoxide; and nitrogen-containing aromatic hydrocarbons such as pyridine, lutidine and quinoline, or mixtures thereof.

The reaction temperature is generally in the range of about -40 to 150°C, preferably 0 to 100°C.

The reaction time is generally in the range of 5 minutes to 24 hours, preferably 10 minutes to 5 hours.

When L¹ is OH, as an alternative method, the compound (IIa) may be reacted with the compound (III) in the presence of an appropriate condensing agent.

The amount of the compound (III) used is generally about 0.8 to about 10 mol, preferably about 0.8 to about 2 mol, per mol of the compound (IIa).

Examples of the "condensing agent" include: N,N'-carbodiimides such as N,N'-dicyclohexylcarbodiimide and 1-ethyl-3-(3-dimethylaminopropyl)carbodiimide hydrochloride salt (WSC); azolides such as N,N'-carbonylimidazole; 2-halogenopyridinium salts such as 2-chloro-1-methylpyridinium iodide and 2-fluoro-1-methylpyridinium iodide; and other compounds such as N-ethoxycarbonyl-2-ethoxy-1,2-dihydroquinoline, 2-(7-aza-1H-benzotriazol-1-yl)-1,1,3,3-tetramethyluronium hexafluorophosphate (HATU), 4-(4,6-dimethoxy-1,3,5-triazin-2-yl)-4-methylmorpholinium chloride n-hydrate (DMTMM), diethyl cyanophosphate, phosphorous oxychloride and acetic anhydride.

The amount of the "condensing agent" used is generally about 0.8 to about 5 mol, preferably about 1 to about 3 mol, per mol of the compound (IIa).

The reaction may be carried out in the presence of base. Examples of the "base" include basic salts such as potassium acetate and sodium acetate; and tertiary amines such as triethylamine, tripropylamine, tributylamine, cyclohexyldimethylamine, 4-dimethylaminopyridine, N-methylpiperidine, N-methylpyrrolidine and N-methylmorpholine. In addition, if required, the reaction may be carried out in the presence of a condensation accelerator such as 1-hydroxy-1H-benzotriazole (HOBt) monohydrate.

The amount of "base" used is generally about 0.5 to about 5 mol, preferably about 2 to about 3 mol, per mol of the compound (IIa).

It is advantageous to carry out the present reaction using a solvent inactive to the reaction. Preferably, examples of such a solvent include: alcohols such as methanol, ethanol and propanol; hydrocarbons such as hexane, cyclohexane, benzene, toluene and xylene; ethers such as diethyl ether, diisopropyl ether, tetrahydrofuran, 1,4-dioxane and 1,2-dimethoxyethane; amides such as N,N-dimethylformamide, N,N-dimethylacetamide, hexamethylphosphoric triamide and 1-methylpyrrolidin-2-one; sulfoxides such as dimethyl sulfoxide; halogenated hydrocarbons such as dichloromethane, chloroform, carbon tetrachloride and 1,2-dichloroethane; nitriles such as acetonitrile and propionitrile; acid anhydride such as acetic anhydride; and mixtures thereof.

The reaction time is generally in the range of about 10 minutes to about 48 hours, preferably about 30 minutes to about 24 hours.

The reaction temperature is generally in the range of about -20 to about 150°C, preferably about 0 to about 100°C.

The reaction time can be shortened using a microwave reactor or the like.

The compound (I₀) thus obtained may be isolated from the reaction mixture by a conventional method and can be easily purified by any of well-known separation techniques, such as concentration, vacuum concentration, solvent extraction, crystallization, recrystallization, transfer dissolution and chromatography.

The compound (I₀) can be produced by the process represented by Reaction Formula 2 as follows. 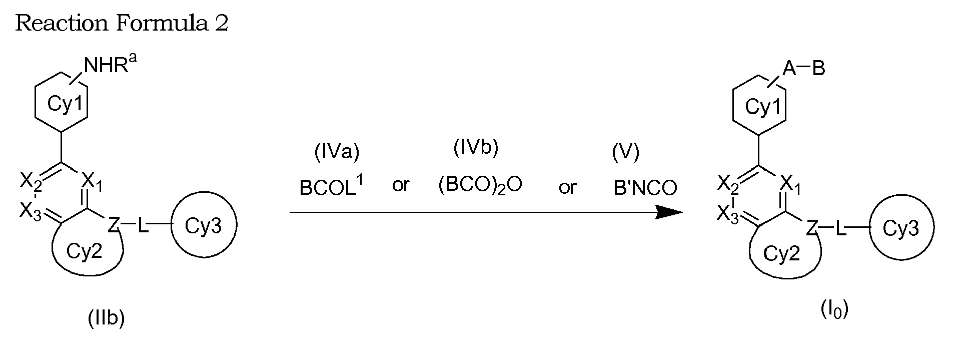 wherein B' represents that, when B is an amino group which may be substituted, an amino group is removed from B; and other symbols are as defined above.

The compound (Iₒ) can be produced by reaction of the compound (IIb) with the compound (IVa), compound (IVb), or compound (V) in the presence of base or acid if required.

The compound (IVa), compound (IVb), or compound (V) may be any of commercially available products or may be produced according to a well-known method or another method based thereon.

The amount of each of the compound (IVa), compound (IVb), or compound (V) used is about 1 to 10 mol, preferably about 1 to 2 mol, per mol of the compound (IIb).

Examples of the "base" include basic salts such as sodium carbonate, potassium carbonate, cesium carbonate and sodium hydrogen carbonate; aromatic amines such as pyridine and lutidine; tertiary amines such as triethylamine, tripropylamine, tributylamine, cyclohexyldimethylamine, 4-dimethylaminopyridine, N-methylpiperidine, N-methylpyrrolidine and N-methylinorpholine; alkaline metal hydrides such as sodium hydride and potassium hydride; metal amides such as sodium amide, lithium diisopropylamide and lithium hexamethyldisilazide; and metal alkoxides such as sodium methoxide, sodium ethoxide and potassium tert-butoxide.

The amount of the "base" used is generally about 0.1 to 10, preferably 0.8 to 2 equivalents per compound (IIb).

Examples of the "acid" include methanesulfonic acid, p-toluenesulfonic acid and camphorsulfonic acid.

The amount of the "acid" used is generally about 0.1 to 10, preferably 0.8 to 3 equivalents per compound (IIb).

It is advantageous to carry out the present reaction in the absence of a solvent or in the presence of a solvent inactive to the reaction. Preferable examples of such a solvent include, but not specifically limited as long as the reaction proceeds, water; ethers such as diethyl ether, tetrahydrofuran, 1,4-dioxane and 1,2-dimethoxyethane; hydrocarbons such as benzene, toluene, cyclohexane and hexane; amides such as N,N-dimethylformamide and N,N-dimethylacetamide; halogenated hydrocarbons such as dichloromethane, chloroform, carbon tetrachloride and 1,2-dichloroethane; nitriles such as acetonitrile and propionitrile; sulfoxides such as dimethyl sulfoxide; and nitrogen-containing aromatic hydrocarbons such as pyridine, lutidine and quinoline, or mixtures thereof.

The reaction temperature is generally in the range of about -40 to 150°C, preferably 0 to 100°C.

The reaction time is generally in the range of 5 minutes to 24 hours, preferably 10 minutes to 5 hours.

As an alternative method, the compound (II) may be reacted with BCOOH in the presence of an appropriate condensing agent.

The amount of the BCOOH used is generally about 0.8 to about 10 mol, preferably about 0.8 to about 2 mol, per mol of the compound (IIb).

Examples of the "condensing agent" include: N,N'-carbodiimides such as N,N'-dicyclohexylcarbodiimide and 1-ethyl-3-(3-dimethylaminopropyl)carbodiimide hydrochloride salt (WSC); azolides such as N,N'-carbonylimidazole; 2-halogenopyridinium salts such as 2-chloro-1-methylpyridinium iodide and 2-fluoro-1-methylpyridinium iodide; and other compounds such as N-ethoxycarbonyl-2-ethoxy-1,2-dihydroquinoline, diethyl cyanophosphate, phosphorous oxychloride and acetic anhydride.

The amount of the "condensing agent" used is generally about 0.8 to about 5 mol, preferably about 1 to about 3 mol, per mol of the compound (IIb).

The reaction may be carried out in the presence of a base if required. Examples of the "base" include basic salts such as potassium acetate and sodium acetate; tertiary amines such as triethylamine, tripropylamine, tributylamine, cyclohexyldimethylamine, 4-dimethylaminopyridine, N-methylpiperidine, N-methylpyrrolidine and N-methylmorpholine. In addition, if required, the reaction may be carried out in the presence of a condensation accelerator such as 1-hydroxy-1H-benzotriazole (HOBt) monohydrate or the like.

The amount of "base" used is generally about 0.5 to about 5 mol, preferably about 2 to about 3 mol, per mol of the compound (IIb).

It is advantageous to carry out the present reaction using a solvent inactive to the reaction. Preferably, examples of such a solvent include: alcohols such as methanol, ethanol and propanol; hydrocarbons such as hexane, cyclohexane, benzene, toluene and xylene; ethers such as diethyl ether, diisopropyl ether, tetrahydrofuran, 1,4-dioxane and 1,2-dimethoxyethane; amides such as N,N-dimethylformamide, N,N-dimethylacetamide, hexamethylphosphoric triamide and 1-methylpyrrolidin-2-one; sulfoxides such as dimethyl sulfoxide; halogenated hydrocarbons such as dichloromethane, chloroform, carbon tetrachloride and 1,2-dichloroethane; nitriles such as acetonitrile and propionitrile; acid anhydride such as acetic anhydride; and mixtures thereof.

The reaction time is generally in the range of about 10 minutes to about 48 hours, preferably about 30 minutes to about 24 hours.

The reaction temperature is generally in the range of about -20 to about 150°C, preferably about 0 to about 100°C.

The reaction time can be shortened using a microwave reactor or the like.

The compound (Iₒ) thus obtained may be isolated from the reaction mixture by a conventional method and can be easily purified by any of well-known separation techniques, such as concentration, vacuum concentration, solvent extraction, crystallization, recrystallization, transfer dissolution and chromatography.

The compound (I₀) in which B is -NHB' can be also produced by the process represented by Reaction Formula 3 below. In other words, the compound (IIb) can be 2,2,2-trichloroethoxycarbonylated with 2,2,2-trichloroethyl chloroformate to prepare compound (I'). Subsequently, the compound (I) is reacted with compound (VI), thereby obtaining the compound (I₀).

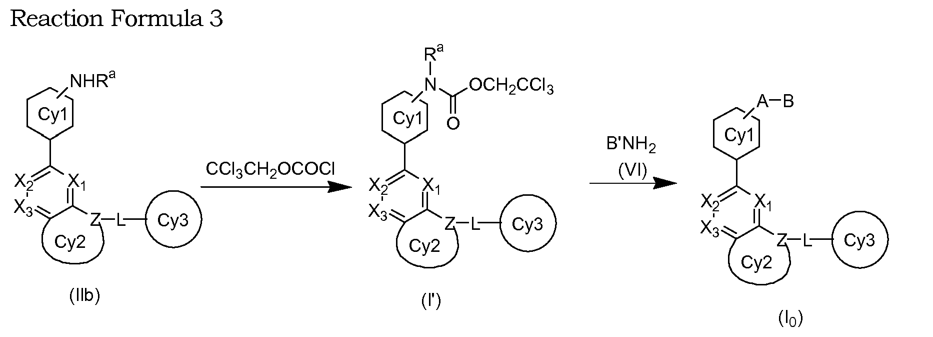

wherein each symbol is as defined above.

The compound (I') can be produced from the compound (IIb) in a manner similar to the production of the compound (I₀) from the compound (IIb).

The compound (I₀) can be produced by reaction of the compound (I) with the compound (VI) in a solvent that does not affect on the reaction under basic conditions.

The compound (VI) may be any of commercially available products or may be produced according to a well-known method or another method based thereon.

The amount of the compound (VI) used is generally about 2 to 10 mol, preferably about 2 to 5 mol, per mol of the compound (I').

The examples of the "base" include pyridine, triethylamine, diisopropylethylamine, potassium carbonate, sodium carbonate, sodium hydride and potassium hydride.

The amount of the "base" used is generally about 2 to 10 mol, preferably about 2 to 5 mol, per mol of the compound (I').

Examples of the solvent that does not affect on the reaction include: ethers such as tetrahydrofuran; halogenated hydrocarbons such as chloroform; aromatic hydrocarbons such as toluene; amides such as N,N-dimethylformamide; and sulfoxides such as dimethyl sulfoxide. Two or more of these solvents may be mixed together at a suitable ratio.

The reaction temperature is generally in the range of about -50 to 200°C, preferably about 0 to 100°C.

The reaction time is generally in the range of about 10 minutes to about 36 hours, preferably about 30 minutes to about 24 hours.

The compound (I₀) thus obtained may be isolated from the reaction mixture by a conventional method and can be easily purified by any of well-known separation techniques, such as concentration, vacuum concentration, solvent extraction, crystallization, recrystallization, transfer dissolution and chromatography.

The compound (I₀) can be produced by the process represented by Reaction Formula as follows.

The compound (I₀) can be produced by the process represented by Reaction Formula 4 as follows.

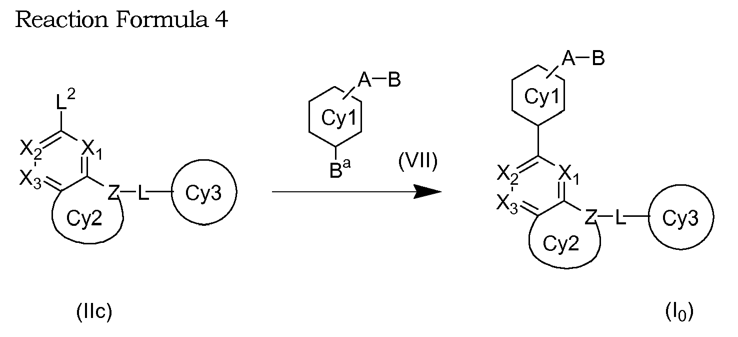

In the reaction formula, L² represents a leaving group; B^{a} represents boronic acids and other symbols are as defined above.
The compound (I₀) is produced by carrying out Suzuki coupling between the compound (IIc) and the compound (VII).
The reaction is carried out by reaction of the compound (IIc) with boronic acid (VII) in a solvent under basic conditions in the presence of a transition metal catalyst.
The compound (VII) may be any of commercially available products or may be produced according to a well-known method or another method based thereon.
Examples of the "leaving group" represented by L² include a halogen atom (e.g. chlorine, bromine, iodine), C₁₋₆ alkylsulfonyloxy group which may be halogenated (e.g., triffuoromethanesulfonyloxy).
Examples of the boronic acid represented by B^{a} include substituted boronic acids and substituted boronic acid esters.
The amount of the "boronic acids" used is about 0.5 to about 10 mol, preferably about 0.9 to about 3 mol, per mol of the compound (IIc).
Examples of the "base" include basic salts such as sodium carbonate, potassium carbonate, cesium carbonate and sodium hydrogen carbonate; aromatic amines such as the pyridine, lutidine; tertiary amines such as triethylamine, tripropylamine, tributylamine, cyclohexyldimethylamine, 4-dimethylaminopyridine, N,N-dimethylaniline, N-methylpiperidine, N-methylpyrrolidine and N-methylmorpholine; and metal alkoxides such as sodium methoxide, sodium ethoxide, sodium tert-butoxide and potassium tert-butoxide.
Examples of the "transition metal catalyst" include palladium catalysts such as palladium acetate, palladium chloride, tetrakis(triphenylphosphine)palladium, 1,1-bis(diphenylphosphino)ferrocene dichloropalladium and dichlorobis(triphenylphosphine)palladium. The amount of the transition metal catalyst used is about 0.001 to about 3 mol, preferably about 0.02 to about 0.2 mol, per mol of the compound (IIc).
Examples of the solvent include: ethers such as diethyl ether, diisopropyl ether, tetrahydrofuran, dioxane, 1,2-dimethoxyethane; alcohols such as methanol, ethanol and propanol; hydrocarbons such as benzene, toluene, carbon disulfide, cyclohexane and hexane; amides such as N, N-dimethylformamide and N,N-dimethylacetamide; halogenated hydrocarbons such as dichloromethane, chloroform, carbon tetrachloride and 1,2-dichloroethane; nitriles such as acetonitrile and propionitrile; sulfoxides such as dimethyl sulfoxide; and water or mixture solvents thereof.

The reaction temperature is generally in the range of 0 to 250°C, preferably 50 to 150°C. The reaction time is generally about 5 minutes to about 48 hours, preferably about 30 minutes to about 24 hours.
The reaction time can be shortened using a microwave reactor or the like.
In addition, compounds obtained in the respective steps may be directly used as a reaction solution or a crude product in the subsequent reaction. Alternatively, it may be isolated from the reaction mixture by a conventional method and can be easily purified by any of well-known separation techniques (e.g., recrystallization, distillation and chromatography).

The compound (Iₒ) can be produced by the process represented by Reaction Formula 5 as follows.

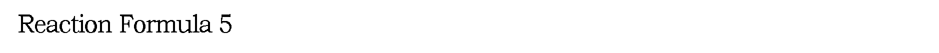

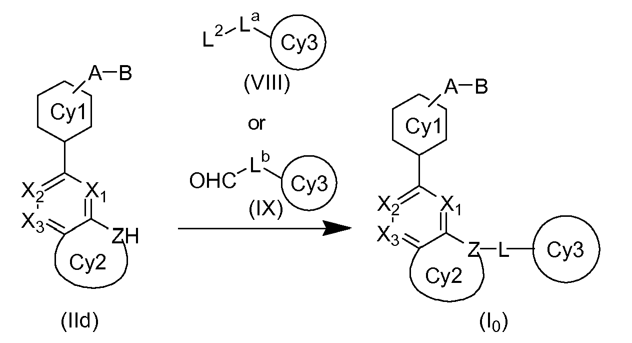

In the reaction formula, L^{a} is a bond, -CH₂- or -(CH₂)₂-, L^{b} is a bond or -CH₂-, and other symbols are as defined above.
The compound (I₀) wherein Z is a nitrogen atom and L is a bond can be produced by reacting compound (IId) with compound (VIII) in the presence of a base when desired. Where necessary, moreover, a copper catalyst such as copper, copper salt and the like may also be used. Alternatively, it can also be produced by Buchwald cross coupling reaction.

The compound (VIII) may be any of commercially available products or may be produced according to a well-known method or another method based thereon.
The amount of the compound (VIII) used is generally about 0.8 to about 10 mol, preferably about 1 to about 5 mol, per mol of the compound (IId).
Examples of the "base" include basic salts such as sodium carbonate, potassium carbonate, cesium carbonate and sodium hydrogen carbonate; aromatic amines such as the pyridine, lutidine; tertiary amines such as triethylamine, tripropylamine, tributylamine, cyclohexyldimethylamine, 4-dimethylaminopyridine, N,N-dimethylaniline, N-methylpiperidine, N-methylpyrrolidine and N-methylmorpholine; alkaline metal hydrides such as sodium hydride and potassium hydride; metal amides such as sodium amide, lithium diisopropylamide and lithium hexamethyldisilazide; and metal alkoxides such as sodium methoxide, sodium ethoxide, sodium tert-butoxide and potassium tert-butoxide.
The amount of the "base" used is generally about 0.8 to about 10 mol, preferably about 1 to about 5 mol, per mol of the compound (IId).

It is advantageous to carry out the present reaction in the presence of a solvent inactive to the reaction. Preferable examples of such a solvent include, but not specifically limited as long as the reaction proceeds, alcohols such as methanol, ethanol and propanol; ethers such as diethyl ether, tetrahydrofuran, 1,4-dioxane and 1,2-dimethoxyethane; hydrocarbons such as benzene, toluene, cyclohexane and hexane; amides such as N,N-dimethylformamide and N,N-dimethylacetamide; halogenated hydrocarbons such as dichloromethane, chloroform, carbon tetrachloride and 1,2-dichloroethane; nitriles such as acetonitrile and propionitrile; and sulfoxides such as dimethyl sulfoxide; or mixture solvents thereof.

Examples of the "copper catalyst" include copper, copper halide (CuI, CuBr, CuCl etc.), copper oxide (CuO) and the like.
The amount of the "copper catalyst" to be used is generally about 0.1 to about 10 mol, preferably about 0.5 to about 2 mol, per mol of the compound (IId).

When compound (I₀) is synthesized by Buchwald reaction, examples of the palladium catalyst include palladium acetate, palladium chloride, tetrakis(triphenylphosphine)palladium, bis(dibenzylideneacetone)palladium, tris(dibenzylideneacetone)dipalladium and the like. As the "ligand", phosphine is preferable, and examples include trialkylphosphine, triarylphosphine, trialkoxyphosphine and the like.
The amount of the palladium catalyst to be used is generally about 0.001 to about 5 mol, preferably about 0.01 to about 0.5 mol, per mol of the compound (IId). The amount of "phosphine" to be used is generally about 0.001 to about 10 mol, preferably about 0.01 to about 1 mol, per mol of the compound (IId).

The reaction time is generally about 30 minutes to about 72 hours, preferably about one hour to about 48 hours
The reaction temperature is generally about -20 to about 200°C, preferably about 0 to about 150°C.

The reaction time of this reaction can be shortened by using a microwave reactor and the like.
The compound (I₀) wherein Z is a nitrogen atom and L is not a bond can be produced by reacting compound (IId) with compound (VIII) in the presence of a base when desired.
The amount of the compound (VIII) used is about 0.8 to about 5.0 mol, preferably 1.0 to about 2.0 mol, per mol of the compound (IId).
Examples of the "base" include basic salts such as sodium carbonate, potassium carbonate, cesium carbonate and sodium hydrogen carbonate; aromatic amines such as the pyridine, lutidine; tertiary amines such as triethylamine, tripropylamine, tributylamine, cyclohexyldimethylamine, 4-dimethylaminopyridine, N,N-dimethylaniline, N-methylpiperidine, N-methylpyrrolidine and N-methylmorpholine; alkaline metal hydrides such as sodium hydride and potassium hydride; metal amides such as sodium amide, lithium diisopropylamide and lithium hexamethyldisilazide; and metal alkoxides such as sodium methoxide, sodium ethoxide and potassium tert-butoxide.
The amount of the base used is about 0.8 to about 5.0 mol, preferably about 1.0 to about 2.0 mol, per mol of the compound (IId).
It is advantageous to carry out the present reaction in the presence of a solvent inactive to the reaction. Preferable examples of such a solvent include, but not specifically limited as long as the reaction proceeds, alcohols such as methanol, ethanol and propanol; ethers such as diethyl ether, tetrahydrofuran, dioxane and 1,2-dimethoxyethane; hydrocarbons such as benzene, toluene, cyclohexane and hexane; amides such as N,N-dimethylformamide and N,N-dimethylacetamide; halogenated hydrocarbons such as dichloromethane, chloroform, carbon tetrachloride and 1,2-dichloroethane; nitriles such as acetonitrile and propionitrile; and sulfoxides such as dimethyl sulfoxide; and mixture solvents thereof.
The reaction time is generally about 30 minutes to about 48 hours, preferably about one hour to about 24 hours. The reaction temperature is generally about -20 to about 200°C, preferably about 0 to about 150°C.

Furthermore, instead of the above reaction, the compound (I₀) may be prepared using the compound (IId) and the compound (IX) by a reductive amination reaction.
The compound (IX) may be any of commercially available products or may be produced according to a well-known method or another method based thereon.

The amount of the compound (IX) used is about 0.8 to about 5.0 mol, preferably about 1.0 to about 2.0 mol, per mol of the compound (IId).
Examples of the "reducing agent" include: metal hydrides such as sodium borohydride, sodium cyanoborohydride, sodium triacetoxyborohydride and lithium aluminum hydride; boranes such as a borane-tetrahydrofuran complex; hydrosilanes such as triethylsilane; or formic acid. If desired, an acid catalyst may be added together with the reducing agent. Examples of the acid catalyst include: mineral acids such as hydrochloric acid, hydrobromic acid and sulfuric acid; sulfonic acids such as methanesulfonic acid and p-toluenesulfonic acid; organic acids such as acetic acid, propionic acid and trifluoroacetic acid; and Lewis acids such as zinc chloride and aluminum chloride.
The amount of the "reducing agent" used is about 0.25 to about 5.0 mol, preferably about 0.5 to about 2.0 mol, per mol of the compound (IId).
The amount of the acid catalyst used is, for example, in the case of mineral acids, generally about 1 to about 100 mol, preferably about 1 to about 20 mol, per mol of the compound (IId) .
It is advantageous to carry out the present reaction in the presence of a solvent inactive to the reaction. Preferable examples of such a solvent include, but not specifically limited as long as the reaction proceeds, alcohols such as methanol, ethanol and propanol; ethers such as diethyl ether, tetrahydrofuran, dioxane and 1,2-dimethoxyethane; hydrocarbons such as benzene, toluene, cyclohexane and hexane; amides such as N,N-dimethylformamide and N,N-dimethylacetamide; and mixture solvents thereof.
The reaction time is generally about 5 minutes to about 48 hours, preferably about 30 minutes to about 24 hours. The reaction temperature is generally about - 20 to about 200°C, preferably about 0 to about 100°C.
After condensation of the compound (IId) and the compound (IX), instead of reduction with a reducing agent, the compound (I₀) may be also produced by a catalytic hydrogenation reaction with any of various catalysts under hydrogen atmosphere. Examples of the catalyst used include platinum oxide, platinum activated carbon, palladium activated carbon, nickel, copper-chromium oxide, rhodium, cobalt and ruthenium. The amount of the catalyst used is about 1 to about 1000% by weight, preferably about 5 to about 50% by weight with respect to the compound (IId).
It is advantageous to carry out the present reaction in the presence of a solvent inactive to the reaction. Preferable examples of such a solvent include, but not specifically limited as long as the reaction proceeds, alcohols such as methanol, ethanol and propanol; ethers such as diethyl ether, tetrahydrofuran, dioxane and 1,2-dimethoxyethane; hydrocarbons such as benzene, toluene, cyclohexane and hexane; amides such as N,N-dimethylformamide and N,N-dimethylacetamide; water; and mixture solvents thereof.
The reaction time is generally about 30 minutes to about 48 hours, preferably about 30 minutes to about 24 hours. The reaction temperature is generally about 0 to about 120°C, preferably about 20 to about 80°C.

In addition, the product may be directly used as a reaction solution or a crude product in the subsequent reaction. Alternatively, the product may be isolated from the reaction mixture by a conventional method and can be easily purified by any of well-known separation techniques (e.g., recrystallization, distillation and chromatography).

The compound (Iₒ) can be produced by the process represented by Reaction Formula 6 as follows.

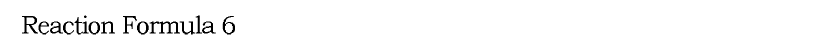

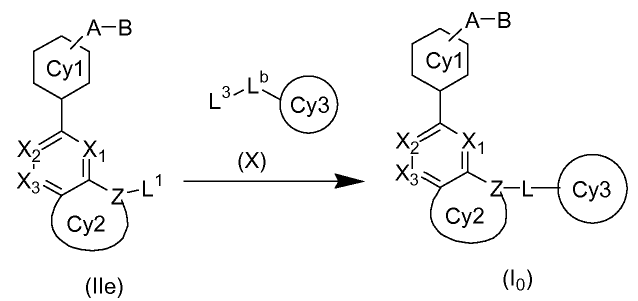

In the reaction formula, L³ represents a leaving group selected from a hydroxy group, an amino group and a mercapto group and other symbols are each as defined above.
When Z is a carbon atom, the compound (I₀) can be produced by reacting the compound (IIe) with the compound (X) in the presence of a base when desired.
The compound (X) may be any of commercially available products or may be produced according to a well-known method or another method based thereon.
The amount of the compound (X) used is about 0.8 to about 5.0 mol, preferably about 1.0 to about 2.0 mol, per mole of the compound (IIe).
Examples of the "base" include: basic salts such as sodium carbonate, potassium carbonate, cesium carbonate and sodium hydrogen carbonate; aromatic amines such as the pyridine and lutidine; tertiary amines such as triethylamine, tripropylamine, tributylamine, cyclohexyldimethylamine, 4-dimethylaminopyridine, N,N-dimethylaniline, N-methylpiperidine, N-methylpyrrolidine and N-methylmorpholine; alkaline metal hydrides such as sodium hydride and potassium hydride; metal amides such as sodium amide, lithium diisopropylamide and lithium hexamethyldisilazide; and metal alkoxides such as sodium methoxide, sodium ethoxide and potassium tert-butoxide.
The amount of the base used is about 0.8 to about 5.0 mol, preferably about 1.0 to about 2.0 mol, per mol of the compound (IIe).
It is advantageous to carry out the present reaction in the presence of a solvent inactive to the reaction. Preferable examples of such a solvent include, but not specifically limited as long as the reaction proceeds, alcohols such as methanol, ethanol and propanol; ethers such as diethyl ether, tetrahydrofuran, dioxane and 1,2-dimethoxyethane; hydrocarbons such as benzene, toluene, cyclohexane and hexane; amides such as N,N-dimethylformamide and N,N-dimethylacetamide; halogenated hydrocarbons such as dichloromethane, chloroform, carbon tetrachloride and 1,2-dichloroethane; nitriles such as acetonitrile and propionitrile; sulfoxides such as dimethyl sulfoxide; and mixture solvents thereof.
The reaction time is generally about 30 minutes to about 48 hours, preferably about one hour to about 24 hours. The reaction temperature is generally about -20 to about 200°C, preferably about 0 to about 150°C.

Instead of the above reaction, the Mitsunobu reaction ("Synthesis", pages 1-27, 1981) may be used.
The compound (IIe) is reacted with the compound (X) in which L¹ and L³ are OH in the presence of azodicarboxylates (e.g., diethyl azodicarboxylate) and phosphines (e.g., triphenylphosphine and tributylphosphine).
The amount of the compound (X) used is about 1.0 to 5.0 mol, preferably about 1.0 to 2.0 mol, per mol of the compound (IIe).
The amounts of "azodicarboxylates" and "phosphines" used are about 1.0 to 5.0 mol, preferably about 1.0 to 2.0 mol, per mol of compound (IIe), respectively.
It is advantageous to carry out the present reaction in the presence of a solvent inactive to the reaction. Preferable examples of such a solvent include, but not specifically limited as long as the reaction proceeds, ethers such as diethyl ether, tetrahydrofuran, dioxane and 1,2-dimethoxyethane; hydrocarbons such as benzene, toluene, cyclohexane and hexane; amides such as N,N-dimethylformamide and N,N-dimethylacetamide; halogenated hydrocarbons such as dichloromethane, chloroform, carbon tetrachloride and 1,2-dichloroethane; nitriles such as acetonitrile and propionitrile; sulfoxides such as dimethyl sulfoxide; and mixture solvents thereof.
The reaction time is generally 5 minutes to 48 hours, preferably 30 minutes to 24 hours. The reaction temperature is generally -20 to 200°C, preferably 0 to 100°C.

The product may be directly used as a reaction solution or a crude product in the subsequent reaction. Alternatively, the product may be isolated from the reaction mixture by a conventional method and can be easily purified by any of well-known separation techniques (e.g., recrystallization, distillation and chromatography).

The compound (IIa) can be produced by the process represented by Reaction Formula 7 as follows. 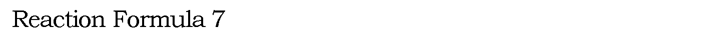 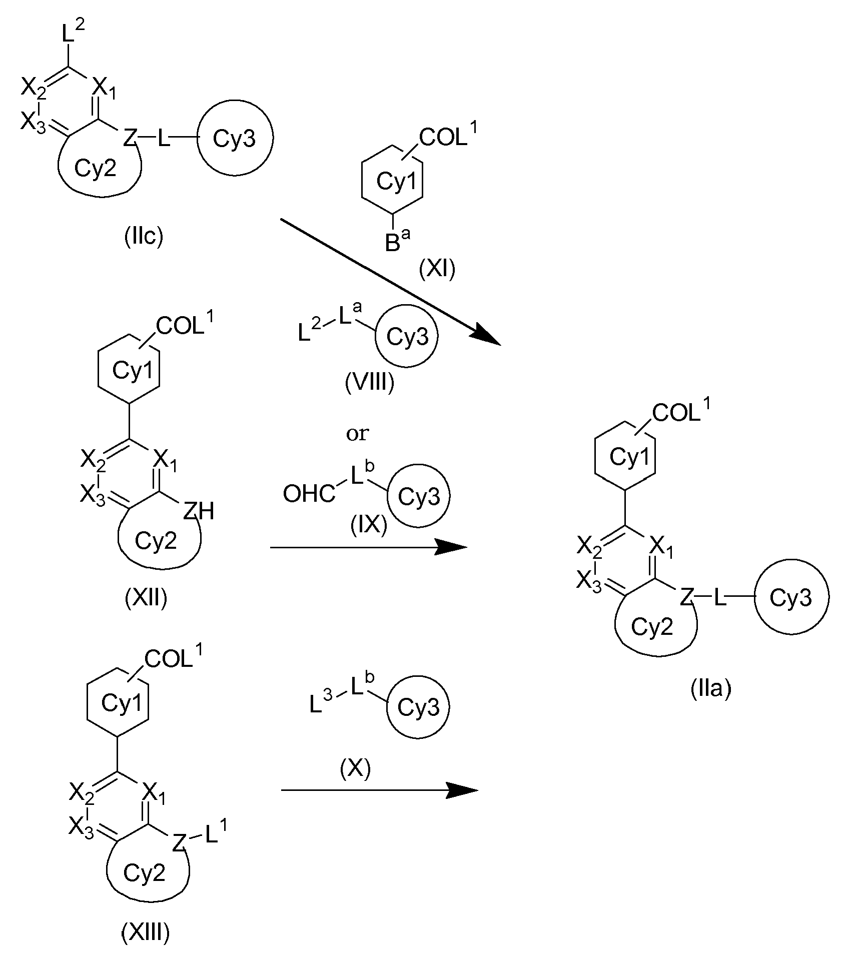 wherein the symbols are as defined above.
The compound (IIa) can be produced from the compound (IIc) and the compound (XI) in a manner similar to the production of the compound (I₀) from the compound (IIc) as described in Reaction Formula 4; from the compound (XII) in a manner similar to the production of the compound (I₀) from the compound (IId) as described in Reaction Formula 5; from the compound (XIII) in a manner similar to the production of the compound (I₀) from the compound (IIe) as described in Reaction Formula 6
The compound (IIa) thus obtained may be isolated from the reaction mixture by a conventional method and can be easily purified by any of well-known separation techniques, such as concentration, vacuum concentration, solvent extraction, crystallization, recrystallization, transfer dissolution and chromatography.

The compound (IId) can be produced by the process represented by Reaction Formula 8 as follows.

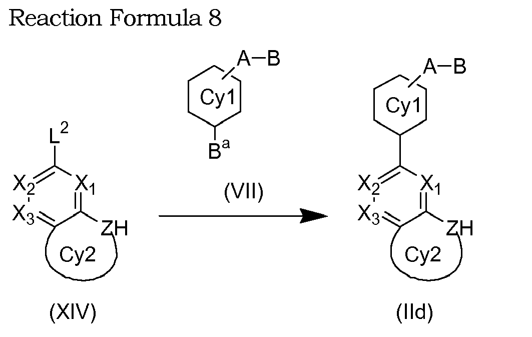

In the reaction formula, symbols are each as defined above.
The compound (IId) can be produced from the compound (XIV) in a manner similar to the production of the compound (I₀) from the compound (IIc) as described above in Reaction Formula 4.
The compound (XIV) may be any of commercially available products or may be produced according to a well-known method or another method based thereon.
The compound (IId) thus obtained may be isolated from the reaction mixture by a conventional method and can be easily purified by any of well-known separation techniques, such as concentration, vacuum concentration, solvent extraction, crystallization, recrystallization, transfer dissolution and chromatography.

The compound (IIe) can be produced from the compound (XV) by the process represented by Reaction Formula 9 as follows.

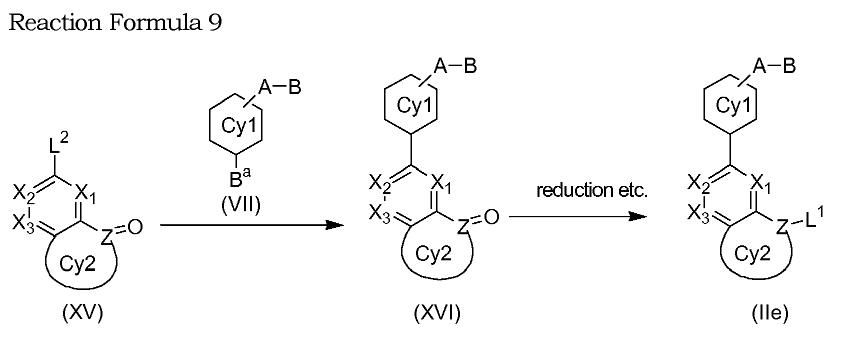

In the reaction formula, symbols are each as defined above.
The compound (XVI) can be produced from the compound (XV) in a manner similar to the production of the compound (I₀) from the compound (IIc) as described in Reaction Formula 4.
The compound (XV) may be any of commercially available products or may be produced according to a well-known method or another method based thereon.
The compound (IIe) can be produced by subjecting compound (XVI) to a reduction reaction.
The reduction reaction can also be performed by a method known per se, for example, the method described in The Fourth Series of Experimental Chemistry, vol. 26 (Ed. Chemical Society of Japan), published by Maruzen Co., Ltd.
The compound (IIe) thus produced may be isolated from the reaction mixture by a conventional method and can be easily purified by any of well-known separation techniques, concentration, vacuum concentration, solvent extraction, crystallization, recrystallization, transfer dissolution and chromatography.

The compound (IIc) can be produced by a well-known method, for example, the method described in JP-A-2005-35993 and the like or a method analogous thereto and the like.
Alternatively, it may be produced by the process represented by Reaction Formula 10 as follows.

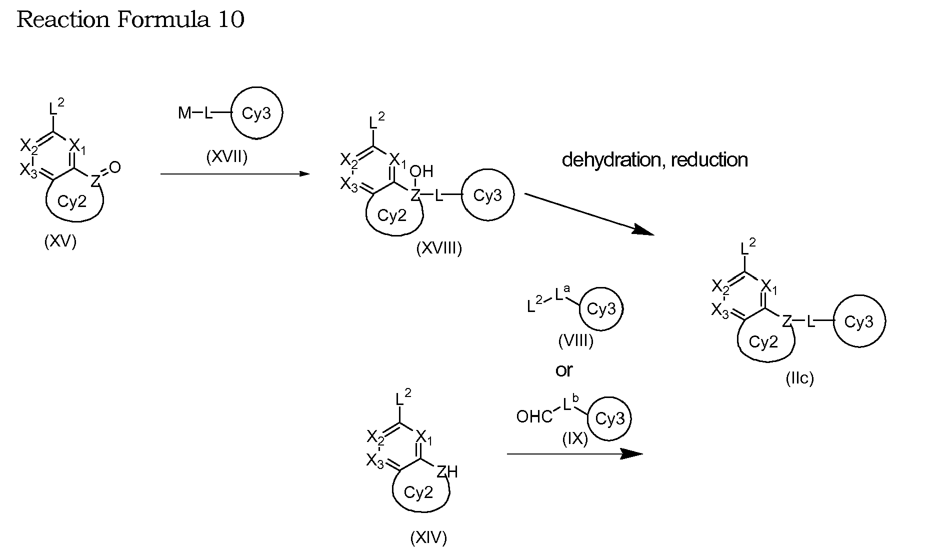

In the reaction formula, M represents a metal and other symbols are as defined above.
When Z is a carbon atom, compound (XVIII) can be produced by reacting compound (XV) with organic metal compound (XVII).
In the formula, the organic metal compound (XVII) can be easily obtained as a commercial product or may be produced according to a well-known method or another method based thereon, such as one described in The Fourth Series of Experimental Chemistry, vol. 25 (Ed. Chemical Society of Japan), published by Maruzen Co., Ltd.
As the organic metal compound (XVII), Grignard reagents and organic lithium reagents are preferable.
The amount of the compound (XVII) used is about 0.8 to about 30 mol, preferably about 1.0 to about 20 mol, per mol of the compound (XV).
It is advantageous to carry out the present reaction in the absence of a solvent or in the presence of a solvent inactive to the reaction. Preferable examples of such a solvent include, but not specifically limited as long as the reaction proceeds, alcohols such as methanol, ethanol and propanol; hydrocarbons such as hexane, cyclohexane, benzene, toluene and xylene; ethers such as diethyl ether, diisopropyl ether, tetrahydrofuran, dioxane and 1,2-dimethoxyethane; amides such as N, N-dimethylformamide, N,N-dimethylacetamide and hexamethylphosphoric triamide; sulfoxides such as dimethyl sulfoxide; halogenated hydrocarbons such as dichloromethane, chloroform, carbon tetrachloride and 1,2-dichloroethane; and mixture solvents thereof.
The reaction time is generally about 10 minutes to about 24 hours, preferably about 30 minutes to about 12 hours. The reaction temperature is generally about -100 to about 120°C, preferably about -80 to about 60°C.
In addition, the product may be directly used as a reaction solution or a crude product in the subsequent reaction. Alternatively, the product may be isolated from the reaction mixture by a conventional method and can be easily purified by any of well-known separation techniques (e.g., recrystallization, distillation and chromatography).

Furthermore, the compound (IIc) can be produced by subjecting the compound (XVIII) to reductive dehydration.
The reductive dehydration may be carried out by a catalytic reduction method, a method using an organic silyl reagent (e.g., alkylsilane reagent), or the like.
In the catalytic reduction method, the compound (XV) can be obtained by reaction of the compound (XVIII) with a metal catalyst under hydrogen atmosphere. The reaction may be carried out in the presence of an appropriate acid catalyst if required.
Examples of the "metal catalyst" include Raney nickel, platinum oxide, metal palladium and palladium activated carbon. The amount of the "metal catalyst" used is generally about 1 to about 1000% by weight, preferably about 5 to about 20% by weight with respect to the compound (XVIII).
Examples of the "acid catalyst" include organic acids such as formic acid, acetic acid, trifluoroacetic acid, p-toluenesulfonic acid; and mineral acid such as sulfuric acid, hydrochloric acid and hydrobromic acid. The amount of the "acid catalyst" used is about 0.1 mol to an excess amount thereof per mol of the compound (XVIII) .

It is advantageous to carry out the present reaction in the presence of a solvent inactive to the reaction. Preferable examples of such a solvent include, but not specifically limited as long as the reaction proceeds, alcohols such as methanol, ethanol and propanol; ethers such as diethyl ether, tetrahydrofuran, dioxane and 1,2-dimethoxyethane; hydrocarbons such as benzene, toluene, cyclohexane and hexane; amides such as N,N-dimethylformamide and N,N-dimethylacetamide; organic acids such as acetic acid; water; and mixture solvents thereof. A hydrogen pressure is generally about 1 to about 100 atm, preferably about 1 to about 5 atm. The reaction time is generally about 30 minutes to about 48 hours, preferably about one hour to about 24 hours. The reaction temperature is generally about 0 to about 120°C, preferably about 20 to about 80°C.
After removal of the catalyst, the product may be isolated from the reaction mixture by a conventional method and can be easily purified by any of well-known separation techniques (e.g., recrystallization, distillation and chromatography).
In the process using an organic silyl reagent (alkylsilane reagent), the compound (XV) can be produced by reaction of the compound (XVIII) with the alkylsilane reagent and an acid.
Examples of the alkylsilane reagent include triethylsilane and phenyldimethylsilane. The amount of the "alkylsilane reagent" used is about 0.8 to about 20 mol, preferably about 1 to about 10 mol, per mol of the compound (XVIII).
The acid used may be an organic acid such as trifluoroacetic acid. The amount of the acid used is about 0.1 mol to an excessive amount per mol of the compound (XVIII).
It is advantageous to carry out the present reaction in the absence of a solvent or in the presence of a solvent inactive to the reaction. Examples of such a solvent include, but not specifically limited as long as the reaction proceeds, ethers such as diethyl ether, tetrahydrofuran, dioxane and 1,2-dimethoxyethane; hydrocarbons such as benzene, toluene, cyclohexane and hexane; organic acids such as acetic acid, trifluoroacetic acid; mixture solvents thereof.
The product may be isolated from the reaction mixture by a conventional method and can be easily purified by any of well-known separation techniques (e.g., recrystallization, distillation and chromatography).

In addition, the compound (IIc) wherein Z is a nitrogen atom can be produced from the compound (XIV) by a method similar to the production of the compound (I₀) from the compound (IId) as described in Reaction Formula 5.

The compound (XIII) may be produced by the process represented by Reaction Formula 11 as follows. 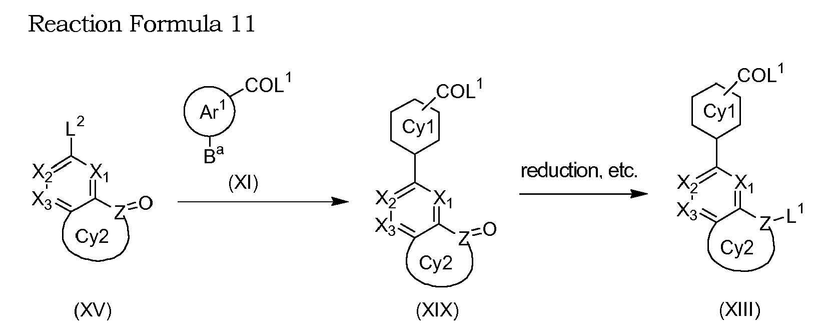 In the reaction formula, symbols are as defined above.

The compound (XIII) is produced from the compound (XV) in a manner similar to the production of the compound (IIe) from the compound (XV) as described in Reaction Formula 9.

The compound (IIb) may be produced by the process represented by Reaction Formula 12 as follows. 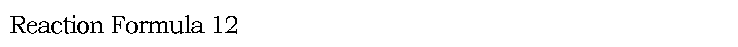 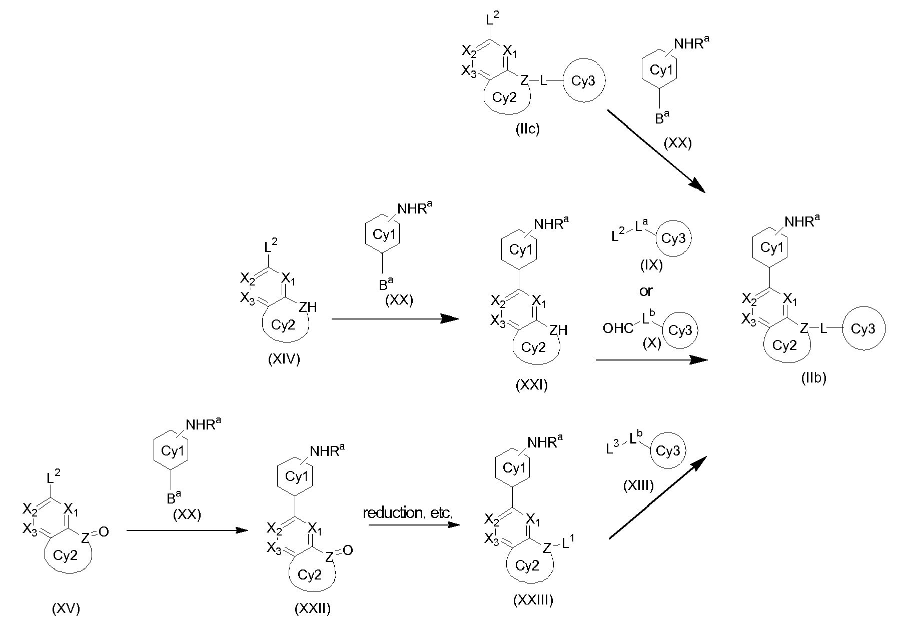 The compound (IIb) can be produced from compound (IIc) and compound (XX) by a method similar to the production of the compound (I₀) from the compound (IIc) as described in Reaction Formula 4; or by producing compound (XXI) from compound (XIV) by a method similar to the production of the compound (I₀) from the compound (IIc) as described in Reaction Formula 4 and then by a method similar to the production of the compound (Io) from the compound (IId) as described in Reaction Formula 5; or by producing compound (XXIII) from compound (XV) by a method similar to the production of the compound (IIe) from the compound (XV) as described in Reaction Formula 9 and then by a method similar to the production of the compound (I₀) from the compound (IIe) as described in Reaction Formula 6.
The compound (IIb) thus obtained may be isolated from the reaction mixture by a conventional method and can be easily purified by any of well-known separation techniques, such as concentration, vacuum concentration, solvent extraction, crystallization, recrystallization, transfer dissolution and chromatography.

In each case, if required, the product is further subjected to one or any combination of well-known reactions, such as deprotection, acylation, alkylation, hydrogenation, oxidation, reduction, carbon-chain extension and substituent change. Consequently, the compound (I₀) can be synthesized.

In the case that the product of interest is obtained in free form, it may be converted into salt form by an ordinary method. If the product of interest is obtained in salt form, it may be converted into a free form or another salt by an ordinary method. The compound (I₀) thus obtained may be isolated and purified from a reaction solution by any of well-known techniques, such as transfer dissolution, concentration, solvent extraction, cracking, crystallization, recrystallization and chromatography.
Furthermore, if the compound (I₀) is present as a configurational isomer, a diastereomer, or a conformer, it may be isolated by any of the separation and purification techniques if required. In addition, if the compound (I₀) is present as a racemic body, it can be separated into a d-isomer and an 1-isomer using a usual optical resolution technique.

In addition to the compound (I₀), the product may be used as a prodrug of the compound (I₀). The prodrug of the compound (I₀) means a compound which can be converted into the compound (I₀) by reaction with an enzyme, gastric acid, or the like under physiological conditions in the living body. In other words, it means a compound which can be converted into the compound (I₀) by enzymatic oxidation, reduction, hydrolysis or the like, or a compound which can be converted into the compound (I₀) by hydrolysis with gastric acid or the like.

Examples of the prodrug of the compound (I₀) include a compound in which an amino group of the compound (I₀) is acylated, alkylated, or phosphorylated (e.g., the amino group of the compound (I₀) is eicosanoylated, alanylated, pentylaminocarbonylated, (5-methyl-2-oxo-1,3-dioxolen-4-yl)methoxycarbonylated, tetrahydrofuranylated, pyrrolidylmethylated, pivaloyloxymethylated, or tertbutylated); a compound in which a hydroxyl group of the compound (I₀) is acylated, alkylated, phosphorylated, or borated (e.g., the hydroxyl group of the compound (I₀) is acetylated, palmitoylated, propanoylated, pivaloylated, succinylated, fumarylated, alanylated, or dimethylaminomethylcarbonylated); a compound in which a carboxy group of compound (I₀) is esterified or amidated (e.g., a compound in which a carboxy group of the compound (I₀) is ethyl esterified, phenyl esterified, carboxymethyl esterified, dimethylaminomethyl esterified, pivaloyloxymethyl esterified, ethoxycarbonyloxyethyl esterified, phthalidyl esterified, (5-methyl-2-oxo-1,3-dioxolen-4-yl)methyl esterified, cyclohexyloxycarbonylethyl esterified, or methylamidated). These compounds can be produced from compound (I₀) by a well-known method. The prodrug of compound (I₀) may be a compound that converts to the compound (Iₒ) under physiological conditions as described in Development of Pharmaceutical Products, vol. 7, Molecule Design, 163-198, Hirokawa Shoten (1990).

The compound of the present invention has an excellent GPR52 agonist activity and can be used as a preventive or therapeutic agent to mammals (e.g., humans, cows, horses, dogs, cats, monkeys, mice and rats, particularly humans among them) for diseases, such as mental diseases (e.g., schizophrenia, depression, anxiety, bipolar disorder or PTSD, aporioneurosis and obsessive-compulsive disorder); and neurodegenerative diseases (e.g., Alzheimer's disease, mild cognitive impairment (MCI), Parkinson's disease, amyotrophic lateral sclerosis (ALS), Huntington's disease, spinocerebellar degeneration, multiple sclerosis (MS), and Pick disease). In particular, the compound of the present invention is useful for improving the medical conditions of schizophrenia, such as (1) positive symptoms such as delusions and hallucination; (2) negative symptoms such as hypesthesia, social withdrawal and disinclination or loss of concentration; and (3) cognitive dysfunction.

The compound of the present invention is superior in metabolic stability, so that the compound of this invention can be expected to have an excellent therapeutic effect on the above diseases even in a small dose.

The compound of the present invention has low toxicity (which is a medicament superior to others with respect to, for example, acute toxicity, chronic toxicity, genetic toxicity, reproductive toxicity, cardiotoxicity, drug interactions and carcinogenicity). The compound of the present invention is directly used as a medicament or a pharmaceutical composition mixed with a pharmaceutically acceptable carrier or the like to be orally or parenterally administered to mammals (e.g., humans, monkeys, cows, horses, pigs, mice, rats, hamsters, rabbits, cats, dogs, sheep and goats) in safety. The term "parenterally" means intravenous, intramuscular, subcutis, intraorgan, intranasal, intracutaneous, eye-drop, intracerebral, rectal, intravaginal, or intraabdominal administration.

The term "pharmaceutically acceptable carrier" means any of various organic or inorganic carriers conventionally used as materials for pharmaceutical preparations, which are added as excipient, lubricant, binder and disintegrant for solid preparations; and solvent, solubilizing agents, suspending agent, isotonicity agent, buffer and soothing agent and the like for liquid preparations. Where necessary, preparation additive such as preservative, antioxidant, colorant, sweetening agent and the like can be used.

Preferable examples of the excipient include lactose, sucrose, D-mannitol, D-sorbitol, starch, pregelatinized starch, dextrin, crystalline cellulose, low-substituted hydroxypropyl cellulose, sodium carboxymethyl cellulose, gum arabic, pullulan, light anhydrous silicic acid, synthetic aluminum silicate and magnesium aluminometasilicate.
Preferable examples of the lubricant include magnesium stearate, calcium stearate, talc, colloidal silica and the like.

Preferable examples of the binder include pregelatinized starch, saccharose, gelatin, gum arabic, methylcellulose, carboxymethyl cellulose, sodium carboxymethyl cellulose, crystalline cellulose, sucrose, D-mannitol, trehalose, dextrin, pullulan, hydroxypropyl cellulose, hydroxypropyl methyl cellulose and polyvinyl pyrrolidone.

Preferable examples of the disintegrant include lactose, sucrose, starch, carboxymethyl cellulose, carboxymethyl cellulose calcium, croscarmellose sodium, sodium carboxymethyl starch, light anhydrous silicic acid and low-substituted hydroxypropylcellulose.

Preferable examples of the solvent include water for injection, physiological saline, Ringer's solution, alcohol, propylene glycol, polyethylene glycol, sesame oil, corn oil, olive oil and cottonseed oil.

Preferable examples of the solubilizing agents include polyethylene glycol, propylene glycol, D-mannitol, trehalose, benzyl benzoate, ethanol, trisaminomethane, cholesterol, triethanolamine, sodium carbonate, sodium citrate, sodium salicylate and sodium acetate.

Preferable examples of the suspending agent include surfactants such as stearyl triethanolamine, sodium lauryl sulfate, lauryl aminopropionate, lecithin, benzalkonium chloride, benzethonium chloride and glycerol monostearate; for example, hydrophilic polymers such as polyvinyl alcohol, polyvinyl pyrrolidone, sodium carboxymethyl cellulose, methylcellulose, hydroxymethyl cellulose, hydroxyethyl cellulose and hydroxypropyl cellulose; polysorbates and polyoxyethylene hydrogenated castor oil.

Preferable examples of an isotonicity agent include sodium chloride, glycerin, D-mannitol, D-sorbitol and glucose.
Preferable examples of the buffer include buffers such as phosphate, acetate, carbonate and citrate.
Preferable examples of the soothing agent include benzyl alcohol.
Preferable examples of the preservative include p-hydroxybenzoic acid esters, chlorobutanol, benzyl alcohol, phenethyl alcohol, dehydroacetic acid and sorbic acid.
Preferable examples of the antioxidant include sulfite and ascorbate.

Preferable examples of the colorant include water-soluble edible tar pigments (e.g., food colors such as Food Color Red Nos. 2 and 3, Food Color Yellow Nos. 4 and 5 and Food Color Blue Nos. 1 and 2), water-insoluble lake pigments (e.g., aluminum salt of the aforementioned water-soluble edible tar pigment) and natural pigments (e.g., β-carotene, chlorophil and colcothar).
Preferable examples of the sweetening agent include saccharin sodium, dipotassium glycyrrhizinate, aspartame and stevia.

Examples of the dosage form of the pharmaceutical composition include oral agents such as tablets (inclusive of sugarcoated tablets, film-coating tablets, sublingual tablets and orally disintegrable tablets), capsules (inclusive of soft capsules and micro capsules), granules, powders, troches, syrups, emulsions, suspensions and films (e.g., film disintegrable in the mouth); and parenteral agents such as injections (e.g., subcutaneous injections, intravenous injections, intramuscular injections, intraperitoneal injections and drip infusion), external agents (e.g., transdermal preparations and ointments), suppositories (e.g., rectal suppositories and vaginal suppositories), pellets, preparations for nasal administration, pulmonary preparations (inhalants) and eye drop. Any of these preparations can be safely administered orally or parenterally (e.g., locally, rectal and intravenous administrations).
In addition, these preparations may also be controlled-release preparations such as rapid-release preparations and sustained-release preparations (e.g., sustained-release microcapsules etc.).

The pharmaceutical composition of the present invention can be produced by a conventional method in the technical field of drug formulation, for example, the method described in the Japanese Pharmacopoeia and the like. Hereinafter, a method for preparing a medicament will be described in detail.
The content of the compound of the present invention in the pharmaceutical composition of the present invention varies among formulations, the dosages of the compound of the present invention and the like. For example, the content of the compound is about 0.01 to 100% by weight, preferably 0.1 to 95% by weight with respect to the total amount of the composition.

The dosage of the compound of the present invention varies among dosage subjects, routes of administration, subject diseases, symptoms and the like. For example, when the compound is orally administered to a schizophrenia patient (adult, about 60 kg in weight), a normal single dosage of about 0.1 to about 20 mg/kg weight, preferably about 0.2 to about 10 mg/kg weight, more preferably about 0.5 to about 10 mg/kg weight is preferably administered one or several times (e.g., three times) a day.

The compound of the present invention may be used in combination with any of other active components. Exemplary active components include: atypical antipsychotic agents (e.g., clozapine, olanzapine, risperidone, aripiprazole, iloperidone, asenapine, ziprasidone, quetiapine and zotepine);
typical antipsychotic agents (e.g., haloperidol and chlorpromazine);
selective serotonin reuptake inhibitors (e.g., paroxetine, sertraline, fluvoxamine and fluoxetine);
selective serotonin-noradrenaline reuptake inhibitors (e.g., milnacipran and venlafaxine);
selective noradrenaline-dopamine reuptake inhibitors (e.g., bupropion);
tetracyclic antidepressants (e.g., amoxapine and clomipramine);
tricyclic antidepressants (e.g., imipramine and amitriptyline);
other antidepressant agents (e.g., NS-2359, Lu AA21004 and DOV21947);
α7-nicotinic receptor partial modifiers (e.g., SSR-180711 and PNU-120596);
NK2 antagonists;
NK3 antagonists;
glycine transporter 1 inhibitors (e.g., ALX5407 and SSR504734);
metabolic glutamate receptor modifiers (e.g., CDPPB and MPEP);
antianxiety agents (benzodiazepine-based agents (e.g., diazepam and etizolam) and serotonin 5-HT_{1A}agonists (e.g., tandospirone));
sleep inducing agents (benzodiazepine-based agents (e.g., estazolam and triazolam), non-benzodiazepine-based agents (e.g., zolpidem) and melatonin receptor agonists (e.g., ramelteon));
β-amyloid vaccines;
β-amyloid degrading enzymes and the like;
brain function activators (e.g., aniracetam and nicergoline);
antiparkinson agents (e.g., dopamine receptor agonists (L-DOPA, bromocriptine, pergolide, talipexole, pramipexole, cabergoline and amantadine), mono-amine oxidase (MAO) inhibitors (e.g., deprenyl, selgiline (selegiline), remacemide and riluzole), anticholinergic agents (e.g., trihexyphenidyl and biperiden), and COMT inhibitors (e.g., entacapone));
therapeutic agents for amyotrophic lateral sclerosis (e.g., neurotrophic factors such as riluzole);
antihyperlipidemic drugs such as cholesterol-lowering drugs (statins (e.g., pravastatin sodium, atorvastatin, simvastatin and rosuvastatin), fibrates (e.g., clofibrate) and squalene synthase inhibitors);
therapeutic agents for abnormal behaviors, wandering symptoms associated with dementia (e.g., a sedative and anxiolytic);
apoptosis inhibitors (e.g., CPI-1189, IDN-6556 and CEP-1347);
neuronal differentiation/regeneration accelerator (e.g., leteprinim, xaliproden (SR-57746-A) and SB-216763);
antihypertensive agents;
therapeutic agents for diabetes mellitus;
nonsteroidal anti-inflammatory agents (e.g., meloxicam, tenoxicam, indomethacin, ibuprofen, celecoxib, rofecoxib, aspirin and indomethacin);
disease modifying antirheumatic drugs (DMARDs);
anticytokine agents (e.g., TNF inhibitors and MAP kinase inhibitors);
steroid agents (e.g., dexamethasone, hexestrol and cortisone acetate);
sex hormones or the derivatives thereof (e.g., progesterone, estradiol and estradiol benzoate);
parathyroid hormone (PTH); and
calcium receptor blockers (hereinafter, also simply referred to as combination drugs).
In particular, the compound of the present invention can be preferably used in combination with any of various central nervous system drugs and therapeutic agents for diseases easily developed with schizophrenia (e.g., therapeutic agents for diabetes mellitus).
In particular, the compound of the present invention can be preferably used in combination with any of various active components that do not act on GPR52.

The dosage forms of the compound of the present invention and the combination drugs thereof are not specifically limited. Any dosage form may be employed as long as the compound of the present invention is combined with any of the combination drugs. Exemplary dosage forms include:
(1) administration of a single medicament prepared by simultaneously formulating the compound of the present invention and the combination drug;
(2) administration of two different medicaments by the same administering route at same time, which are independently formulated from the compound of the present invention and the combination drug;
(3) administration of two different medicaments by the same administering route at different times, which are independently formulated from the compound of the present invention and the combination drug;
(4) administration of two different medicaments by different administering routes at same time, which are independently formulated from the compound of the present invention and the combination drug;
(5) administration of two different medicaments by different administering routes at different times, which are independently formulated from the compound of the present invention and the combination drug (e.g., the compound of the present invention and the combination drug are administered in this order and vice versa); and the like. Hereinafter, these dosage forms are collectively referred to as a combination agent of the present invention.

When the combination agent of the present invention is administered, both the combination drug and the compound of the present invention may simultaneously administered. Alternatively, after the administration of a combination drug, the compound of the present invention may be administered. Alternatively, the combination drug may be administered after the administration of the compound of the present invention. In the case of administration at different times, the time difference may vary among active ingredients, dosage forms and medication methods. For instance, there is a method in which, when the combination drug is administered first, the compound of the present invention is administered after one minute or more but not more than three days, preferably 10 minutes to one day, more preferably 15 minutes to one hour from the administration of the combination drug. For instance, there is another method in which, when the combination drug is administered after one minute or more but not more than one day, preferably 10 minutes to 6 hours, more preferably 15 minutes to one hour from the administration of the compound of the present invention.

The combination drug may be contained in any amount as long as a side effect does not pose a problem. The daily dose of the combination drug may vary depending on the target of administration, route of administration, target diseases, symptoms, and so on. For example, when orally administering to a schizophrenia patient (adult, about 60 kg in weight), it is desirable to administer the combination drug in general at a unit dose of abut 0.1 to about 20 mg/kg weight, preferably about 0.2 to about 10 mg/kg weight, more preferably about 0.5 to about 10 mg/kg weight. The unit dose of the combination drug may be preferably administered one to several times (e.g., three times) a day.
When the compound of the present invention is administered in combination with the combination drug, the amounts of the respective agents may be reduced within their safe ranges in consideration of their opposing effects.

The combination agent of the present invention is less toxic, so that it can be administered in safety in the form of a pharmaceutical composition prepared by mixing the compound of the present invention and/or the above combination drug with a pharmaceutically acceptable carrier according to a well-known method. Specifically, for example, it may be orally or parenterally (e.g., locally, rectal, or intravenous) administered in the form of a tablet (e.g., sugar-coated tablet or a film-coating tablet), powders, granules, capsules (inclusive of soft capsules), a liquid drug, an injection agent, a suppository agent, a sustained-release agent, or the like.

The pharmaceutically acceptable carrier to be used in the production of the combination agent of the present invention may be any of those used for the pharmaceutical composition of the present invention.

A blending ratio of the compound of the present invention to the combination drug in the combination agent of the present invention can be appropriately determined depending on the target of administration, the route of administration, diseases and the like.
Two or more of the combination drugs as described above may be combined together at an appropriate ratio.
The dosage of the combination drug can be appropriately determined on the basis of a clinically used dosage. A blending ratio of the compound of the present invention to the combination drug can be appropriately determined depending on the target of administration, the route of administration, target diseases, symptoms, combination and the like. For example, if the target of administration is a human, 0.01 to 100 parts by weight of the combination drug may be used for one part by weight of the compound of the present invention.

For instance, the content of the compound of the present invention in the combination agent of the present invention varies among the dosage forms. In general, however, the content of the compound of the present invention is in the range of about 0.01 to 99.9% by weight, preferably about 0.1 to 50% by weight, more preferably about 0.5 to 20% by weight with respect to the whole amount of the medicament.

The content of the combination drug in the combination agent of the present invention varies among the dosage forms. In general, however, the content of the combination drug is in the range of about 0.01 to 99.9% by weight, preferably about 0.1 to 50% by weight, more preferably about 0.5 to 20% by weight with respect to the whole amount of the medicament.

The content of any additive such as a carrier in the combination agent of the present invention varies among the dosage forms. In general, however, the content of the additive is in the range of about 1 to 99.99% by weight, preferably about 10 to 90% by weight with respect to the whole amount of the medicament.

The contents of the compound of the present invention and the combination drug may be equal to those described above even if they are independently formulated.

As described above, the dosage varies under various conditions, so that the contents of the compound of the present invention and the combination drug may be less than the above dosages or may be higher than the above dosages in some cases. Examples

The present invention will be illustrated in further detail by the following Reference Examples, Examples, Formulation Example and Experimental Example, but these examples, which are merely embodiments, do not limit the present invention and may be modified without departing from the scope of the invention.
In the following Reference Examples and Examples, "room temperature" ordinarily indicates a temperature from about 10°C to about 35°C. Percentages for yield indicate mol/mol% and percentages for media used in chromatography indicate percent by volume, but otherwise indicate percent by weight. Broad peaks such as OH and NH protons that could not be confirmed in the proton NMR spectra are not included in the data. Kiesselgel 60 by Merck was used in silica gel chromatography and Chromatorex NH by Fuji Silysia Chemical Ltd. was used in basic silica gel chromatography.

Other abbreviations used in this document are defined below.
s: singlet
d: doublet
dd: doublet of doublets
dt: doublet of triplets
t: triplet
tt: triplet of triplets
td: triplet of doublets
q: quartet
septet
m: multiplet
br: broad
J: coupling constant
Hz: Hertz
CDCl₃ : deuterated chloroform
DMSO-d₆ : deuterated dimethyl sulfoxide
¹H-NMR: proton nuclear magnetic resonance
HPLC: high performance liquid chromatography
THF: tetrahydrofuran
DMF: N,N-dimethylformamide
DMSO: dimethyl sulfoxide
NMP: N-methylpyrrolidone
HOBt: 1-hydroxybenzotriazole
WSC: 1-ethyl-3-(3-dimethylaminopropyl)carbodiimide hydrochloride
HATU: 2-(7-aza-1H-benzotriazol-1-yl)-1,1,3,3-tetramethyluronium hexafluorophosphate
DMTMM: 4-(4,6-dimethoxy-1,3,5-triazin-2-yl)-4-methylmorpholinium chloride n-hydrate
DBU: 1,8-diazabicyclo[5.4.0]-7-undecene
LC-MS: liquid chromatography-mass spectrometry
ESI: electrospray ionization

### Reference Example 1

### methyl 3-(1H-indol-6-yl)benzoate

A mixture of 6-bromo-1H-indole (1.00 g, 5.10 mmol), [3-(methoxycarbonyl)phenyl]boronic acid (1.10 g, 6.12 mmol) and tetrakis(triphenylphosphine)palladium(0) (295 mg, 0.255 mmol) in 2 N aqueous sodium carbonate solution (20 mL)-1,2-dimethoxyethane (30 mL) was reacted under a nitrogen atmosphere at 90°C for 5 hr. To the reaction mixture was added saturated brine and the mixture was extracted with ethyl acetate. The organic layer was washed with water, dried over anhydrous sodium sulfate, and concentrated under reduced pressure. The residue was purified by silica gel column chromatography to give the title compound (638 mg, yield 50%) as crystals.
¹H-NMR (CDCl₃) δ : 3.95 (3H, s), 6.58 (1H, t, J = 2.1 Hz), 7.25 (1H, t, J = 2.8 Hz), 7.41 (1H, dd, J = 8.3, 1.7 Hz), 7.49 (1H, t, J = 7.8 Hz), 7.62 (1H, s), 7.71 (1H, d, J = 8.3 Hz), 7.79 - 7.87 (1H, m), 7.95 - 8.02 (1H, m), 8.30 (1H, brs), 8.34 (1H, t, J = 1.8 Hz).

### Reference Example 2

### methyl 3-(2,3-dihydro-1 H-indol-6-yl)benzoate

To a solution of methyl 3-(1H-indol-6-yl)benzoate (620 mg, 2.47 mmol) obtained in Reference Example 1 in acetic acid (6 mL) was added sodium cyanotrihydroborate (310 mg, 4.94 mmol), and the mixture was stirred at room temperature for 15 hr. Water was added to the reaction mixture, aqueous sodium hydroxide solution was added under ice-cooling to adjust the pH to 12 and the mixture was extracted with ethyl acetate. The organic layer was dried over anhydrous sodium sulfate, and concentrated under reduced pressure. The residue was purified by silica gel column chromatography, and recrystallized from ethyl acetate-hexane to give the title compound (572 mg, yield 91%) as crystals. Melting point 104 - 105°C.
¹H-NMR (CDCl₃) δ : 3.08 (2H, t, J = 8.3 Hz), 3.62 (2H, t, J = 8.4 Hz), 3.93 (3H, s), 6.89 (1H, d, J = 1.3 Hz), 6.95 (1H, dd, J = 7.5, 1.7 Hz), 7.19 (1H, d, J = 7.5 Hz), 7.46 (1H, t, J = 7.7 Hz), 7.70 - 7.77 (1H, m), 7.95 - 8.00 (1H, m), 8.23 (1H, t, J = 1.7 Hz).

### Reference Example 3

### methyl 3-[1-(2,4-dichlorophenyl)-2,3-dihydro-1H-indol-6-yl]benzoate

To a solution of methyl 3-(2,3-dihydro-1H-indol-6-yl)benzoate (300 mg, 1.18 mmol) obtained in Reference Example 2, 1,3-dichloro-4-iodobenzene (193 µL, 1.42 mmol) and cesium carbonate (577 mg, 1.77 mmol) in toluene (3 mL) were added tris(dibenzylideneacetone)dipalladium(0) (25.6 mg, 0.028 mmol) and 2-dicyclohexylphosphino-2',4',6'-triisopropylbiphenyl (16.7 mg, 0.035 mmol), and the mixture was stirred with heating under a nitrogen atmosphere at 100°C for 40 hr. Water was added to the reaction mixture and the mixture was extracted with ethyl acetate. The organic layer was washed with water and saturated brine, dried over anhydrous sodium sulfate, and concentrated under reduced pressure. The residue was purified by silica gel column chromatography to give the title compound (350 mg, yield 74%) as an oil.
¹H-NMR (CDCl₃) δ : 3.22 (2H, t, J = 8.3 Hz), 3.86 - 4.02 (5H, m), 6.62 (1H, d, J = 1.3 Hz), 7.01 (1H, dd, J = 7.5, 1.5 Hz), 7.21 - 7.29 (2H, m), 7.37 - 7.47 (2H, m), 7.50 (1H, d, J = 2.4 Hz), 7.64 - 7.72 (1H, m), 7.91 - 7.99 (1H, m), 8.17 (1H, t, J = 1.6 Hz).

### Reference Example 4

### 3-[1-(2,4-dichlorophenyl)-2,3-dihydro-1H-indol-6-yl]benzoic acid

To a mixed solution of methyl 3-[1-(2,4-dichlorophenyl)-2,3-dihydro-1H-indol-6-yl]benzoate (350 mg, 0.88 mmol) obtained in Reference Example 3 in THF (3 mL)-methanol (1.5 mL) was added 2 N aqueous sodium hydroxide solution (0.88 mL, 1.76 mmol), and the mixture was stirred at room temperature for 6 hr. The reaction mixture was neutralized with 6 N hydrochloric acid, and diluted with water and the mixture was extracted with ethyl acetate. The organic layer was washed with water, dried over anhydrous magnesium sulfate, and concentrated under reduced pressure. The precipitated crystals were filtrated with diethyl ether to give the title compound (290 mg, yield 75%) as crystals. Melting point 234 - 235°C.
¹H-NMR (DMSO-d₆) δ : 3.16 (2H, t, J = 8.3 Hz), 3.93 (2H, t, J = 8.3 Hz), 6.49 (1H, d, J = 1.1 Hz), 7.03 (1H, dd, J = 7.6, 1.5 Hz), 7.29 (1H, d, J = 7.6 Hz), 7.45 - 7.49 (1H, m), 7.52 (1H, t, J = 7.8 Hz), 7.56 - 7.61 (1H, m), 7.72 - 7.79 (2H, m), 7.88 (1H, d, J = 8.0 Hz), 8.03 (1H, s), 13.04 (1H, s).

### Reference Example 5

### ethyl 3-(1H-indol-6-yl)benzoate

In the same manner as in Reference Example 1 and using 6-bromo-1H-indole and [3-(ethoxycarbonyl)phenyl]boronic acid, the title compound was obtained. Yield 39%, melting point 120 - 121°C (ethyl acetate-hexane).
¹H-NMR (CDC13) δ : 1.42 (3H, t, J = 7.2 Hz), 4.42 (2H, q, J = 7.2 Hz), 6.59 (1H, s), 7.23 - 7.28 (1H, m), 7.41 (1H, dd, J = 8.3, 1.5 Hz), 7.50 (1H, t, J = 7.8 Hz), 7.63 (1H, s), 7.72 (1H, d, J = 8.0 Hz), 7.83 (1H, d, J = 8.3 Hz), 7.99 (1H, d, J = 7.6 Hz), 8.28 (1H, brs), 8.34 (1H, s).

### Reference Example 6

### ethyl 3-(2,3-dihydro-1H-indol-6-yl)benzoate

In the same manner as in Reference Example 2 and using ethyl 3-(1H-indol-6-yl)benzoate obtained in Reference Example 5, the title compound was obtained as an oil. Yield 80%.
¹H-NMR (CDC13) δ : 1.40 (3H, t, J = 7.1 Hz), 3.06 (2H, t, J = 8.3 Hz), 3.60 (2H, t, J = 8.4 Hz), 3.83 (1H, brs), 4.39 (2H, q, J = 7.2 Hz), 6.87 (1H, d, J = 1.5 Hz), 6.95 (1H, dd, J = 7.5, 1.7 Hz), 7.18 (1H, d, J = 7.5 Hz), 7.45 (1H, t, J = 7.7 Hz), 7.66 - 7.77 (1H, m), 7.92 - 8.02 (1H, m), 8.23 (1H, t, J = 1.6 Hz).

### Reference Example 7

### ethyl 3-[1-(2,5-dichlorophenyl)-2,3-dihydro-1H-indol-6-yl]benzoate

In the same manner as in Reference Example 3 and using ethyl 3-(2,3-dihydro-1H-indol-6-yl)benzoate obtained in Reference Example 6 and 1,4-dichloro-2-iodobenzene, the title compound was obtained as an oil. Yield 76%.
¹H-NMR (CDCl₃) δ : 1.40 (3H, t, J = 7.2 Hz), 3.22 (2H, t, J = 8.5 Hz), 3.96 (2H, brs), 4.38 (2H, q, J = 7.2 Hz), 6.68 (1H, d, J = 1.5 Hz), 7.03 (1H, dd, J = 7.6, 1.5 Hz), 7.12 (1H, dd, J = 8.7, 2.3 Hz), 7.23 - 7.29 (1H, m), 7.39 - 7.49 (3H, m), 7.68 (1H, d, J = 7.6 Hz), 7.97 (1H, d, J = 7.6 Hz), 8.18 (1H, s).

### Reference Example 8

### 3-[1-(2,5-dichlorophenyl)-2,3-dihydro-1H-indol-6-yl]benzoic acid

In the same manner as in Reference Example 4 and using ethyl 3-[1-(2,5-dichlorophenyl)-2,3-dihydro-1H-indol-6-yl]benzoate obtained in Reference Example 7, the title compound was obtained. Yield 55%, melting point 219 - 220°C (ethyl acetate).
¹H-NMR (DMSO-d₆) δ : 3.16 (2H, t, J = 8.1 Hz), 3.98 (2H, t, J = 8.1 Hz), 6.49 (1H, d, J = 1.5 Hz), 7.05 (1H, dd, J = 7.6, 1.5 Hz), 7.27 - 7.36 (2H, m), 7.53 (1H, t, J = 7.8 Hz), 7.61 (1H, d, J = 2.3 Hz), 7.63 (1H, d, J = 8.7 Hz), 7.75 (1H, d, J = 8.3 Hz), 7.88 (1H, d, J = 7.6 Hz), 8.03 (1H, s), 13.04 (1H, brs).

### Reference Example 9

### ethyl 3-[1-(3,4-dichlorophenyl)-2,3-dihydro-1H-indol-6-yl]benzoate

In the same manner as in Reference Example 3 and using ethyl 3-(2,3-dihydro-1H-indol-6-yl)benzoate obtained in Reference Example 6 and 1,2-dichloro-4-iodobenzene, the title compound was obtained as an oil. Yield 30%.
¹H-NMR (CDCh) δ : 1.41 (3H, t, J = 7.0 Hz), 3.19 (2H, t, J = 8.1 Hz), 3.99 (2H, t, J = 8.5 Hz), 4.40 (2H, q, J=7.2Hz), 7.01-7.10 (1H, m), 7.15 (1H, dd, J=9.1, 2.7 Hz), 7.24 - 7.29 (2H, m), 7.31 (1H, d, J = 2.7 Hz), 7.38 (1H, d, J = 8.7 Hz), 7.49 (1H, t, J = 7.8 Hz), 7.71 (1H, d, J = 7.6 Hz), 8.01 (1H, d, J = 8.0 Hz), 8.22 (1H, s).

### Reference Example 10

### 3-[1-(3,4-dichlorophenyl)-2,3-dihydro-1H-indol-6-yl]benzoic acid

In the same manner as in Reference Example 4 and using ethyl 3-[1-(3,4-dichlorophenyl)-2,3-dihydro-1H-indol-6-yl]benzoate obtained in Reference Example 9, the title compound was obtained. Yield 49%, melting point 205 - 206°C (ethyl acetate).
¹H-NMR (DMSO-d₆) δ : 3.15 (2H, t, J = 8.3 Hz), 4.01 (2H, t, J = 8.4 Hz), 7.09 (1H, dd, J = 7.5, 1.3 Hz), 7.26 - 7.36 (2H, m), 7.37 - 7.46 (2H, m), 7.57 (2H, t, J = 7.7 Hz), 7.87 (1H, d, J = 8.3 Hz), 7.92 (1H, d, J = 7.7 Hz), 8.10 (1H, s), 13.07 (1H, brs).

### Reference Example 11

### ethyl 3-[1-(3,5-dichlorophenyl)-2,3-dihydro-1 H-indol-6-yl]benzoate

In the same manner as in Reference Example 3 and using ethyl 3-(2,3-dihydro-1H-indol-6-yl)benzoate obtained in Reference Example 6 and 1,3-dichloro-5-iodobenzene, the title compound was obtained as an oil. Yield 25%.
¹H-NMR (CDCh) δ : 1.41 (3H, t, J = 7.2 Hz), 3.19 (2H, t, J = 8.2 Hz), 4.00 (2H, t, J = 8.4 Hz), 4.41 (2H, q, J = 7.0 Hz), 6.94 (1H, t, J = 1.8 Hz), 7.07 (1H, dd, J = 7.5, 1.5 Hz), 7.14 (2H, d, J = 1.7 Hz), 7.24 - 7.29 (1H, m), 7.34 (1H, d, J = 1.3 Hz), 7.51 (1H, t, J = 7.7 Hz), 7.70 - 7.76 (1H, m), 8.03 (1H, d, J = 7.9 Hz), 8.23 (1H, t, J = 1.6 Hz).

### Reference Example 12

### 3-[1-(3,5-dichlorophenyl)-2,3-dihydro-1H-indol-6-yl]benzoic acid

In the same manner as in Reference Example 4 and using ethyl 3-[1-(3,5-dichlorophenyl)-2,3-dihydro-1H-indol-6-yl]benzoate obtained in Reference Example 11, the title compound was obtained. Yield 59%, melting point 210 - 211°C (ethyl acetate).
¹H-NMR (DMSO-d₆) δ : 3.15 (2H, t, J = 8.2 Hz), 4.03 (2H, t, J = 8.4 Hz), 7.07 - 7.15 (2H, m), 7.30 (2H, d, J = 1.9 Hz), 7.32 - 7.36 (2H, m), 7.58 (1H, t, J = 7.7 Hz), 7.83 - 7.89 (1H, m), 7.89 - 7.95 (1H, m), 8.12 (1H, t, J = 1.6 Hz), 13.07 (1H, brs).

### Reference Example 13

### ethyl 3-[1-(2,3-dichlorophenyl)-2,3-dihydro-1H-indol-6-yl]benzoate

In the same manner as in Reference Example 3 and using ethyl 3-(2,3-dihydro-1H-indol-6-yl)benzoate obtained in Reference Example 6 and 1,2-dichloro-3-iodobenzene, the title compound was obtained as an oil. Yield 71%.
¹H-NMR (CDCl₃)δ: 1.39 (3H, t, J = 7.0 Hz), 3.23 (2H, t, J = 8.3 Hz), 3.96 (2H, brs), 4.38 (2H, q, J = 6.9 Hz), 6.63 (1H, d, J = 1.5 Hz), 7.01 (1H, dd, J = 7.6, 1.5 Hz), 7.17 - 7.24 (1H, m), 7.24 - 7.28 (1H, m), 7.32 - 7.36 (1H, m), 7.39 (1H, dd, J = 8.0, 1.5 Hz), 7.43 (1H, t, J = 7.8 Hz), 7.67 (1H, d, J = 7.2 Hz), 7.96 (1H, d, J = 8.0 Hz), 8.17 (1H, t, J = 1.7 Hz).

### Reference Example 14

### 3-[-(2,3-dichlorophenyl)-2,3-dihydro-1H-indol-6-yl]benzoic acid

In the same manner as in Reference Example 4 and using ethyl 3-[1-(2,3-dichlorophenyl)-2,3-dihydro-1H-indol-6-yl]benzoate obtained in Reference Example 13, the title compound was obtained. Yield 84%, melting point 223 - 224°C (ethyl acetate).
¹H-NMR (DMSO-d₆) δ : 3.18 (2H, t, J = 8.3 Hz), 3.95 (2H, brs), 6.48 (1H, s), 7.04 (1H, dd, J = 7.6, 1.5 Hz), 7.30 (1H, d, J = 7.6 Hz), 7.37 - 7.46 (1H, m), 7.50 - 7.59 (3H, m), 7.75 (1H, d, J = 8.0 Hz), 7.87 (1H, d, J = 8.0 Hz), 8.02 (1H, s), 13.03 (1H, brs).

### Reference Example 15

### methyl 3-[1-(2,4-dichlorobenzyl)-2,3-dihydro-1H-indol-6-yl]benzoate

A mixed solution of methyl 3-(2,3-dihydro-1H-indol-6-yl)benzoate (270 mg, 1.07 mmol) obtained in Reference Example 2 and 2,4-dichlorobenzaldehyde (282 mg, 1.61 mmol) in THF (15 mL)-DMF (1.5 mL) was stirred at room temperature for 20 min. Sodium triacetoxyhydroborate (454 mg, 2.14 mmol) was added, and the mixture was stirred under a nitrogen atmosphere at room temperature for 20 hr. The reaction mixture was treated with 1 N aqueous sodium hydroxide solution and extracted with ethyl acetate. The organic layer was washed with saturated brine, dried over anhydrous sodium sulfate, and concentrated under reduced pressure. The residue was purified by silica gel column chromatography to give the title compound (360 mg, yield 82%) as an oil.
¹H-NMR (CDCl₃) δ : 3.08 (2H, t, J = 8.3 Hz), 3.48 (2H, t, J = 8.4 Hz), 3.93 (3H, s), 4.38 (2H, s), 6.62 (1H, d, J = 1.3 Hz), 6.94 (1H, dd, J = 7.5, 1.5 Hz), 7.19 (1H, d, J = 7.5 Hz), 7.23 (1H, dd, J = 8.3, 2.1 Hz), 7.38 - 7.43 (2H, m), 7.44 - 7.49 (1H, m), 7.68 - 7.74 (1H, m), 7.94 - 8.00 (1H, m), 8.21 (1H, t, J = 1.6 Hz).

### Reference Example 16

### 3-[1-(2,4-dichlorobenzyl)-2,3-dihydro-1H-indol-6-yl]benzoic acid

In the same manner as in Reference Example 4 and using methyl 3-[1-(2,4-dichlorobenzyl)-2,3-dihydro-1H-indol-6-yl]benzoate obtained in Reference Example 15, the title compound was obtained. Yield 50%, melting point 175 - 176°C (ethyl acetate).
¹H-NMR (DMSO-d₆) δ : 2.98 (2H, t, J = 8.3 Hz), 3.38 (2H, t, J = 8.4 Hz), 4.45 (2H, s), 6.85 (1H, d, J = 1.1 Hz), 6.91 (1H, dd, J = 7.5, 1.3 Hz), 7.17 (1H, d, J = 7.5 Hz), 7.41 - 7.46 (1H, m), 7.50 (1H, d, J = 8.1 Hz), 7.53 - 7.57 (1H, m), 7.66 (1H, d, J = 1.9 Hz), 7.80 - 7.85 (1H, m), 7.86 - 7.91 (1H, m), 8.11 (1H, t, J = 1.6 Hz), 13.02 (1H, s).

### Reference Example 17

### ethyl 3- [1-(3,4-dichlorobenzyl)-2,3-dihydro-1H-indol-6-yl]benzoate

In the same manner as in Reference Example 15 and using ethyl 3-(2,3-dihydro-1H-indol-6-yl)benzoate obtained in Reference Example 6 and 3,4-dichlorobenzaldehyde, the title compound was obtained as an oil. Yield 84%. ¹H-NMR (CDCl₃) δ: 1.40 (3H, t, J = 7.2 Hz), 3.04 (2H, t, J = 8.1 Hz), 3.37 (2H, t, J = 8.3 Hz), 4.27 (2H, s), 4.40 (2H, q, J = 6.9 Hz), 6.67 (1H, d, J = 1.1 Hz), 6.94 (1H, dd, J = 7.6, 1.5 Hz), 7.18 (1H, d, J = 7.6 Hz), 7.22 (1H, dd, J = 8.3, 2.3 Hz), 7.41 (1H, d, J = 8.3 Hz), 7.46 (1H, t, J = 7.8 Hz), 7.49 (1H, d, J = 2.3 Hz), 7.71 (1H, d, J = 7.2 Hz), 7.98 (1H, d, J = 7.6 Hz), 8.21 (1H, t, J = 1.7 Hz).

### Reference Example 18

### 3-[1-(3,4-dichlorobenzyl)-2,3-dihydro-1H-indol-6-yl]benzoic acid

In the same manner as in Reference Example 4 and using ethyl 3-[1-(3,4-dichlorobenzyl)-2,3-dihydro-1H-indol-6-yl]benzoate obtained in Reference Example 17, the title compound was obtained. Yield 88%, melting point 167 - 168°C (ethyl acetate).
¹H-NMR (DMSO-d₆) δ : 2.96 (2H, t, J = 8.3 Hz), 3.33 (2H, t, J = 8.3 Hz), 4.41 (2H, s), 6.88 - 6.95 (2H, m), 7.15 (1H, d, J = 8.0 Hz), 7.38 (1H, dd, J = 8.3, 1.9 Hz), 7.55 (1H, t, J = 7.8 Hz), 7.62 (1H, d, J = 8.3 Hz), 7.65 (1H, d, J = 1.9 Hz), 7.84 (1H, d, J = 8.0 Hz), 7.89 (1H, d, J = 7.6 Hz), 8.12 (1H, s), 13.04 (1H, brs).

### Reference Example 19

### ethyl 3-[1-(3,5-dichlorobenzyl)-2,3-dihydro-1H-indol-6-yl]benzoate

In the same manner as in Reference Example 15 and using ethyl 3-(2,3-dihydro-1H-indol-6-yl)benzoate obtained in Reference Example 6 and 3,5-dichlorobenzaldehyde, the title compound was obtained as an oil. Yield 82%. ¹H-NMR (CDCl₃) δ: 1.40 (3H, t, J = 7.0 Hz), 3.05 (2H, t, J = 8.1 Hz), 3.38 (2H, t, J = 8.1 Hz), 4.26 (2H, s), 4.39 (2H, q, J = 7.1 Hz), 6.65 (1H, d, J = 1.1 Hz), 6.95 (1H, dd, J = 7.2, 1.5 Hz), 7.18 (1H, d, J = 7.6 Hz), 7.23 - 7.30 (3H, m), 7.46 (1H, t, J = 7.8 Hz), 7.70 (1H, d, J = 7.6 Hz), 7.98 (1H, d, J = 7.6 Hz), 8.21 (1H, s).

### Reference Example 20

### 3-[1-(3,5-dichlorobenzyl)-2,3-dihydro-1H-indol-6-yl]benzoic acid

In the same manner as in Reference Example 4 and using ethyl 3-[1-(3,5-dichlorobenzyl)-2,3-dihydro-1H-indol-6-yl]benzoate obtained in Reference Example 19, the title compound was obtained. Yield 87%, melting point 207 - 208°C (ethyl acetate).
¹H-NMR (DMSO-d₆) δ : 2.97 (2H, t, J = 8.3 Hz), 3.27 - 3.40 (2H, m), 4.42 (2H, s), 6.92 (2H, d, J = 4.5 Hz), 7.16 (1H, d, J = 8.0 Hz), 7.44 (2H, d, J = 1.9 Hz), 7.49 - 7.59 (2H, m), 7.84 (1H, d, J = 8.3 Hz), 7.89 (1H, d, J = 8.0 Hz), 8.12 (1H, s), 13.04 (1H, brs).

### Reference Example 21

### ethyl 3-[1-(2,3-dichlorobenzyl)-2,3-dihydro-1H-indol-6-yl]benzoate

In the same manner as in Reference Example 15 and using ethyl 3-(2,3-dihydro-1H-indol-6-yl)benzoate obtained in Reference Example 6 and 2,3-dichlorobenzaldehyde, the title compound was obtained as an oil. Yield 87%. ¹H-NMR (CDCl₃) δ: 1.39 (3H, t, J = 7.2 Hz), 3.09 (2H, t, J = 8.1 Hz), 3.51 (2H, t, J = 8.3 Hz), 4.32 - 4.45 (4H, m), 6.60 (1H, s), 6.93 (1H, d, J = 7.6 Hz), 7.13 - 7.22 (2H, m), 7.39 (2H, dd, J = 7.6, 1.9 Hz), 7.44 (1H, t, J = 7.8 Hz), 7.69 (1H, d, J = 7.6 Hz), 7.97 (1H, d, J = 7.6 Hz), 8.20 (1H, s).

### Reference Example 22

### 3-[-(2,3-dichlorobenzyl)-2,3-dihydro-1H-indol-6-yl]benzoic acid

In the same manner as in Reference Example 4 and using ethyl 3-[1-(2,3-dichlorobenzyl)-2,3-dihydro-1H-indol-6-yl]benzoate obtained in Reference Example 21, the title compound was obtained. Yield 69%, melting point 185-186°C (ethyl acetate).
¹H-NMR (DMSO-d₆) δ : 3.00 (2H, t, J = 8.3 Hz), 3.41 (2H, t, J = 8.5 Hz), 4.51 (2H, s), 6.84 (1H, s), 6.91 (1H, dd, J = 7.4, 1.3 Hz), 7.17 (1H, d, J = 7.6 Hz), 7.34 - 7.41 (1H, m), 7.43 - 7.49 (1H, m), 7.53 (1H, t, J = 7.6 Hz), 7.59 (1H, dd, J = 7.8, 1.7 Hz), 7.83 (1H, d, J = 8.3 Hz), 7.88 (1H, d, J = 8.0 Hz), 8.10 (1H, s), 13.03 (1H, brs).

### Reference Example 23

### ethyl 3-[1-(2,5-dichlorobenzyl)-2,3-dihydro-1H-indol-6-yl]benzoate

To a solution of ethyl 3-(2,3-dihydro-1H-indol-6-yl)benzoate (500 mg, 1.81 mmol) obtained in Reference Example 6 in DMF (2 mL) was added potassium carbonate (250 mg, 1.81 mmol) under ice-cooling, and the mixture was stirred for 20 min. To the reaction mixture was added dropwise a solution of 2-(bromomethyl)-1,4-dichlorobenzene (521 mg, 2.17 mmol) in DMF (1 mL), and the mixture was stirred at room temperature for 3 hr. Water was added to the reaction mixture and the mixture was extracted with ethyl acetate. The organic layer was washed with water and saturated brine, dried over anhydrous sodium sulfate, and concentrated under reduced pressure. The residue was purified by silica gel column chromatography to give the title compound (650 mg, yield 84%) as an oil.
¹H-NMR (CDCl₃) δ : 1.40 (3H, t, J = 7.0 Hz), 3.10 (2H, t, J = 8.1 Hz), 3.50 (2H, t, J = 8.3 Hz), 4.32 - 4.44 (4H, m), 6.62 (1H, d, J = 1.5 Hz), 6.95 (H, dd, J = 7.2, 1.5 Hz), 7.16 - 7.23 (2H, m), 7.33 (1H, d, J = 8.3 Hz), 7.45 (1H, t, J = 7.8 Hz), 7.49 (1H, d, J = 2.3Hz),7.71 (1H,d,J=8.3Hz), 7.98(1H,d,J=7.6Hz),8.21 (1H,t,J=1.7Hz).

### Reference Example 24

### 3-[-(2,5-dichlorobenzyl)-2,3-dihydro-1H-indol-6-yl]benzoic acid

In the same manner as in Reference Example 4 and using ethyl 3-[1-(2,5-dichlorobenzyl)-2,3-dihydro-1H-indol-6-yl]benzoate obtained in Reference Example 23, the title compound was obtained. Yield 99%, melting point 198 - 199°C (ethyl acetate).
¹H-NMR (DMSO-d₆) δ : 3.00 (2H, t, J = 8.3 Hz), 3.40 (2H, t, J = 8.3 Hz), 4.45 (2H, s), 6.86 (1H, d, J = 1.1 Hz), 6.93 (1H, dd, J = 7.6, 1.5 Hz), 7.18 (1H, d, J = 7.6 Hz), 7.38-7.44 (1H, m), 7.51 - 7.57 (3H, m), 7.83 (1H, d, J = 8.3 Hz), 7.88 (1H, d, J = 7.6 Hz), 8.11 (1H, s), 13.03 (1H, brs).

### Reference Example 25

### ethyl 3-[1-(2,4-dichlorophenyl)-1H-indol-6-yl]benzoate

A suspension of ethyl 3-(1H-indol-6-yl)benzoate (1.00 g, 3.77 mmol) obtained in Reference Example 5, 1,3-dichloro-4-iodobenzene (1.02 mL, 7.54 mmol), copper powder (240 mg, 3.77 mmol) and potassium carbonate (1.04 g, 7.54 mmol) in NMP (8 mL) was stirred with heating under a nitrogen atmosphere at 150°C for 20 hr. Water was added to the reaction mixture and the mixture was extracted with ethyl acetate. The organic layer was washed with saturated brine, dried over anhydrous sodium sulfate, and concentrated under reduced pressure. The residue was purified by silica gel column chromatography to give the title compound (930 mg, yield 60%) as an oil.
¹H-NMR (CDCl₃) δ : 1.40 (3H, t, J = 7.1 Hz), 4.40 (2H, q, J = 7.0 Hz), 6.73 (1H, dd, J = 3.3, 0.8 Hz), 7.24 (1H, d, J = 3.4 Hz), 7.29 - 7.32 (1H, m), 7.40 - 7.43 (2H, m), 7.44 - 7.51 (2H, m), 7.64 (1H, dd, J = 1.8, 0.8 Hz), 7.73 - 7.81 (2H, m), 7.93 - 8.01 (1H, m), 8.27(1H, t, J = 1.6 Hz).

### Reference Example 26

### 3-[1-(2,4-dichlorophenyl)-1H-indol-6-yl]benzoic acid

In the same manner as in Reference Example 4 and using ethyl 3-[1-(2,4-dichlorophenyl)-1H-indol-6-yl]benzoate obtained in Reference Example 25, the title compound was obtained. Yield 70%.
¹H-NMR (DMSO-d₆) δ : 6.77 (1H, dd, J = 3.3, 0.7 Hz), 7.30 (1H, s), 7.47 (1H, dd, J = 8.3, 1.5 Hz), 7.51 - 7.59 (2H, m), 7.63 - 7.68 (1H, m), 7.68 - 7.72 (1H, m), 7.78 (1H, d, J = 8.3 Hz), 7.87 (1H, d, J = 1.5 Hz), 7.90 (1H, d, J = 1.7 Hz), 7.97 (1H, d, J = 2.3 Hz), 8.13 (1H, t, J = 1.6 Hz), 13.05 (1H, brs).

### Reference Example 27

### ethyl 3-[1-(2,4-dichlorobenzyl)-1H-indol-6-yl]benzoate

To a solution of ethyl 3-(1H-indol-6-yl)benzoate (500mg, 1.88 mmol) obtained in Reference Example 5 in DMF (4 mL) was added sodium hydride (97.6 mg, 2.44 mmol) under ice-cooling, and the mixture was stirred at room temperature for 30 min. To the reaction mixture was added dropwise a solution of 1-(bromomethyl)-2,4-dichlorobenzene (542 mg, 2.26 mmol) in DMF (1 mL), and the mixture was stirred at room temperature for 15 hr. The reaction mixture was poured into ice and the mixture was extracted with ethyl acetate. The organic layer was washed with water, dried over anhydrous sodium sulfate, and concentrated under reduced pressure. The residue was purified by silica gel column chromatography to give the title compound (677 mg, yield 85%) as an oil.
¹H-NMR (CDCl₃) δ: 1.41 (3H, t, J = 7.2 Hz), 4.40 (2H, q, J = 7.2 Hz), 5.43 (2H, s), 6.53 (1H, d, J = 8.3 Hz), 6.62 (1H, d, J = 3.4 Hz), 7.03 - 7.09 (1H, m), 7.15 (1H, d, J = 3.0 Hz), 7.39 - 7.53 (4H, m), 7.74 (1H, d, J = 8.7 Hz), 7.78 (1H, d, J = 8.3 Hz), 7.98 (1H, d, J = 8.0 Hz), 8.29 (1H, s).

### Reference Example 28

### 3-[1-(2,4-dichlorobenzyl)-1H-indol-6-yl]benzoic acid

In the same manner as in Reference Example 4 and using ethyl 3-[1-(2,4-dichlorobenzyl)-1H-indol-6-yl]benzoate obtained in Reference Example 27, the title compound was obtained an amorphous solid. Yield 88%.
¹H-NMR (CDCl₃) δ: 5.44 (2H, s), 6.54 (1H, d, J = 8.3 Hz), 6.63 (1H, d, J = 3.4 Hz), 7.08 (1H, dd, J = 8.3, 1.9 Hz), 7.16 (1H, d, J = 3.0 Hz), 7.39 - 7.47 (3H, m), 7.53 (1H, t, J = 7.8 Hz), 7.75 (1H, d, J = 9.1 Hz), 7.85 (1H, d, J = 8.0 Hz ), 8.06 (1H, d, J = 7.6 Hz), 8.36 (1H, s).

### Reference Example 29

### 6-chloro-1-[2-(3,4-dimethoxyphenyl)ethyl]-1H-pyrrolo[2,3-b]pyridine

To a solution of 6-chloro-1H-pyrrolo[2,3-b]pyridine (300 mg, 1.97 mmol) in N,N-dimethylformamide (3.0 ml) was added sodium hydride (86.5 mg, 2.16 mmol) at room temperature, and the mixture was stirred at room temperature for 15 min. A solution of 1-(2-bromoethyl)-3,4-dimethoxybenzene (723 mg, 2.95 mmol) in N,N-dimethylformamide (3.0 ml) was added dropwise at room temperature. The reaction solution was stirred overnight at 120°C, water was poured into the reaction mixture and the mixture was extracted with ethyl acetate. The extract was washed with water, and dried over anhydrous magnesium sulfate, and the solvent was evaporated under reduced pressure. The residue was purified by silica gel column chromatography (ethyl acetate:hexane = 1:5) to give the title compound (312 mg, yield 50%) as an oil.
¹H NMR (CDCl₃) δ: 3.08 (2H, t, J = 7.0 Hz), 3.75 (3H, s), 3.85 (3H, s), 4.48 (2H, t, J = 7.0 Hz), 6.34 (1H, d, J = 3.6 Hz), 6.48 (1H, d, J = 1.9 Hz), 6.61 - 6.68 (1H, m), 6.72 - 6.79 (1H, m), 6.90 (1H, d, J = 3.6 Hz), 7.06 (1H, d, J = 8.3 Hz), 7.81 (1H, d, J = 8.3 Hz).

### Reference Example 30

### ethyl 3-[1-[2-(3,4-dimethoxyphenyl)ethyl]-1H-pyrrolo[2,3-b]pyridin-6-yl]benzoate

A mixture of 6-chloro-1-[2-(3,4-dimethoxyphenyl)ethyl]-1H-pyrrolo[2,3-b]pyridine (600 mg, 1.89 mmol) obtained in Reference Example 29, [3-(ethoxycarbonyl)phenyl]boronic acid (441 mg, 2.27 mmol) and tetrakis(triphenylphosphine)palladium(O) (263 mg, 0.227 mmol) in 2 N aqueous sodium carbonate solution (3.8 ml)-1,2-dimethoxyethane (18 ml) was stirred overnight under a nitrogen atmosphere at 90°C. Water was poured into the reaction mixture and the mixture was extracted with ethyl acetate. The extract was washed with water, and dried over anhydrous magnesium sulfate, and the solvent was evaporated under reduced pressure. The residue was purified by silica gel column chromatography (ethyl acetate:hexane = 1:9) to give the title compound (606 mg, yield 74%) as an oil.
¹H-NMR (CDCl₃) δ: 1.44 (3H, t, J = 7.1 Hz), 3.17 (2H, t, J = 7.1 Hz), 3.71 (3H, s), 3.82 (3H, s), 4.44 (2H, q, J = 7.1 Hz), 4.59 (2H, t, J = 7.1 Hz), 6.39 (1H, d, J = 3.4 Hz), 6.47 - 6.52 (1H, m), 6.68 - 6.81 (2H, m), 7.02 (1H, d, J = 3.4 Hz), 7.55 (1H, t, J = 7.8 Hz), 7.61 (1H, d, J = 8.3 Hz), 7.96 (1H, d, J = 8.3 Hz), 8.02 - 8.08 (1H, m), 8.32 - 8.38 (1H, m), 8.77 (1H, t, J = 1.5 Hz).

### Reference Example 31

### 3-[1-[2-(3,4-dimethoxyphenyl)ethyl]-1H-pyrrolo[2,3-b]pyridin-6-yl]benzoic acid

To a mixture of ethyl 3-[1-[2-(3,4-dimethoxyphenyl)ethyl]-1H-pyrrolo[2,3-b]pyridin-6-yl]benzoate (600 mg, 1.39 mmol) obtained in Reference Example 30 in tetrahydrofuran (8 ml)-methanol (4 ml) was added 1 N aqueous sodium hydroxide solution (2.1 ml) at room temperature, and the mixture was stirred overnight. Water was poured into the reaction mixture, and the mixture was adjusted to pH 2-3 with 1 N aqueous hydrochloric acid solution, and extracted with ethyl acetate. The extract was washed with water, and dried over anhydrous magnesium sulfate, and the solvent was evaporated under reduced pressure. The residue was filtrated with diethyl ether to give the title compound (493 mg, yield 88%) as a solid.
¹H-NMR (DMSO-d₆) δ: 3.18 (2H, t, J = 7.0 Hz), 3.74 (3H, s), 3.83 (3H, s), 4.60 (2H, t, J = 7.0 Hz), 6.40 (1H, d, J = 3.4 Hz), 6.53 (1H, d, J = 1.9 Hz), 6.73 - 6.83 (2H, m), 7.05 (1H, d, J = 3.4 Hz), 7.56 - 7.65 (2H, m), 7.94 - 8.00 (1H, m), 8.10 - 8.17 (1H, m), 8.37 - 8.43 (1H, m), 8.89 (1H, t, J = 1.7 Hz).

### Reference Example 32

### ethyl 3-[1-(2,4-dichlorobenzyl)-1H-pyrrolo[2,3-b]pyridin-6-yl]benzoate

In the same manner as in Reference Example 29 and using 6-chloro-1H-pyrrolo[2,3-b]pyridine and 1-(bromomethyl)-2,4-dichlorobenzene, 6-chloro-1-(2,4-dichlorobenzyl)-1H-pyrrolo[2,3-b]pyridine was obtained as an oil. In the same manner as in Reference Example 30 and using 6-chloro-1-(2,4-dichlorobenzyl)-1H-pyrrolo[2,3-b]pyridine and [3-(ethoxycarbonyl)phenyl]boronic acid, the title compound was obtained as an oil. Yield 79%
¹H-NMR (CDCl₃) δ: 1.43 (3H, t, J = 7.2 Hz), 4.43 (2H, q, J = 7.2 Hz), 5.64 (2H, s), 6.52 (1H, d, J = 3.4 Hz), 7.06 - 7.18 (2H, m), 7.28 (1H, d, J = 3.4 Hz), 7.43 (1H, d, J = 1.9 Hz), 7.54 (1H, t, J = 7.8 Hz), 7.65 (1H, d, J = 7.8 Hz), 7.95 - 8.09 (2H, m), 8.27 - 8.36 (1H, m), 8.71 - 8.78 (1H, m).

### Reference Example 33

### 3-[1-(2,4-dichlorobenzyl)-1H-pyrrolo[2,3-b]pyridin-6-yl]benzoic acid

In the same manner as in Reference Example 31 and using ethyl 3-[1-(2,4-dichlorobenzyl)-1H-pyrrolo[2,3-b]pyridin-6-yl]benzoate obtained in Reference Example 32, the title compound was obtained as a solid. Yield 77%
¹H-NMR (DMSO-d₆) δ: 5.64 (2H, s), 6.60 (1H, d, J = 3.6 Hz), 7.10 (1H, d, J = 8.5 Hz), 7.38 (1H, dd, J = 8.5, 2.1 Hz), 7.61 (1H, t, J = 7.7 Hz), 7.66 - 7.71 (2H, m), 7.77 (1H, d, J = 8.3 Hz), 7.96 (1H, d, J = 7.7 Hz), 8.11 (1H, d, J = 8.3 Hz), 8.33 (1H, d, J = 7.7 Hz), 8.66 - 8.72 (1H, m).

### Reference Example 34

### ethyl 3-(1H-pyrrolo[2,3-b]pyridin-6-yl)benzoate

In the same manner as in Reference Example 30 and using methyl 6-chloro-1H-pyrrolo[2,3-b]pyridine-1-carboxylate and [3-(ethoxycarbonyl)phenyl]boronic acid, the title compound was obtained as an oil. Yield 64%
¹H-NMR (CDCl₃) δ: 1.40 (3H, t, J = 7.2 Hz), 4.43 (2H, q, J = 7.2 Hz), 6.51 (1H, dd, J = 3.5, 2.0 Hz), 7.23 (1H, dd, J = 3.5, 2.4 Hz), 7.55 - 7.64 (2H, m), 8.05 (1H, d, J = 8.1 Hz), 8.09 - 8.16 (1H, m), 8.21 - 8.28 (1H, m), 8.65 - 8.72 (1H, m), 11.13 (1H, brs).

### Reference Example 35

### ethyl 3-(2,3-dihydro-1H-pyrrolo[2,3-b]pyridin-6-yl)benzoate

To a solution of ethyl 3-(1H-pyrrolo[2,3-b]pyridin-6-yl)benzoate (280 mg, 1.05 mmol) obtained in Reference Example 34 and concentrated hydrochloric acid (0.30 ml) in ethanol (10 ml) was added palladium carbon (100 mg) under a nitrogen atmosphere and the reaction solution was stirred under a hydrogen atmosphere at 3 atm and 60°C for 5 hr. The reaction solution was filtered, and the filtrate was concentrated. The residue was dissolved in water, and the mixture was neutralized with 1 N aqueous sodium hydroxide solution and extracted with ethyl acetate. The extract was washed with water, and dried over anhydrous magnesium sulfate, and the solvent was evaporated under reduced pressure to give the title compound (198 mg, yield 70%) as a solid.
¹H-NMR (CDCl₃) δ: 1.41 (3H, t, J = 7.1 Hz), 3.10 (2H, t, J = 8.3 Hz), 3.65 (2H, t, J = 8.3 Hz), 4.40 (2H, q, J = 7.1 Hz), 4.69 (1H, brs), 7.01 (1H, d, J = 7.3 Hz), 7.48 (1H, t, J = 7.7 Hz), 8.02 (1H, d, J = 7.7 Hz), 8.10 (1H, dd, J = 7.7, 1.0 Hz), 8.53 (1H, s).

### Reference Example 36

### ethyl 3-[1-(2,4-dichlorobenzyl)-2,3-dihydro-1H-pyrrolo[2,3-b]pyridin-6-yl]benzoate

To a solution of ethyl 3-(2,3-dihydro-1H-pyrrolo[2,3-b]pyridin-6-yl)benzoate (450 mg, 1.68 mmol) obtained in Reference Example 35 and 2,4-dichlorobenzaldehyde (323 mg, 1.84 mmol) in acetic acid (1.4 ml)-methanol (18 ml) was added sodium cyanotrihydroborate (632 mg, 10.1 mmol) by small portions at room temperature, and the mixture was stirred overnight. Water was poured into the reaction solution, and the mixture was neutralized with 8 N aqueous sodium hydroxide solution and extracted with ethyl acetate. The extract was washed with water, and dried over anhydrous magnesium sulfate, and the solvent was evaporated under reduced pressure. The residue was purified by silica gel column chromatography (ethyl acetate:hexane = 1:5) to give the title compound (445 mg, yield 62%) as an oil.
¹H-NMR (CDCl₃) δ: 1.41 (3H, t, J = 7.0 Hz), 3.03 (2H, q, J = 8.0 Hz), 3.53 (2H, t, J = 8.0 Hz), 4.40 (2H, q, J = 7.0 Hz), 4.76 (2H, s), 7.03 (1H, d, J = 7.6 Hz), 7.12 - 7.34 (2H, m), 7.41 (1H, d, J = 1.9 Hz), 7.44 - 7.53 (2H, m), 7.97 - 8.04 (1H, m), 8.16 - 8.25 (1H, m), 8.59 - 8.64 (1H, m).

### Reference Example 37

### 3-[1-(2,4-dichlorobenzyl)-2,3-dihydro-1H-pyrrolo[2,3-b]pyridin-6-yl]benzoic acid

In the same manner as in Reference Example 31 and using ethyl 3-[1-(2,4-dichlorobenzyl)-2,3-dihydro-1H-pyrrolo[2,3-b]pyridin-6-yl]benzoate obtained in Reference Example 36, the title compound was obtained as a solid. Yield 86% ¹H-NMR (DMSO-d₆) δ: 3.03 (2H, t, J = 8.2 Hz), 3.53 (2H, t, J = 8.2 Hz), 4.67 (2H, s), 7.14 (1H, d, J = 7.7 Hz), 7.37 - 7.47 (2H, m), 7.50 - 7.59 (2H, m), 7.62 - 7.68 (1H, m), 7.92 (1H, d, J = 7.7 Hz), 8.20 (1H, d, J = 7.7 Hz ), 8.56 (1H, s).

### Reference Example 38

### ethyl 3-[1-(2,4-dichlorophenyl)-1H-pyrrolo[2,3-b]pyridin-6-yl]benzoate

A mixture of ethyl 3-(1H-pyrrolo[2,3-b]pyridin-6-yl)benzoate (200 mg, 0.751 mmol) obtained in Reference Example 34, 1,3-dichloro-4-iodobenzene (153 µl, 1.12 mmol), potassium phosphate (351 mg, 1.65 mmol), copper iodide (7.2 mg, 0.038 mmol), N,N'-dimethylcyclohexane-1,2-diamine (23.7 µl, 0.150 mmol) and 1,4-dioxane (2.0 ml) was stirred in a microwave reactor (Initiator (trade name), Biotage AB) at 120°C for 30 min. Copper iodide (72 mg, 0.38 mmol) and N,N-dimethylcyclohexyldiamine (94.8 µl, 0.600 mmol) were further added and the mixture was stirred in a microwave reactor (Initiator (trade name), Biotage AB) at 120°C for 30 min. Water was poured into the reaction solution and the mixture was extracted with ethyl acetate. The extract was washed with water, and dried over anhydrous magnesium sulfate, and the solvent was evaporated under reduced pressure. The residue was purified by silica gel column chromatography (ethyl acetate:hexane = 1:9) to give the title compound (59.6 mg , yield 19%) as a solid.
¹H-NMR (CDCl₃) δ:1.39 - 1.45 (3H, m), 4.40 (2H, q, J = 6.9 Hz), 6.67 (1H, d, J = 3.8 Hz), 7.39 - 7.53 (3H, m), 7.57 - 7.64 (2H, m), 7.68 - 7.74 (1H, m), 7.99 - 8.07 (2H, m), 8.21 - 8.27 (1H, m), 8.64 - 8.69 (1H, m).

### Reference Example 39

### 3-[1-(2,4-dichlorophenyl)-1H-pyrrolo[2,3-b]pyridin-6-yl]benzoic acid

In the same manner as in Reference Example 31 and using ethyl 3-[1-(2,4-dichlorophenyl)-1H-pyrrolo[2,3-b]pyridin-6-yl]benzoate obtained in Reference Example 38, the title compound was obtained as a solid. Yield 76%
¹H-NMR (DMSO-d₆) δ: 6.78 (1H, d, J = 3.6 Hz), 7.58 (1H, t, J = 7.7 Hz), 7.63 - 7.70 (1H, m), 7.71 - 7.78 (2H, m), 7.84 (1H, d, J = 8.3 Hz), 7.90 - 7.99 (2H, m), 8.16 - 8.27 (2H, m), 8.53 (1H, s).

### Reference Example 40

### ethyl 3-[1-(2,4-dichlorophenyl)-2,3-dihydro-1H-pyrrolo[2,3-b]pyridin-6-yl]benzoate

A mixture of ethyl 3-(2,3-dihydro-1H-pyrrolo[2,3-b]pyridin-6-yl)benzoate (190 mg, 0.708 mmol) obtained in Reference Example 35, 1,3-dichloro-4-iodobenzene (144 µl, 1.06 mmol), sodium tert-butoxide (102 mg, 1.06 mmol), tris(dibenzylideneacetone)dipalladium(O) (13.0 mg, 0.014 mmol), 2-(dicyclohexylphosphino)-2',4',6'-triisopropyl-1,1'-biphenyl (20.3 mg, 0.042 mmol) and toluene (3.8 ml) was stirred at 100°C for 1 day. Water was poured into the reaction solution and the mixture was extracted with ethyl acetate. The extract was washed with water, and dried over anhydrous magnesium sulfate, and the solvent was evaporated under reduced pressure. The residue was purified by silica gel column chromatography (ethyl acetate:hexane = 1:9) to give the title compound (72.8 mg, yield 25%) as a solid.
¹H-NMR (CDCl₃) δ: 1.41 (3H, t, J = 7.2 Hz), 3.17 - 3.25 (2H, m), 4.05 (2H, t, J = 8.4 Hz), 4.38 (2H, q, J = 7.2 Hz), 7.13 - 7.19 (1H, m), 7.30 (1H, dd, J = 8.7, 2.4 Hz), 7.40-7.51 (3H, m), 7.57 (1H, d, J = 8.7 Hz), 7.95 - 8.01 (1H, m), 8.06 - 8.13 (1H, m), 8.53 (1H, t, J = 1.6 Hz).

### Reference Example 41

### 3-[1-(2,4-dichlorophenyl)-2,3-dihydro-1H-pyrrolo[2,3-b]pyridin-6-yl]benzoic acid

In the same manner as in Reference Example 31 and using ethyl 3-[1-(2,4-dichlorophenyl)-2,3-dihydro-1H-pyrrolo[2,3-b]pyridin-6-yl]benzoate obtained in Reference Example 40, the title compound was obtained as a solid. Yield 77% ¹H-NMR (DMSO-d₆) δ: 3.20 (2H, t, J = 8.3 Hz), 4.01 (2H, t, J = 8.3 Hz), 7.28 (1H, d, J = 7.5 Hz), 7.45 - 7.53 (2H, m), 7.53 - 7.60 (1H, m), 7.64 (1H, d, J = 8.7 Hz), 7.75 (1H, d, J = 2.4 Hz), 7.86 - 7.93 (1H, m), 8.05 - 8.12 (1H, m), 8.40 (1H, brs).

### Reference Example 42

### ethyl 3-[1-(3,5-dichloropyridin-2-yl)-1H-pyrrolo[2,3-b]pyridin-6-yl]benzoate

To a solution of ethyl 3-(1H-pyrrolo[2,3-b]pyridin-6-yl)benzoate (100 mg, 0.376 mmol) obtained in Reference Example 34 in N,N-dimethylformamide (1.0 ml) was added sodium hydride (16.5 mg, 0.413 mmol) at room temperature, and the mixture was stirred at room temperature for 10 min. A solution of 3,5-dichloro-2-fluoropyridine (93.5 mg, 0.563 mmol) in N,N-dimethylformamide (1.0 ml) was added dropwise at room temperature. The reaction solution was stirred overnight at 120°C, water was poured into the reaction mixture and the mixture was extracted with ethyl acetate. The extract was washed with water, and dried over anhydrous magnesium sulfate, and the solvent was evaporated under reduced pressure. The residue was purified by silica gel column chromatography (ethyl acetate:hexane =1:9) to give the title compound (127 mg, yield 82%) as a solid.
¹H-NMR (CDCl₃) δ: 1.43 (3H, t, J = 7.2 Hz), 4.41 (2H, q, J = 7.2 Hz), 6.73 (1H, d, J = 3.8 Hz), 7.47 - 7.57 (2H, m), 7.71 (1H, d, J = 8.3 Hz), 7.99 - 8.08 (3H, m), 8.22 - 8.30 (1H, m), 8.50 (1H, d, J = 2.3 Hz), 8.70 (1H, t, J = 1. 5 Hz).

### Reference Example 43

### 3-[1-(3,5-dichloropyridin-2-yl)-1H-pyrrolo[2,3-b]pyridin-6-yl]benzoic acid

In the same manner as in Reference Example 31 and using ethyl 3-[1-(3,5-dichloropyridin-2-yl)-1H-pyrrolo[2,3-b]pyridin-6-yl]benzoate obtained in Reference Example 42, the title compound was obtained as a solid. Yield 85%
¹H-NMR (DMSO-d₆) δ: 6.82 (1H, d, J = 3.8 Hz), 7.59 (1H, t, J = 7.8 Hz), 7.82 (1H, d, J = 3.8 Hz), 7.87 (1H, d, J = 7.8 Hz), 7.91 - 7.98 (1H, m), 8.21 (1H, d, J = 8.3 Hz), 8.23 - 8.29 (1H, m), 8.53 - 8.57 (1H, m), 8.65 (1H, d, J = 2.3 Hz), 8.74 (1H, d, J = 2.3 Hz).

### Reference Example 44

### ethyl 3-[1-(2,4-dichlorophenyl)-1H-pyrazolo[3,4-b]pyridin-6-yl]benzoate

A mixture of 2,6-dichloropyridine-3-carbaldehyde (800 mg, 4.55 mmol), 2,4-dichlorophenylhydrazine hydrochloride (1.07 g, 5.00 mmol) and ethanol (16 ml) was stirred at 90°C for 3 hr, and the reaction solution was concentrated. Diethyl ether was added to the residue and 2,6-dichloro-3-[(E)-[(2,4-dichlorophenyl)hydrazono]methyl]pyridine (1.49 g, yield 98%) was collected by filtration to give a solid. A mixture of the compound (1.49 g), sodium tert-butoxide (641 mg, 6.67 mmol), tris(dibenzylideneacetone)dipalladium(O) (81.5 mg, 0.089 mmol), 2-(dicyclohexylphosphino)-2',4',6'-triisopropyl-1,1'-biphenyl (127 mg, 0.267 mmol) and 1,4-dioxane (20 ml) was stirred in a microwave reactor (Initiator (trade name), Biotage AB) at 120°C for 30 min. Water was poured into the reaction solution and the mixture was extracted with ethyl acetate. The extract was washed with water, and dried over anhydrous magnesium sulfate, and the solvent was evaporated under reduced pressure. The residue was purified by silica gel column chromatography (ethyl acetate:hexane = 1:19) to give 6-chloro-1-(2,4-dichlorophenyl)-1H-pyrazolo[3,4-b]pyridine (339 mg, yield 26%) as a solid. To a solution of the compound (365 mg), [3-(ethoxycarbonyl)-phenyl]boronic acid (261 mg, 1.34 mmol) and 2 N aqueous sodium carbonate solution (2.4 ml) in 1,2-dimethoxyethane (12 ml) was added under a nitrogen atmosphere tetrakis(triphenylphosphine)palladium(O) (170 mg, 0.147 mmol) at room temperature, and the mixture was stirred overnight at 100°C. Water was poured into the reaction mixture and the mixture was extracted with ethyl acetate. The extract was washed with water, and dried over anhydrous magnesium sulfate, and the solvent was evaporated under reduced pressure. The residue was purified by silica gel column chromatography (ethyl acetate:hexane= 1:9) to give the title compound (321 mg, yield 64%) as an oil.
¹H-NMR (CDCl₃) δ: 1.43 (3H, t, J = 7.2 Hz), 4.36 - 4.46 (2H, m), 7.44 (1H, dd, J = 8.6, 2.4 Hz), 7.51 - 7.62 (2H, m), 7.66 (1H, d, J = 2.4 Hz), 7.78 (1H, d, J = 8.6 Hz), 8.10 (1H, dd, J = 7.7, 1.1 Hz), 8.17 - 8.34 (3H, m), 8.70 (1H, s).

### Reference Example 45

### 3-[1-(2,4-dichlorophenyl)-1H-pyrazolo[3,4-b]pyridin-6-yl]benzoic acid

In the same manner as in Reference Example 31 and using ethyl 3-[1-(2,4-dichlorophenyl)-1H-pyrazolo[3,4-b]pyridin-6-yl]benzoate obtained in Reference Example 44, the title compound was obtained as a solid. Yield 71 %
¹H-NMR (DMSO-d₆) δ: 7.60 - 7.74 (2H, m), 7.75 - 7.83 (1H, m), 7.96 - 8.07 (3H, m), 8.32 (1H, d, J = 8.0 Hz), 8.49 (1H, d, J = 8.7 Hz), 8.52 (1H, s), 8.58 - 8.63 (1H, m).

### Reference Example 46

### ethyl 3-[1-(2,4-dichlorophenyl)-1H-indazol-6-yl]benzoate

In the same manner as in Reference Example 44 and using 4-bromo-2-fluorobenzaldehyde and 2,4-dichlorophenylhydrazine hydrochloride, the title compound was obtained as an oil. Yield 8%
¹H-NMR (CDCl₃) δ: 1.41 (3H, t, J = 7.2 Hz), 4.41 (2H, q, J = 7.2 Hz), 7.38 - 7.56 (5H, m), 7.65 (1H, d, J = 2.1 Hz), 7.76 - 7.83 (1H, m), 7.89 (1H, d, J = 8.5 Hz), 8.04 (1H, d, J = 7.9 Hz), 8.24 - 8.32 (2H, m).

### Reference Example 47

### 3-[1-(2,4-dichlorophenyl)-1H-indazol-6-yl]benzoic acid

In the same manner as in Reference Example 31 and using ethyl 3-[1-(2,4-dichlorophenyl)-1H-indazol-6-yl]benzoate obtained in Reference Example 46, the title compound was obtained as a solid. Yield 60%
¹H-NMR (DMSO-d₆) δ: 7.52 - 7.64 (3H, m), 7.65 - 7.71 (1H, m), 7.71 - 7.77 (1H, m), 7.92 - 8.04 (4H, m), 8.18 - 8.23 (1H, m), 8.46 (1H, s).

### Reference Example 48

### 2-chloro-7-(2,4-dichlorobenzyl)-7H-pyrrolo[2,3-d]pyrimidine

In the same manner as in Reference Example 29 and using 2-chloro-7H-pyrrolo[2,3-d]pyrimidine and 1-(bromomethyl)-2,4-dichlorobenzene, the title compound was obtained as an oil. Yield 61%
¹H-NMR (CDCl₃) δ: 5.51 (2H, s), 6.60 (1H, d, J = 3.8 Hz), 7.02 (1H, d, J = 8.3 Hz), 7.18 (1H, d, J = 1.9 Hz), 7.21 (1H, d, J = 3.8 Hz), 7.45 (1H, d, J = 1.9 Hz), 8.83 (1H, s).

### Reference Example 49

### ethyl 3-[7-(2,4-dichlorobenzyl)-7H-pyrrolo[2,3-d]pyrimidin-2-yl]benzoate

In the same manner as in Reference Example 30 and using 2-chloro-7-(2,4-dichlorobenzyl)-7H-pyrrolo[2,3-d]pyrimidine obtained in Reference Example 48 and (3-(ethoxycarbonyl)phenyl)boronic acid, the title compound was obtained as an oil. Yield 82%
¹H-NMR (CDCl₃) δ: 1.44 (3H, t, J = 7.0 Hz), 4.44 (2H, q, J = 7.0 Hz), 5.63 (2H, s), 6.61 (1H, d, J = 3.4 Hz), 7.19 (2H, s), 7.24 - 7.29 (1H, m), 7.45 (1H, s), 7.57 (1H, t, J = 7.8 Hz), 8.10 - 8.16 (1H, m), 8.69 - 8.75 (1H, m), 9.07 (1H, s), 9.17 - 9.20 (1H, m).

### Reference Example 50

### 3-[7-(2,4-dichlorobenzyl)-7H-pyrrolo[2,3-d]pyrimidin-2-yl]benzoic acid

In the same manner as in Reference Example 31 and using ethyl 3-[7-(2,4-dichlorobenzyl)-7H-pyrrolo[2,3-d]pyrimidin-2-yl]benzoate obtained in Reference Example 49, the title compound was obtained as a solid. Yield 86%
¹H-NMR (DMSO-d₆) δ: 5.66 (2H, s), 6.75 (1H, d, J = 3.6 Hz), 7.17 (1H, d, J = 8.3 Hz), 7.41 (1H, dd, J = 8.3, 2.0 Hz), 7.64 (1H, t, J = 7.7 Hz), 7.70 (1H, d, J = 2.O Hz), 7.73 (1H, d, J = 3.6 Hz), 7.99 - 8.06 (1H, m), 8.63 - 8.70 (1H, m), 9.05 (1H, t, J = 2.0 Hz), 9.17 (1H, s).

### Reference Example 51

### ethyl 3-(2,3-dihydro- H-pyrrolo[3,2-c]pyridin-6-yl)benzoate

In the same manner as in Reference Example 30 and using 6-bromo-1H-pyrrolo[3,2-c]pyridine and [3-(ethoxycarbonyl)phenyl]boronic acid, the title compound was obtained as an oil. Yield 74%
¹H-NMR (CDCl₃) δ: 1.41 (3H, t, J = 7.2 Hz), 3.12 (2H, t, J = 8.4 Hz), 3.73 (2H, td, J = 8.6, 1.2 Hz), 4.29 (1H, brs), 4.40 (2H, q, J = 7.2 Hz), 6.95 (1H, brs), 7.51 (1H, t, J = 8.0 Hz), 8.05 (1H, ddd, J = 7.7, 1.2, 1.2 Hz), 8.14 (1H, ddd, J = 7.9, 1.6, 1.6 Hz), 8.24 (1H, brs), 8.51 (1H, dd, J = 1.6, 1.6 Hz).

### Reference Example 52

### ethyl 3-(1 H-pyrrolo[3,2-c]pyridin-6-yl)benzoate

A solution of ethyl 3-(2,3-dihydro-1H-pyrrolo[3,2-c]pyridin-6-yl)benzoate (2.82 g, 10.5 mmol) obtained in Reference Example 51 and manganese(IV) dioxide (4.57 g, 52.6 mmol) in methylene chloride (35 ml) was heated under reflux for 3 hr. Manganese(IV) dioxide (9.14 g, 105 mmol) was further added, and the mixture was heated under reflux for 27 hr. The reaction solution was cooled, and passed through a small amount of silica gel, and the solvent was evaporated under reduced pressure. The residue was purified by silica gel column chromatography (ethanol:methylene chloride = 1:2) to give the title compound (1.87 g, yield 67%) as a solid.
¹H-NMR (CDCl₃) δ: 1.43 (3H, t, J = 7.2 Hz), 4.42 (2H, q, J = 7.2 Hz), 6.68 - 6.70 (1H, m), 7.29 - 7.30 (1H, m), 7.55 (1H, t, J = 7.6 Hz), 7.80 (1H, brs), 8.05 (1H, ddd, J = 8.6, 1.2, 1.2 Hz), 8.29 (1H, ddd, J = 7.7, 1.6, 1.6 Hz), 8.60 (1H, brs), 8.65 (1H, dd, J = 1.8, 1.6 Hz), 9.05 (1H, brs).

### Reference Example 53

### ethyl 3-[1-(2,4-dichlorobenzyl)-1H-pyrrolo[3,2-c]pyridin-6-yl]benzoate

In the same manner as in Reference Example 29 and using ethyl 3-(1H-pyrrolo[3,2-c]pyridin-6-yl)benzoate obtained in Reference Example 52 and 1-(bromomethyl)-2,4-dichlorobenzene, the title compound was obtained as an oil.
Yield 83%
¹H-NMR (CDCl₃) δ: 1.42 (3H, t, J = 7.0 Hz), 4.42 (2H, q, J = 7.0 Hz), 5.44 (2H, s), 6.58 (1H, d, J = 8.3 Hz), 6.72 (1H, d, J = 3.4 Hz), 7.06 - 7.19 (2H, m), 7.43 - 7.58 (2H, m), 7.63 (1H, s), 8.00 - 8.07 (1H, m), 8.22 - 8.29 (1H, m), 8.59 - 8.64 (1H, m), 9.04 (1H, s).

### Reference Example 54

### 3-[1-(2,4-dichlorobenzyl)-1H-pyrrolo[3,2-c]pyridin-6-yl]benzoic acid

In the same manner as in Reference Example 31 and using ethyl 3-[1-(2,4-dichlorobenzyl)-1H-pyrrolo[3,2-c]pyridin-6-yl]benzoate obtained in Reference Example 53, the title compound was obtained as a solid. Yield 44%
¹H-NMR (DMSO-d₆) δ: 5.81 (2H, s), 6.85 (1H, d, J = 8.7 Hz), 7.09 (1H, d, J = 3.0 Hz), 7.39(1H, dd, J = 8.7, 1.9 Hz), 7.68 - 7.80 (2H, m), 7.88 (1H, d, J = 3.0 Hz), 8.11 (1H, d, J = 7.6 Hz), 8.28 (1H, d, J = 7.6 Hz), 8.53 - 8.69 (2H, m), 9.28 (1H, s).

### Reference Example 55

### 5-chloro-3-(2,4-dichlorobenzyl)-3H-imidazo[4,5-b]pyridine

To a solution of 2,6-dichloro-3-nitropyridine (3.0 g, 15.5 mmol) in ethanol (30 ml) were added 2,4-dichlorobenzylamine (3.0 g, 17.1 mmol) and potassium carbonate (2.36 g, 17.1 mmol), and the mixture was heated under reflux for 2 hr. The reaction mixture was added to water and the mixture was extracted with ethyl acetate. The organic layer was washed with water and saturated brine, dried over anhydrous sodium sulfate, and concentrated under reduced pressure. The residue was crystallized from methanol to give 6-chloro-N-(2,4-dichlorobenzyl)-3-nitropyridin-2-amine as a crude product (5.0 g). A mixture of the compound and iron (4.3 g, 77.5 mmol) in acetic acid (50 mL) was heated at 80°C for 3 hr. The solid was removed by filtration, and the filtrate was concentrated under reduced pressure. The obtained residue was diluted with ethyl acetate, and the solution was washed with water, saturated aqueous sodium hydrogen carbonate solution and saturated brine, dried over anhydrous sodium sulfate, and concentrated under reduced pressure to give 6-chloro-N²-(2,4-dichlorobenzyl)pyridine-2,3-diamine as a crude product (3.7 g). To the compound was added formic acid (20 mL), and the mixture was heated under reflux for 16 hr. The solvent was evaporated and the residue was diluted with water. The resultant product was extracted with ethyl acetate, and the organic layer was washed with water, saturated aqueous sodium hydrogen carbonate solution and saturated brine, dried over anhydrous sodium sulfate, and concentrated under reduced pressure. The residue was purified by silica gel column chromatography (hexane-ethyl acetate 10:0→A:6) to give the title compound (3.49 g, yield 72%). Melting point 149 - 150°C (ethyl acetate-hexane).
¹H-NMR (DMSO-d₆) δ: 5.77 (2H, s), 6.60 (1H, brs), 7.47 (1H, dd, J = 8.1, 2.1 Hz), 7.56 - 7.72 (3H, m), 8.02 (1H, d, J = 7.8 Hz), 8.12 (1H, d, J = 8.4 Hz), 8.30 - 8.37 (2H, m), 8.70 (1H, s), 9.28 (1H, s).

### Reference Example 56

### 3-[3-(2,4-dichlorobenzyl)-3H-imidazo[4,5-b]pyridin-5-yl]benzoic acid

A mixture of 5-chloro-3-(2,4-dichlorobenzyl)-3H-imidazo[4,5-b]pyridine (3.6 g, 11.5 mmol) obtained in Reference Example 55, [3-(ethoxycarbonyl)phenyl]boronic acid (2.67 g, 13.8 mmol) and tetrakis(triphenylphosphine)palladium(0) (664 mg, 0.58 mmol) in 2 N aqueous sodium carbonate solution (30 mL)-1,2-dimethoxyethane (30 mL) was reacted under a nitrogen atmosphere at 90°C for 16 hr. Water was added to the reaction mixture and the mixture was extracted with ethyl acetate. The organic layer was washed with saturated brine, dried over anhydrous sodium sulfate, and concentrated under reduced pressure. The residue was purified by silica gel column chromatography (ethyl acetate-hexane 2:3) to give ethyl 3-[3-(2,4-dichlorobenzyl)-3H-imidazo[4,5-b]pyridin-5-yl]benzoate (1.72 g). To a solution of the compound in ethanol (50 mL) was added 1 N aqueous sodium hydroxide solution (10 mL, 10 mmol) at room temperature, and the mixture was stirred at 60°C for 2 hr, and concentrated under reduced pressure. Water and hydrochloric acid were added to the reaction mixture to acidify the aqueous layer, and the mixture was extracted with ethyl acetate. The organic layer was washed with water and saturated brine, and dried over anhydrous sodium sulfate. The solvent was evaporated under reduced pressure and the obtained residue was crystallized from ethyl acetate-hexane to give the title compound (1.1 g, yield 24%). Melting point 258 - 260°C.
¹H-NMR (DMSO-d₆) δ: 5.77 (2H, s), 6.60 (1H, brs), 7.47 (1H, dd, J = 8.1, 2.1 Hz), 7.56 - 7.72 (3H, m), 8.02 (1H, d, J = 7.8 Hz), 8.12 (1H, d, J = 8.4 Hz), 8.30 - 8.37 (2H, m), 8.70 (1H, s), 9.28 (1H, s).

### Reference Example 57

### 6-chloro-N-[2-(3,4-dimethoxyphenyl)ethyl]-3-nitropyridin-2-amine

To a suspension of 2,6-dichloro-3-nitropyridine (2.00 g, 10.4 mmol) and potassium carbonate (1.44 g, 10.4 mmol) in ethanol (60 mL) was added dropwise 2-(3,4-dimethoxyphenyl)ethanamine (1.75 mL, 10.4 mmol) under ice-cooling, and the mixture was stirred at room temperature for 30 min. After stirring, the mixture was stirred at 40°C for 3 hr. The reaction mixture was filtered, and the obtained crystals were washed with water and ethanol, and dried to give the title compound (3.02 g, yield 86%) as crystals. Melting point 132 - 133°C.
¹H-NMR (CDCl₃) δ: 2.93 (2H, t, J = 6.9 Hz), 3.82 - 3.86 (2H, m), 3.87 (3H, s), 3.89 (3H, s), 6.61 (1H, d, J = 8.5 Hz), 6.79 (1H, d, J = 1.7 Hz), 6.81 (1H, d, J = 1.7 Hz), 6.82 (1H, s), 8.33 (1H, d, J = 8.5 Hz), 8.38 (1H, brs).

### Reference Example 58

### 6-chloro-N²-[2-(3,4-dimethoxyphenyl)ethyl]pyridine-2,3-diamine

To a solution of 6-chloro-N-[2-(3,4-dimethoxyphenyl)ethyl]-3-nitropyridin-2-amine (500 mg, 1.48 mmol) obtained in Reference Example 57 in acetic acid (7 mL) was added iron powder (331 mg, 5.92 mmol), and the mixture was stirred with heating at 65°C for 3 hr. The reaction mixture was poured into water, aqueous ammonia solution was added to adjust pH to 9, and the mixture was extracted with ethyl acetate. The organic layer was dried over anhydrous sodium sulfate, and concentrated under reduced pressure. The residue was purified by silica gel column chromatography to give the title compound (246 mg, yield 54%) as crystals. Melting point 90 - 91°C (ethyl acetate-hexane).
¹H-NMR (CDCl₃) δ: 2.88 (2H, t, J = 6.8 Hz), 3.02 (2H, brs), 3.63 - 3.72 (2H, m), 3.86 (3H, s), 3.87 (3H, s), 4.29 (1H, brs), 6.49 (1H, d, J = 7.6 Hz), 6.74 - 6.85 (4H, m)

### Reference Example 59

### 5-chloro-3-[2-(3,4-dimethoxyphenyl)ethyl]-3H-imidazo[4,5-b]pyridine

A solution of 6-chloro-N²-[2-(3,4-dimethoxyphenyl)ethyl]pyridine-2,3-diamine (240 mg, 0.78 mmol) obtained in Reference Example 58 in formic acid (1.5 mL) was refluxed for 2 hr. The reaction mixture was poured into aqueous sodium carbonate solution, and the mixture was extracted with ethyl acetate-THF (80/20) mixed solution. The organic layer was washed with saturated brine, dried over anhydrous sodium sulfate, and concentrated under reduced pressure. The residue was purified by silica gel column chromatography to give the title compound (226 mg, yield 91%) as crystals. Melting point 100 - 101°C (ethyl acetate-hexane).
¹H-NMR (CDCl₃) δ: 3.12 (2H, t, J = 6.8 Hz), 3.76 (3H, s), 3.84 (3H, s), 4.49 (2H, t, J = 6.8 Hz), 6.50 (1H, d, J = 1.9 Hz), 6.52 - 6.59 (1H, m), 6.75 (1H, d, J = 8.1 Hz), 7.25 (1H, d, J = 8.3 Hz), 7.66 (1H, s), 7.98 (1H, d, J = 8.3 Hz).

### Reference Example 60

### ethyl 3-[3-[2-(3,4-dimethoxyphenyl)ethyl]-3H-imidazo[4,5-b]pyridin-5-yl]benzoate

In the same manner as in Reference Example 1 and using 5-chloro-3-[2-(3,4-dimethoxyphenyl)ethyl]-3H-imidazo[4,5-b]pyridine obtained in Reference Example 59 and [3-(ethoxycarbonyl)phenyl]boronic acid, the title compound was obtained as an oil. Yield 86%.
¹H-NMR (CDCl₃) δ: 1.44 (3H, t, J = 7.1 Hz), 3.22 (2H, t, J = 6.8 Hz), 3.74 (3H, s), 3.83 (3H, s), 4.44 (2H, q, J = 7.2 Hz), 4.59 (2H, t, J = 6.8 Hz), 6.51 (1H, d, J = 2.1 Hz), 6.60 - 6.68 (1H, m), 6.78 (1H, d, J = 8.1 Hz), 7.58 (1H, t, J = 7.7 Hz), 7.74 (1H, s), 7.79 (1H, d, J = 8.5 Hz), 8.07 - 8.15 (2H, m), 8.30 - 8.36 (1H, m), 8.77 (1H, t, J = 1.6 Hz).

### Reference Example 61

### 3-[3-[2-(3,4-dimethoxyphenyl)ethyl]-3H-imidazo[4,5-b]pyridin-5-yl]benzoic acid

In the same manner as in Reference Example 4 and using ethyl 3-[3-[2-(3,4-dimethoxyphenyl)ethyl]-3H-imidazo[4,5-b]pyridin-5-yl]benzoate obtained in Reference Example 60, the title compound was obtained. Yield 80%, melting point 105 - 106°C (ethyl acetate).
¹H-NMR (DMSO-d₆) δ : 3.21 (2H, t, J = 7.0 Hz), 3.62 (3H, s), 3.66 (3H, s), 4.61 (2H, t, J = 6.9 Hz), 6.65 - 6.70 (1H, m), 6.71 (1H, d, J = 1.7 Hz), 6.81 (1H, d, J = 8.1Hz), 7.66 (1H, t, J = 7.7 Hz), 7.96 (1H, d, J = 8.3 Hz), 8.01 (1H, d, J = 7.7 Hz), 8.18 (1H, d, J = 8.3 Hz), 8.35 - 8.41 (1H, m), 8.47 (1H, brs), 8.75 (1H, t, J = 1.7 Hz), 13.14 (1H, brs).

### Reference Example 62

### 4-(2,4-dichlorophenyl)-6-methoxy-3,4-dihydro-2H-1,4-benzoxazine

To a solution of 6-methoxy-3,4-dihydro-2H-1,4-benzoxazine (6.0 g, 36.4 mmol) in toluene were added 2,4-dichloro-1-iodobenzene (12.0 g, 43.6 mmol), tris(dibenzylideneacetone)dipalladium (330 mg, 0.36 mmol) and 4,5-bis(diphenylphosphino)-9,9-dimethylxanthene (624 mg, 1.08 mmol) at room temperature, and the mixture was stirred under an argon stream for 15 min. To the reaction mixture was added cesium carbonate (18 g, 55.6 mmol) at room temperature, and the mixture was heated under an argon stream at 110°C for 16 hr. Water was added to the reaction mixture and the mixture was extracted with ethyl acetate. The organic layer was washed with water and saturated brine, dried over anhydrous sodium sulfate, filtered, and concentrated under reduced pressure. The obtained residue was purified by silica gel column chromatography (hexane-ethyl acetate 1:9) to give the title compound (3.76 g, yield 33%). Oil.
¹H-NMR (CDCl₃) δ: 3.50-3.70 (5H, m), 4.27 (2H, brs), 5.88 (1H, d, J = 2.7 Hz), 6.27 (1H, dd, J = 9.0, 2.7 Hz), 6.79 (1H, d, J = 9.0 Hz), 7.10 - 7.33 (2H, m), 7. 50 (1H, t, J = 1.5 Hz).

### Reference Example 63

### 4-(2,4-dichlorophenyl)-3,4-dihydro-2H-1,4-benzoxazin-6-yl trifluoromethanesulfonate

To a solution of 4-(2,4-dichlorophenyl)-6-methoxy-3,4-dihydro-2H-1,4-benzoxazine (3.76 g, 12.0 mmol) obtained in Reference Example 62 in methylene chloride (50 mL) was added dropwise under an argon atmosphere at 0°C boron tribromide (1.0 M methylene chloride solution, 15.0 mL, 15.0 mmol), and the mixture was stirred for 3 hr. The reaction mixture was added to saturated aqueous sodium hydrogen carbonate solution, the organic layer was separated, and the aqueous layer was extracted with ethyl acetate. All organic layers were washed with saturated brine, dried over anhydrous sodium sulfate, filtered and concentrated under reduced pressure to give 4-(2,4-dichlorophenyl)-3,4-dihydro-2H-1,4-benzoxazin-6-ol as a crude product (1.86 g). To a solution of the compound (1.86 g, 6.28 mmol) and 4-dimethylaminopyridine (1.5 g, 12.5 mmol) in pyridine (30 mL) was added trifluoromethanesulfonic anhydride (1.16 mL, 6.91 mmol) at 0°C, and the mixture was stirred at room temperature for 4 hr. Water was added to the reaction mixture, the organic layer was separated, and the aqueous layer was extracted with ethyl acetate. The organic layer was washed with 1 N hydrochloric acid, washed with saturated aqueous sodium hydrogen carbonate solution, dried over magnesium sulfate, filtered and concentrated under reduced pressure to give the title compound (1.32 g, yield 26%). Oil.
¹H-NMR (CDCl₃) δ: 3.64 (2H, brs), 4.34 (2H, brs), 6.15 (1H, d, J = 2.7 Hz), 6.58 (1H, dd, J = 8.7, 2.7 Hz), 6.86 (1H, d, J = 8.7 Hz), 7.20 - 7.34 (2H, m), 7.54 (1H, d, J = 2.1 Hz).

### Reference Example 64

### 3-[4-(2,4-dichlorophenyl)-3,4-dihydro-2H-1,4-benzoxazin-6-yl]benzoic acid

In the same manner as in Reference Example 56 and using 4-(2,4-dichlorophenyl)-3,4-dihydro-2H-1,4-benzoxazin-6-yl trifluoromethanesulfonate obtained in Reference Example 63, the title compound was obtained. Yield 34%, melting point 222 - 223°C (ethyl acetate-hexane).
¹H-NMR (CDCl₃) δ: 3.66 (2H, brs), 4.76 (2H, brs), 6.59 (1H, d, J = 1.5 Hz), 6.94 - 7.02 (2H, m), 7.24 - 7.33 (2H, m), 7.43 (1H, t, J = 7.8 Hz), 7.53 (1H, d, J = 1.5 Hz), 7.60 (1H, dd, J = 6.3, 1.8 Hz), 7.98 (1H, d, J = 7.5 Hz), 8.14 (1H, s), 1H unconfirmed.

### Reference Example 65

### 8-methoxy-2,3,4, 5-tetrahydro-1 H-1-benzazepine

To a suspension of lithium aluminum hydride (725 mg, 19.1 mmol) in THF (15 mL) was added dropwise a solution of 8-methoxy-1,3,4, 5-tetrahydro-2H-1-benzazepin-2-one (1.46 g, 7.63 mmol) in THF (20 mL) under ice-cooling, and the mixture was refluxed for 4 hr. The reaction mixture was treated by adding dropwise water (0.75 mL), 15% aqueous sodium hydroxide solution (0.75 mL) and water (2.25 mL). The precipitate was filtered and the obtained solid was washed with ethyl acetate. The filtrate was concentrated under reduced pressure, and the residue was purified by silica gel column chromatography to give the title compound (1.20 g, yield 89%) as an oil.
¹H-NMR (CDCl₃) δ: 1.55 - 1.69 (2H, m), 1.72 - 1.89 (2H, m), 2.64 - 2.78 (2H, m), 2.99 - 3.12 (2H, m), 3.75 (4H, s), 6.30 (1H, d, J = 2.6 Hz), 6.38 (1H, dd, J = 8.2, 2.5 Hz), 6.99 (1H, d, J = 8.1 Hz).

### Reference Example 66

### 1-(2,4-dichlorophenyl)-8-methoxy-2,3,4,5-tetrahydro-1H-1-benzazepine

To a solution of 8-methoxy-2,3,4,5-tetrahydro-1H-1-benzazepine (1.17 g, 6.60 mmol) obtained in Reference Example 65, 1,3-dichloro-4-iodobenzene (996 µL, 6.60 mmol) and sodium t-butoxide (1.59 g, 16.5 mmol) in toluene (80 mL) were added tris(dibenzylideneacetone)dipalladium(0) (604 mg, 0.66 mmol) and 2'-(dicyclohexylphosphino)-N,N-dimethylbiphenyl-2-amine (519 mg, 1.32 mmol), and the mixture was stirred with heating under a nitrogen atmosphere at 100°C for 13 hr. Ethyl acetate was added to the reaction mixture, and the mixture was filtered through celite, and the filtrate was concentrated under reduced pressure. The residue was purified by silica gel column chromatography to give the title compound (1.07 g, yield 50%) as an oil.
¹H-NMR (CDCl₃) δ: 1.64 - 1.74 (2H, m), 1.74 - 1.89 (2H, m), 2.85 - 2.93 (2H, m), 3.52 - 3.59 (2H, m), 3.63 (3H, s), 6.00 (1H, d, J = 2.7 Hz), 6.47 (1H, dd, J = 8.3, 2.7 Hz), 7.07 (1H, d, J = 8.3 Hz), 7.22 (2H, d, J = 3.0 Hz), 7.33 (1H, d, J = 1.9 Hz).

### Reference Example 67

### 1-(2,4-dichlorophenyl)-2,3,4,5-tetrahydro-1H-1-benzazepin-8-ol

To a solution of 1-(2,4-dichlorophenyl)-8-methoxy-2,3,4,5-tetrahydro-1H-1-benzazepine (1.05 g, 3.26 mmol) obtained in Reference Example 66 in dichloromethane (4 mL) was added dropwise 1 M boron tribromide dichloromethane solution (6.25 mL, 6.25 mmol) at -78°C, and the mixture was stirred at room temperature for 2 hr. The reaction mixture was poured into a mixture of ice and aqueous sodium hydrogen carbonate solution and the mixture was extracted with ethyl acetate. The organic layer was washed with water and saturated brine, dried over anhydrous magnesium sulfate, and concentrated under reduced pressure. The residue was purified by silica gel column chromatography to give the title compound (510 mg, yield 51 %) as an oil.
¹H-NMR (CDCl₃) δ: 1.65 - 1.73 (2H, m), 1.74 - 1.84 (2H, m), 2.83 - 2.96 (2H, m), 4.53 (1H, s), 5.92 (1H, d, J = 2.6 Hz), 6.39 (1H, dd, J = 8.1, 2.6 Hz), 7.01 (1H, d, J = 8.3 Hz), 7.17 - 7.28 (2H, m), 7.34 (1H, d, J = 2.1 Hz).

### Reference Example 68

### 1-(2,4-dichlorophenyl)-2,3,4,5-tetrahydro-1H-1-benzazepin-8-yl trifluoromethanesulfonate

To a solution of 1-(2,4-dichlorophenyl)-2,3,4,5-tetrahydro-1H-1-benzazepin-8-ol (300 mg, 0.97 mmol) obtained in Reference Example 67 in pyridine (1 mL) was added dropwise trifluoromethanesulfonic anhydride (180 µL, 1.07 mmol) under ice-cooling, and the mixture was stirred at room temperature for 2 hr. The reaction mixture was poured into a mixture of ice and 6 N hydrochloric acid, and the mixture was extracted with diethyl ether. The organic layer was washed with saturated aqueous sodium hydrogen carbonate solution, dried over anhydrous magnesium sulfate, and concentrated under reduced pressure to give the title compound (400 mg) as an oil. The obtained compound was used for the next reaction without purification.

### Reference Example 69

### ethyl 3-[1-(2,4-dichlorophenyl)-2,3,4,5-tetrahydro-1H-1-benzazepin-8-yl]benzoate

In the same manner as in Reference Example 1 and using 1-(2,4-dichlorophenyl)-2,3,4,5-tetrahydro-1H-1-benzazepin-8-yltrifluoromethanesulfonate obtained in Reference Example 68 and [3-(ethoxycarbonyl)phenyl]boronic acid, the title compound was obtained as an oil. Yield 50%.
¹H-NMR (CDCl₃) δ: 1.38 (3H, t, J = 7.2 Hz), 1.69 - 1.89 (4H, m), 2.95 - 3.06 (2H, m), 3.59 - 3.67 (2H, m), 4.37 (2H, q, J = 7.0 Hz), 6.68 (1H, d, J = 1.9 Hz), 7.16 - 7.21 (1H, m), 7.26 (3H, q, J = 2.5 Hz), 7.34 (1H, dd, J = 1.9, 0.9 Hz), 7.40 (1H, t, J = 7.7 Hz), 7.51 - 7.57 (1H, m), 7.90 - 7.98 (1H, m), 8.07 (1H, t, J = 1.6 Hz).

### Reference Example 70

### 3-[1-(2,4-dichlorophenyl)-2,3,4,5-tetrahydro-1H-1-benzazepin-8-yl]benzoic acid

In the same manner as in Reference Example 4 and using ethyl 3-[1-(2,4-dichlorophenyl)-2,3,4,5-tetrahydro-1H-1-benzazepin-8-yl]benzoate obtained in Reference Example 69, the title compound was obtained. Yield 81 %, melting point 131 - 132°C (ethyl acetate).
¹H-NMR (DMSO-d₆) δ: 1.72 (4H, brs), 2.94 (2H, brs), 3.60 (2H, brs), 6.59 (1H, d, J = 1.7 Hz), 7.22 - 7.27 (1H, m), 7.31 - 7.36 (1H, m), 7.44 - 7.54 (3H, m), 7.58 (1H, d, J = 8.7 Hz), 7.59 - 7.64 (1H, m), 7.85 (1H, d, J = 7.7 Hz), 7.89 (1H, s), 13.03 (1H, brs).

### Reference Example 71

### 1-(2,4-dichlorobenzyl)-8-methoxy-2,3,4,5-tetrahydro-1H-1-benzazepine

In the same manner as in Reference Example 15 and using 8-methoxy-2,3,4,5-tetrahydro-1H-1-benzazepine obtained in Reference Example 65 and 2,4-dichlorobenzaldehyde, the title compound was obtained as an oil. Yield 66%. ¹H-NMR (CDCl₃) δ: 1.58 - 1.71 (4H, m), 2.76 - 2.87 (2H, m), 2.94 - 3.02 (2H, m), 3.73 (3H, s), 4.38 (2H, s), 6.38 - 6.47 (2H, m), 7.01 (1H, d, J = 8.0 Hz), 7.19 (1H, dd, J = 8.3, 2.3 Hz), 7.37 (1H, d, J = 1.9 Hz), 7.41 (1H, d, J = 8.3 Hz).

### Reference Example 72

### 1-(2,4-dichlorobenzyl)-2,3,4,5-tetrahydro-1H-1-benzazepin-8-ol

In the same manner as in Reference Example 67 and using 1-(2,4-dichlorobenzyl)-8-methoxy-2,3,4,5-tetrahydro-1H-1-benzazepine obtained in Reference Example 71, the title compound was obtained as an oil. Yield 70%.
¹H-NMR (CDCl₃) δ: 1.56 - 1.72 (4H, m), 2.73 - 2.87 (2H, m), 2.94 - 3.02 (2H, m), 4.36 (2H, s), 4.76 (1H, s), 6.32 (1H, dd, J = 7.9, 2.4 Hz), 6.36 (1H, d, J = 2.4 Hz), 6.94 (1H, d, J = 7.9 Hz), 7.20 (1H, dd, J = 8.3, 2.1 Hz), 7.38 (1H, d, J = 2.1 Hz), 7.40 (1H, d, J = 8.3 Hz).

### Reference Example 73

### 1-(2,4-dichlorobenzyl)-2,3,4,5-tetrahydro-1H-1-benzazepin-8-yl trifluoromethanesulfonate

In the same manner as in Reference Example 68 and using 1-(2,4-dichlorobenzyl)-2,3,4,5-tetrahydro-1H-1-benzazepin-8-ol obtained in Reference Example 72, the title compound was obtained as an oil. The obtained compound was used for the next reaction without purification.

### Reference Example 74

### ethyl 3-[1-(2,4-dichlorobenzyl)-2,3,4,5-tetrahydro-1H-1-benzazepin-8-yl]benzoate

In the same manner as in Reference Example 1 and using 1-(2,4-dichlorobenzyl)-2,3,4,5-tetrahydro-1H-1-benzazepin-8-yl trifluoromethanesulfonate obtained in Reference Example 73 and [3-(ethoxycarbonyl)phenyl]boronic acid, the title compound was obtained as an oil. The obtained compound was used for the next reaction without purification.

### Reference Example 75

### 3-[1-(2,4-dichlorobenzyl)-2,3,4,5-tetrahydro-1H-1-benzazepin-8-yl]benzoic acid

In the same manner as in Reference Example 4 and using ethyl 3-[1-(2,4-dichlorobenzyl)-2,3,4,5-tetrahydro-1H-1-benzazepin-8-yl]benzoate obtained in Reference Example 74, the title compound was obtained. The obtained compound was used for the next reaction without purification.

### Reference Example 76

### 1-(2,5-dichlorophenyl)-6-methoxyindan-1-ol

To a solution of 1-bromo-2,5-dichlorobenzene (4.18 g, 18.5 mmol) in THF (25 mL) was added dropwise 1.6 M n-butyllithium hexane solution (13.8 mL, 22.1 mmol) at -78°C, and the mixture was stirred for 30 min. Then, a solution of 6-methoxyindan-1-one (2.00 g, 12.3 mmol) in THF (15 mL) was added dropwise, and the mixture was stirred for 1 hr. To the reaction mixture was added saturated aqueous ammonium chloride solution and the mixture was extracted with ethyl acetate. The organic layer was washed with saturated brine, dried over anhydrous sodium sulfate, and concentrated under reduced pressure to give the title compound (1.95 g, yield 51%) as an oil.
¹H-NMR (CDCl₃) δ: 2.29 - 2.42 (1H, m), 2.52 (1H, s), 2.79 - 3.00 (2H, m), 3.04 - 3.23 (1H, m), 3.73 (3H, s), 6.53 (1H, d, J = 2.4 Hz), 6.87 (1H, dd, J = 8.3, 2.4 Hz), 7.16 - 7.30 (3H, m), 7.70 (1H, d, J = 2.3 Hz).

### Reference Example 77

### 1-(2,5-dichlorophenyl)-6-methoxyindane

To a solution of 1-(2,5-dichlorophenyl)-6-methoxyindan-1-ol (1.95 g, 6.31 mmol) obtained in Reference Example 76 in trifluoroacetic acid (15 mL) was added triethylsilane (3.0 mL, 18.9 mmol), and the mixture was stirred at room temperature for 13 hr. The reaction mixture was concentrated under reduced pressure, saturated aqueous sodium hydrogen carbonate solution was added and the mixture was extracted with ethyl acetate. The organic layer was washed with water and saturated brine, dried over anhydrous sodium sulfate, and concentrated under reduced pressure. The residue was purified by silica gel column chromatography to give the title compound (1.56 g, yield 84%) as an oil.
¹H-NMR (CDCl₃) δ: 1.87 - 2.07 (1H, m), 2.58 - 2.72 (1H, m), 2.85 - 2.98 (2H, m), 3.74 (3H, s), 4.79 (1H, t, J = 7.8 Hz), 6.55 (1H, d, J = 1.9 Hz), 6.79 (1H, dd, J = 8.2, 2.0 Hz), 6.94 (1H, d, J = 2.4 Hz), 7.12 (1H, dd, J = 8.5, 2.4 Hz), 7.20 (1H, d, J = 8.3 Hz), 7.33 (1H, d, J = 8.5 Hz).

### Reference Example 78

### 3-(2,5-dichlorophenyl)indan-5-ol

In the same manner as in Reference Example 67 and using 1-(2,5-dichlorophenyl)-6-methoxyindane obtained in Reference Example 77, the title compound was obtained as an oil. Yield 98%.
¹H-NMR (CDCl₃) δ: 1.86 - 2.02 (1H, m), 2.55 - 2.73 (1H, m), 2.79 - 3.05 (2H, m), 4.77 (1H, t, J = 7.9 Hz), 4.87 (1H, s), 6.48 (1H, d, J = 1.3 Hz), 6.64 - 6.78 (1H, m), 6.95 (1H, d, J = 2.6 Hz), 7.07 - 7.19 (2H, m), 7.32 (1H, d, J = 8.5 Hz).

### Reference Example 79

### 3-(2,5-dichlorophenyl)-2,3-dihydro-1H-inden-5-yl trifluoromethanesulfonate

In the same manner as in Reference Example 68 and using 3-(2,5-dichlorophenyl)indan-5-ol obtained in Reference Example 78, the title compound was obtained as an oil. Yield 87%.
¹H-NMR (CDCl₃) δ: 1.91 - 2.12 (1H, m), 2.61 - 2.84 (1H, m), 2.88 - 3.15 (2H, m), 4.86 (1H, t, J = 8.2 Hz), 6.90 (2H, d, J = 2.4 Hz), 7.10 - 7.21 (2H, m), 7.36 (2H, d, J = 8.7 Hz).

### Reference Example 80

### methyl 3-[3-(2,5-dichlorophenyl)-2,3-dihydro-1H-inden-5-yl]benzoate

In the same manner as in Reference Example 1 and using 3-(2,5-dichlorophenyl)-2,3-dihydro-1H-inden-5-yl trifluoromethanesulfonate obtained in Reference Example 79 and [3-(methoxycarbonyl)phenyl]boronic acid, the title compound was obtained as an oil. Yield 81 %.
¹H-NMR (CDCl₃) δ: 1.92 - 2.08 (1H, m), 2.60 - 2.80 (1H, m), 2.92 - 3.13 (2H, m), 3.93 (3H, d, J = 0.9 Hz), 4.90 (1H, t, J = 7.8 Hz), 6.98 (1H, d, J = 2.4 Hz), 7.09 - 7.16 (1H, m), 7.25 (1H, s), 7.35 (1H, d, J = 8.5 Hz), 7.39 (1H, d, J = 7.7 Hz), 7.43 - 7.54 (2H, m), 7.72 (1H, dd, J = 7.8, 1.0 Hz), 7.97 (1H, dd, J = 7.7, 0.9 Hz), 8.21 (1H, d, J = 1.1 Hz).

### Reference Example 81

### 3-[3-(2,5-dichlorophenyl)-2,3-dihydro-1H-inden-5-yl]benzoic acid

In the same manner as in Reference Example 4 and using methyl 3-[3-(2,5-dichlorophenyl)-2,3-dihydro-1H-inden-5-yl]benzoate obtained in Reference Example 80, the title compound was obtained. Yield 72%, melting point 229 - 230°C (ethyl acetate).
¹H-NMR (DMSO-d₆) δ: 1.96 - 2.11 (1H, m), 2.56 - 2.71 (1H, m), 2.91 - 3.14 (2H, m), 4.85 (1H, t, J = 7.8 Hz), 7.00 (1H, d, J = 2.3 Hz), 7.26 (1H, s), 7.36 (1H, dd, J = 8.5, 2.5 Hz), 7.44 - 7.49 (1H, m), 7.51 - 7.61 (3H, m), 7.83 (1H, d, J = 8.3 Hz), 7.90 (1H, d, J = 7.6 Hz), 8.09 (1H, s), 13.05 (1H, s).

### Reference Example 82

### 1-(2,4-dichlorophenyl)-6-methoxyindan-1-ol

To a mixture of magnesium (3.89 g, 160 mmol) and THF (100 mL) was added dropwise a solution of 1-bromo-2,4-dichlorobenzene (4.18 g, 18.5 mmol) in THF (50 mL), and the mixture was stirred at room temperature for 2 hr to give a Grignard reagent. Then, a solution of 6-methoxyindan-1-one (10.0 g, 61.7 mmol) in THF (50 mL) was added dropwise thereto under ice-cooling, and the mixture was stirred at room temperature for 15 hr. The reaction mixture was poured into a mixture of ice and ammonium chloride, and the mixture was extracted with diethyl ether. The organic layer was washed with water, dried over anhydrous sodium sulfate, and concentrated under reduced pressure. The residue was purified by silica gel column chromatography to give the title compound (12.6 g, yield 66%) as an oil.
¹H-NMR (CDCl₃) δ: 2.29 - 2.42 (1H, m), 2.57 (1H, s), 2.88 (2H, q, J = 10.2 Hz), 3.01 - 3.20 (1H, m), 3.73 (3H, s), 6.55 (1H, d, J = 2.3 Hz), 6.87 (1H, dd, J = 8.3, 2.3 Hz), 7.17 - 7.25 (2H, m), 7.37 (1H, d, J = 2.3 Hz), 7.53 (1H, d, J = 8.7 Hz).

### Reference Example 83

### 1-(2,4-dichlorophenyl)-6-methoxyindane

In the same manner as in Reference Example 77 and using 1-(2,4-dichlorophenyl)-6-methoxyindan-1-ol obtained in Reference Example 82, the title compound was obtained as an oil. Yield 77%.
¹H-NMR (CDCl₃) δ: 1.84 - 2.01 (1H, m), 2.56 - 2.74 (1H, m), 2.81 - 3.02 (2H, m), 3.73 (3H, s), 4.78 (1H, t, J = 7.8 Hz), 6.53 (1H, d, J = 2.3 Hz), 6.78 (1H, dd, J = 8.3, 2.3 Hz), 6.89 (1H, d, J = 8.3 Hz), 7.12 (1H, dd, J = 8.5, 2.1 Hz), 7.19 (1H, d, J = 8.3 Hz), 7.42 (1H, d, J = 2.3Hz).

### Reference Example 84

### 3-(2,4-dichlorophenyl)indan-5-ol

A mixed solution of 1-(2,4-dichlorophenyl)-6-methoxyindane (11.4 g, 38.9 mmol) obtained in Reference Example 83 in acetic acid (70 mL)-48% hydrobromic acid (50 mL) was refluxed for 7 hr. The reaction mixture was concentrated under reduced pressure, saturated aqueous sodium hydrogen carbonate solution was added and the mixture was extracted with ethyl acetate. The organic layer was washed with water and saturated brine, dried over anhydrous sodium sulfate, and concentrated under reduced pressure. The residue was purified by silica gel column chromatography to give the title compound (7.80 g, yield 72%) as an oil.
¹H-NMR (CDCl₃) δ : 1.83 - 1.99 (1H, m), 2.51 - 2.73 (1H, m), 2.80 - 3.01 (2H, m), 4.76 (1H, t, J = 8.0 Hz), 5.09 (1H, s), 6.45 (1H, d, J = 2.3 Hz), 6.70 (1H, dd, J = 7.8, 2.1 Hz), 6.90 (1H, d, J = 8.3 Hz), 7.07 - 7.17 (2H, m), 7.41 (1H, d, J = 2.3 Hz).

### Reference Example 85

### 3-(2,4-dichlorophenyl)-2,3-dihydro-1H-inden-5-yl trifluoromethanesulfonate

In the same manner as in Reference Example 68 and using 3-(2,4-dichlorophenyl)indan-5-ol obtained in Reference Example 84, the title compound was obtained as an oil. Yield 89%.
¹H-NMR (CDCl₃) δ : 1.90 - 2.08 (1H, m), 2.63 - 2.82 (1H, m), 2.88 - 3.15 (2H, m), 4.85 (1H, t, J = 8.2 Hz), 6.84 - 6.92 (2H, m), 7.09 - 7.20 (2H, m), 7.35 (1H, d, J = 8.3 Hz), 7.45(1H, d, J = 2.3 Hz).

### Reference Example 86

### methyl 3-[3-(2,4-dichlorophenyl)-2,3-dihydro-1H-inden-5-yl]benzoate

In the same manner as in Reference Example 1 and using 3-(2,4-dichlorophenyl)-2,3-dihydro-1H-inden-5-yl trifluoromethanesulfonate obtained in Reference Example 85 and [3-(methoxycarbonyl)phenyl]boronic acid, the title compound was obtained as an oil. Yield 83%.
¹H-NMR (CDCl₃) δ : 1.40 (3H, t, J = 7.1 Hz), 1.90 - 2.03 (1H, m), 2.63 - 2.78 (1H, m), 2.93 - 3.14 (2H, m), 4.39 (2H, q, J = 7.0 Hz), 4.89 (1H, t, J = 7.9 Hz), 6.93 (1H, d, J = 8.3 Hz), 7.12 (1H, dd, J = 8.4, 2.2 Hz), 7.23 (1H, s), 7.36 - 7.41 (1H, m), 7.42 - 7.52 (3H, m), 7.66 - 7.74 (1H, m), 7.95 - 8.00 (1H, m), 8.20 (1H, t, J = 1.7 Hz).

### Reference Example 87

### 3-[3-(2,4-dichlorophenyl)-2,3-dihydro-1H-inden-5-yl]benzoic acid

In the same manner as in Reference Example 4 and using methyl 3-[3-(2,4-dichlorophenyl)-2,3-dihydro-1H-inden-5-yl]benzoate obtained in Reference Example 86, the title compound was obtained. Yield 71 %, melting point 157 - 158°C (ethyl acetate).
¹H-NMR (DMSO-d₆) δ : 1.87 - 2.10 (1H, m), 2.55 - 2.74 (1H, m), 2.94 - 3.09 (2H, m), 4.84 (1H, t, J = 7.8 Hz), 7.02 (1H, d, J = 8.3 Hz), 7.24 (1H, s), 7.35 (1H, dd, J = 8.3, 2.3 Hz), 7.42 - 7.49 (1H, m), 7.50 - 7.60 (2H, m), 7.67 (1H, d, J = 2.3 Hz), 7.82 (1H, d, J = 8.0 Hz), 7.89 (1H, d, J = 8.0 Hz), 8.08 (1H, s), 13.06 (1H, brs).

### Reference Example 88

### 2-(4-bromophenoxy)-1-(2,4-dichlorophenyl)ethanone

To a solution of 4-bromophenol (2.0 g, 11.6 mmol) in acetonitrile (50 mL) were added 2-bromo-1-(2,4-dichlorophenyl)ethanone (3.42 g, 11.6 mmol) and potassium carbonate (1.77 g, 12.8 mmol) at room temperature, and the mixture was heated under reflux for 3 hr. The reaction mixture was added to water and the mixture was extracted with ethyl acetate. The organic layer was washed with water and saturated brine, dried over anhydrous sodium sulfate, and concentrated under reduced pressure. The residue was purified by silica gel column chromatography (hexane-ethyl acetate 90:10→40:60) to give the title compound (3.1 g, yield 74%) as an oil.
¹H-NMR (CDCl₃) δ : 5.12 (2H, s), 6.75 (2H, d, J = 9.0 Hz), 7.31 - 7.38 (3H, m), 7.47 (1H, d, J = 1.8 Hz), 7.55(1H, d, J = 8.1 Hz).

### Reference Example 89

### 5-bromo-3-(2,4-dichlorophenyl)-1-benzofuran

To a solution of 2-(4-bromophenoxy)-1-(2,4-dichlorophenyl)ethanone (4.0 g, 11.1 mmol) obtained in Reference Example 88 in toluene (30 mL) was added methanesulfonic acid (3 mL) at room temperature, and the mixture was heated under reflux for 6 hr. The reaction mixture was added to water and the mixture was extracted with ethyl acetate. The organic layer was washed with water and saturated brine, dried over anhydrous sodium sulfate, and concentrated under reduced pressure. The residue was crystallized from methanol to give the title compound (2.3 g, yield 61%). Melting point 116 - 117°C.
¹H-NMR (CDCl₃) δ : 7.35 (1H, dd, J = 8.1, 2.1 Hz), 7.40 (1H, s), 7.42 - 7.45 (2H, m), 7.63 - 7.65 (1H, m), 7.81 (1H, s).

### Reference Example 90

### 3-[3-(2,4-dichlorophenyl)-1-benzofuran-5-yl]benzoic acid

In the same manner as in Reference Example 56 and using 5-bromo-3-(2,4-dichlorophenyl)-1-benzofuran obtained in Reference Example 89, the title compound was obtained. Yield 59%, melting point 271 - 272°C (ethyl acetate-hexane). ¹H-NMR (DMSO-d₆) δ : 7.54 - 7.61 (2H, m), 7.66 - 7.74 (3H, m), 7.80 (1H, d, J = 8.7 Hz), 7.83 (1H, d, J = 1.8 Hz), 7.92 (2H, d, J = 7.2 Hz), 8.16 (1H, s), 8.33 (1H, s), 13.1 (1H, brs).

### Reference Example 91

### (2,4-dichlorophenyl)(hydroxy)acetic acid

To a mixture of 2,4-dichlorobenzaldehyde (15 g, 85.7 mmol) and zinc iodide (2.49 g, 7.79 mmol) in methylene chloride (50 mL) was added trimethylsilyl cyanide (25 mL, 187 mmol), and the mixture was stirred for 3 hr. The mixture was diluted with water and the mixture was extracted with ethyl acetate. The extract was washed with water and dried over anhydrous sodium sulfate. The solvent was evaporated under reduced pressure to give (2,4-dichlorophenyl)[(trimethylsilyl)oxy]acetonitrile as a crude product (22 g) as an oil. To the compound was added concentrated hydrochloric acid (50 mL) and the mixture was heated under reflux for 2 hr. The mixture was diluted with water and the resultant product was extracted with ethyl acetate. The organic layer was washed with water and saturated brine, dried over anhydrous sodium sulfate, and concentrated under reduced pressure. The obtained residue was crystallized from hexane-diethyl ether to give the title compound (12.5 g, yield 66%).
¹H-NMR (CDCl₃) δ : 5.00 (2H, brs), 5.61 (1H, s), 7.24 - 7.30 (1H, m), 7.37 (1H, d, J = 8.1 Hz), 7.42 (1H, d, J = 1.8 Hz).

### Reference Example 92

### 5-bromo-3-(2,4-dichlorophenyl)-1-benzofuran-2 (3H)-one

A mixture of 4-bromophenol (5.16 g, 29.8 mmol) and (2,4-dichlorophenyl)(hydroxy)acetic acid (6.0 g, 27.1 mmol) synthesized in Reference Example 91 in sulfuric acid (21 mL) and acetic acid (9 mL) was stirred at 110°C for 3 hr. The mixture was added to ice water, and the resultant product was extracted with ethyl acetate. The extract was washed with water and saturated aqueous sodium hydrogen carbonate solution, and concentrated. The residue was purified by silica gel column chromatography (hexane-ethyl acetate 10:0→A:6) to give the title compound (2.8 g, yield 29%). Melting point 149 - 150°C (methanol).
¹H-NMR (CDCl₃) δ : 5.28 (1H, brs), 7.06 (2H, d, J= 8.7 Hz), 7.23 - 7.29 (2H, m), 7.45 - 7.50 (2H, m).

### Reference Example 93

### 5-bromo-3-(2,4-dichlorophenyl)-2,3-dihydro-1-benzofuran

To a solution of 5-bromo-3-(2,4-dichlorophenyl)-1-benzofuran-2(3H)-one (2.8 g, 7.82 mmol) synthesized in Reference Example 92 in THF (50 mL) was added lithium aluminum hydride (444 mg, 11.7 mmol) at 0°C, and the mixture was heated under reflux for 1 hr. Water was added to the reaction mixture, and the resultant product was extracted with ethyl acetate. The extract was washed with water, dried over magnesium sulfate, and concentrated under reduced pressure. The obtained residue was purified by silica gel column chromatography (hexane-ethyl acetate 9:1→3:7) to give 4-bromo-2-[1-(2,4-dichlorophenyl)-2-hydroxyethyl]phenol (1.28 g). To a solution of the compound (1.28 g, 3.54 mmol) and triphenylphosphine (1.02 g, 3.89 mmol) in THF (50 mL) was added diethyl azodicarboxylate (40% toluene solution; 1.85 g, 4.25 mmol) under ice-cooling, and the mixture was stirred at room temperature for 1 hr. The solvent was concentrated under reduced pressure, and the obtained residue was purified by silica gel column chromatography (hexane-ethyl acetate 10:0→0:10) to give the title compound (850 mg, yield 32%) as an oil. ¹H-NMR (CDCl₃) δ : 4.34 (1H, dd, J = 9.0, 6.3 Hz), 4.90 - 4.99 (1H, m), 5.10 (1H, dd, J = 9.6, 6.3 Hz), 6.76 (1H, d, J = 8.4 Hz), 6.88 - 7.00 (1H, m), 7.10-7.31 (3H, m), 7.42 (1H, d, J = 2.1 Hz).

### Reference Example 94

### 3-[3-(2,4-dichlorophenyl)-2,3-dihydro-1-benzofuran-5-yl]benzoic acid

In the same manner as in Reference Example 56 and using 5-bromo-3-(2,4-dichlorophenyl)-2,3-dihydro-1-benzofuran obtained in Reference Example 93, the title compound was obtained. Yield 63%, melting point 192 - 193°C (ethyl acetate-hexane).
¹H-NMR (CDCl₃) δ : 4.39 (1H, dd, J = 8.4, 6.0 Hz), 5.02 (1H, t, J = 9.0 Hz), 5.19 (1H, dd, J = 9.0, 6.98 (1H, d, J = 8.4 Hz), 7.04 (1H, d, J = 8.4 Hz), 7.18 (1H, dd, J = 8.4, 2.1 Hz), 7.35 (1H, s), 7.43 - 7.53 (3H, m), 7.74 (1H, d, J = 7.8 Hz), 8.02 (1H, d, J = 7.8 Hz), 8.24 (1H, d, J = 1.8 Hz), 1H unconfirmed.

### Reference Example 95

### ethyl 3-[1-[(4-methylphenyl)sulfonyl]-1H-indol-5-yl]benzoate

In the same manner as in Reference Example 1 and using 5-bromo-1-[(4-methylphenyl)sulfonyl]-1H-indole and [3-(ethoxycarbonyl)phenyl]boronic acid, the title compound was obtained as an oil. Yield 97%.
¹H-NMR (CDCl₃) δ : 1.41 (3H, t, J = 7.2 Hz), 2.34 (3H, s), 4.41 (2H, q, J = 7.1 Hz), 6.71 (1H, d, J = 3.6 Hz), 7.19 - 7.27 (2H, m), 7.49 (1H, t, J = 7.7 Hz), 7.56 (1H, dd, J = 8.7, 1.7 Hz), 7.60 (1H, d, J = 3.6 Hz), 7.75 (1H, s), 7.75 - 7.81 (3H, m), 7.98 - 8.03 (1H, m), 8.06 (1H, d, J = 8.7 Hz), 8.26 (1H, t, J = 1.6 Hz).

### Reference Example 96

### ethyl 3-[3-bromo-1-[(4-methylphenyl)sulfonyl]-1H-indol-5-yl]benzoate

To a solution of ethyl 3-[1-[(4-methylphenyl)sulfonyl]-1H-indol-5-yl]benzoate (5.82 g, 13.9 mmol) obtained in Reference Example 95 in DMF (10 mL) was added a solution of bromine (2.22 g, 13.9 mmol) in DMF (5 mL) and the mixture was stirred for several minutes. The reaction mixture was poured into a mixture of ethyl acetate and water, and the organic layer was washed with water, dried over anhydrous magnesium sulfate, and concentrated under reduced pressure. The residue was purified by silica gel column chromatography to give the title compound (3.44 g, yield 50%) as an amorphous solid.
¹H-NMR (CDCl₃) δ : 1.42 (3H, t, J = 7.2 Hz), 2.35 (3H, s), 4.42 (2H, q, J = 7.2 Hz), 7.25 (2H, d, J = 8.5 Hz), 7.51 (1H, t, J = 7.7 Hz), 7.63 (1H, dd, J = 8.7, 1.7 Hz), 7.66 (1H, s), 7.69 (1H, d, J = 1.9 Hz), 7.75 - 7.83 (3H, m), 8.03 (1H, d, J = 7.7 Hz), 8.06 (1H, d, J = 8.7 Hz), 8.28 (1H, t, J = 1.8 Hz).

### Reference Example 97

### ethyl 3-[3-(2,4-dichlorophenyl)-1-[(4-methylphenyl)sulibnyl]-1H-indol-5-yl]benzoate

In the same manner as in Reference Example 1 and using ethyl 3-[3-bromo-1-[(4-methylphenyl)sulfonyl]-1H-indol-5-yl]benzoate obtained in Reference Example 96 and (2,4-dichlorophenyl)boronic acid, the title compound was obtained as an amorphous solid. Yield 90%.
¹H-NMR (CDCl₃) δ : 1.40 (3H, t, J = 7.0 Hz), 2.37 (3H, s), 4.39 (2H, q, J = 7.2 Hz), 7.24 - 7.31 (2H, m), 7.32 - 7.38 (1H, m), 7.40 - 7.44 (1H, m), 7.47 (1H, t, J = 7.8 Hz), 7.57 (1H, d, J = 2.3 Hz), 7.58 - 7.64 (2H, m), 7.74 (1H, s), 7.77 (1H, s), 7.84 (2H, d, J = 8.3 Hz), 7.99 (1H, d, J = 8.0 Hz), 8.11 (1H, d, J = 9.5 Hz), 8.21 (1H, s).

### Reference Example 98

### 3-[3-(2,4-dichlorophenyl)-1H-indol-5-yl]benzoic acid

To a mixed solution of ethyl 3-[3-(2,4-dichlorophenyl)-1-[(4-methylphenyl)sulfonyl]-1H-indol-5-yl]benzoate (1.25 g, 2.21 mmol) obtained in Reference Example 97 in methanol (28 mL)-water (5 mL) was added potassium hydroxide (870 mg, 15.5 mmol) and the mixture was stirred at 80°C for 16 hr. The reaction mixture was concentrated under reduced pressure, diluted with water, adjusted to pH 2 to 3 with hydrochloric acid and the mixture was extracted with ethyl acetate. The organic layer was washed with water, dried over anhydrous magnesium sulfate, and concentrated under reduced pressure. The precipitated crystals were filtrated with diethyl ether to give the title compound (780 mg, yield 92%) as crystals. Melting point 303 - 304°C (ethyl acetate).
¹H-NMR (DMSO-d₆) δ : 7.48 - 7.54 (2H, m), 7.55 - 7.62 (2H, m), 7.66 (1H, d, J = 8.3 Hz), 7.68 (1H, d, J = 2.4 Hz), 7.70 (1H, d, J = 1.1 Hz), 7.75 (1H, d, J = 2.1 Hz), 7.83 - 7.95 (2H, m), 8.16 (1H, t, J = 1.6 Hz), 11.63 (1H, d, J = 2.1 Hz), 13.03 (1H, brs).

### Reference Example 99

### 3-oxo-2,3-dihydro-1H-inden-5-yl trifluoromethanesulfonate

In the same manner as in Reference Example 68 and using 6-hydroxyindan-1-one, the title compound was obtained as an oil. Yield quantitatively.
¹H-NMR (CDCl₃) δ : 2.74 - 2.85 (2H, m), 3.16 - 3.25 (2H, m), 7.46 - 7.52 (1H, m), 7.57 - 7.61 (1H, m), 7.63 (1H, d, J = 2.3 Hz).

### Reference Example 100

### ethyl 3-(3-oxo-2,3-dihydro-1H-inden-5-yl)benzoate

In the same manner as in Reference Example 1 and using 3-oxo-2,3-dihydro-1H-inden-5-yl trifluoromethanesulfonate obtained in Reference Example 99 and [3-(ethoxycarbonyl)phenyl]boronic acid, the title compound was obtained as an oil. Yield 97%.
¹H-NMR (CDCl₃) δ : 1.43 (3H, t, J = 7.2 Hz), 2.73 - 2.80 (2H, m), 3.16 - 3.24 (2H, m), 4.42 (2H, q, J = 6.9 Hz), 7.53 (1H, t, J = 7.8 Hz), 7.57 (1H, d, J = 8.0 Hz), 7.79 (1H, d, J = 8.3 Hz), 7.87 (1H, dd, J = 8.0, 1.9 Hz), 8.02 (1H, d, J = 1.5 Hz), 8.05 (1H, d, J = 8.0 Hz), 8.29 (1H, s).

### Reference Example 101

### ethyl 3-(3-hydroxy-2,3-dihydro-1H-inden-5-yl)benzoate

To a mixed solution of ethyl 3-(3-oxo-2,3-dihydro-1H-inden-5-yl)benzoate (3.68 g, 13.1 mmol) obtained in Reference Example 100 in THF (30 mL)-methanol (30 mL) was added sodium borohydride (991 mg, 26.2 mmol), and the mixture was stirred at room temperature for 3 hr. Water was added to the reaction mixture and the mixture was extracted with ethyl acetate. The organic layer was washed with saturated brine, dried over anhydrous sodium sulfate, and concentrated under reduced pressure. The residue was purified by silica gel column chromatography to give the title compound (3.00 g, yield 81%) as an oil.
¹H-NMR (CDCl₃) δ : 1.41 (3H, t, J = 7.2 Hz), 1.92 (1H, d, J = 6.8 Hz), 1.94 - 2.08 (1H, m), 2.49 - 2.64 (1H, m), 2.79 - 2.94 (1H, m), 3.04 - 3.17 (1H, m), 4.41 (2H, q, J = 7.2 Hz), 5.32 (1H, q, J = 6.1 Hz), 7.34 (1H, d, J = 8.0 Hz), 7.45 - 7.55 (2H, m), 7.67 (1H, s), 7.77 (1H, d, J = 7.2 Hz), 8.01 (1H, d, J = 7.6 Hz), 8.27 (1H, s).

### Reference Example 102

### ethyl 3-[3-(2,4-dichlorophenoxy)-2,3-dihydro-1H-inden-5-yl]benzoate

To a solution of ethyl 3-(3-hydroxy-2,3-dihydro-1H-inden-5-yl)benzoate (1.50 g, 5.31 mmol) obtained in Reference Example 101, 2,4-dichlorophenol (952 mg, 5.84 mmol) and triphenylphosphine (1.53 g, 5.84 mmol) in THF (25 mL) was added dropwise a 40% solution of diethyl azodicarboxylate in toluene (2.84 mL, 6.37 mmol) under ice-cooling, and the mixture was stirred at room temperature for 15 hr. The reaction mixture was concentrated under reduced pressure, diethyl ether and hexane were added and the mixture was filtered through celite. The filtrate was concentrated under reduced pressure, and the residue was purified by silica gel column chromatography (hexane-ethyl acetate 85:15 and NH, 80:20) to give the title compound (1.54 g, yield 68%) as an oil.
¹H-NMR (CDCl₃) δ : 1.41 (3H, t, J = 7.0 Hz), 2.22 - 2.38 (1H, m), 2.52 - 2.70 (1H, m), 2.87 - 3.05 (1H, m), 3.14 - 3.33 (1H, m), 4.40 (2H, q, J = 7.2 Hz), 5.76 (1H, dd, J = 6.4, 4.5 Hz), 7.05 (1H, d, J = 9.1 Hz), 7.21 (1H, dd, J = 8.7, 2.7 Hz), 7.35-7.42 (2 H, m), 7.49 (1H, t, J = 7.6 Hz), 7.58 (1H, dd, J = 8.0, 1.9 Hz), 7.65 (1H, s), 7.74 (1H, d, J = 8.3 Hz), 8.01 (1H, d, J = 8.0 Hz), 8.25 (1H, s).

### Reference Example 103

### 3-[3-(2,4-dichlorophenoxy)-2,3-dihydro-1H-inden-5-yl]benzoic acid

In the same manner as in Reference Example 4 and using ethyl 3-[3-(2,4-dichlorophenoxy)-2,3-dihydro-1H-inden-5-yl]benzoate obtained in Reference Example 102, the title compound was obtained. Yield 81 %, melting point 173 - 174°C (ethyl acetate).
¹H-NMR (DMSO-d₆) δ : 2.03 - 2.21 (1H, m), 2.54 - 2.74 (1H, m), 2.86 - 3.03 (1H, m), 3.05 - 3.20 (1H, m), 5.98 (1H, dd, J = 6.1, 3.8 Hz), 7.39 - 7.51 (3H, m), 7.55 - 7.63 (2H, m), 7.69 (1H, d, J = 8.0 Hz), 7.71 (1H, s), 7.88 (1H, d, J = 8.3 Hz), 7.93 (1H, d, J = 7.6 Hz), 8.16 (1H, s), 13.15 (1H, brs).

### Reference Example 104

### 2-chloro-5,6-dihydro-7H-cyclopenta[b]pyridin-7-one

To a solution of oxalyl chloride (4.20 mL, 44.2 mmol) in dichloromethane (127 mL) was added dropwise DMSO (6.28 mL, 88.0 mmol) at -78°C over 5 min, and the mixture was stirred for 3 hr. A solution of 2-chloro-6,7-dihydro-5H-cyclopenta[b]pyridin-7-ol (2.50 g, 14.7 mmol) in dichloromethane (20 mL) was added at -78°C, and the mixture was stirred for 3 hr. Triethylamine was added to the reaction mixture and the mixture was warmed to room temperature, diluted with dichloromethane (100 mL), washed with saturated brine, dried over anhydrous magnesium sulfate, and concentrated under reduced pressure. The residue was purified by silica gel column chromatography (hexane-ethyl acetate-methanol 6:2:1) to give the title compound (2.10 g, yield 85%) as crystals.
¹H-NMR (CDCl₃) δ : 2.79 - 2.82 (2H, m), 3.14-3.17 (2H, m), 7.49 (1H, d, J = 8.0 Hz), 7.86 (1H, d, 8.0 Hz).

### Reference Example 105

### ethyl 3-(7-oxo-6,7-dihydro-5H-cyclopenta[b]pyridin-2-yl)benzoate

A mixture of 2-chloro-5,6-dihydro-7H-cyclopenta[b]pyridin-7-one (2.83 g, 16.9 mmol) obtained in Reference Example 104, [3-(ethoxycarbonyl)phenyl]boronic acid (4.91 g, 25.3 mmol), sodium carbonate (3.58 g, 33.8 mmol) and tetrakis(triphenylphosphine)palladium(0) (1.74 g, 1.69 mmol) in water (10 mL)-toluene (40 mL)-ethanol (20 mL) was reacted under a nitrogen atmosphere at 90°C for 2 days. Water was added to the reaction mixture, and the mixture was extracted with dichloromethane. The organic layer was dried over anhydrous sodium sulfate, and concentrated under reduced pressure. The residue was purified by silica gel column chromatography (hexane-ethyl acetate 8:1) to give the title compound (1.10 g, yield 23%) as crystals.
¹H-NMR (CDCl₃) δ : 1.43 (3H, t, J = 7.2 Hz), 2.83 - 2.86 (2H, m), 3.21 - 3.24 (2H, m), 4.43 (2H, q, J = 7.2 Hz), 7.58 (1H, t, J = 7.6 Hz), 7.99 (2H, s), 8.13 (1H, ddd, J = 7.7, 1.6, 1.6 Hz), 8.40 (1H, ddd, J = 4.8, 2.0, 1.2 Hz), 8.64 (1H, dd, J = 1.4, 1.4 Hz).

### Reference Example 106

### ethyl 3-(7-hydroxy-6,7-dihydro-5H-cyclopenta[b]pyridin-2-yl)benzoate

In the same manner as in Reference Example 101 and using ethyl 3-(7-oxo-6,7-dihydro-5H-cyclopenta[b]pyridin-2-yl)benzoate obtained in Reference Example 105, the title compound was obtained. Yield 89%, melting point 46 - 47°C (ethyl acetate-hexane).
¹H-NMR (CDCl₃) δ : 1.43 (3H, t, J = 7.2 Hz), 1.99 - 2.18 (1H, m), 2.54 - 2.68 (1H, m), 2.82 - 2.94 (2H, m), 3.01 - 3.14 (1H, m), 4.43 (2H, q, J = 7.2 Hz), 5.20 - 5.32 (1H, m), 7.54 (1H, t, J = 7.7 Hz), 7.66 (2H, s), 8.05 - 8.11 (1H, m), 8.20 - 8.26 (1H, m), 8.62 (1H, t, J = 1.7 Hz).

### Reference Example 107

### ethyl 3-[7-(2,4-dichlorophenoxy)-6,7-dihydro-5H-cyclopenta[b]pyridin-2-yl]benzoate

In the same manner as in Reference Example 102 and using ethyl 3-(7-hydroxy-6,7-dihydro-5H-cyclopenta[b]pyridin-2-yl)benzoate obtained in Reference Example 106, the title compound was obtained. Yield 77%, melting point 129 - 130°C (ethyl acetate-hexane).
¹H-NMR (CDCl₃) δ : 1.42 (3H, t, J = 7.1 Hz), 2.42 - 2.54 (1H, m), 2.54 - 2.69 (1H, m), 2.88 - 3.04 (1H, m), 3.22 - 3.37 (1H, m), 4.43 (2H, q, J = 7.2 Hz), 5.71 (1H, dd, J = 6.7, 3.3 Hz), 7.25 - 7.31 (1H, m), 7.36 (1H, d, J = 2.4 Hz), 7.52 (1H, t, J = 7.7 Hz), 7.68 - 7.74(3H,m),8.04-8.11 (1H, m), 8.12 - 8.19 (1H, m), 8.61 (1H, t, J = 1.6 Hz).

### Reference Example 108

### 3-[7-(2,4-dichlorophenoxy)-6,7-dihydro-5H-cyclopenta[b]pyridin-2-yl]benzoic acid

In the same manner as in Reference Example 4 and using ethyl 3-[7-(2,4-dichlorophenoxy)-6,7-dihydro-5H-cyclopenta[b]pyridin-2-yl]benzoate obtained in Reference Example 107, the title compound was obtained. Yield 91 %, melting point 198 - 204°C (ethyl acetate).
¹H-NMR (DMSO-d₆) δ : 2.12 - 2.28 (1H, m), 2.55 - 2.74 (1H, m), 2.89 - 3.03 (1H, m), 3.07 - 3.21 (1H, m), 5.92 (1H, dd, J = 6.8, 3.0 Hz), 7.46 (1H, dd, J = 8.7, 2.7 Hz), 7.52 - 7.61 (2H, m), 7.76 (1H, d, J = 8.7 Hz), 7.86 - 7.92 (1H, m), 7.93 - 8.02 (2H, m), 8.18 (1H, d, J = 8.0 Hz), 8.64 (1H, s), 13.28 (1H, brs).

### Reference Example 109

### 5-bromo-N-(2,4-dichlorophenyl)-2,3-dihydro-1-benzofuran-3-amine

A solution of 5-bromosalicylaldehyde (5.0 g, 24.9 mmol) and 2,4-dichloroaniline (4.0 g, 24.9 mmol) in ethanol (20 mL) was heated under reflux for 1 hr. The reaction mixture was cooled and the resulting crystals were collected by filtration to give 4-bromo-2-[(2,4-dichlorophenyl)imino]methylphenol as crude crystals (6.1 g). Under a nitrogen atmosphere, sodium hydride (60% dispersion in liquid paraffin, 1.16 g, 29.0 mmol) was added to a solution of trimethylsulfoxonium iodide (6.38 g, 29.0 mmol) in DMSO (20 mL) at 0°C, and the mixture was stirred at room temperature for 10 min. To the mixture was added dropwise a solution of the above-mentioned compound (4.0 g, 11.6 mmol) in DMSO (10 mL) and the mixture was stirred at room temperature for 3 hr. Water was added to the reaction mixture, and the resultant product was extracted with ethyl acetate. The extract was washed with water, dried over magnesium sulfate, and concentrated under reduced pressure. The obtained residue was purified by silica gel column chromatography (hexane-ethyl acetate 20:80→80:20) to give the title compound (1.5 g, yield 17%). Melting point 112 - 113°C (ethyl acetate-hexane).
¹H-NMR (CDCl₃) δ : 4.38 (1H, dd, J = 9.6, 4.5 Hz), 4.55 (1H, d, J = 7.5 Hz), 4.75 (1H, dd, J = 9.6, 7.5 Hz), 5.15 - 5.25 (1H, m), 6.59 (1H, d, J = 8.4 Hz), 6.79 (1H, d, J = 8.7 Hz), 7.14 (1H, dd, J = 8.4, 2.1 Hz), 7.30 (1H, d, J = 2.1 Hz), 7.37 (1H, dd, J = 8.4, 2.1 Hz), 7.46 (1H, d, J = 1.8 Hz).

### Reference Example 110

### 3-[3-[(2,4-dichlorophenyl)amino]-2,3-dihydro-1-benzofuran-5-yl]benzoic acid

In the same manner as in Reference Example 56 and using 5-bromo-N-(2,4-dichlorophenyl)-2,3-dihydro-1-benzofuran-3-amine obtained in Reference Example 109, the title compound was obtained. Yield 60%, melting point 164 - 165°C (ethyl acetate-hexane).
¹H-NMR (CDCl₃) δ : 4.38 (1H, dd, J = 6.6, 5.1 Hz), 4.87 (1H, t, J = 9.0 Hz), 5.44 - 5.55 (1H, m), 5.75 (1H, d, J = 7.5 Hz), 6.98 (2H, d, J = 8.4 Hz), 7.23 (1H, dd, J = 8.7, 2.4 Hz), 7.41 (1H, d, J = 2.4 Hz), 5.50 - 5.61 (2H, m), 7.65 (1H, s), 7.80 - 7.88 (2H, m), 8.10 (1H, s), 1H unconfirmed.

### Reference Example 111

### 6-bromo-4-(2,4-dichlorophenyl)chroman-2-one

A mixture of 4-bromophenol (1.73 g, 10 mmol) and 3-(2,4-dichlorophenyl)phenylacrylic acid (12.4 g, 91.2 mmol) in sulfuric acid (14 mL) and acetic acid (6 mL) was stirred at 150°C for 1 hr. The mixture was poured into ice water, and the resultant product was extracted with ethyl acetate. The extract was washed with water and saturated aqueous sodium hydrogen carbonate solution, and concentrated. The residue was purified by silica gel column chromatography (hexane-ethyl acetate 10:0→0:10) to give the title compound (2.1 g, yield 56%). Melting point 117 - 118°C (ethyl acetate-hexane).
¹H-NMR (CDCl₃) δ : 3.04 (2H, d, J = 6.0 Hz), 4.80 (1H, t, J = 6.0 Hz), 6.80 (1H, d, J= 8.1 Hz), 7.06 (1H, d, J= 8.4 Hz), 7.14 (1H, d, J = 2.4 Hz), 7.16 (1H, dd, J = 8.1, 2.4 Hz), 7.45 (1H, d, J = 2.1 Hz), 7.46 (1H, d, J = 2.1 Hz).

### Reference Example 112

### 6-bromo-4-(2,4-dichlorophenyl)chromane

To a solution of 6-bromo-4-(2,4-dichlorophenyl)chroman-2-one (2.1 g, 5.64 mmol) synthesized in Reference Example 111 in THF (30 mL) was added lithium aluminum hydride (214 mg, 5.64 mmol) at 0°C, and the mixture was heated under reflux for 1 hr. Water was added to the reaction mixture, and the resultant product was extracted with ethyl acetate. The extract was washed with water, dried over magnesium sulfate, and concentrated under reduced pressure. The obtained residue was crystallized from hexane-ethyl acetate to give 4-bromo-2-[1-(2,4-dichlorophenyl)-3-hydroxypropyl]phenol (1.4 g). To a solution of the compound (1.40 g, 2.66 mmol) and triphenylphosphine (768 mg, 2.93 mmol) in THF (30 mL) was added diethyl azodicarboxylate (556 mg, 3.19 mmol) under ice-cooling, and the mixture was stirred at room temperature for 1 hr. The solvent was concentrated under reduced pressure, and the obtained residue was purified by silica gel column chromatography (hexane-ethyl acetate 10:0→0:10) to give 910 mg (yield 95%) as an oil.
¹H-NMR (CDCl₃) δ : 1.96 - 2.08 (1H, m), 2.23 - 2.41 (1H, m), 4.01 - 4.25 (2H, m), 4.57 (1H, t, J = 6.0 Hz), 6.77 (1H, d, J= 8.7 Hz), 6.81 (1H, d, J= 8.4 Hz), 6.92 (1H, dd, J = 2.4, 0.9 Hz), 7.15 (1H, dd, J = 8.4, 2.4 Hz), 7.24 (1H, dd, J = 8.4, 2.4 Hz), 7.43 (1H, d, J = 2.4 Hz).

### Reference Example 113

### 3-[4-(2,4-dichlorophenyl)-3,4-dihydro-2H-chromen-6-yl]benzoic acid

In the same manner as in Reference Example 56 and using 6-bromo-4-(2,4-dichlorophenyl)chromane obtained in Reference Example 112, the title compound was obtained. Yield 66%, melting point 223 - 224°C (ethyl acetate-hexane). ¹H-NMR (CDCl₃) δ : 2.00 - 2.12 (1H, m), 2.30 - 2.42 (1H, m), 4.02 - 4.18 (1H, m), 4.20 - 4.31 (1H, m), 4.68 (1H, t, J = 5.7 Hz), 6.86 (1H, d, J= 8.7 Hz), 6.98 (1H, d, J= 8.7 Hz), 7.08 (1H, d, J = 2.1 Hz), 7.13 (1H, dd, J = 8.4, 2.1 Hz), 7.42 - 7.49 (3H, m), 7.68 (1H, d, J = 7.8 Hz), 7.99 (1H, d, J = 7.8 Hz), 8.18 (1H, s), 1H unconfirmed.

### Reference Example 114

### ethyl 3-(8-oxo-5,6,7,8-tetrahydronaphthalen-2-yl)benzoate

In the same manner as in Reference Example 1 and using 8-oxo-5,6,7,8-tetrahydronaphthalen-2-yl trifluoromethanesulfonate and [3-(ethoxycarbonyl)phenyl]boronic acid, the title compound was obtained. Yield 83%, melting point 89 - 90°C (ethyl acetate-hexane).
¹H-NMR (CDCl₃) δ : 1.42 (3H, t, J = 7.2 Hz), 2.12 - 2.25 (2H, m), 2.63 - 2.80 (2H, m), 3.02 (2H, t, J = 6.0 Hz), 4.42 (2H, q, J = 7.0 Hz), 7.36 (1H, d, J = 7.9 Hz), 7.52 (1H, t, J = 7.7 Hz), 7.75 (1H, dd, J = 7.9, 2.1 Hz), 7.78 - 7.83 (1H, m), 8.00 - 8.07 (1H, m), 8.29 (1H, t, J = 1.6 Hz), 8.31 (1H, d, J = 2.1 Hz).

### Reference Example 115

### ethyl 3-(8-hydroxy-5,6,7,8-tetrahydronaphthalen-2-yl)benzoate

In the same manner as in Reference Example 101 and using ethyl 3-(8-oxo-5,6,7,8-tetrahydronaphthalen-2-yl)benzoate obtained in Reference Example 114, the title compound was obtained. Yield 76%, melting point 88 - 100°C (ethyl acetate-hexane).
¹H-NMR (CDCl₃) δ : 1.41 (3H, t, J = 7.2 Hz), 1.74 - 1.90 (1H, m), 1.90 - 2.11 (4H, m), 2.68 - 2.97 (2H, m), 4.40 (2H, q, J = 6.9 Hz), 4.85 (1H, d, J = 3.8 Hz), 7.19 (1H, d, J = 8.0 Hz), 7.41 - 7.52 (2H, m), 7.71 (1H, d, J = 1.9 Hz), 7.76 (1H, d, J = 8.3 Hz), 8.00 (1H, d, J = 8.0 Hz), 8.26 (1H, s).

### Reference Example 116

### ethyl 3-[8-(2,4-dichlorophenoxy)-5,6,7,8-tetrahydronaphthalen-2-yl]benzoate

In the same manner as in Reference Example 102 and using ethyl 3-(8-hydroxy-5,6,7,8-tetrahydronaphthalen-2-yl)benzoate obtained in Reference Example 115, the title compound was obtained as an oil. The obtained crude product was directly used for the next reaction.
¹H-NMR (CDCl₃) δ : 1.41 (3H, t, J = 7.2 Hz), 1.77 - 1.93 (1H, m), 1.98 - 2.24 (3H, m), 2.72 - 2.89 (1H, m), 2.89 - 3.04 (1H, m), 4.40 (2H, q, J = 7.0 Hz), 5.39 (1H, t, J = 4.7 Hz), 7.06 (1H, d, J = 8.9 Hz), 7.21 (1H, dd, J = 8.8, 2.5 Hz), 7.25 (1H, d, J = 7.9 Hz), 7.41 (1H, d, J = 2.4 Hz), 7.44 - 7.55 (2H, m), 7.60 - 7.66 (1H, m), 7.68 - 7.76 (1H, m), 7.95 - 8.03 (1H, m), 8.24 (1H, t, J = 1.7 Hz).

### Reference Example 117

### 3-[8-(2,4-dichlorophenoxy)-5,6,7,8-tetrahydronaphthalen-2-yl]benzoic acid

In the same manner as in Reference Example 4 and using ethyl 3-[8-(2,4-dichlorophenoxy)-5,6,7,8-tetrahydronaphthalen-2-yl]benzoate obtained in Reference Example 116, the title compound was obtained. Yield 83%, melting point 176 - 181 °C (ethyl acetate).
¹H-NMR (DMSO-d₆) δ:1.68 - 1.88 (1H, m), 1.89 - 2.07 (3H, m), 2.68 - 2.85 (1H, m), 2.86 - 2.99 (1H, m), 5.69 (1H, brs), 7.31 (1H, d, J = 7.9 Hz), 7.39 - 7.46 (1H, m), 7.52 - 7.65 (4H, m), 7.68 (1H, d, J = 1.7 Hz), 7.84 - 7.89 (1H, m), 7.89 - 7.95 (1H, m), 8.15 (1H, t, J = 1.6 Hz), 13.08 (1H, s).

### Reference Example 118

### 2-chloro-4-(3-nitrophenyl)pyrimidine

To a solution of 2,4-dichloropyrimidine (2.0 g, 13.4 mmol) in dimethoxyethane (100 ml) were added 3-nitrophenylboronic acid (2.2 g, 13.2 mmol), tetrakis(triphenylphosphine)palladium(0) (0.77 g, 0.67 mmol) and 2 M aqueous sodium carbonate solution (8 ml), and the mixture was heated under reflux under an argon atmosphere for 13 hr. Ethyl acetate was added to the reaction mixture, and the mixture was washed with water and saturated brine, dried and concentrated. The residue was purified by silica gel column chromatography (THF) and recrystallized from ethyl acetate to give the title compound (1.2 g, yield 39%). ¹H-NMR (CDCl₃) δ : 7.72-7.77 (2H, m), 8.39-8.43 (1H, m), 8.47-8.51 (1H, m), 8.77 (1H, d, J=5.1 Hz), 8.93 (1H, t, J=2.1 Hz).

### Reference Example 119

### (4-(3-nitrophenyl)pyrimidin-2-yl)-(2-(3,4-dimethoxyphenyl)ethyl)amine

To a solution of 2-chloro-4-(3-nitrophenyl)pyrimidine (1.1 g, 4.7 mmol) synthesized in Reference Example 118 in n-butanol (15 ml) were added 2-(3,4-dimethoxyphenyl)ethylamine (1.3 g, 7.2 mmol) and ethyldiisopropylamine (1.7 ml, 9.5 mmol), and the mixture was heated under microwave irradiation at 130°C for 30 min. The reaction mixture was dissolved in ethyl acetate, washed with water and saturated brine, dried and concentrated. The residue was purified by silica gel column chromatography (ethyl acetate) and recrystallized from ethyl acetate-hexane to give the title compound (1.5 g, yield 84%).
¹H-NMR (CDCl₃) δ : 2.93 (2H, t, J=6.9 Hz), 3.75-3.82 (2H, m), 3.87 (3H, s), 3.88 (3H, s), 5.30 (1H, br t, J=5.7 Hz), 6.78 (1H, s), 6.84 (2H, d, J=0.6 Hz), 7.03 (1H, d, J=5.4 Hz), 7.65 (1H, t, J=8.1 Hz), 8.30-8.37 (2H, m), 8.41 (1H, d, J=5.1 Hz), 8.92 (1H, brs).

### Reference Example 120

### (4-(3-aminophenyl)pyrimidin-2-yl)-(2-(3,4-dimethoxyphenyl)ethyl)amine

To a solution of (4-(3-nitrophenyl)pyrimidin-2-yl)-(2-(3,4-dimethoxyphenyl)ethyl)amine (1.3 g, 3.4 mmol) synthesized in Reference Example 119 in THF-ethanol (1:1 40 ml) was added 10% palladium carbon (0.13 g), and the mixture was stirred at normal pressure under a hydrogen atmosphere at room temperature for 1 day. The reaction mixture was filtered and concentrated. The residue was purified by basic silica gel column chromatography (hexane-ethyl acetate 10:0→0:10) and recrystallized from ethyl acetate-hexane to give the title compound (1.2 g, quantitative).
¹H-NMR (CDCl₃) δ : 2.91 (2H, t, J=6.9 Hz), 3.72-3.82 (4H, m), 3.87 (6H, s), 5.21 (1H, br t, J=5.7 Hz), 6.78-6.84 (4H, m), 6.93 (1H, d, J=5.1 Hz), 7.22-7.27 (1H, m), 7.36-7.39 (2H, m), 8.31 (1H, d, J=5.1 Hz).

### Reference Example 121

### 2-chloro-6-(2-(3,4-dimethoxyphenyl)ethoxy)pyridine

A solution of 6-chloropyridin-2-ol (1.0 g, 7.72 mmol), 2-(3,4-dimethoxyphenyl)ethanol (1.55 g, 8.44 mmol), triphenylphosphine (2.23 g, 8.44 mmol) and diethyl azodicarboxylate (1.61 g, 8.44 mmol) in tetrahydrofuran (30 mL) was stirred at room temperature for 1 hr. The reaction mixture was concentrated under reduced pressure, and the residue was subjected to silica gel column chromatography (hexane-ethyl acetate 80:20) to give the title compound (1.76 g, yield 78%) as an oil.
¹H-NMR (CDCl₃) δ : 3.01 (2H, t, J = 6.9 Hz), 3.86 (3H, s), 3.88 (3H, s), 4.49 (2H, t, J = 6.9 Hz), 6.60-6.89 (5H, m), 7.45-7.92 (1H, m).

### Reference Example 122

### N-(3-(2-(2-(3,4-dimethoxyphenyl)ethylamino)pyrimidin-4-yl)phenyl)-2-methoxyacetamide

To a solution of (4-(3-aminophenyl)pyrimidin-2-yl)-(2-(3,4-dimethoxyphenyl)ethyl)amine (0.15 g, 0.43 mmol) synthesized in Reference Example 120 in DMF (3 ml) were added methoxyacetic acid (46 mg, 0.51 mmol), WSC (99 mg, 0.52 mmol) and HOBt (70 mg, 0.52 mmol) and the mixture was stirred at room temperature for 16 hr. Ethyl acetate was added to the reaction mixture, and the mixture was washed with water and saturated brine, and concentrated. The residue was purified by silica gel column chromatography (hexane-ethyl acetate 10:0→0:10) and recrystallized from ethyl acetate-hexane to give the title compound (0.14 g, yield 77%) as crystals. Melting point 140 - 141°C.
¹H-NMR (CDCl₃) → : 2.92 (2H, t, J=6.9 Hz), 3.53 (3H, s), 3.74-3.80 (2H, m), 3.87 (6H, s), 4.05 (2H,s), 5.22 (1H, br t, J=5.7 Hz), 6.78 (1H, s), 6.82 (2H, s), 6.98 (1H, d, J=5.1 Hz), 7.44 (1H, d, J=8.1 Hz), 7.76-7.80 (2H, m), 8.19 (1H, brs), 8.33-8.36 (2H, m).

### Reference Example 123

### 3-(6-(2-(3,4-dimethoxyphenyl)ethoxy)pyridin-2-yl)benzoic acid

A mixture of 2-chloro-6-(2-(3,4-dimethoxyphenyl)ethoxy)pyridine (1.76 g, 5.99 mmol) obtained in Reference Example 121, [3-(ethoxycarbonyl)phenyl]boronic acid (1.28 g, 6.59 mmol) and tetrakis(triphenylphosphine)palladium(0) (207 mg, 0.18 mmol) in 2 N aqueous sodium carbonate solution (20 mL)-1,2-dimethoxyethane (20 mL) was allowed to react under a nitrogen atmosphere at 90°C for 16 hr. Water was added to the reaction mixture and the mixture was extracted with ethyl acetate. The organic layer was washed with saturated brine, dried over anhydrous sodium sulfate, and concentrated under reduced pressure. The residue was purified by silica gel column chromatography (ethyl acetate-hexane 2:3) to give ethyl 3-(6-(2-(3,4-dimethoxyphenyl)ethoxy)pyridin-2-yl)benzoate (1.36 g). To a solution of the compound in ethanol (50 mL) was added 1 N aqueous sodium hydroxide solution (10 mL, 10 mmol) at room temperature, and the mixture was stirred at 60°C for 2 hr and concentrated under reduced pressure. The aqueous layer was acidified with water and hydrochloric acid and the mixture was extracted with ethyl acetate. The organic layer was washed with water and saturated brine, and dried over anhydrous sodium sulfate. The solvent was evaporated under reduced pressure and the obtained residue was crystallized from ethyl acetate-hexane to give the title compound (820 mg, yield 36%). Melting point 147 - 148°C.
¹H-NMR (CDCl₃) δ : 3.11 (2H, t, J = 7.2 Hz), 3.86 (3H, s), 3.88 (3H, s), 4.66 (2H, t, J = 7.2 Hz), 6.72 (1H, d, J = 8.1 Hz), 6.82-6.91 (3H, m), 7.41 (1H, d, J = 7.5 Hz), 7.57 (1H, t, J = 7.5 Hz), 7.66 (1H, t, J = 7.5 Hz), 8.14 (1H, dd, J = 7.5, 1.2 Hz).8.30 (1H, d, J = 6.6 Hz), 8.76 (1H, s), 1H unconfirmed.

### Reference Example 124

### 3-(6-(2-(3,4-dimethoxyphenyl)ethoxy)pyridin-2-yl)-N-(2-pyrrolidin-1-ylethyl)benzamide

In the same manner as in Reference Example 122 and using 3-(6-(2-(3,4-dimethoxyphenyl)ethoxy)pyridin-2-yl)benzoic acid obtained in Reference Example 123 and 2-pyrrolidin-1-ylethanamine, the title compound was obtained. Yield 45%, melting point 126 - 127°C (ethyl acetate-hexane).
¹H-NMR (CDCl₃) δ : 1.73-1.82 (4H, m), 2.47-2.61 (4H, m), 2.70 (2H, t, J = 6.0 Hz), 3.09 (2H, t, J = 6.9 Hz), 3.57 (2H, q, J = 6.0 Hz), 3.86 (3H, s), 3.87 (3H, s), 4.63 (2H, t, J = 6.9 Hz), 6.70 (1H, d, J = 7.8 Hz), 6.81-6.88 (4H, m), 7.38 (1H, d, J = 7.2 Hz), 7.50 (1H, t, J = 7.5 Hz), 7.64 (1H, t, J = 8.1 Hz), 7.76 (1H, d, J = 8.4 Hz), 8.16 (1H, d, J = 8.4 Hz), 8.42 (1H, t, J = 1.5 Hz).

### Reference Example 125

### ethyl 3-[1-[2-(2,4-dichlorophenyl)ethyl]-2,3-dihydro-1H-indol-6-yl]benzoate

In the same manner as in Reference Example 15 and using ethyl 3-(2,3-dihydro-1H-indol-6-yl)benzoate obtained in Reference Example 6 and (2,4-dichlorophenyl)acetaldehyde, the title compound was obtained. The obtained compound was directly used for the next reaction.

### Reference Example 126

### ethyl 3-(1-[[3-(triffuoromethyl)phenyl]acetyl]-2,3-dihydro-1H-indol-6-yl)benzoate

To a solution of ethyl 3-(2,3-dihydro-1H-indol-6-yl)benzoate (500 mg, 1.87 mmol) obtained in Reference Example 6, WSC (429 mg, 2.24 mmol) and HOBt (303 mg, 2.24 mmol) in DMF (5 ml) was added amine (457 mg, 2.24 mmol), and the mixture was stirred at room temperature for 3 hr. To the reaction mixture was added saturated aqueous sodium hydrogen carbonate solution and the mixture was extracted with ethyl acetate. The combined organic layer was washed with water, dried over anhydrous magnesium sulfate, and concentrated under reduced pressure. The residue was purified by silica gel column chromatography (hexane-ethyl acetate 100:0→70:30) to give the title compound (790 mg, yield 93%) as crystals. Melting point 222 - 223°C.
¹H-NMR (CDCl₃) δ : 1.40 (3H, t, J = 7.2 Hz), 3.27 (2H, t, J = 8.3 Hz), 3.89 (2H, s), 4.19 (2H, t, J = 8.3 Hz), 4.39 (2H, q, J = 7.2 Hz), 7.28 (1H, brs), 7.29 - 7.34 (1H, m), 7.41 - 7.52 (2H, m), 7.52 - 7.59 (3H, m), 7.78 (1H, d, J = 7.6 Hz), 7.99 (1H, d, J = 8.0 Hz), 8.24 (1H, s), 8.55 (1H, s).

### Reference Example 127

### ethyl 3-(1-[2-[3-(trifluoromethyl)phenyl] ethyl]-2,3-dihydro-1H-indol-6-yl)benzoate

To a solution of ethyl 3-(1-[[3-(triffuoromethyl)phenyl]acetyl]-2,3-dihydro-1H-indol-6-yl)benzoate (200 mg, 0.44 mmol) obtained in Reference Example 126 in THF (4 mL) were added boron trifluoride diethyl ether complex (86.2 µL, 0.66 mmol) and sodium borohydride (16.6 mg, 0.44 mmol), and the mixture was stirred at 50°C for 14 hr. To the reaction mixture were added water and aqueous sodium hydrogen carbonate solution and the mixture was extracted with ethyl acetate. The organic layer was dried over anhydrous magnesium sulfate, and concentrated under reduced pressure. The residue was purified by silica gel column chromatography (hexane-ethyl acetate 100:0→90:10) to give the title compound (157 mg, yield 81%) as an oil. ¹H-NMR (CDCl₃) δ : 1.41 (3H, t, J = 7.2 Hz), 2.94 - 3.08 (4H, m), 3.38 - 3.50 (4H, m), 4.40 (2H, q, J = 7.2 Hz), 6.63 (1H, s), 6.89 (1H, dd, J = 7.2, 1.5 Hz), 7.15 (1H, d, J = 7.6 Hz), 7.39 - 7.50 (4H, m), 7.52 (1H, s), 7.72 (1H, d, J = 8.0 Hz), 7.99 (1H, d, J = 8.0 Hz), 8.23 (1H, s).

### Reference Example 128

### ethyl 3-[3-[3-(trifluoromethyl)phenoxy]-2,3-dihydro-1H-inden-5-yl]benzoate

In the same manner as in Reference Example 102 and using ethyl 3-(3-hydroxy-2,3-dihydro-1H-inden-5-yl)benzoate obtained in Reference Example 101 and 3-hydroxybenzene trifluoride, the title compound was obtained. Yield 62%. ¹H-NMR (CDCl₃) δ : 1.41 (3H, t, J = 7.2 Hz), 2.19 - 2.34 (1H, m), 2.57 - 2.74 (1H, m), 2.94 - 3.07 (1H, m), 3.14 - 3.29 (1H, m), 4.40 (2H, q, J = 7.2 Hz), 5.86 (1H, dd, J = 6.6, 4.1 Hz), 7.20 (1H, dd, J = 8.4, 2.4 Hz), 7.22 - 7.29 (2H, m), 7.38 - 7.46 (2H, m), 7.49 (1H, t, J = 7.8 Hz), 7.60 (1H, dd, J = 7.8, 1.8 Hz), 7.67 (1H, s), 7.73 - 7.79 (1H, m), 7.97 - 8.05 (1H, m), 8.26 (1H, t, J = 1.6 Hz).

### Reference Example 129

### ethyl 3-[3-[(2,4-dichlorobenzyl)amino]-2,3-dihydro-1H-inden-5-yl]benzoate

In the same manner as in Reference Example 15 and using ethyl 3-(3-oxo-2,3-dihydro-1H-inden-5-yl)benzoate obtained in Reference Example 100 and 2,4-dichlorobenzylamine, the title compound was obtained. Yield 73%.
¹H-NMR (CDCl₃) δ : 1.42 (3H, t, J = 7.2 Hz), 1.84 - 2.02 (1H, m), 2.40 - 2.60 (1H, m), 2.76 - 2.96 (1H, m), 2.99 - 3.16 (1H, m), 3.93 - 4.06 (2H, m), 4.31 - 4.38 (1H, m), 4.36 - 4.46 (2H, m), 7.23 (1H, dd, J = 8.1, 2.1 Hz), 7.32 (1H, d, J = 8.0 Hz), 7.38 (1H, d, J = 1.9 Hz), 7.44 - 7.54 (3H, m), 7.60 (1H, s), 7.76 (1H, d, J = 8.3 Hz), 8.00 (1H, d, J = 8.0 Hz), 8.27 (1H, t, J = 1.7 Hz).

### Reference Example 130

### 2-[3-(2,4-dichlorophenyl)-2,3-dihydro-1H-inden-5-yl]-4,4,5,5-tetramethyl-1,3,2-dioxaborolane

To a solution of 3-(2,5-dichlorophenyl)-2,3-dihydro-1H-inden-5-yl trifluoromethanesulfonate (3.50 g, 8.51 mmol) obtained in Reference Example 79, 4,4,4',4',5,5,5',5'-octamethyl-2,2'-bi-1,3,2-dioxaborolane (2.59 g, 10.2 mmol) and potassium acetate (1.00 g, 10.2 mmol) in DMSO (40 mL) was added (1,1-bis(diphenylphosphino)ferrocene)dichloropalladium(II) dichloromethane adduct (351 mg, 0.43 mmol) and the mixture was stirred with heating at 85°C for 15 hr. The reaction mixture was poured into ethyl acetate, washed with saturated brine, dried over anhydrous magnesium sulfate, and concentrated under reduced pressure. The residue was purified by silica gel column chromatography (hexane-ethyl acetate 100:0→95:5) to give the title compound (2.28 g, yield 69%) as an oil.
¹H-NMR (CDCl₃) δ : 1.31 (12H, s), 1.80 - 1.96 (1H, m), 2.63 (1H, m), 2.90 - 3.08 (2H, m), 4.82 (1H, t, J = 8.1 Hz), 6.84 (1H, d, J = 8.3 Hz), 7.11 (1H, dd, J = 8.7, 2.3 Hz), 7.32 (1H, d, J = 7.5 Hz), 7.41 (1H, d, J = 2.3 Hz), 7.43 (1H, s), 7.69 (1H, d, J = 7. 5 Hz).

### Reference Example 131

### methyl 2-[3-(2,4-dichlorophenyl)-2,3-dihydro-1H-inden-5-yl] pyridine-4-carboxylate

In the same manner as in Reference Example 1 and using 2-[3-(2,4-dichlorophenyl)-2,3-dihydro-1H-inden-5-yl]-4,4,5,5-tetramethyl-1,3,2-dioxaborolane obtained in Reference Example 130 and methyl 2-bromopyridine-4-carboxylate, the title compound was obtained as an oil. Yield 21 %.
¹H-NMR (CDCl₃) δ : 1.96 (1H, dq, J = 12.8, 8.0 Hz), 2.71 (1H, m), 2.92 - 3.17 (2H, m), 3.97 (3H, s), 4.91 (1H, t, J = 7.9 Hz), 6.91 (1H, d, J = 8.3 Hz), 7.12 (1H, dd, J = 8.5, 2.1 Hz), 7.38 - 7.49 (2H, m), 7.68 (1H, s), 7.72 (1H, dd, J = 4.9, 1.5 Hz), 7.93 (1H, d, J = 7.9 Hz), 8.21 (1H, s), 8.77 (1H, d, J = 6.0 Hz).

### Reference Example 132

### 3-[-[2-(2,4-dichlorophenyl)ethyl]-2,3-dihydro-1H-indol-6-yl]benzoic acid

In the same manner as in Reference Example 4 and using ethyl 3-[1-[2-(2,4-dichlorophenyl)ethyl]-2,3-dihydro-1H-indol-6-yl]benzoate obtained in Reference Example 125, the title compound was obtained as crude crystals. The obtained compound was directly used for the next reaction.

### Reference Example 133

### 3-(1-[2-[3-(trifluoromethyl)phenyl]ethyl]-2,3-dihydro-1H-indol-6-yl)benzoic acid

In the same manner as in Reference Example 4 and using ethyl 3-(1-[2-[3-(trifluoromethyl)phenyl]ethyl]-2,3-dihydro-1H-indol-6-yl)benzoate obtained in Reference Example 127, the title compound was obtained. Yield 93%.
¹H-NMR (CDCl₃) δ : 2.94 - 3.07 (4H, m), 3.39 - 3.46 (4H, m), 6.63 (1H, d, J = 1.5 Hz), 6.91 (1H, dd, J = 7.6, 1. 5 Hz), 7.15 (1H, d, J = 7.6 Hz), 7.39 - 7.58 (5H, m), 7.79 (1H, d, J = 7.6 Hz), 8.07 (1H, d, J = 7.6 Hz), 8.31 (1H, s).

### Reference Example 134

### 3-[3-[3-(trifluoromethyl)phenoxy]-2,3-dihydro-1H-inden-5-yl]benzoic acid

In the same manner as in Reference Example 4 and using ethyl 3-[3-[3-(trifluoromethyl)phenoxy]-2,3-dihydro-1H-inden-5-yl]benzoate obtained in Reference Example 128, the title compound was obtained. Yield 32%, melting point 175 - 185°C.
¹H-NMR (DMSO-d₆) δ : 2.01 - 2.17 (1H, m), 2.58 - 2.74 (1H, m), 2.87 - 3.02 (1H, m), 3.03 - 3.20 (1H, m), 6.05 (1H, dd, J = 6.5, 3.7 Hz), 7.32 (1H, d, J = 7.5 Hz), 7.36 - 7.44 (2H, m), 7.47 (1H, t, J = 7.9 Hz), 7.52 - 7.63 (2H, m), 7.68 (1H, dd, J = 7.7, 1.7 Hz), 7.73 (1H, s), 7.86 - 7.97 (2H, m), 8.16 (1H, t, J = 1.6 Hz), 13.08 (1H, s).

### Reference Example 135

### 3-[3-[(2,4-dichlorobenzyl)amino]-2,3-dihydro-1H-inden-5-yl]benzoic acid

In the same manner as in Reference Example 4 and using ethyl 3-[3-[(2,4-dichlorobenzyl)amino]-2,3-dihydro-1H-inden-5-yl]benzoate obtained in Reference Example 129, the title compound was obtained. Yield 76%, melting point 190 - 191°C (ethyl acetate).
¹H-NMR (DMSO-d₆) δ : 1.99 (1H, brs), 2.44 (2H, brs), 2.86 (1H, brs), 3.00 (1H, brs), 4.00 (2H, brs), 4.44 (1H, brs), 7.38 (1H, d, J = 7.9 Hz), 7.46 (1H, dd, J = 8.0, 1.6 Hz), 7.59 (3H, t, J = 7.7 Hz), 7.70 (1H, brs), 7.87 - 7.96 (2H, m), 8.19 (1H, s), 1H unconfirmed.

### Reference Example 136

### 2-[3-(2,4-dichlorophenyl)-2,3-dihydro-1H-inden-5-yl]pyridine-4-carboxylic acid

In the same manner as in Reference Example 4 and using methyl 2-[3-(2,4-dichlorophenyl)-2,3-dihydro-1H-inden-5-yl]pyridine-4-carboxylate obtained in Reference Example 131, the title compound was obtained. Yield 96%, melting point 205 - 206°C (ethyl acetate).
¹H-NMR (DMSO-d₆) δ : 1.90 - 2.12 (1H, m), 2.55 - 2.74 (1H, m), 2.90 - 3.15 (2H, m), 4.85 (1H, t, J = 8.0 Hz), 7.05 (1H, d, J = 8.3 Hz), 7.36 (1H, dd, J = 8.3, 1.9 Hz), 7.47 (1H, d, J = 8.0 Hz), 7.62 - 7.78 (3H, m), 7.98 (1H, d, J = 8.0 Hz), 8.19 (1H, s), 8.76 (1H, d, J = 4.9 Hz), 13.81 (1H, brs).

### Reference Example 137

### 3-[3-(2,4-dichlorophenyl)-2,3-dihydro-1H-inden-5-yl]aniline

In the same manner as in Reference Example 1 and using 3-(2,5-dichlorophenyl)-2,3-dihydro-1H-inden-5-yl trifluoromethanesulfonate obtained in Reference Example 79 and 3-aminophenylboronic acid, the title compound was obtained. Yield 52%.
¹H-NMR (CDCl₃) δ : 1.86 - 2.06 (1H, m), 2.61 - 2.77 (1H, m), 2.94 - 3.10 (2H, m), 3.70 (2H, brs), 4.87 (1H, t, J = 8.1 Hz), 6.61 - 6.66 (1H, m), 6.83 (1H, t, J = 2.0 Hz), 6.89-6.93 (1H, m), 6.95 (1H, d, J = 8.5 Hz), 7.12 (1H, dd, J = 8.5, 2.1 Hz), 7.15 - 7.21 (2H, m), 7.31 - 7.36 (1H, m), 7.41 - 7.46 (2H, m).

### Example 1

### 3-[1-(2,4-dichlorophenyl)-2,3-dihydro-1H-indol-6-yl]-N-[2-(dimethylamino)ethyl]benzamide

To a solution of 3-[1-(2,4-dichlorophenyl)-2,3-dihydro-1H-indol-6-yl]benzoic acid (70 mg, 0.18 mmol) obtained in Reference Example 4, WSC (42.2 mg, 0.22 mmol) and HOBt (29.7 mg, 0.22 mmol) in DMF (1 ml) was added N,N-dimethylethane-1,2-diamine (24.0 µL, 0.22 mmol), and the mixture was stirred at room temperature for 16 hr. To the reaction mixture was added saturated aqueous sodium hydrogen carbonate solution and the mixture was extracted with ethyl acetate. The organic layer was washed with water, dried over anhydrous magnesium sulfate, and concentrated under reduced pressure. The residue was purified by silica gel column chromatography to give the title compound (50 mg , yield 61%) as an amorphous solid.
¹H-NMR (CDCl₃) δ : 2.25 (6H, s), 2.51 (2H, t, J = 5.9 Hz), 3.22 (2H, t, J = 8.3 Hz), 3.47 - 3.58 (2H, m), 3.94 (2H, brs), 6.61 (1H, s), 6.80 (1H, brs), 7.00 (1 H, dd, J = 7.6, 1.5 Hz), 7.21 - 7.31 (2H, m), 7.36 - 7.47 (2H, m), 7.50 (1H, d, J = 2.7 Hz), 7.61 (1H, d, J = 7.6 Hz), 7.68 (1H, d, J = 7.6 Hz), 7.90 (1H, s).

### Example 2

### N-(2-cyanoethyl)-3-[1-(2,4-dichlorophenyl)-2,3-dihydro-1H-indol-6-yl]benzamide

To a solution of 3-[1-(2,4-dichlorophenyl)-2,3-dihydro-1H-indol-6-yl]benzoic acid (105 mg, 0.27 mmol) obtained in Reference Example 4 and DMTMM (94.3 mg, 0.32 mmol) in DMF (1.5 ml) was added 3-aminopropanenitrile (23.6 µL, 0.32 mmol), and the mixture was stirred at room temperature for 4 hr. To the reaction mixture was added saturated aqueous sodium hydrogen carbonate solution and the mixture was extracted with ethyl acetate. The organic layer was washed with water, dried over anhydrous magnesium sulfate, and concentrated under reduced pressure. The residue was recrystallized from ethyl acetate-hexane to give the title compound (100 mg , yield 85%) as crystals. Melting point 148 - 154°C.
¹H-NMR (CDCl₃) δ : 2.76 (2H, t, J = 6.3 Hz), 3.22 (2H, t, J = 8.3 Hz), 3.73 (2H, q, J = 6.2 Hz), 3.94 (2H, brs), 6.60 (2H, d, J = 1.5 Hz), 6.99 (1H, dd, J = 7.6, 1.6 Hz), 7.21 - 7.29 (2H, m), 7.39 (1H, d, J = 8.5 Hz), 7.45 (1H, t, J = 7.7 Hz), 7.51 (1H, d, J = 2.3 Hz), 7.62 - 7.69 (2H, m), 7.89 (1H, t, J = 1.7 Hz).

### Example 3

### 3-[1-(2,4-dichlorophenyl)-2,3-dihydro-1H-indol-6-yl]-N-(2-hydroxyethyl)benzamide

To a solution of 3-[1-(2,4-dichlorophenyl)-2,3-dihydro-1H-indol-6-yl]benzoic acid (250 mg, 0.65 mmol) obtained in Reference Example 4 and DMTMM (230 mg, 0.78 mmol) in DMF (5 ml) was added 2-aminoethanol (32.7 µL, 0.78 mmol), and the mixture was stirred at room temperature for 15 hr. To the reaction mixture was added saturated aqueous sodium hydrogen carbonate solution and the mixture was extracted with ethyl acetate. The organic layer was washed with water, dried over anhydrous magnesium sulfate, and concentrated under reduced pressure. The residue was purified by silica gel column chromatography, and recrystallized from ethyl acetate-hexane to give the title compound (170 mg, yield 61 %) as crystals. Melting point 140 - 141 °C.
¹H-NMR (CDCl₃) δ : 2.46 (1H, t, J = 4.9 Hz), 3.22 (2H, t, J = 8.5 Hz), 3.59 - 3.69 (2H, m), 3.85 (2H, q, J = 5.2 Hz), 3.94 (2H, brs), 6.60 (2H, d, J = 1.5 Hz), 6.99 (1H, dd, J = 7.6, 1.5 Hz), 7.21 - 7.29 (2H, m), 7.37 - 7.47 (2H, m), 7.50 (1H, d, J = 2.3 Hz), 7.63 (1H, d, J = 8.3 Hz), 7.68 (1H, d, J = 7.6 Hz), 7.90 (1H, t, J = 1.7 Hz).

### Example 4

### N-(2-cyanoethyl)-3-[1-(2,5-dichlorophenyl)-2,3-dihydro-1H-indol-6-yl]benzamide

In the same manner as in Example 3 and using 3-[1-(2,5-dichlorophenyl)-2,3-dihydro-1H-indol-6-yl]benzoic acid obtained in Reference Example 8 and 3-aminopropanenitrile, the title compound was obtained. Yield 91 %, melting point 189 - 190°C (THF-hexane).
¹H-NMR (CDCl₃) δ : 2.75 (2H, t, J = 6.3 Hz), 3.22 (2H, t, J = 8.4 Hz), 3.67 - 3.79 (2H, m), 3.97 (2H, brs), 6.55 - 6.68 (2H, m), 7.01 (1H, dd, J = 7.7, 1.5 Hz), 7.13 (1H, dd, J = 8.5, 2.4 Hz), 7.21 - 7.29 (1H, m), 7.35 - 7.50 (3H, m), 7.59 - 7.72 (2H, m), 7.90 (1H, t, J = 1.7 Hz).

### Example 5

### 3-[1-(2,5-dichlorophenyl)-2,3-dihydro-1H-indol-6-yl]-N-(2-hydroxyethyl)benzamide

To a solution of 3-[1-(2,5-dichlorophenyl)-2,3-dihydro-1H-indol-6-yl]benzoic acid (300 mg, 0.78 mmol) obtained in Reference Example 8 and DMTMM (277 mg, 0.94 mmol) in methanol (8 ml) was added c (39.5 µL, 0.94 mmol), and the mixture was stirred at room temperature for 6 hr. The reaction mixture was concentrated under reduced pressure, saturated aqueous sodium hydrogen carbonate solution was added, and the mixture was extracted with ethyl acetate. The organic layer was dried over anhydrous magnesium sulfate, and concentrated under reduced pressure. The residue was recrystallized from THF-hexane to give the title compound (150 mg, yield 45%) as crystals. Melting point 206 - 207°C.
¹H-NMR (CDCl₃) δ : 2.48 (1H, brs), 3.22 (2H, t, J = 8.4 Hz), 3.59 - 3.69 (2H, m), 3.80 - 3.88 (2H, m), 3.97 (2H, brs), 6.62 (1H, brs), 6.65 (1H, d, J = 1.3 Hz), 7.01 (1H, dd, J = 7.5, 1.5 Hz), 7.13 (1H, dd, J = 8.5, 2.4 Hz), 7.20 - 7.30 (1H, m), 7.38 - 7.49 (3H, m), 7.61 - 7.67 (1H, m), 7.67 - 7.73 (1H, m), 7.90 (1H, t, J = 1.6 Hz).

### Example 6

### N-(2-cyanoethyl)-3-[1-(3,4-dichlorophenyl)-2,3-dihydro-1H-indol-6-yl]benzamide

In the same manner as in Example 3 and using 3-[1-(3,4-dichlorophenyl)-2,3-dihydro-1H-indol-6-yl]benzoic acid obtained in Reference Example 10 and 3-aminopropanenitrile, the title compound was obtained. Yield 74%, melting point 166 - 196°C (ethyl acetate-hexane).
¹H-NMR (CDCl₃) δ : 2.77 (2H, t, J = 6.2 Hz), 3.19 (2H, t, J = 8.3 Hz), 3.74 (2H, q, J = 6.2 Hz), 3.99 (2H, t, J = 8.3 Hz), 6.62 (1H, brs), 7.02 (1H, dd, J = 7.6, 1.5 Hz), 7.17 (1H, dd, J = 8.9, 2.8 Hz), 7.23 - 7.30 (3H, m), 7.39 (1H, d, J = 8.7 Hz), 7.50 (1H, t, J = 7.6 Hz), 7.70 (2H, t, J = 7.2 Hz), 7.94 (1H, s).

### Example 7

### 3-[1-(3,4-dichlorophenyl)-2,3-dihydro-1H-indol-6-yl]-N-(2-hydroxyethyl)benzamide

To a solution of 3-[1-(3,4-dichlorophenyl)-2,3-dihydro-1H-indol-6-yl]benzoic acid (42.0 mg, 0.11 mmol) obtained in Reference Example 10 and DMTMM (38.3 mg, 0.13 mmol) in methanol (1 ml) was added 2-aminoethanol (5.46 µL, 0.13 mmol), and the mixture was stirred at room temperature for 16 hr. The reaction mixture was concentrated under reduced pressure, saturated aqueous sodium hydrogen carbonate solution was added, and the mixture was extracted with ethyl acetate. The organic layer was dried over anhydrous magnesium sulfate, and concentrated under reduced pressure. The residue was purified by silica gel column chromatography (hexane-ethyl acetate 90:10→0:100) and recrystallized from ethyl acetate-hexane to give the title compound (23 mg, yield 49%) as crystals. Melting point 167 - 168°C.
¹H-NMR (CDCl₃) δ : 2.47 (1H, t, J = 5.1 Hz), 3.19 (2H, t, J = 8.3 Hz), 3.66 (2H, q, J = 5.0 Hz), 3.86 (2H, q, J = 5.0 Hz), 3.98 (2H, t, J = 8.3 Hz), 6.64 (1H, brs), 7.02 (1H, d, J = 8.7 Hz), 7.16 (1H, dd, J = 8.7, 2.7 Hz), 7.23 - 7.30 (3H, m), 7.39 (1H, d, J = 9.1 Hz), 7.49 (1H, t, J = 7.8 Hz), 7.67 (1H, d, J = 8.0 Hz), 7.73 (1H, d, J = 7.6 Hz), 7.94 (1H, s).

### Example 8

### N-(2-cyanoethyl)-3-[1-(3,5-dichlorophenyl)-2,3-dihydro-1H-indol-6-yl]benzamide

In the same manner as in Example 3 and using 3-[1-(3,5-dichlorophenyl)-2,3-dihydro-1H-indol-6-yl]benzoic acid obtained in Reference Example 12 and 3-aminopropanenitrile, the title compound was obtained. Yield 60%, melting point 196 - 197°C (ethyl acetate-hexane).
¹H-NMR (CDCl₃) δ : 2.77 (2H, t, J = 6.3 Hz), 3.19 (2H, t, J = 8.4 Hz), 3.75 (2H, q, J = 6.3 Hz), 4.00 (2H, t, J = 8.4 Hz), 6.63 (1H, brs), 6.94 (1H, t, J = 1.8 Hz), 7.05 (1H, dd, J = 7.5, 1.5 Hz), 7.12 (2H, d, J = 1.7 Hz), 7.22 - 7.29 (1H, m), 7.31 (1H, d, J = 1.3 Hz), 7.52 (1H, t, J = 7.7 Hz), 7.66 - 7.76 (2H, m), 7.94 (1H, t, J = 1.7 Hz).

### Example 9

### 3-[1-(3,5-dichlorophenyl)-2,3-dihydro-1H-indol-6-yl]-N-(2-hydroxyethyl)benzamide

In the same manner as in Example 7 and using 3-[1-(3,5-dichlorophenyl)-2,3-dihydro-1H-indol-6-yl]benzoic acid obtained in Reference Example 12 and 2-aminoethanol, the title compound was obtained. Yield 29%, melting point 108 - 109°C (ethyl acetate-hexane).
¹H-NMR (CDCl₃) δ : 2.49 (1H, brs), 3.18 (2H, t, J = 8.2 Hz), 3.62 - 3.71 (2H, m), 3.81 - 3.91 (2H, m), 3.99 (2H, t, J = 8.4 Hz), 6.65 (1H, brs), 6.93 (1H, t, J = 1.8 Hz), 7.05 (1H, dd, J = 7.5, 1.5 Hz), 7.12 (2H, d, J = 1.9 Hz), 7.23 - 7.29 (1H, m), 7.31 (1H, d, J = 1.1 Hz), 7.50 (1H, t, J = 7.7 Hz), 7.65 - 7.71 (1H, m), 7.72 - 7.79 (1H, m), 7.94 (1H, t, J = 1.6 Hz).

### Example 10

### N-(2-cyanoethyl)-3-[1-(2,3-dichlorophenyl)-2,3-dihydro-1H-indol-6-yl]benzamide

In the same manner as in Example 3 and using 3-[1-(2,3-dichlorophenyl)-2,3-dihydro-1H-indol-6-yl]benzoic acid obtained in Reference Example 14 and 3-aminopropanenitrile, the title compound was obtained. Yield 84%, melting point 155 - 156°C (ethyl acetate-hexane).
¹H-NMR (CDCl₃) δ : 2.75 (2H, t, J = 6.2 Hz), 3.23 (2H, t, J = 8.3 Hz), 3.72 (2H, q, J = 6.4 Hz), 3.99 (2H, brs), 6.59 (1H, brs), 6.61 (1H, d, J = 1.1 Hz), 6.99 (1H, dd, J = 7.6, 1.5 Hz), 7.18 - 7.28 (2H, m), 7.32 - 7.37 (1H, m), 7.37 - 7.41 (1H, m), 7.45 (1H, t, J = 7.6 Hz), 7.66 (2H, t, J = 6.6 Hz), 7.88 (1H, s).

### Example 11

### 3-[1-(2,3-dichlorophenyl)-2,3-dihydro-1H-indol-6-yl]-N-(2-hydroxyethyl)benzamide

In the same manner as in Example 7 and using 3-[1-(2,3-dichlorophenyl)-2,3-dihydro-1H-indol-6-yl]benzoic acid obtained in Reference Example 14 and 2-aminoethanol, the title compound was obtained. Yield 54%, melting point 162 - 163°C (ethyl acetate-hexane).
¹H-NMR (CDCl₃) δ : 2.52 (1H, t, J = 5.1 Hz), 3.23 (2H, t, J = 8.3 Hz), 3.59 - 3.69 (2H, m), 3.84 (2H, q, J = 5.2 Hz), 3.94 (2H, brs), 6.61 (2H, d, J = 1.5 Hz), 7.17 - 7.28 (3H, m), 7.34 (1H, dd, J = 8.3, 1.5 Hz), 7.36 - 7.40 (1H, m), 7.43 (1H, t, J = 7.8 Hz), 7.62 (1H, d, J = 8.3 Hz), 7.68 (1H, d, J = 8.0 Hz), 7.88 (1H, s).

### Example 12

### 3-[1-(2,4-dichlorobenzyl)-2,3-dihydro-H-indol-6-yl]-N-[2-(dimethylamino)ethyl]benzamide

In the same manner as in Example 1 and using 3-[1-(2,4-dichlorobenzyl)-2,3-dihydro-1H-indol-6-yl]benzoic acid obtained in Reference Example 16 and N,N-dimethylethane-1,2-diamine, the title compound was obtained. Yield 65%, melting point 122 - 123°C (ethyl acetate-hexane).
¹H-NMR (DMSO-d₆) δ : 2.18 (6H, s), 2.41 (2H, t, J = 6.8 Hz), 2.98 (2H, t, J = 8.1 Hz), 3.33 - 3.42 (4H, m), 4.44 (2H, s), 6.86 (1H, s), 6.96 (1H, d, J = 7.6 Hz), 7.17 (1H, d, J = 7.6 Hz), 7.40 - 7.46 (1H, m), 7.46 - 7.53 (2H, m), 7.66 (1H, d, J = 1.9 Hz), 7.74 (2H, t, J = 8.7 Hz), 8.01 (1H, s), 8.46 (1H, t, J = 5.3 Hz).

### Example 13

### N-(2-cyanoethyl)-3-[1-(2,4-dichlorobenzyl)-2,3-dihydro-1H-indol-6-yl]benzamide

In the same manner as in Example 1 and using 3-[1-(2,4-dichlorobenzyl)-2,3-dihydro-1H-indol-6-yl]benzoic acid obtained in Reference Example 16 and 3-aminopropanenitrile, the title compound was obtained. Yield 73%, melting point 148 - 149°C (ethyl acetate-hexane).
¹H-NMR (DMSO-d₆) δ : 2.79 (2H, t, J = 6.4 Hz), 2.99 (2H, t, J = 8.3 Hz), 3.38 (2H, t, J = 8.3 Hz), 3.51 (2H, q, J = 6.4 Hz), 4.45 (2H, s), 6.87 (1H, s), 6.96 (1H, d, J = 7.6 Hz), 7.18 (1H, d, J = 7.6 Hz), 7.41 - 7.46 (1H, m), 7.48 - 7.56 (2H, m), 7.66 (1H, d, J = 2.3 Hz), 7.77 (2H, t, J = 7.6 Hz), 8.04 (1H, s), 8.91 (1H, t, J = 5.7 Hz).

### Example 14

### N-(2-cyanoethyl)-3-[1-(3,4-dichlorobenzyl)-2,3-dihydro-1H-indol-6-yl]benzamide

In the same manner as in Example 3 and using 3-[1-(3,4-dichlorobenzyl)-2,3-dihydro-1H-indol-6-yl]benzoic acid obtained in Reference Example 18 and 3-aminopropanenitrile, the title compound was obtained. Yield 55%, melting point 135 - 136°C (ethyl acetate-hexane).
¹H-NMR (CDCl₃) δ : 2.77 (2H, t, J = 6.2 Hz), 3.05 (2H, t, J = 8.1 Hz), 3.38 (2H, t, J = 8.3 Hz), 3.74 (2H, q, J = 6.1 Hz), 4.27 (2H, s), 6.59 (1H, t, J = 5.9 Hz), 6.65 (1H, s), 6.93 (1H, dd, J = 7.4, 1.3 Hz), 7.18 (1H, d, J = 7.6 Hz), 7.22 (1H, dd, J = 8.3, 1.9 Hz), 7.42 (1H, d, J = 8.0 Hz), 7.44 - 7.51 (2H, m), 7.69 (2H, dd, J = 8.0, 1.5 Hz), 7.93 (1H, s).

### Example 15

### N-(2-cyanoethyl)-3-[1-(3,5-dichLorobenzyl)-2,3-dihydro-1H-indol-6-yl]benzamide

In the same manner as in Example 3 and using 3-[1-(3,5-dichlorobenzyl)-2,3-dihydro-1H-indol-6-yl]benzoic acid obtained in Reference Example 20 and 3-aminopropanenitrile, the title compound was obtained. Yield 44%, melting point 179 - 180°C (ethyl acetate-hexane).
¹H-NMR (CDCl₃) δ : 2.77 (2H, t, J = 6.2 Hz), 3.06 (2H, t, J = 8.1 Hz), 3.40 (2H, t, J = 8.3 Hz), 3.74 (2H, q, J = 6.2 Hz), 4.27 (2H, s), 6.58 (1H, brs), 6.64 (1H, s), 6.94 (1H, d, J = 6.4 Hz), 7.19 (1H, d, J = 7.6 Hz), 7.28 (3H, s), 7.48 (1H, t, J = 7.8 Hz), 7.69 (2H, d, J = 7.6 Hz), 7.94 (1H, s).

### Example 16

### 3-[1-(3,5-dichlorobenzyl)-2,3-dihydro-1H-indol-6-yl]-N-(2-hydroxyethyl)benzamide

In the same manner as in Example 7 and using 3-[1-(3,5-dichlorobenzyl)-2,3-dihydro-1H-indol-6-yl]benzoic acid obtained in Reference Example 20 and 2-aminoethanol, the title compound was obtained. Yield 57%, melting point 121 - 128°C (ethyl acetate-hexane).
¹H-NMR (CDCl₃) δ : 2.51 (1H, t, J = 4.9 Hz), 3.06 (2H, t, J = 8.3 Hz), 3.39 (2H, t, J = 8.3 Hz), 3.60 - 3.71 (2H, m), 3.85 (2H, q, J = 4.8 Hz), 4.27 (2H, s), 6.64 (2H, s), 6.93 (1H, dd, J = 7.6, 1.5 Hz), 7.18 (1H, d, J = 7.6 Hz), 7.28 (3H, s), 7.46 (1H, t, J = 7.8 Hz), 7.64 - 7.73 (2H, m), 7.94 (1H, s).

### Example 17

### N-(2-cyanoethyl)-3-[1-(2,3-dichlorobenzyl)-2,3-dihydro-1H-indol-6-yl]benzamide

In the same manner as in Example 3 and using 3-[1-(2,3-dichlorobenzyl)-2,3-dihydro-1H-indol-6-yl]benzoic acid obtained in Reference Example 22 and 3-aminopropanenitrile, the title compound was obtained. Yield 87%, melting point 160 - 161 °C (ethyl acetate-hexane).
¹H-NMR (CDCl₃) δ : 2.76 (2H, t, J = 6.2 Hz), 3.09 (2H, t, J = 8.5 Hz), 3.51 (2H, t, J = 8.3 Hz), 3.73 (2H, q, J = 6.3 Hz), 4.44 (2H, s), 6.59 (2H, s), 6.87 - 6.96 (1H, m), 7.14 - 7.24 (2H, m), 7.39 (2H, t, J = 6.6 Hz), 7.43 - 7.50 (1H, m), 7.67 (2H, dd, J = 7.6, 1.9 Hz), 7.92 (1H, s).

### Example 18

### 3-[1-(2,3-dichlorobenzyl)-2,3-dihydro-1H-indol-6-yl]-N-(2-hydroxyethyl)benzamide

In the same manner as in Example 7 and using 3-[1-(2,3-dichlorobenzyl)-2,3-dihydro-1H-indol-6-yl]benzoic acid obtained in Reference Example 22 and 2-aminoethanol, the title compound was obtained. Yield 59%, melting point 158 - 159°C (ethyl acetate-hexane).
¹H-NMR (CDCl₃) δ : 2.45 (1H, t, J = 5.1 Hz), 3.09 (2H, t, J = 8.3 Hz), 3.51 (2H, t, J = 8.3 Hz), 3.60 - 3.70 (2H, m), 3.85 (2H, q, J = 4.9 Hz), 4.44 (2H, s), 6.59 (2H, s), 6.92 (1H, dd, J = 7.4, 1.7 Hz), 7.14 - 7.22 (2H, m), 7.36 - 7.48 (3H, m), 7.62 - 7.71 (2H, m), 7.93 (1H, s).

### Example 19

### N-(2-cyanoethyl)-3-[1-(2,5-dichlorobenzyl)-2,3-dihydro-1H-indol-6-yl] benzamide

In the same manner as in Example 3 and using 3-[1-(2,5-dichlorobenzyl)-2,3-dihydro-1H-indol-6-yl]benzoic acid obtained in Reference Example 24 and 3-aminopropanenitrile, the title compound was obtained. Yield 71 %, melting point 152 - 153°C (ethyl acetate-hexane).
¹H-NMR (CDCl₃) δ : 2.76 (2H, t, J = 6.2 Hz), 3.10 (2H, t, J = 8.3 Hz), 3.50 (2H, t, J = 8.3 Hz), 3.74 (2H, q, J = 6.1 Hz), 4.37 (2H, s), 6.58 (1H, brs), 6.61 (1H, s), 6.93 (1H, dd, J = 7.4, 1.3 Hz), 7.17 - 7.24 (2H, m), 7.31 - 7.36 (1H, m), 7.44 - 7.51 (2H, m), 7.69 (2H, dd, J = 7.8, 1.7 Hz), 7.93 (1H, s).

### Example 20

### 3-[1-(2,5-dichlorobenzyl)-2,3-dihydro-1H-indol-6-yl]-N-(2-hydroxyethyl)benzamide

In the same manner as in Example 7 and using 3-[1-(2,5-dichlorobenzyl)-2,3-dihydro-1H-indol-6-yl]benzoic acid obtained in Reference Example 24 and 2-aminoethanol, the title compound was obtained. Yield 73%, melting point 149 - 150°C (ethyl acetate-hexane).
¹H-NMR (CDCl₃) δ : 2.46 (1H, brs), 3.10 (2H, t, J = 8.3 Hz), 3.50 (2H, t, J = 8.3 Hz), 3.60 - 3.74 (2H, m), 3.85 (2H, t, J = 4.7 Hz), 4.37 (2H, s), 6.61 (2H, d, J = 1.5 Hz), 6.90 - 6.97 (1H, m), 7.18 - 7.23 (2H, m), 7.30 - 7.36 (1H, m), 7.42 - 7.50 (2H, m), 7.68 (2H, dd, J = 12.1, 8.0 Hz), 7.93 (1H, s).

### Example 21

### N-(2-cyanoethyl)-3-[1-(2,4-dichlorophenyl)-1H-indol-6-yl]benzamide

In the same manner as in Example 1 and using 3-[1-(2,4-dichlorophenyl)-1H-indol-6-yl]benzoic acid obtained in Reference Example 26 and 3-aminopropanenitrile, the title compound was obtained as an amorphous solid. Yield 75%.
¹H-NMR (CDCl₃) δ : 2.76 (2H, t, J = 6.2 Hz), 3.73 (2H, q, J = 6.3 Hz), 6.63 (1H, t, J = 5.7 Hz), 6.74 (1H, dd, J = 3.2, 0.8 Hz), 7.25 (1H, d, J = 3.2 Hz), 7.30 (1H, s), 7.40 - 7.53 (4H, m), 7.61 - 7.69 (2H, m), 7.72 - 7.80 (2H, m), 8.00 (1H, t, J = 1.6 Hz).

### Example 22

### 3-[1-(2,4-dichlorophenyl)-1H-indol-6-yl]-N-(2-hydroxyethyl)benzamide

In the same manner as in Example 7 and using 3-[1-(2,4-dichlorophenyl)-1H-indol-6-yl]benzoic acid obtained in Reference Example 26 and 2-aminoethanol, the title compound was obtained. Yield 41%, melting point 163 - 164°C (ethyl acetate).
¹H-NMR (CDCl₃) δ : 2.52 (1H, t, J = 5.1 Hz), 3.60 - 3.70 (2H, m), 3.85 (2H, q, J = 4.9 Hz), 6.65 (1H, brs), 6.73 (1H, dd, J = 3.0, 0.8 Hz), 7.24 (1H, d, J = 3.4 Hz), 7.30 (1H, s), 7.41 - 7.49 (4H, m), 7.64 (1H, t, J = 1.3 Hz), 7.66 (1H, d, J = 8.0 Hz), 7.70 - 7.78 (2H, m), 8.00 (1H, t, J = 1.7 Hz).

### Example 23

### N-(2-cyanoethyl)-3-[1-(2,4-dichlorobenzyl)-1H-indol-6-yl]benzamide

In the same manner as in Example 1 and using 3-[1-(2,4-dichlorobenzyl)-1H-indol-6-yl]benzoic acid obtained in Reference Example 28 and 3-aminopropanenitrile, the title compound was obtained. Yield 89%, melting point 153 - 154°C (ethyl acetate-hexane).
¹H-NMR (CDCl₃) δ : 2.77 (2H, t, J = 6.3 Hz), 3.74 (2H, q, J = 6.3 Hz), 5.44 (2H, s), 6.52 (1H, d, J = 8.3 Hz), 6.59 - 6.69 (2H, m), 7.07 (1H, dd, J = 8.4, 2.2 Hz), 7.16 (1H, d, J = 3.2 Hz), 7.39 - 7.45 (3H, m), 7.50 (1H, t, J = 7.7 Hz), 7.64 - 7.70 (1H, m), 7.71 - 7.80 (2H, m), 8.03 (1H, t, J = 1.6 Hz).

### Example 24

### 3-[1-(2,4-dichlorobenzyl)-1H-indol-6-yl]-N-(2-hydroxyethyl)benzamide

In the same manner as in Example 7 and using 3-[1-(2,4-dichlorobenzyl)-1H-indol-6-yl]benzoic acid obtained in Reference Example 28 and 2-aminoethanol, the title compound was obtained. Yield 46%, melting point 124 - 125°C (ethyl acetate-hexane).
¹H-NMR (CDCl₃) δ : 2.51 (1H, t, J = 4.7 Hz), 3.61 - 3.71 (2H, m), 3.86 (2H, q, J = 4.5 Hz), 5.43 (2H, s), 6.51 (1H, d, J = 8.3 Hz), 6.63 (1H, d, J = 3.0 Hz), 6.65 (1H, brs), 7.07 (1H, dd, J = 8.5, 2.1 Hz), 7.15 (1H, d, J = 3.0 Hz), 7.36 - 7.46 (3H, m), 7.46 - 7.52 (1H, m), 7.68 (1H, d, J = 8.0 Hz), 7.74 (2H, d, J = 8.0 Hz), 8.03 (1 H, s).

### Example 25

### 3-[1-[2-(3,4-dimethoxyphenyl)ethyl]-1H-pyrrolo[2,3-b]pyridin-6-yl]-N-[2-(dimethylamino)ethyl]benzamide

In the same manner as in Example 1 and using 3-[1-[2-(3,4-dimethoxyphenyl)ethyl]-1H-pyrrolo[2,3-b]pyridin-6-yl]benzoic acid obtained in Reference Example 31 and N,N-dimethylethylenediamine, the title compound was obtained. Yield 59%, melting point 97 - 98°C (ethyl acetate-hexane).
¹H NMR (DMSO-d₆) δ: 2.39 - 2.48 (2H, m), 3.13 (2H, t, J = 7.1 Hz), 3.41 (2H, q, J = 6.4 Hz), 3.62 (3H, s), 3.66 (3H, s), 4.57 (2H, t, J = 7.1 Hz), 6.46 (1H, d, J = 3.4 Hz), 6.66 - 6.76 (2H, m), 6.77 - 6.85 (1H, m), 7.50 (1H, d, J = 3.4 Hz), 7.58 (1H, t, J = 7.8 Hz), 7.75 (1H, d, J = 8.3 Hz), 7.85 (1H, d, J = 7.8 Hz), 8.06 (1H, d, J = 8.3 Hz), 8.30 (1H, d, J = 7.8 Hz), 8.47 - 8.62 (2H, m).

### Example 26

### N-(2-cyanoethyl)-3-[1-[2-(3,4-dimethoxyphenyl)ethyl]-1H-pyrrolo[2,3-b]pyridin-6-yl]benzamide

In the same manner as in Example 1 and using 3-[1-[2-(3,4-dimethoxyphenyl)ethyl]-1H-pyrrolo[2,3-b]pyridin-6-yl]benzoic acid obtained in Reference Example 31 and 3-aminopropanenitrile, the title compound was obtained. Yield 76%, melting point 138 -139°C (ethyl acetate-hexane).
¹H-NMR (DMSO-d₆) δ: 2.83 (2H, t, J = 6.4 Hz), 3.13 (2H, t, J = 7.0 Hz), 3.56 (2H, q, J = 6.4 Hz), 3.62 (3H, s), 3.66 (3H, s), 4.58 (2H, t, J = 7.0 Hz), 6.46 (1H, d, J = 3.4 Hz), 6.68 - 6.76 (2H, m), 6.77 - 6.84 (1H, m), 7.50 (1H, d, J = 3.4 Hz), 7.61 (1H, t, J = 7.7 Hz), 7.75 (1H, d, J = 8.3 Hz), 7.87 (1H, d, J = 7.7 Hz), 8.06 (1H, d, J = 8.3 Hz), 8.33 (1H, d, J = 7.9 Hz), 8.61 (1H, s), 9.00 (1H, t, J = 5.6 Hz).

### Example 27

### N-(2-cyanoethyl)-3-[1-(2,4-dichlorobenzyl)-1H-pyrrolo[2,3-b]pyridin-6-yl]benzamide

In the same manner as in Example 1 and using 3-[1-(2,4-dichlorobenzyl)-1H-pyrrolo[2,3-b]pyridin-6-yl]benzoic acid obtained in Reference Example 33 and 3-aminopropanenitrile, the title compound was obtained. Yield 72%, melting point 186 -187°C (ethyl acetate-hexane).
¹H-NMR (DMSO-d₆) δ: 2.82 (2H, t, J = 6.4 Hz), 3.55 (2H, q, J = 6.4 Hz), 5.65 (2H, s), 6.60 (1H, d, J = 3.6 Hz), 7.05 (1H, d, J = 8.5 Hz), 7.38 (1H, dd, J = 8.5, 2.2 Hz), 7.59 (1H, t, J = 7.7 Hz), 7.64 - 7.70 (2H, m), 7.77 - 7.89 (2H, m), 8.14 (1H, d, J = 8.5 Hz), 8.26 - 8.32 (1H, m), 8.57 (1H, s), 8.98 (1H, t, J = 5.6 Hz).

### Example 28

### 3-[1-(2,4-dichlorobenzyl)-1H-pyrrolo[2,3-b]pyridin-6-yl]-N-(2-hydroxyethyl)benzamide

In the same manner as in Example 1 and using 3-[1-(2,4-dichlorobenzyl)-1H-pyrrolo[2,3-b]pyridin-6-yl]benzoic acid obtained in Reference Example 33 and 2-aminoethanol, the title compound was obtained. Yield 99%, melting point 161 - 162°C (ethyl acetate-hexane).
¹H-NMR (DMSO-d₆) δ: 3.38 (2H, q, J = 6.2 Hz), 3.55 (2H, q, J = 6.2 Hz), 4.75 (1H, t, J = 5.6 Hz), 5.65 (2H, s), 6.60 (1H, d, J = 3.4 Hz), 7.05 (1H, d, J = 8.3 Hz), 7.38 (1H, dd, J = 8.3, 2.3 Hz), 7.56 (1H, t, J = 7.8 Hz), 7.63 - 7.70 (2H, m), 7.78 - 7.89 (2H, m), 8.13 (1H, d, J = 8.3 Hz), 8.26 (1H, d, J = 7.7 Hz), 8.52 - 8.62 (2H, m).

### Example 29

### 3-[1-(2,4-dichlorobenzyl)-2,3-dihydro-1H-pyrrolo[2,3-b]pyridin-6-yl]-N-(2-hydroxyethyl)benzamide

In the same manner as in Example 1 and using 3-[1-(2,4-dichlorobenzyl)-2,3-dihydro-1H-pyrrolo[2,3-b]pyridin-6-yl]benzoic acid obtained in Reference Example 37 and 2-aminoethanol, the title compound was obtained. Yield 54%, melting point 142 -143°C (ethyl acetate-hexane).
¹H-NMR (DMSO-d₆) δ: 3.03 (2H, t, J = 8.1 Hz), 3.30 - 3.40 (2H, m), 3.47 - 3.57 (4H, m), 4.68 (2H, s), 4.73 (1H, t, J = 5.7 Hz), 7.18 (1H, d, J = 7.6 Hz), 7.38 - 7.55 (4H, m), 7.65 (1H, d, J = 2.3 Hz), 7.77 - 7.84 (1H, m), 8.10 - 8.16 (1H, m), 8.39 - 8.44 (1H, m), 8.52 (1H, t, J = 5.7 Hz).

### Example 30

### N-(2-cyanoethyl)-3-[1-(2,4-dichlorobenzyl)-2,3-dihydro-1H-pyrrolo[2,3-b]pyridin-6-yl]benzamide

A mixture of 3-[1-(2,4-dichlorobenzyl)-2,3-dihydro-1H-pyrrolo[2,3-b]pyridin-6-yl]benzoic acid (100 mg, 0.250 mmol) obtained in Reference Example 37, 3-aminopropanenitrile (18.3 µl, 0.276 mmol) and DMTMM (88.6 mg, 0.301 mmol) in methanol (2.5 ml) was stirred at room temperature for 6 hr. Water was poured into the reaction mixture and the mixture was extracted with ethyl acetate. The extract was washed with water, and dried over anhydrous magnesium sulfate, and the solvent was evaporated under reduced pressure. The residue was purified by basic silica gel column chromatography (ethyl acetate:hexane = 1:1) and recrystallized from hexane and ethyl acetate to give the title compound (81.6 mg, yield 72%) as a solid. Melting point 156 -157°C
¹H-NMR (DMSO-d₆) δ: 2.80 (2H, t, J = 6.5 Hz), 3.03 (2H, q, J = 8.3 Hz), 3.45 - 3.58 (4H, m), 4.69 (2H, s), 7.17 (1H, d, J = 7.3 Hz), 7.38 - 7.47 (2H, m), 7.49 - 7.57 (2H, m), 7.66 (1H, d, J = 1.9 Hz), 7.82 (1H, d, J = 7.4 Hz), 8.16 (1H, d, J = 7.4 Hz), 8.43 (1H, s), 8.93 (1H, t, J = 5.6 Hz).

### Example 31

### 3-[1-(2,4-dichlorobenzyl)-2,3-dihydro-1H-pyrrolo[2,3-b]pyridin-6-yl]-N-(3-hydroxypropyl)benzamide

In the same manner as in Example 30 and using 3-[1-(2,4-dichlorobenzyl)-2,3-dihydro-1H-pyrrolo[2,3-b]pyridin-6-yl]benzoic acid obtained in Reference Example 37 and 3-aminopropan-1-ol, the title compound was obtained as a solid. Yield 52%, melting point 145 -146°C (tetrahydrofuran-hexane).
¹H-NMR (DMSO-d₆) δ: 1.61 - 1.77 (2H, m), 3.03 (2H, t, J = 8.2 Hz), 3.29 - 3.38 (2H, m), 3.43 - 3.56 (4H, m), 4.48 (1H, t, J = 5.2 Hz), 4.68 (2H, s), 7.17 (1H, d, J = 7.3 Hz), 7.39 - 7.55 (4H, m), 7.66 (1H, d, J = 2.1 Hz), 7.75 - 7.83 (1H, m), 8.03 - 8.15 (1H, m), 8.37 - 8.43 (1H, m), 8.53 (1H, t, J = 5.2 Hz).

### Example 32

### N-(2-cyanoethyl)-3-[1-(2,4-dichlorophenyl)-1H-pyrrolo[2,3-b]pyridin-6-yl]benzamide

In the same manner as in Example 1 and using 3-[1-(2,4-dichlorophenyl)-1H-pyrrolo[2,3-b]pyridin-6-yl]benzoic acid obtained in Reference Example 39 and 3-aminopropanenitrile, the title compound was obtained. Yield 75%, melting point 215 -216°C (tetrahydrofuran-hexane).
¹H-NMR (DMSO-d₆) δ: 2.80 (2H, t, J = 6.4 Hz), 3.53 (2H, q, J = 6.4 Hz), 6.78 (1H, d, J = 3.6 Hz), 7.56 (1H, t, J = 7.7 Hz), 7.63 - 7.70 (1H, m), 7.71 - 7.77 (2H, m), 7.80 - 7.90 (2H, m), 7.96 (1H, d, J = 2.3 Hz), 8.11 - 8.25 (2H, m), 8.45 (1H, s), 8.96 (1H, t, J = 5.5 Hz).

### Example 33

### 3-[1-(2,4-dichlorophenyl)-1H-pyrrolo[2,3-b]pyridin-6-yl]-N-(2-hydroxyethyl)benzamide

In the same manner as in Example 1 and using 3-[1-(2,4-dichlorophenyl)-1H-pyrrolo[2,3-b]pyridin-6-yl]benzoic acid obtained in Reference Example 39 and 2-aminoethanol, the title compound was obtained. Yield 58%, melting point 159 - 160°C (tetrahydrofuran-hexane).
¹H-NMR (DMSO-d₆) δ: 3.32 - 3.40 (2H, m), 3.54 (2H, q, J = 6.0 Hz), 4.74 (1H, t, J = 6.0 Hz), 6.78 (1H, d, J = 3.6 Hz), 7.53 (1H, t, J = 7.7 Hz), 7.64 - 7.69 (1H, m), 7.71 - 7.77 (2H, m), 7.81 - 7.91 (2H, m), 7.96 (1H, d, J = 2.3 Hz), 8.10 - 8.16 (1H, m), 8.21 (1H, d, J = 7.7 Hz), 8.45 (1H, s), 8.56 (1H, t, J = 5.6 Hz).

### Example 34

### N-(2-cyanoethyl)-3-[1-(2,4-dichlorophenyl)-2,3-dihydro-1H-pyrrolo[2,3-b]pyridin-6-yl]benzamide

In the same manner as in Example 1 and using 3-[1-(2,4-dichlorophenyl)-2,3-dihydro-1H-pyrrolo[2,3-b]pyridin-6-yl]benzoic acid obtained in Reference Example 41 and 3-aminopropanenitrile, the title compound was obtained. Yield 36%, melting point 219 - 220°C (tetrahydrofuran-hexane).
¹H-NMR (DMSO-d₆) δ: 2.79 (2H, t, J = 6.4 Hz), 3.20 (2H, t, J = 8.3 Hz), 3.44 - 3.56 (2H, m), 4.01 (2H, t, J = 8.3 Hz), 7.30 (1H, d, J = 7.5 Hz), 7.45 - 7.55 (2H, m), 7.56 - 7.68 (2H, m), 7.75 (1H, d, J = 2.3 Hz), 7.79 (1H, d, J = 7.7 Hz), 8.02 (1H, d, J = 7.7 Hz), 8.31 (1H, s), 8.91 (1H, t, J = 5.3 Hz).

### Example 35

### 3-[1-(2,4-dichlorophenyl)-2,3-dihydro-1H-pyrrolo[2,3-b]pyridin-6-yl]-N-(2-hydroxyethyl)benzamide

In the same manner as in Example 1 and using 3-[1-(2,4-dichlorophenyl)-2,3-dihydro-1H-pyrrolo[2,3-b]pyridin-6-yl]benzoic acid obtained in Reference Example 41 and 2-aminoethanol, the title compound was obtained. Yield 59%, melting point 168 -169°C (tetrahydrofuran-hexane).
¹H-NMR (DMSO-d₆) δ: 3.20 (2H, t, J = 8.2 Hz), 3.29 - 3.39 (2H, m), 3.52 (2H, q, J = 5.8 Hz), 4.01 (2H, t, J = 8.2 Hz), 4.73 (1H, t, J = 5.8 Hz), 7.32 (1H, d, J = 7.3 Hz), 7.42 - 7.53 (2H, m), 7.58 (1H, d, J = 7.4 Hz), 7.65 (1H, d, J = 8.7 Hz), 7.72 - 7.82 (2H, m), 7.99 (1H, d, J = 7.4 Hz), 8.31 (1H, s), 8.50 (1H, t, J = 5.8 Hz).

### Example 36

### 3-[1-(3,5-dichloropyridin-2-yl)-1H-pyrrolo[2,3-b]pyridin-6-yl]-N-(2-hydroxyethyl)benzamide

In the same manner as in Example 1 and using 3-[1-(3,5-dichloropyridin-2-yl)-1H-pyrrolo[2,3-b]pyridin-6-yl]benzoic acid obtained in Reference Example 43 and 2-aminoethanol, the title compound was obtained. Yield 75%, melting point 196-197°C (tetrahydrofuran-hexane)
¹H-NMR (DMSO-d₆) δ: 3.27 - 3.43 (2H, m), 3.47 - 3.63 (2H, m), 4.75 (1H, t, J = 5.5 Hz), 6.82 (1H, d, J = 3.4 Hz), 7.53 (1H, t, J = 7.4 Hz), 7.76 - 7.97 (3H, m), 8.11 - 8.28 (2H, m), 8.47 (1H, brs), 8.56 (1H, brs), 8.64 (1H, brs), 8.73 (1H, s).

### Example 37

### N-(2-cyanoethyl)-3-[1-(2,4-dichlorophenyl)-1H-pyrazolo [3,4-b]pyridin-6-yl]benzamide

In the same manner as in Example 1 and using 3-[1-(2,4-dichlorophenyl)-1H-pyrazolo[3,4-b]pyridin-6-yl]benzoic acid obtained in Reference Example 45 and 3-aminopropanenitrile, the title compound was obtained. Yield 74%, melting point 158 -159°C (tetrahydrofuran-hexane)
¹H-NMR (DMSO-d₆) δ: 2.81 (2H, t, J = 6.5 Hz), 3.53 (2H, q, J = 6.5 Hz), 7.62 (1H, t, J = 7.7 Hz), 7.67 - 7.73 (1H, m), 7.75 - 7.82 (1H, m), 7.94 (1H, t, J = 7.7 Hz), 7.98 - 8.04 (2H, m), 8.25 (1H, d, J = 8.3 Hz), 8.47 - 8.55 (3H, m), 9.00 (1H, t, J = 5.5 Hz).

### Example 38

### 3-[1-(2,4-dichlorophenyl)-1H-pyrazolo[3,4-b]pyridin-6-yl]-N-(2-hydroxyethyl)benzamide

In the same manner as in Example 1 and using 3-[1-(2,4-dichlorophenyl)-1H-pyrazolo[3,4-b]pyridin-6-yl]benzoic acid obtained in Reference Example 45 and 2-aminoethanol, the title compound was obtained. Yield 63%, melting point 169-170°C (tetrahydrofuran-hexane).
¹H-NMR (DMSO-d₆) δ: 3.32 - 3.41 (2H, m), 3.54 (2H, q, J = 6.0 Hz), 4.75 (1H, t, J = 6.0 Hz), 7.59 (1H, t, J = 7.5 Hz), 7.67 - 7.73 (1H, m), 7.75 - 7.82 (1H, m), 7.93 (1H, d, J = 7.5 Hz), 7.98 - 8.06 (2H, m), 8.21 (1H, d, J = 7.5 Hz), 8.46 - 8.55 (3H, m), 8.61 (1H, t, J = 5.6 Hz).

### Example 39

### 3-[1-(2,4-dichlorophenyl)-1H-indazol-6-yl]-N-(2-hydroxyethyl)benzamide

In the same manner as in Example 1 and using 3-[1-(2,4-dichlorophenyl)-1H-indazol-6-yl]benzoic acid obtained in Reference Example 47 and 2-aminoethanol, the title compound was obtained. Yield 79%, melting point 191 -192°C (tetrahydrofuran-hexane).
¹H-NMR (DMSO-d₆) δ: 3.31 - 3.39 (2H, m), 3.52 (2H, q, J = 5.9 Hz), 4.74 (1H, t, J = 5.9 Hz), 7.49 - 7.58 (2H, m), 7.62 - 7.77 (3H, m), 7.82 - 7.89 (2H, m), 7.96 - 8.04 (2H, m), 8.14 (1H, s), 8.45 (1H, d, J = 0.9 Hz), 8.58 (1H, t, J = 5.4 Hz).

### Example 40

### N-(2-cyanoethyl)-3-[7-(2,4-dichlorobenzyl)-7H-pyrrolo[2,3-d]pyrimidin-2-yl]benzamide

In the same manner as in Example 1 and using 3-[7-(2,4-dichlorobenzyl)-7H-pyrrolo[2,3-d]pyrimidin-2-yl]benzoic acid obtained in Reference Example 50 and 3-aminopropanenitrile, the title compound was obtained. Yield 65%, melting point 198 -199°C (ethyl acetate-hexane).
¹H-NMR (DMSO-d₆) δ: 2.82 (2H, t, J = 6.4 Hz), 3.54 (2H, q, J = 6.4 Hz), 5.66 (2H, s), 6.75 (1H, d, J = 3.6 Hz), 7.15 (1H, d, J = 8.3 Hz), 7.41 (1H, dd, J = 8.3, 2.2 Hz), 7.62 (1H, t, J = 8.3 Hz), 7.69 - 7.74 (2H, m), 7.90 - 7.96 (1H, m), 8.57 - 8.63 (1H, m), 8.95 (1H, t, J = 2.2 Hz), 9.00 (1H, t, J = 6.4 Hz), 9.18 (1H, s).

### Example 41

### 3-[7-(2,4-dichlorobenzyl)-7H-pyrrolo[2,3-d]pyrimidin-2-yl]-N-(2-hydroxyethyl)benzamide

In the same manner as in Example 1 and using 3-[7-(2,4-dichlorobenzyl)-7H-pyrrolo[2,3-d]pyrimidin-2-yl]benzoic acid obtained in Reference Example 50 and 2-aminoethanol, the title compound was obtained. Yield 63%, melting point 194 - 195°C (ethyl acetate-hexane).
¹H-NMR (DMSO-d₆) δ: 3.29 - 3.43 (2H, m), 3.55 (2H, q, J = 6.0 Hz), 4.76 (1H, t, J = 6.0 Hz), 5.66 (2H, s), 6.75 (1H, d, J = 3.6 Hz), 7.15 (1H, d, J = 8.3 Hz), 7.41 (1H, dd, J = 8.3, 2.1 Hz), 7.59 (1H, t, J = 8.3 Hz), 7.68 - 7.74 (2H, m), 7.89 - 7.96 (1H, m), 8.53 - 8.62 (2H, m), 8.93 (1H, t, J = 2.1 Hz), 9.17 (1H, s).

### Example 42

### N-(2-cyanoethyl)-3-[1-(2,4-dichlorobenzyl)-1H-pyrrolo [3,2-c] pyridin-6-yl] benzamide

In the same manner as in Example 1 and using 3-[1-(2,4-dichlorobenzyl)-1H-pyrrolo[3,2-c]pyridin-6-yl]benzoic acid obtained in Reference Example 54 and 3-aminopropanenitrile, the title compound was obtained. Yield 67%, melting point 199 - 200°C (tetrahydrofuran-hexane).
¹H-NMR (DMSO-d₆) δ: 2.81 (2H, t, J = 6.4 Hz), 3.53 (2H, q, J = 6.4 Hz), 5.66 (2H, s), 6.63 - 6.81 (2H, m), 7.35 (1H, dd, J = 8.3, 1.7 Hz), 7.49 - 7.62 (2H, m), 7.72 (1H, d, J = 1.7 Hz), 7.82 (1H, d, J = 8.3 Hz), 8.21 (1H, s), 8.27 (1H, d, J = 7.3 Hz), 8.62 (1H, s), 8.85 - 9.05 (2H, m).

### Example 43

### 3-[1-(2,4-dichlorobenzyl)-1H-pyrrolo[3,2-c]pyridin-6-yl]-N-(2-hydroxyethyl)benzamide

In the same manner as in Example 1 and using 3-[1-(2,4-dichlorobenzyl)-1H-pyrrolo[3,2-c]pyridin-6-yl]benzoic acid obtained in Reference Example 54 and 2-aminoethanol, the title compound was obtained. Yield 44%, melting point 198 - 199°C (tetrahydrofuran-hexane).
¹H-NMR (DMSO-d₆) δ: 3.36 (2H, q, J = 6.2 Hz), 3.54 (2H, q, J = 6.2 Hz), 4.74 (1H, t, J = 6.2 Hz), 5.66 (2H, s), 6.68 (1H, d, J = 8.3 Hz), 6.74 (1H, d, J = 3.0 Hz), 7.35 (1H, dd, J = 8.3, 2.2 Hz), 7.49 - 7.57 (2H, m), 7.71 (1H, d, J = 2.2 Hz), 7.81 (1H, d, J = 8.3 Hz), 8.20 (1H, s), 8.22 - 8.29 (1H, m), 8.51 (1H, t, J = 6.2 Hz), 8.59 (1H, brs), 8.98 (1H, s).

### Example 44

### N-(2-cyanoethyl)-3-[3-(2,4-dichlorobenzyl)-3H-imidazo [4,5-b]pyridin-5-yl]benzamide

In the same manner as in Example 1 and using 3-[3-(2,4-dichlorobenzyl)-3H-imidazo[4,5-b]pyridin-5-yl]benzoic acid obtained in Reference Example 56 and 3-aminopropanenitrile, the title compound was obtained. Yield 51 %, melting point 218 - 219°C (ethyl acetate-hexane).
¹H-NMR (CDCl₃) δ : 2.81 (2H, t, J = 6.3 Hz), 3.78 (2H, q, J = 6.3 Hz), 5.62 (2H, s), 6.71 (1H, brs), 7.07 - 7.15 (1H, m), 7.36 (1H, d, J = 8.1 Hz), 7.46 (1H, d, J = 1.8 Hz), 7.58 (1H, t, J = 7.8 Hz), 7.75 - 7.81 (2H, m), 8.11 - 8.16 (2H, m), 8.23 (1H, d, J = 8.1 Hz), 8.82 (1H, s).

### Example 45

### 3-[3-[2-(3,4-dimethoxyphenyl)ethyl]-3H-imidazo[4,5-b]pyridin-5-yl]-N-(2-pyrrolidin-1-ylethyl)benzamide

In the same manner as in Example 1 and using 3-[3-[2-(3,4-dimethoxyphenyl)ethyl]-3H-imidazo[4,5-b]pyridin-5-yl]benzoic acid obtained in Reference Example 61 and 2-pyrrolidin-1-ylethanamine, the title compound was obtained. Yield 38%, melting point 106 - 107°C (ethyl acetate-hexane).
¹H-NMR (DMSO-d₆) δ : 1.64 - 1.71 (4H, m), 2.46 - 2.54 (4H, m), 2.61 (2H, t, J = 7.0 Hz), 3.20 (2H, t, J = 7.0 Hz), 3.43 (2H, q, J = 6.8 Hz), 3.62 (3H, s), 3.66 (3H, s), 4.59 (2H, t, J = 7.0 Hz), 6.63 - 6.68 (1H, m), 6.71 (1H, d, J = 1.9 Hz), 6.80 (1H, d, J = 8.3 Hz), 7.60 (1H, t, J = 7.8 Hz), 7.88 (1H, d, J = 8.0 Hz), 7.93 (1H, d, J = 8.3 Hz), 8.16 (1H, d, J = 8.3 Hz), 8.27 - 8.33 (2H, m), 8.55 - 8.63 (2H, m).

### Example 46

### 3-[3-[2-(3,4-dimethoxyphenyl)ethyl]-3H-imidazo[4,5-b]pyridin-5-yl]-N-ethylbenzamide

In the same manner as in Example 1 and using 3-[3-[2-(3,4-dimethoxyphenyl)ethyl]-3H-imidazo[4,5-b]pyridin-5-yl]benzoic acid obtained in Reference Example 61 and ethylamine, the title compound was obtained. Yield 20%, melting point 152 - 153°C (ethyl acetate-hexane).
¹H-NMR (CDCl₃) δ : 1.30 (3H, t, J = 7.2 Hz), 3.21 (2H, t, J = 6.6 Hz), 3.50 - 3.62 (2H, m), 3.73 (3H, s), 3.82 (3H, s), 4.58 (2H, q, J = 6.6 Hz), 6.22 (1H, brs), 6.49 (1H, d, J = 1.8 Hz), 6.60 (1H, dd, J = 7.5, 1.8 Hz), 6.76 (1H, d, J = 8.4 Hz), 7.55 (1H, t, J = 7.5 Hz), 7.70 - 7.82 (3H, m), 8.10 (1H, d, J = 8.7 Hz), 8.22 (1H, d, J = 8.1 Hz), 8.49 (1H, s).

### Example 47

### N-(2-cyanoethyl)-3-[4-(2,4-dichlorophenyl)-3,4-dihydro-2H-1,4-benzoxazin-6-yl]benzamide

In the same manner as in Example 1 and using 3-[4-(2,4-dichlorophenyl)-3,4-dihydro-2H-1,4-benzoxazin-6-yl]benzoic acid obtained in Reference Example 64 and 3-aminopropanenitrile, the title compound was obtained. Yield 53%, melting point 147 - 148°C (ethyl acetate-hexane).
¹H-NMR (CDCl₃) δ : 2.74 (2H, t, J = 6.0 Hz), 3.50 - 3.82 (4H, m), 4.35 (2H, brs), 6.57 (1H, s), 6.61 (1H, brs), 6.96 (2H, d, J = 1.2 Hz), 7.20 - 7.30 (2H, m), 7.39 (1H, t, J = 7.8 Hz), 7.49 - 7.53 (2H, m), 7.61 (1H, d, J = 7.5 Hz), 7.79 (1H, s).

### Example 48

### N-(2-cyanoethyl)-3-[1-(2,4-dichlorophenyl)-2,3,4,5-tetrahydro-1H-1-benzazepin-8-yl]benzamide

In the same manner as in Example 1 and using 3-[1-(2,4-dichlorophenyl)-2,3,4,5-tetrahydro-1H-1-benzazepin-8-yl]benzoic acid obtained in Reference Example 70 and 3-aminopropanenitrile, the title compound was obtained. Yield 58%, melting point 139 - 142°C (ethyl acetate-hexane).
¹H-NMR (CDCl₃) δ : 1.79 (4H, brs), 2.75 (2H, t, J = 6.3 Hz), 2.97 - 3.05 (2H, m), 3.60 - 3.66 (2H, m), 3.72 (2H, q, J = 6.2 Hz), 6.57 (1H, brs), 6.66 (1H, d, J = 1.9 Hz), 7.14 - 7.19 (1H, m), 7.23 - 7.29 (3H, m), 7.34 (1H, dd, J = 1.8, 1.0 Hz), 7.38 - 7.46 (1H, m), 7.48 - 7.55 (1H, m), 7.61 - 7.67 (1H, m), 7.80 (1H, t, J = 1.6 Hz).

### Example 49

### 3-[1-(2,4-dichlorophenyl)-2,3,4,5-tetrahydro-1H-1-benzazepin-8-yl]-N-(2-hydroxyethyl)benzamide

In the same manner as in Example 7 and using 3-[1-(2,4-dichlorophenyl)-2,3,4,5-tetrahydro-1H-1-benzazepin-8-yl]benzoic acid obtained in Reference Example 70 and 2-aminoethanol, the title compound was obtained. Yield 61 %, melting point 172 - 173°C (ethyl acetate-hexane).
¹H-NMR (CDCl₃) δ : 1.79 (4H, brs), 2.45 (1H, t, J = 5.0 Hz), 2.96 - 3.06 (2H, m), 3.63 (4H, q, J = 5.3 Hz), 3.84 (2H, q, J = 4.8 Hz), 6.58 (1H, brs), 6.66 (1H, d, J = 1.7 Hz), 7.14 - 7.20 (1H, m), 7.23 - 7.30 (3H, m), 7.34 (1H, dd, J = 1.7, 0.8 Hz), 7.40 (1H, t, J = 7.6 Hz), 7.46 - 7.53 (1H, m), 7.65 (1H, dd, J = 7.6, 1.6 Hz), 7.80 (1H, t, J = 1.5 Hz).

### Example 50

### N-(2-cyanoethyl)-3-[1-(2,4-dichlorobenzyl)-2,3,4,5-tetrahydro-1H-1-benzazepin-8-yl]benzamide

In the same manner as in Example 1 and using 3-[1-(2,4-dichlorobenzyl)-2,3,4,5-tetrahydro-1H-1-benzazepin-8-yl]benzoic acid obtained in Reference Example 75 and 3-aminopropanenitrile, the title compound was obtained. Yield 31%, melting point 139 - 140°C (ethyl acetate-hexane).
¹H-NMR (CDCl₃) δ : 1.68 (4H, brs), 2.78 (2H, t, J = 6.2 Hz), 2.91 (2H, brs), 3.00 (2H, brs), 3.75 (2H, q, J = 6.2 Hz), 4.48 (2H, s), 6.60 (1H, brs), 7.07 - 7.14 (2H, m), 7.18 - 7.24 (2H, m), 7.40 (1H, d, J = 2.1 Hz), 7.44 - 7.53 (2H, m), 7.64 - 7.74 (2H, m), 7.91 (1H, t, J = 1.7 Hz).

### Example 51

### 3-[3-(2,5-dichlorophenyl)-2,3-dihydro-1H-inden-5 yl]-N-[2-(dimethylamino)ethyl]benzamide

In the same manner as in Example 1 and using 3-[3-(2,5-dichlorophenyl)-2,3-dihydro-1H-inden-5-yl]benzoic acid obtained in Reference Example 81 and N,N-dimethylethane-1,2-diamine, the title compound was obtained as an amorphous solid. Yield 68%.
¹H-NMR (CDCl₃) δ : 2.01 - 2.05 (1H, m), 2.25 (6H, s), 2.51 (2H, t, J = 5.9 Hz), 2.62 - 2.78 (1H, m), 2.94 - 3.17 (2H, m), 3.48 - 3.59 (2H, m), 4.90 (1H, t, J = 8.0 Hz), 6.81 (1H, brs), 6.99 (1H, d, J = 2.7 Hz), 7.14 (1H, dd, J = 8.5, 2.5 Hz), 7.24 (1H, s), 7.35 (1H, d, J = 8.7 Hz), 7.38 - 7.42 (1H, m), 7.45 (1H, t, J = 7.6 Hz), 7.48 - 7.53 (1H, m), 7.62 - 7.67 (1H, m), 7.69 (1H, d, J = 7.6 Hz), 7.95 (1H, t, J = 1.7 Hz)

### Example 52

### N-(2-cyanoethyl)-3-[3-(2,5-dichlorophenyl)-2,3-dihydro-1H-inden-5-yl]benzamide

In the same manner as in Example 1 and using 3-[3-(2,5-dichlorophenyl)-2,3-dihydro-1H-inden-5-yl]benzoic acid obtained in Reference Example 81 and 3-aminopropanenitrile, the title compound was obtained. Yield 74%, melting point 135 - 136°C (ethyl acetate-hexane).
¹H-NMR (CDCl₃) δ : 1.97 - 2.04 (1H, m), 2.62 - 2.81 (3H, m), 2.97 - 3.15 (2H, m), 3.73 (2H, q, J = 6.4 Hz), 4.90 (1H, t, J = 8.0 Hz), 6.61 (1H, brs), 6.97 (1H, d, J = 2.3 Hz), 7.14 (1H, dd, J = 8.7, 2.7 Hz), 7.24 (1H, s), 7.35 (1H, d, J = 8.3 Hz), 7.38 - 7.43 (1H, m), 7.44 - 7.52 (2H, m), 7.65 - 7.73 (2H, m), 7.94 (1H, t, J = 1.7 Hz).

### Example 53

### (-)-N-(2-cyanoethyl)-3-[3-(2,5-dichlorophenyl)-2,3-dihydro-1H-inden-5-yl]benzamide

N-(2-Cyanoethyl)-3-[3-(2,5-dichlorophenyl)-2,3-dihydro-1H-inden-5-yl]benzamide (941 mg) obtained in Example 52 was fractionated by high performance liquid chromatography (column: CHIRALPAK AD-H, mobile phase: A) carbon dioxide, B) methanol, mixing ratio: A/B=700/300, flow rate: 50 mL/min, column temperature: 35°C, sample concentration: 10 mg/mL (methanol 100%), injection volume: 1.5 mL). The fraction solution containing an optically active form having a shorter retention time under the above-mentioned high performance liquid chromatography conditions was concentrated to give the title compound (463 mg, 99.9 %ee). Melting point 146°C (ethyl acetate-hexane).
¹H-NMR (CDCl₃) δ : 1.94 - 2.11 (1H, m), 2.64 - 2.83 (3H, m), 2.97 - 3.14 (2H, m), 3.73 (2H, q, J = 6.3 Hz), 4.90 (1H, t, J = 8.0 Hz), 6.58 (1H, brs), 6.96 (1H, d, J = 2.5 Hz), 7.14 (1H, dd, J = 8.5, 2.5 Hz), 7.23 (1H, s), 7.31 - 7.52 (4H, m), 7.64 - 7.73 (2H, m), 7.93 (1H, t, J = 1.6 Hz).
[α]D^{20:} -248.9° (c 0.4975, methanol).

### Example 54

### (+)-N-(2-cyanoethyl)-3-[3-(2,5-dichlorophenyl)-2,3-dihydro-1H-inden-5-yl]benzamide

N-(2-Cyanoethyl)-3-[3-(2,5-dichlorophenyl)-2,3-dihydro-1H-inden-5-yl]benzamide (941 mg) obtained in Example 52 was fractionated by high performance liquid chromatography (column: CHIRALPAK AD-H, mobile phase: A) carbon dioxide, B) methanol, mixing ratio: A/B=700/300, flow rate: 50 mL/min, column temperature: 35°C, sample concentration: 10 mg/mL (methanol 100%), injection volume: 1.5 mL). The fraction solution containing an optically active form having a longer retention time under the above-mentioned high performance liquid chromatography conditions was concentrated to give the title compound (463 mg, 99.9 %ee). Melting point 145°C (ethyl acetate-hexane).
¹H-NMR (CDCl₃) δ : 1.93 - 2.09 (1H, m), 2.64 - 2.83 (3H, m), 2.97 - 3.14 (2H, m), 3.73 (2H, q, J = 6.3 Hz), 4.90 (1H, t, J = 8.0 Hz), 6.58 (1H, brs), 6.96 (1H, d, J = 2.5 Hz), 7.14 (1H, dd, J = 8.5, 2.5 Hz), 7.23 (1H, s), 7.31 - 7.52 (4H, m), 7.64 - 7.73 (2H, m), 7.93 (1H, t, J = 1.6 Hz).
[a]D^{20:} +247.7° (c 0.514, methanol).

### Example 55

### 3-[3-(2,5-dichlorophenyl)-2,3-dihydro-1H-inden-5-yl]-N-(2-hydroxyethyl)benzamide

In the same manner as in Example 7 and using 3-[3-(2,5-dichlorophenyl)-2,3-dihydro-1H-inden-5-yl]benzoic acid obtained in Reference Example 81 and 2-aminoethanol, the title compound was obtained as an amorphous solid. Yield 66%. ¹H-NMR (CDCl₃) δ : 1.91 - 2.11 (1H, m), 2.63 - 2.81 (2H, m), 2.90 - 3.17 (2H, m), 3.58 - 3.68 (2H, m), 3.82 (2H, t, J = 4.9 Hz), 4.89 (1H, t, J = 7.8 Hz), 6.72 (1H, brs), 6.97 (1H, d, J = 2.7 Hz), 7.13 (1H, dd, J = 8.5, 2.5 Hz), 7.23 (1H, s), 7.31 - 7.50 (4H, m), 7.62 - 7.72 (2H, m), 7.93 (1H, s).

### Example 56

### 3-[3-(2,5-dichlorophenyl)-2,3-dihydro-1H-inden-5-yl]-N-[2-(methylsulfanyl)ethyl]benzamide

In the same manner as in Example 1 and using 3-[3-(2,5-dichlorophenyl)-2,3-dihydro-1H-inden-5-yl]benzoic acid obtained in Reference Example 81 and 2-(methylsulfanyl)ethanamine, the title compound was obtained. Yield 88%
¹H-NMR (CDCl₃) δ : 1.95 - 2.11 (1H, m), 2.15 (3H, s), 2.63 - 2.87 (3H, m), 3.07 (2H, ddd, J = 8.7, 4.3, 4.1 Hz), 3.64 - 3.76 (2H, m), 4.90 (1H, t, J = 8.0 Hz), 6.62 (1H, brs), 6.98 (1H, d, J = 2.7 Hz), 7.14 (1H, dd, J = 8.5, 2.5 Hz), 7.24 (1H, s), 7.32 - 7.56 (4H, m), 7.68 (2H, td, J = 9.0, 1.5 Hz), 7.94 (1H, t, J = 1.5 Hz).

### Example 57

### 3-[3-(2,5-dichlorophenyl)-2,3-dihydro-1H-inden-5-yl]-N-[2-(methylsulfinyl)ethyl]benzamide

To a solution of 3-[3-(2,5-dichlorophenyl)-2,3-dihydro-1H-inden-5-yl]-N-[2-(methylsulfanyl)ethyl]benzamide (521 mg, 1.14 mmol) obtained in Example 56 in dichloromethane was added m-chloroperbenzoic acid (309 mg, 1.26 mmol) under ice-cooling, and the mixture was stirred for 1 hr and at room temperature for 3 hr. The reaction solution was diluted with saturated aqueous sodium hydrogen carbonate solution and the mixture was extracted with ethyl acetate. The extract was dried over anhydrous sodium sulfate, and concentrated under reduced pressure. The residue was purified by silica gel column chromatography (ethyl acetate-methanol 1:0→1:1) to give the title compound (438 mg, yield 81%). Melting point 136 - 138°C (ethyl acetate-hexane).
¹H-NMR (CDCl₃) δ: 1.93 - 2.10 (1H, m), 2.61 - 2.77 (4H, m), 2.83 - 2.94 (1H, m), 2.94 - 3.10 (2H, m),3.10 - 3.25 (1H, m), 4.01 (2H, q, J = 5.5 Hz), 4.89 (1H, t, J = 8.0 Hz), 6.96 (1H, d, J = 2.5 Hz), 7.13 (1H, dd, J = 8.4, 2.6 Hz), 7.24 (1H, s), 7.30 - 7.53 (5H, m), 7.62 - 7.74 (2H, m), 7.98 (1H, s).

### Example 58

### 3-[3-(2,5-dichlorophenyl)-2,3-dihydro-1H-inden-5-yl]-N-[2-hydroxy-1-(hydroxymethyl)ethyl]benzamide

In the same manner as in Example 7 and using 3-[3-(2,5-dichlorophenyl)-2,3-dihydro-1H-inden-5-yl]benzoic acid obtained in Reference Example 81 and 2-aminopropane-1,3-diol, the title compound was obtained. Yield 87%, melting point 166°C (ethanol-hexane).
¹H-NMR (CDCl₃) δ : 1.92 - 2.09 (1H, m), 2.53 - 2.61 (2H, m), 2.64 - 2.78 (1H, m), 2.94 - 3.16 (1H, m), 3.86 - 4.05 (4H, m), 4.12 - 4.24 (1H, m), 4.89 (1H, t, J = 8.1 Hz), 6.68 - 7.03 (2H, m), 7.13 (1H, dd, J = 8.5, 2.5 Hz), 7.23 (1H, s), 7.32 - 7.54 (4H, m), 7.62 - 7.77 (2H, m), 7.95 (1H, t, J = 1.8 Hz).

### Example 59

### 3-[3-(2,5-dichlorophenyl)-2,3-dihydro-1H-inden-5-yl]-N-(tetrahydrofuran-2-ylmethyl)benzamide

In the same manner as in Example 1 and using 3-[3-(2,5-dichlorophenyl)-2,3-dihydro-1H-inden-5-yl]benzoic acid obtained in Example 81 and 1-(tetrahydrofuran-2-yl)methanamine, the title compound was obtained. Yield 61%, melting point 122°C (ethyl acetate-hexane).
¹H-NMR (CDCl₃) δ : 1.60 - 1.69 (1H, m), 1.86 - 2.10 (4H, m), 2.64 - 2.78 (1H, m), 2.94 - 3.16 (2H, m), 3.31 - 3.43 (1H, m), 3.72 - 3.93 (3H, m), 4.02 - 4.14 (1H, m), 4.90 (1H, t, J = 8.0 Hz), 6.52 (1H, brs), 6.97 (1H, d, J = 2.5 Hz), 7.13 (1H, dd, J = 8.5, 2.7 Hz), 7.23 (1H, s), 7.32 - 7.52 (4H, m), 7.61 - 7.72 (2H, m), 7.94 (1H, s).

### Example 60

### 3-[3-(2,5-dichlorophenyl)-2,3-dihydro-1H-inden-5-yl]-N-(2-methoxyethyl)benzamide

In the same manner as in Example 1 and using 3-[3-(2,5-dichlorophenyl)-2,3-dihydro-1H-inden-5-yl]benzoic acid obtained in Reference Example 81 and 2-methoxyethanamine, the title compound was obtained. Yield 62%, melting point 116°C (ethyl acetate-hexane).
¹H-NMR (CDCl₃) δ : 1.93 - 2.10 (1H, m), 2.63 - 2.79 (1H, m), 2.94 - 3.17 (2H, m), 3.38 (3H, s), 3.53 - 3.62 (2H, m), 3.62 - 3.72 (2H, m), 4.90 (1H, t, J = 7.7 Hz), 6.53 (1H, brs), 6.97 (1H, d, J = 2.5 Hz), 7.13 (1H, dd, J = 8.5, 2.7 Hz), 7.23 (1H, s), 7.31 - 7.55 (4H, m), 7.62 - 7.71 (2H, m), 7.93 (1H, t, J = 1.6 Hz).

### Example 61

### N-(2-cyanoethyl)-3-[3-(2,4-dichlorophenyl)-2,3-dihydro-1H-inden-5-yl]benzamide

In the same manner as in Example 1 and using 3-[3-(2,4-dichlorophenyl)-2,3-dihydro-1H-inden-5-yl]benzoic acid obtained in Reference Example 87 and 3-aminopropanenitrile, the title compound was obtained. Yield 88%, melting point 112 - 114°C (ethyl acetate-hexane).
¹H-NMR (CDCl₃) δ : 1.89 - 2.06 (1H, m), 2.64 - 2.73 (1H, m), 2.76 (2H, t, J = 6.2 Hz), 2.99 - 3.11 (2H, m), 3.73 (2H, q, J = 6.3 Hz), 4.89 (1H, t, J = 8.0 Hz), 6.61 (1H, brs), 6.93 (1H, d, J = 8.5 Hz), 7.13 (1H, dd, J = 8.5, 2.1 Hz), 7.22 (1H, s), 7.36 - 7.42 (1H, m), 7.42 -7.51 (3H, m), 7.68 (2H, dd, J = 7.8, 1.4 Hz), 7.93 (1H, t, J = 1.6 Hz).

### Example 62

### (-)-N-(2-cyanoethyl)-3-[3-(2,4-dichlorophenyl)-2,3-dihydro-1H-inden-5-yl]benzamide

N-(2-Cyanoethyl)-3-[3-(2,4-dichlorophenyl)-2,3-dihydro-1H-inden-5-yl]benzamide (240 mg) obtained in Example 61 was fractionated by high performance liquid chromatography (column: CHIRALPAK AD-H, mobile phase: A) hexane, B) ethanol, mixing ratio: A/B=900/ 100, flow rate: 80 mL/min, column temperature: 35°C, sample concentration: 2.5 mg/mL (hexane/ethanol=900/ 100), injection volume: 25 mL). The fraction solution containing an optically active form having a shorter retention time under the above-mentioned high performance liquid chromatography conditions was concentrated to give the title compound (120 mg, 99.9 %ee).
¹H-NMR (CDCl₃) δ : 1.89 - 2.05 (1H, m), 2.64 - 2.74 (1H, m), 2.76 (2H, t, J = 6.2 Hz), 2.96 - 3.09 (2H, m), 3.73 (2H, q, J = 6.2 Hz), 4.89 (3H, t, J = 7.8 Hz), 6.58 (1H, brs), 6.93 (1H, d, J = 8.3 Hz), 7.13 (1H, dd, J = 8.3, 1.9 Hz), 7.22 (1H, s), 7.36 - 7.42 (1H, m), 7.44 (1H, d, J = 2.3 Hz), 7.48 (2H, d, J = 7.6 Hz), 7.68 (2H, d, J = 7.2 Hz), 7.93 (1H, s).
[α]D²⁰: -183.5° (c 0.5075, methanol).

### Example 63

### (+)-N-(2-cyanoethyl)-3-[3-(2,4-dichlorophenyl)-2,3-dihydro-1H-inden-5-yl]benzamide

N-(2-Cyanoethyl)-3-[3-(2,4-dichlorophenyl)-2,3-dihydro-1H-inden-5-yl]benzamide (240 mg) obtained in Example 61 was fractionated by high performance liquid chromatography (column: CHIRALPAK AD-H, mobile phase: A) hexane, B) ethanol, mixing ratio: A/B=900/ 100, flow rate: 80 mL/min, column temperature: 35°C, sample concentration: 2.5 mg/mL (hexane/ethanol=900/ 100), injection volume: 25 mL). The fraction solution containing an optically active form having a longer retention time under the above-mentioned high performance liquid chromatography conditions was concentrated to give the title compound (120 mg, 99.9 %ee).
¹H-NMR (CDCl₃) δ : 1.89 - 2.08 (1H, m), 2.63 - 2.73 (1H, m), 2.76 (2H, t, J = 6.2 Hz), 2.98 - 3.10 (2H, m), 3.73 (2H, q, J = 6.2 Hz), 4.89 (1H, t, J = 7.8 Hz), 6.59 (1H, brs), 6.93 (1H, d, J = 8.7 Hz), 7.13 (1H, dd, J = 8.3, 2.3 Hz), 7.22 (1H, s), 7.36 - 7.42 (1H, m), 7.43 - 7.51 (2H, m), 7.68 (2H, d, J = 7.2 Hz), 7.93 (1H, s).
[α]D²⁰: +187.8° (c 0.5155, methanol).

### Example 64

### 3-[3-(2,4-dichlorophenyl)-2,3-dihydro-1H-inden-5-yl]-N-(2-hydroxyethyl)benzamide

In the same manner as in Example 7 and using 3-[3-(2,4-dichlorophenyl)-2,3-dihydro-1H-inden-5-yl]benzoic acid obtained in Reference Example 87 and 2-aminoethanol, the title compound was obtained as an amorphous solid. Yield 66%. ¹H-NMR (CDCl₃) δ : 1.86 - 2.10 (1H, m), 2.50 (1H, t, J = 5.0 Hz), 2.62 - 2.81 (1H, m), 2.92 - 3.14 (2H, m), 3.58 - 3.70 (2H, m), 3.85 (2H, q, J = 4.8 Hz), 4.89 (1H, t, J = 8.0 Hz), 6.63 (1H, brs), 6.93 (1H, d, J = 8.5 Hz), 7.13 (1H, dd, J = 8.5, 2.1 Hz), 7.22 (1H, s), 7.36 - 7.41 (1H, m), 7.42 - 7.51 (3H, m), 7.61 - 7.71 (2H, m), 7.94 (1H, t, J = 1.7 Hz).

### Example 65

### 3-[3-(2,4-dichlorophenyl)-2,3-dihydro-1H-inden-5-yl]-N-[2-hydroxy-1-(hydroxymethyl)ethyl]benzamide

In the same manner as in Example 7 and using 3-[3-(2,4-dichlorophenyl)-2,3-dihydro-1H-inden-5-yl]benzoic acid obtained in Reference Example 87 and 2-aminopropane-1,3-diol, the title compound was obtained. Yield 76%, melting point 135 - 157°C (ethyl acetate-hexane).
¹H-NMR (CDCl₃) δ : 1.89 - 2.05 (1H, m), 2.58 - 2.66 (2H, m), 2.66 - 2.78 (1H, m), 2.94 - 3.12 (2H, m), 3.85 - 4.04 (4H, m), 4.13 - 4.24 (1H, m), 4.89 (1H, t, J = 7.9 Hz), 6.93 (2H, d, J = 8.5 Hz), 7.13 (1H, dd, J = 8.4, 2.2 Hz), 7.21 (1H, s), 7.36 - 7.41 (1H, m), 7.41 - 7.51 (3H, m), 7.62 - 7.68 (1H, m), 7.68 - 7.74 (1H, m), 7.95 (1H, t, J = 1.7 Hz).

### Example 66

### 3-[3-(2,4-dichlorophenyl)-2,3-dihydro-1H-inden-5-yl]-N-[2-(methylsulfinyl)ethyl]benzamide

To a solution of 3-[3-(2,4-dichlorophenyl)-2,3-dihydro-1H-inden-5-yl]benzoic acid (500 mg, 1.30 mmol) obtained in Reference Example 87, WSC (299 mg, 1.56 mmol) and HOBt (211 mg, 1.56 mmol) in DMF (5 ml) was added 2-(methylthio)ethanamine (142 mg, 1.56 mmol), and the mixture was stirred at room temperature for 4 hr. To the reaction mixture was added saturated aqueous sodium hydrogen carbonate solution and the mixture was extracted with ethyl acetate. The organic layer was washed with water, dried over anhydrous magnesium sulfate, and concentrated under reduced pressure. The residue was purified by silica gel column chromatography to give 3-[3-(2,4-dichlorophenyl)-2,3-dihydro-1H-inden-5-yl]-N-[2-(methylthio)ethyl]benzamide (470 mg) as an amorphous solid. To a solution of the compound in dichloromethane (4 mL) was added m-chloroperbenzoic acid (279 mg, 1.13 mmol) under ice-cooling, and the mixture was stirred for 1 hr. To the reaction mixture was added saturated aqueous sodium hydrogen carbonate solution and the mixture was extracted with ethyl acetate. The organic layer was dried over anhydrous sodium sulfate, and concentrated under reduced pressure. The residue was purified by basic silica gel column chromatography (hexane-ethyl acetate 70:30→0:100), and a fraction solution containing a high polar component was concentrated and recrystallized from ethyl acetate-hexane to give the title compound (282 mg, yield 58%) as crystals. Melting point 112 - 113°C.
¹H-NMR (CDCl₃) δ : 1.89 - 2.06 (1H, m), 2.67 (3H, s), 2.68 - 2.78 (1H, m), 2.83 - 2.94 (1H, m), 2.97 - 3.09 (2H, m), 3.11 - 3.25 (1H, m), 3.98 - 4.10 (2H, m), 4.89 (1H, t, J = 8.1 Hz), 6.94 (1H, d, J = 8.7 Hz), 7.13 (1H, dd, J = 8.3, 1.9 Hz), 7.22 (1H, s), 7.38 (2H, d, J = 8.0 Hz), 7.41 - 7.52 (3H, m), 7.66 (2H, t, J = 8.1 Hz), 7.97 (1H, s).

### Example 67

### 3-[3-(2,4-dichlorophenyl)-2,3-dihydro-1H-inden-5-yl]-N-[2-(methylsulfonyl)ethyl]benzamide

To a solution of 3-[3-(2,4-dichlorophenyl)-2,3-dihydro-1H-inden-5-yl]benzoic acid (600 mg, 1.57 mmol) obtained in Reference Example 87, WSC (360 mg, 1.88 mmol) and HOBt (254 mg, 1.88 mmol) in DMF (6 ml) was added 2-(methylthio)ethanamine (176 µL, 1.88 mmol), and the mixture was stirred at room temperature for 15 hr. To the reaction mixture was added saturated aqueous sodium hydrogen carbonate solution and the mixture was extracted with ethyl acetate. The organic layer was washed with water, dried over anhydrous magnesium sulfate, and concentrated under reduced pressure. The residue was purified by silica gel column chromatography to give 3-[3-(2,4-dichlorophenyl)-2,3-dihydro-1H-inden-5-yl]-N-[2-(methylthio)ethyl]benzamide (710 mg) as an amorphous solid. To a solution of the compound in dichloromethane (6 mL) was added m-chloroperbenzoic acid (424 mg, 1.72 mmol) under ice-cooling, and the mixture was stirred for 1 hr. To the reaction mixture was added saturated aqueous sodium hydrogen carbonate solution and the mixture was extracted with ethyl acetate. The organic layer was dried over anhydrous sodium sulfate, and concentrated under reduced pressure. The residue was purified by basic silica gel column chromatography (hexane-ethyl acetate 70:30→0:100), and a fraction solution containing a low polar component was concentrated and recrystallized from THF-hexane to give the title compound (100 mg, yield 13%) as crystals. Melting point 193 - 194°C.
¹H-NMR (CDCl₃) δ : 1.90 - 2.06 (1H, m), 2.57 - 2.78 (1H, m), 3.00 (3H, s), 3.02 - 3.09 (2H, m), 3.31 - 3.39 (2H, m), 3.96 - 4.07 (2H, m), 4.89 (1H, t, J = 8.0 Hz), 6.94 (1H, d, J = 8.3 Hz), 7.00 (1H, t, J = 6.4 Hz), 7.14 (1H, dd, J = 8.3, 2.3 Hz), 7.22 (1H, s), 7.35 - 7.41 (1H, m), 7.42 - 7.50 (3H, m), 7.67 (2H, d, J = 8.3 Hz), 7.94 (1H, s).

### Example 68

### N-(2-cyanoethyl)-3-[3-(2,4-dichlorophenyl)-1-benzofuran-5-yl]benzamide

In the same manner as in Example 1 and using 3-[3-(2,4-dichlorophenyl)-1-benzofuran-5-yl]benzoic acid obtained in Reference Example 90 and 3-aminopropanenitrile, the title compound was obtained. Yield 73%, melting point 159 - 161°C (ethyl acetate-hexane).
¹H-NMR (CDCl₃) δ : 2.77 (2H, t, J = 6.0 Hz), 3.74 (2H, q, J = 6.0 Hz), 6.65 (1H, brs), 7.33 - 7.39 (1H, m), 7.44 - 7.77 (8H, m), 7.85 (1H, s), 7.99 (1H, d, J = 1.8 Hz).

### Example 69

### N-(2-cyanoethyl)-3-[3-(2,4-dichlorophenyl)-2,3-dihydro-1-benzofuran-5-yl]benzamide

In the same manner as in Example 1 and using 3-[3-(2,4-dichlorophenyl)-2,3-dihydro-1-benzofuran-5-yl]benzoic acid obtained in Reference Example 94 and 3-aminopropanenitrile, the title compound was obtained. Yield 69%, melting point 130 - 131°C (ethyl acetate-hexane).
¹H-NMR (CDCl₃) δ : 2.75 (2H, t, J = 6.0 Hz), 3.71 (2H, q, J = 6.0 Hz), 4.38 (1H, dd, J = 8.4, 6.0 Hz), 5.01 (1H, t, J = 9.0 Hz), 5.17 (1H, dd, J = 9.0, 6.0 Hz), 6.71 (1H, brs), 6.91 (1H, d, J = 8.7 Hz), 7.02 (1H, d, J = 8.4 Hz), 7.17 (1H, d, J = 8.1 Hz), 7.32 (1H, s), 7.39 - 7.51 (3H, m), 7.61 - 7.69 (2H, m), 7.91 (1H, s).

### Example 70

### 3-[3-(2,4-dichlorophenyl)-2,3-dihydro-1-benzofuran-5-yl]-N-(2-hydroxyethyl)benzamide

In the same manner as in Example 1 and using 3-[3-(2,4-dichlorophenyl)-2,3-dihydro-1-benzofuran-5-yl]benzoic acid obtained in Reference Example 94 and 2-aminoethanol, the title compound was obtained. Yield 49%, amorphous powder. ¹H-NMR (CDCl₃) δ : 2.47 (1H, brs), 3.61-3.70 (2H, m), 3.78-3.89 (2H, m), 4.38 (1H, dd, J = 8.7, 6.0 Hz), 5.01 (1H, t, J = 9.0 Hz), 5.18 (1H, dd, J = 9.0, 6.0 Hz), 6.60 (1H, brs), 6.96 (1H, d, J = 8.7 Hz), 7.03 (1H, d, J = 8.1 Hz), 7.17 (1H, dd, J = 8.1, 1.8 Hz), 7.33 (1H, s), 7.39 - 7.51 (3H, m), 7.58 - 7.67 (2H, m), 7.92 (1H, s).

### Example 71

### N-(2-cyanoethyl)-3-[3-(2,4-dichlorophenyl)-1 H-indol-5-yl]benzamide

In the same manner as in Example 1 and using 3-[3-(2,4-dichlorophenyl)-1H-indol-5-yl]benzoic acid obtained in Reference Example 98 and 3-aminopropanenitrile, the title compound was obtained as an amorphous solid. Yield 88%.
¹H-NMR (CDCl₃) δ : 2.77 (2H, t, J = 6.2 Hz), 3.74 (2H, q, J = 6.3 Hz), 6.62 (1H, brs), 7.34 (1H, dd, J = 8.3, 1.9 Hz), 7.45 - 7.58 (6H, m), 7.68 (1H, d, J = 7.6 Hz), 7.78 (2H, brs), 8.02 (1H, s), 8.44 (1H, brs).

### Example 72

### 3-[3-(2,4-dichlorophenyl)-1H-indol-5-yl]-N-(2-hydroxyethyl)benzamide

In the same manner as in Example 7 and using 3-[3-(2,4-dichlorophenyl)-1H-indol-5-yl]benzoic acid obtained in Reference Example 98 and 2-aminoethanol, the title compound was obtained. Yield 42%, melting point 140 - 141°C (ethyl acetate-hexane).
¹H-NMR (CDCl₃) δ : 2.54 (1H, t, J = 4.9 Hz), 3.66 (2H, q, J = 5.3 Hz), 3.86 (2H, q, J = 4.5 Hz), 6.65 (1H, brs), 7.33 (1H, dd, J = 8.3, 1.9 Hz), 7.44 - 7.54 (5H, m), 7.56 (1H, d, J = 1.5 Hz), 7.69 (1H, d, J = 7.6 Hz), 7.73 - 7.80 (2H, m), 8.02 (1H, s), 8.46 (1H, brs).

### Example 73

### N-(2-cyanoethyl)-3-[3-(2,4-dichlorophenoxy)-2,3-dihydro-1H-inden-5-yl]benzamide

To a solution of 3-[3-(2,4-dichlorophenoxy)-2,3-dihydro-1H-inden-5-yl]benzoic acid (300 mg, 0.75 mmol) obtained in Reference Example 103, WSC (173 mg, 0.90 mmol), HOBt (122 mg, 0.90 mmol) and triethylamine (157 µL, 1.13 mmol) in DMF (3 ml) was added 3-aminopropanenitrile (66.4 µL, 0.90 mmol), and the mixture was stirred at room temperature for 15 hr. To the reaction mixture was added saturated aqueous sodium hydrogen carbonate solution and the mixture was extracted with ethyl acetate. The organic layer was washed with water, dried over anhydrous magnesium sulfate, and concentrated under reduced pressure. The residue was purified by silica gel column chromatography (hexane-ethyl acetate 100:0→60:40) and recrystallized from ethyl acetate-hexane to give the title compound (260 mg, yield 77%) as crystals. Melting point 139 - 145°C.
¹H-NMR (CDCl₃) δ : 2.24 - 2.38 (1H, m), 2.50 - 2.67 (1H, m), 2.77 (2H, t, J = 6.2 Hz), 2.89 - 3.06 (1H, m), 3.16 - 3.30 (1H, m), 3.74 (2H, q, J = 6.1 Hz), 5.77 (1H, dd, J = 6.8, 4.5 Hz), 6.66 (1H, brs), 7.06 (1H, d, J = 8.7 Hz), 7.20 - 7.25 (1H, m), 7.36 - 7.43 (2H, m), 7. 51 (1H, t, J = 7.8 Hz), 7.57 (1H, dd, J = 7.8, 1.7 Hz), 7.63 (1H, s), 7.72 (2H, dd, J = 7.8, 1.7 Hz), 7.97 (1H, s).

### Example 74

### 3-[3-(2,4-dichlorophenoxy)-2,3-dihydro-1H-inden-5-yl]-N-(2-hydroxyethyl)benzamide

In the same manner as in Example 7 and using 3-[3-(2,4-dichlorophenoxy)-2,3-dihydro-1H-inden-5-yl]benzoic acid obtained in Reference Example 103 and 2-aminoethanol, the title compound was obtained. Yield 45%, melting point 155 - 156°C (ethyl acetate-hexane).
¹H-NMR (CDCl₃) δ : 2.23 - 2.36 (1H, m), 2.50 - 2.69 (2H, m), 2.90 - 3.04 (1H, m), 3.16 - 3.29 (1H, m), 3.61 - 3.70 (2H, m), 3.86 (2H, q, J = 5.0 Hz), 5.76 (1H, dd, J = 6.4, 4.5 Hz), 6.67 (1H, brs), 7.05 (1H, d, J = 9.1 Hz), 7.20 - 7.25 (1H, m), 7.36 - 7.42 (2H, m), 7.49 (1H, t, J = 7.8 Hz), 7.57 (1H, dd, J = 7.8, 1.7 Hz), 7.63 (1H, s), 7.67 - 7.75 (2H, m), 7.97 (1H, s).

### Example 75

### 3-[3-(2,4-dichlorophenoxy)-2,3-dihydro-1H-inden-5-yl]-N-[2-(methylthio)ethyl]benzamide

In the same manner as in Example 1 and using 3-[3-(2,4-dichlorophenoxy)-2,3-dihydro-1H-inden-5-yl]benzoic acid obtained in Reference Example 103 and 2-(methylthio)ethanamine, the title compound was obtained. Yield 85%, melting point 141 - 142°C (ethyl acetate-hexane).
¹H-NMR (CDCl₃) δ : 2.15 (3H, s), 2.24 - 2.39 (1H, m), 2.54 - 2.69 (1H, m), 2.78 (2H, t, J = 6.2 Hz), 2.90 - 3.04 (1H, m), 3.14 - 3.30 (1H, m), 3.70 (2H, q, J = 5.8 Hz), 5.77 (1H, dd, J = 6.4, 4.5 Hz), 6.62 (1H, brs), 7.06 (1H, d, J = 8.7 Hz), 7.22 (1H, dd, J = 8.9, 2.5 Hz), 7.36 - 7.43 (2H, m), 7.50 (1H, t, J = 7.6 Hz), 7.58 (1H, dd, J = 7.8, 1.7 Hz), 7.65 (1H, s), 7.67 - 7.76 (2H, m), 7.98 (1H, s).

### Example 76

### 3-[3-(2,4-dichlorophenoxy)-2,3-dihydro-1H-inden-5-yl]-N-[2-(methylsulfinyl)ethyl]benzamide

To a solution of 3-[3-(2,4-dichlorophenoxy)-2,3-dihydro-1H-inden-5-yl]-N-[2-(methylthio)ethyl]benzamide (415 mg, 0.88 mmol) obtained in Example 75 in dichloromethane (4 mL) was added m-chloroperbenzoic acid (239 mg, 0.97 mmol) under ice-cooling, and the mixture was stirred for 1 hr. To the reaction mixture was added saturated aqueous sodium hydrogen carbonate solution and the mixture was extracted with ethyl acetate. The organic layer was dried over anhydrous sodium sulfate, and concentrated under reduced pressure. The residue was purified by basic silica gel column chromatography (hexane-ethyl acetate 70:30→0:100), and a fraction solution containing a high polar component was concentrated and recrystallized from methanol-hexane to give the title compound (240 mg, yield 56%) as crystals. Melting point 149 - 150°C.
¹H-NMR (CDCl₃) δ : 2.19 - 2.38 (1H, m), 2.52 - 2.64 (1H, m), 2.66 (3H, s), 2.82 - 3.04 (2H, m), 3.10 - 3.30 (2H, m), 4.04 (2H, d, J = 5.3 Hz), 5.71 - 5.82 (1H, m), 7.06 (1H, d, J = 8.9 Hz), 7.22 (1H, dd, J = 8.9, 2.4 Hz), 7.34 - 7.44 (3H, m), 7.48 (1H, t, J = 7.7 Hz), 7.57 (1H, d, J = 7.5 Hz), 7.64 (1H, s), 7.71 (2H, t, J = 8.9 Hz), 8.01 (1H, s).

### Example 77

### 3-[3-(2,4-dichlorophenoxy)-2,3-dihydro-1H-inden-5-yl]-N-[2-(methylsulfonyl)ethyl]benzamide

To a solution of 3-[3-(2,4-dichlorophenoxy)-2,3-dihydro-1H-inden-5-yl]-N-[2-(methylthio)ethyl]benzamide (415 mg, 0.88 mmol) obtained in Example 75 in dichloromethane (4 mL) was added m-chloroperbenzoic acid (239 mg, 0.97 mmol) under ice-cooling, and the mixture was stirred for 1 hr. To the reaction mixture was added saturated aqueous sodium hydrogen carbonate solution and the mixture was extracted with ethyl acetate. The organic layer was dried over anhydrous sodium sulfate, and concentrated under reduced pressure. The residue was purified by basic silica gel column chromatography (hexane-ethyl acetate 70:30→0:100), and a fraction solution containing a low polar component was concentrated and recrystallized from ethyl acetate-hexane to give the title compound (57 mg, yield 13%) as crystals. Melting point 140 - 143°C.
¹H-NMR (CDCl₃) δ : 2.22 - 2.39 (1H, m), 2.50 - 2.70 (1H, m), 2.89 - 3.06 (1H, m), 3.00 (3H, s), 3.16 - 3.31 (1H, m), 3.34 - 3.42 (2H, m), 4.03 (2H, q, J = 5.8 Hz), 5.76 (1H, dd, J = 6.5, 4.4 Hz), 7.03 (1H, brs), 7.06 (1H, d, J = 8.9 Hz), 7.23 (1H, dd, J = 8.8, 2.5 Hz), 7.36 - 7.42 (2H, m), 7.50 (1H, t, J = 7.7 Hz), 7.57 (1H, dd, J = 7.9, 1.7 Hz), 7.64 (1H, s), 7.70 (1H, t, J = 1.6 Hz), 7.72 (1H, s), 7.99 (1H, t, J = 1.7 Hz).

### Example 78

### N-cyclopropyl-3-[3-(2,4-dichlorophenoxy)-2,3-dihydro-1H-inden-5-yl]benzamide

In the same manner as in Example 1 and using 3-[3-(2,4-dichlorophenoxy)-2,3-dihydro-1H-inden-5-yl]benzoic acid obtained in Reference Example 103 and cyclopropylamine, the title compound was obtained. Yield 64%, melting point 179 - 180°C (ethyl acetate-hexane).
¹H-NMR (CDCl₃) δ : 0.58 - 0.69 (2H, m), 0.82 - 0.95 (2H, m), 2.23 - 2.38 (1H, m), 2.52 - 2.68 (1H, m), 2.87 - 3.04 (2H, m), 3.14 - 3.30 (1H, m), 5.76 (1H, dd, J = 6.4, 4. 5 Hz), 6.28 (1H, brs), 7.05 (1H, d, J = 9.1 Hz), 7.19 - 7.25 (1H, m), 7.35 - 7.43 (2H, m), 7.47 (1H, t, J = 7.8 Hz), 7.56 (1H, dd, J = 7.8, 1.7 Hz), 7.63 (1H, s), 7.66 (1H, s), 7.68 (1H, d, J = 1.5Hz), 7.92 (1H, s).

### Example 79

### 3-[3-(2,4-dichlorophenoxy)-2,3-dihydro-1H-inden-5-yl]-N-pyridin-2-ylbenzamide

To a solution of 3-[3-(2,4-dichlorophenoxy)-2,3-dihydro-1H-inden-5-yl]benzoic acid (300 mg, 0.75 mmol) obtained in Reference Example 103, HATU (342 mg, 0.90 mmol) and N-ethyldiisopropylamine (154 µL, 0.90 mmol) in DMF (3 ml) was added 2-aminopyridine (84.7 mg, 0.90 mmol), and the mixture was stirred at 80°C for 13 hr. To the reaction mixture was added saturated aqueous sodium hydrogen carbonate solution and the mixture was extracted with ethyl acetate. The organic layer was washed with water and saturated brine, dried over anhydrous magnesium sulfate, and concentrated under reduced pressure. The residue was purified by silica gel column chromatography (hexane-ethyl acetate 100:0→50:50) to give the title compound (210 mg, yield 59%) as an amorphous solid.
¹H-NMR (CDCl₃) δ : 2.24 - 2.43 (1H, m), 2.53 - 2.70 (1H, m), 2.91 - 3.08 (1H, m), 3.12 - 3.34 (1H, m), 5.78 (1H, dd, J = 6.4, 4. 5 Hz), 7.03 - 7.13 (2H, m), 7.23 (1H, dd, J = 8.8, 2.5 Hz), 7.38 - 7.44 (2H, m), 7.53 - 7.64 (2H, m), 7.67 (1H, s), 7.73 - 7.81 (2H, m), 7.85 - 7.92 (1H, m), 8.12 (1H, t, J = 1.7 Hz), 8.31 (1H, dd, J = 5.0, 1.0 Hz), 8.41 (1H, d, J = 8.5 Hz), 8.67 (1H, brs).

### Example 80

### 3-[3-(2,4-dichlorophenoxy)-2,3-dihydro-1H-inden-5-yl]-N-pyridin-3-ylbenzamide

In the same manner as in Example 79 and using 3-[3-(2,4-dichlorophenoxy)-2,3-dihydro-1H-inden-5-yl]benzoic acid obtained in Reference Example 103 and 3-aminopyridine, the title compound was obtained. Yield 84%, melting point 188 - 189°C (THF-hexane).
¹H-NMR (CDCl₃) δ : 2.23 - 2.40 (1H, m), 2.53 - 2.72 (1H, m), 2.92 - 3.06 (1H, m), 3.15 - 3.32 (1H, m), 5.77 (1H, dd, J = 6.4, 4.5 Hz), 7.06 (1H, d, J = 8.9 Hz), 7.22 (1H, dd, J = 8.8, 2.5 Hz), 7.34 (1H, dd, J = 8.4, 4.8 Hz), 7.38 - 7.45 (2H, m), 7.53 - 7.61 (2H, m), 7.67 (1H, s), 7.73 - 7.79 (1H, m), 7.80 - 7.85 (1H, m), 7.94 (1H, s), 8.06 (1H, t, J = 1.6 Hz), 8.29 - 8.35 (1H, m), 8.41 (1H, dd, J = 4.7, 1.3 Hz), 8.70 (1H, d, J = 2.4 Hz).

### Example 81

### 3-[3-(2,4-dichlorophenoxy)-2,3-dihydro-1H-inden-5-yl]-N-pyridin-4-ylbenzamide

In the same manner as in Example 79 and using 3-[3-(2,4-dichlorophenoxy)-2,3-dihydro-1H-inden-5-yl]benzoic acid obtained in Reference Example 103 and 4-aminopyridine, the title compound was obtained. Yield 70%, melting point 226 - 227°C (THF-hexane).
¹H-NMR (CDCl₃) δ : 2.22 - 2.42 (1H, m), 2.54 - 2.72 (1H, m), 2.90 - 3.07 (1H, m), 3.13 - 3.32 (1H, m), 5.77 (1H, dd, J = 6.4, 4.5 Hz), 7.06 (1H, d, J = 9.1 Hz), 7.23 (1H, dd, J = 8.7, 2.7 Hz), 7.36 - 7.44 (2H, m), 7.53 - 7.60 (2H, m), 7.62 (2H, d, J = 6.4 Hz), 7.66 (1H, s), 7.77 (1H, d, J = 8.3 Hz), 7.81 (1H, d, J = 8.0 Hz), 8.03 (2H, t, J = 1.7 Hz), 8.56 (2H, d, J = 6.4 Hz).

### Example 82

### 3-[3-(2,4-dichlorophenoxy)-2,3-dihydro-1H-inden-5-yl]-N-1,3-thiazol-2-ylbenzamide

In the same manner as in Example 79 and using 3-[3-(2,4-dichlorophenoxy)-2,3-dihydro-1H-inden-5-yl]benzoic acid obtained in Reference Example 103 and 2-thiazolamine, the title compound was obtained. Yield 84%, melting point 180 - 181°C (THF-hexane).
¹H-NMR (CDCl₃) δ : 2.20 - 2.39 (1H, m), 2.53 - 2.68 (1H, m), 2.88 - 3.06 (1H, m), 3.14 - 3.31 (1H, m), 5.75 (1H, dd, J = 6.5, 4.4 Hz), 6.90 (1H, d, J = 3.6 Hz), 7.05 (1H, d, J = 8.7 Hz), 7.11 (1H, d, J = 3.6 Hz), 7.23 (1H, dd, J = 8.8, 2.5 Hz), 7.36 - 7.42 (2H, m), 7.52 - 7.64 (3H, m), 7.82 (1H, d, J = 7.9 Hz), 7.95 (1H, d, J = 7.7 Hz), 8.18 (1H, t, J = 1.5 Hz), 11.92 (1H, s).

### Example 83

### N-(2-amino-2-oxoethyl)-3-[3-(2,4-dichlorophenoxy)-2,3-dihydro-1H-inden-5-yl]benzamide

In the same manner as in Example 73 and using 3-[3-(2,4-dichlorophenoxy)-2,3-dihydro-1H-inden-5-yl]benzoic acid obtained in Reference Example 103 and glycinamide hydrochloride, the title compound was obtained. Yield 76%, melting point 180 - 181°C (THF-hexane).
¹H-NMR (CDCl₃) δ: 2.22 - 2.37 (1H, m), 2.50 - 2.67 (1H, m), 2.89 - 3.05 (1H, m), 3.12 - 3.33 (1H, m), 4.20 (2H, d, J = 4.9 Hz), 5.49 (1H, brs), 5.76 (1H, dd, J = 6.4, 4.5 Hz), 6.07 (1H, brs), 7.02 (1H, brs), 7.05 (1H, d, J = 8.7 Hz), 7.22 (1H, dd, J = 8.7, 2.7 Hz), 7.36 - 7.42 (2H, m), 7.50 (1H, t, J = 7.8 Hz), 7.57 (1H, dd, J = 7.8, 1.7 Hz), 7.64 (1H, s), 7.71 (1H, d, J = 8.7 Hz), 7.77 (1H, d, J = 8.0 Hz), 8.01 (1H, s).

### Example 84

### N-(2-cyanoethyl)-3-[7-(2,4-dichlorophenoxy)-6,7-dihydro-5H-cyclopenta[b]pyridin-2-yl]benzamide

In the same manner as in Example 1 and using 3-[7-(2,4-dichlorophenoxy)-6,7-dihydro-5H-cyclopenta[b]pyridin-2-yl]benzoic acid obtained in Reference Example 108 and 3-aminopropanenitrile, the title compound was obtained. Yield 66%, melting point 193 - 194°C (ethyl acetate-hexane).
¹H-NMR (CDCl₃) δ: 2.39 - 2.52 (1H, m), 2.53 - 2.68 (1H, m), 2.74 - 2.82 (2H, m), 2.91 - 3.04 (1H, m), 3.20 - 3.35 (1H, m), 3.67 - 3.83 (2H, m), 5.73 (1H, dd, J = 6.8, 3.4 Hz), 6.64 (1H, brs), 7.27 - 7.32 (1H, m), 7.37 (1H, d, J = 2.3 Hz), 7.54 (1H, t, J = 8.0 Hz), 7.60 (1H, d, J = 8.7 Hz), 7.72 (2H, s), 7.83 (1H, d, J = 8.0 Hz), 8.09 (1H, d, J = 8.0 Hz), 8.36 (1H, s).

### Example 85

### 3-[7-(2,4-dichlorophenoxy)-6,7-dihydro-5H-cyclopenta[b]pyridin-2-yl]-N-(2-hydroxyethyl)benzamide

In the same manner as in Example 7 and using 3-[7-(2,4-dichlorophenoxy)-6,7-dihydro-5H-cyclopenta[b]pyridin-2-yl]benzoic acid obtained in Reference Example 108 and 2-aminoethanol, the title compound was obtained. Yield 56%, melting point 181 - 182°C (ethyl acetate-hexane).
¹H-NMR (CDCl₃) δ: 2.40 - 2.52 (1H, m), 2.55 - 2.65 (1H, m), 2.68 (1H, t, J = 5.1 Hz), 2.90 - 3.04 (1H, m), 3.21 - 3.35 (1H, m), 3.61 - 3.71 (2H, m), 3.86 (2H, q, J = 5.2 Hz), 5.72 (1H, dd, J = 6.8, 3.4 Hz), 6.71 (1H, brs), 7.27 - 7.32 (1H, m), 7.37 (1H, d, J = 2.3 Hz), 7.51 (1H, t, J = 7.8 Hz), 7.62 (1H, d, J = 8.7 Hz), 7.70 (2H, s), 7.83 (1H, d, J = 8.0 Hz), 8.05 (1H, d, J = 8.0 Hz), 8.35 (1H, s).

### Example 86

### N-(2-cyanoethyl)-3-[3-[(2,4-dichlorophenyl)amino]-2,3-dihydro-1-benzofuran-5-yl]benzamide

In the same manner as in Example 1 and using 3-[3-[(2,4-dichlorophenyl)amino]-2,3-dihydro-1-benzofuran-5-yl]benzoic acid obtained in Reference Example 109 and 3-aminopropanenitrile, the title compound was obtained.
Yield 58%, melting point 165 - 166°C (ethyl acetate-hexane).
¹H-NMR (CDCl₃) δ: 2.77 (2H, t, J = 6.0 Hz), 3.74 (2H, q, J = 6.0 Hz), 4.44 (1H, dd, J = 9.6,3.9 Hz), 4.63 (1H, d, J = 7.8 Hz), 4.76 - 4.84 (1H, m), 5.20 - 5.31 (1H, m), 6.58 - 6.67 (2H, m), 6.98 (1H, d, J = 8.4 Hz), 7.15 (1H, dd, J = 8.7, 2.1 Hz), 7.29 (1H, d, J = 2.1 Hz), 7.45 - 7.56 (2H, m), 7.61 (1H, s), 7.60 - 7.72 (2H, m), 7.95 (1H, s).

### Example 87

### 3-[3-[(2,4-dichlorophenyl)amino]-2,3-dihydro-1-benzofuran-5 yl]-N-(2-hydroxyethyl)benzamide

In the same manner as in Example 1 and using 3-[3-[(2,4-dichlorophenyl)amino]-2,3-dihydro-1-benzofuran-5-yl]benzoic acid obtained in Reference Example 109 and 2-aminoethanol, the title compound was obtained. Yield 29%, amorphous powder.
¹H-NMR (CDCl₃) δ : 2.50 (1H, brs), 3.65 (2H, q, J = 5.7 Hz), 3.86 (2H, q, J = 5.7 Hz), 4.43 (1H, dd, J = 9.6, 4.2 Hz), 4.63 (1H, d, J = 7.5 Hz), 4.80 (1H, dd, J = 9.6, 7.5 Hz), 5.21 - 5.38 (1H, m), 6.57 - 6.79 (2H, m), 6.97 (1H, d, J = 8.4 Hz), 7.15 (1H, dd, J = 8.4, 2.1 Hz), 7.29 (1H, d, J = 2.1 Hz), 7.47 (1H, t, J = 7.8 Hz), 7.53 (1H, dd, J = 8.4, 2.1 Hz), 7.59 - 7.71 (3H, m), 7.96 (1H, s).

### Example 88

### 3-[3-[(2,4-dichlorophenyl)amino]-2,3-dihydro-1-benzofuran-5-yl]-N-1,3-thiazol-2-ylbenzamide

In the same manner as in Example 1 and using 3-[3-[(2,4-dichlorophenyl)amino]-2,3-dihydro-1-benzofuran-5-yl]benzoic acid obtained in Reference Example 110 and 2-aminothiazole, the title compound was obtained. Yield 54%, melting point 221 - 222°C (ethyl acetate-hexane).
¹H-NMR (CDCl₃) δ: 4.44 (1H, dd, J = 9.9, 4.2 Hz), 4.61 (1H, d, J = 9.9 Hz), 4.81 (1H, dd, J = 9.9, 7.2 Hz), 5.20 - 5.32 (1H, m), 6.63 (1H, d, J = 9.0 Hz), 6.92 (1H, d, J = 3.3 Hz), 6.98 (1H, d, J = 8.1 Hz), 7.12 - 7.20 (2H, m), 7.29 (1H, d, J = 2.4 Hz), 7.49 - 7.58 (3H, m), 7.78 (1H, d, J = 7.5 Hz), 7.91 (1H, t, J = 8.7 Hz), 8.15 (1H, s), 1H unconfirmed.

### Example 89

### N-(2-cyanoethyl)-3-[4-(2,4-dichlorophenyl)-3,4-dihydro-2H-chromen-6-yl]benzamide

In the same manner as in Example 1 and using 3-[4-(2,4-dichlorophenyl)-3,4-dihydro-2H-chromen-6-yl]benzoic acid obtained in Reference Example 113 and 3-aminopropanenitrile, the title compound was obtained. Yield 45%, melting point 154 - 155°C (ethyl acetate-hexane).
¹H-NMR (CDCl₃) δ: 2.00 - 2.10 (1H, m), 2.32 - 2.48 (1H, m), 2.75 (2H, t, J = 6.3 Hz), 3.72 (2H, q, J = 6.3 Hz), 4.05 - 4.14 (1H, m), 4.18 - 4.27 (1H, m), 4.67 (1H, t, J = 5.4 Hz), 6.60 (1H, brs), 6.85 (1H, d, J = 8.7 Hz), 6.98 (1H, d, J = 8.4 Hz), 7.07 (1H, d, J = 2.1 Hz), 7.13 (1H, dd, J = 8.4, 2.1 Hz), 7.40 - 7.43 (3H, m), 7.56 - 7.64 (2H, m), 7.86 (1H, s).

### Example 90

### 3-[4-(2,4-dichlorophenyl)-3,4-dihydro-2H-chromen-6 yl]-N-(2-hydroxyethyl)benzamide

In the same manner as in Example 1 and using 3-[4-(2,4-dichlorophenyl)-3,4-dihydro-2H-chromen-6-yl]benzoic acid obtained in Reference Example 113 and 2-aminoethanol, the title compound was obtained. Yield 36%, amorphous powder. H-NMR (CDCl₃) δ : 2.00 - 2.15 (1H, m), 2.30 - 2.55 (2H, m), 3.60 - 3.67 (2H, m), 3.84 (2H,t,J=4.8Hz),4.05-4.18(1H,m),4.21-4.29(1H,m),4.67(1H,t,J=5.7Hz), 6.61 (1H, brs), 6.85 (1H, d, J = 8.7 Hz), 6.98 (1H, d, J = 8.4 Hz), 7.07 (1H, d, J = 2.1 Hz), 7.12 (1H, dd, J = 8.4, 2.1 Hz), 7.37 - 7.45 (3H, m), 7.55 - 7.65 (2H, m), 7.87 (1H, t, J = 1.8 Hz).

### Example 91

### N-(2-cyanoethyl)-3-[8-(2,4-dichlorophenoxy)-5,6,7,8-tetrahydronaphthalen-2-yl]benzamide

In the same manner as in Example 1 and using 3-[8-(2,4-dichlorophenoxy)-5,6,7,8-tetrahydronaphthalen-2-yl]benzoic acid obtained in Reference Example 117 and 3-aminopropanenitrile, the title compound was obtained. Yield 85%, melting point 150 - 151°C (ethyl acetate-hexane).
¹H-NMR (CDCl₃) δ: 1.76 - 1.91 (1H, m), 1.97 - 2.09 (1H, m), 2.10 - 2.24 (2H, m), 2.77 (2H, t, J = 6.2 Hz), 2.82 - 2.90 (1H, m), 2.92 - 3.04 (1H, m), 3.74 (2H, q, J = 6.1 Hz), 5.39 (1H, t, J = 4.7 Hz), 6.60 (1H, brs), 7.06 (1H, d, J = 8.7 Hz), 7.22 (1H, dd, J = 8.7, 2.7 Hz), 7.24 - 7.28 (1H, m), 7.41 (1H, d, J = 2.7 Hz), 7.46 - 7.53 (2H, m), 7.61 (1H, d, J = 1.9 Hz), 7.69 (1H, s), 7.71 (1H, d, J = 1.5 Hz), 7.95 (1H, s).

### Example 92

### 3-[8-(2,4-dichlorophenoxy)-5,6,7,8-tetrahydronaphthalen-2-yl]-N-(2-hydroxyethyl)benzamide

In the same manner as in Example 7 and using 3-[8-(2,4-dichlorophenoxy)-5,6,7,8-tetrahydronaphthalen-2-yl]benzoic acid obtained in Reference Example 117 and 2-aminoethanol, the title compound was obtained. Yield 87%, melting point 171 - 172°C (THF-hexane).
¹H-NMR (CDCl₃) δ: 1.75 - 1.92 (1H, m), 1.97 - 2.09 (1H, m), 2.10 - 2.26 (2H, m), 2.51 (1H, t, J = 4.9 Hz), 2.75 - 2.89 (1H, m), 2.90 - 3.02 (1H, m), 3.66 (2H, q, J = 5.0 Hz), 3.86 (2H, q, J = 4.9 Hz), 5.39 (1H, t, J = 4.4 Hz), 6.63 (1H, brs), 7.06 (1H, d, J = 8.7 Hz), 7.18 - 7.25 (2H, m), 7.41 (1H, d, J = 2.7 Hz), 7.43 - 7.54 (2H, m), 7.61 (1H, s), 7.67 (1H, d, J = 8.0 Hz), 7.71 (1H, d, J = 8.0 Hz), 7.96 (1H, s).

The structures of the compounds of Examples 1 to 92 are shown below.

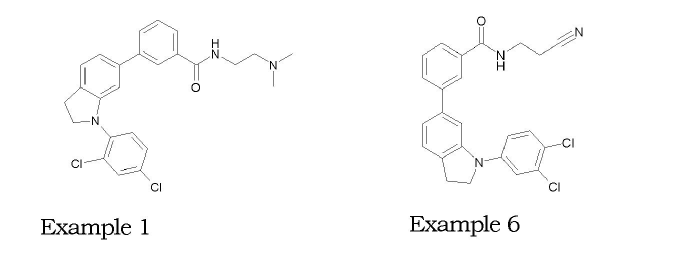

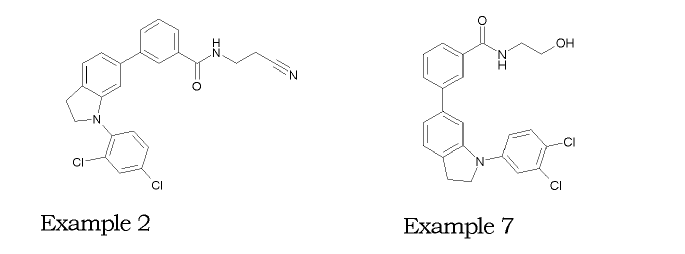

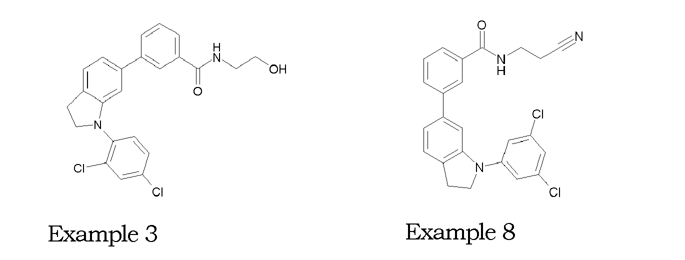

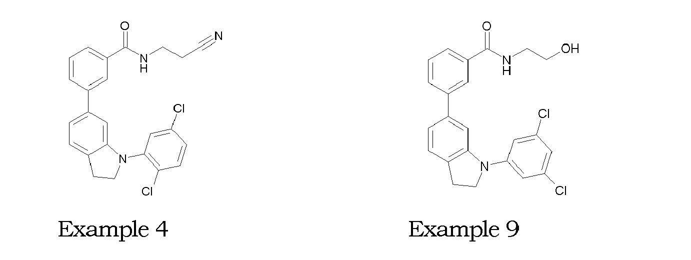

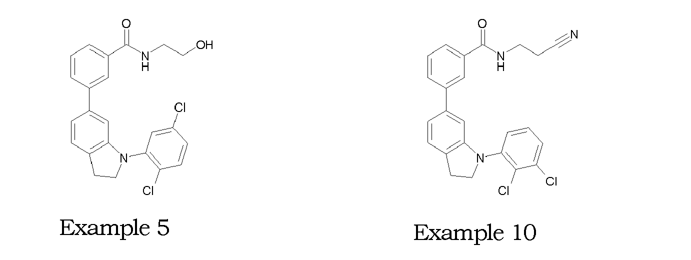

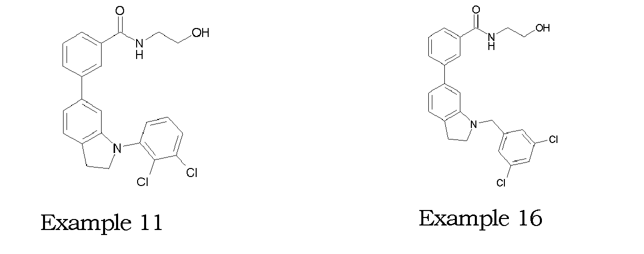

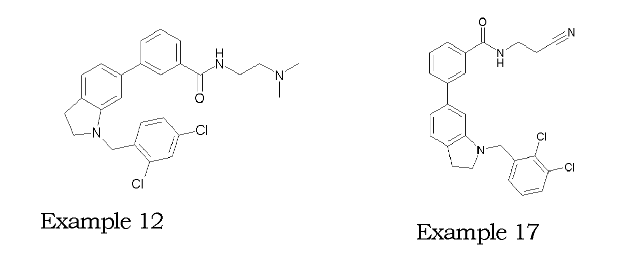

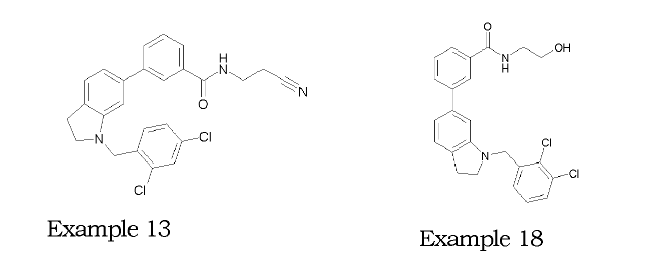

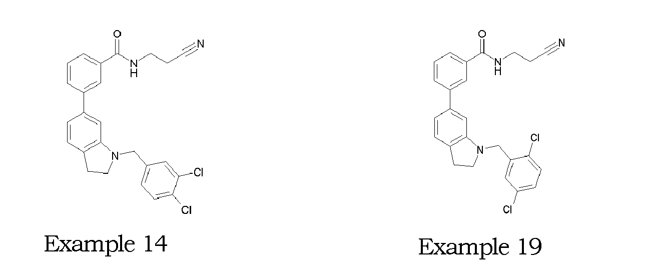

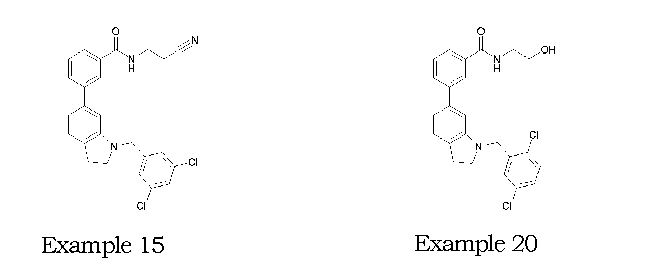

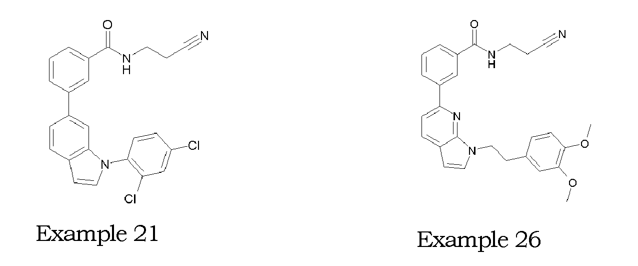

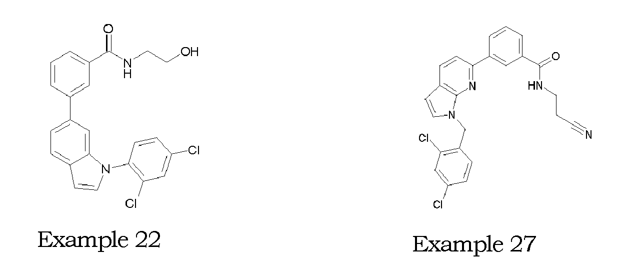

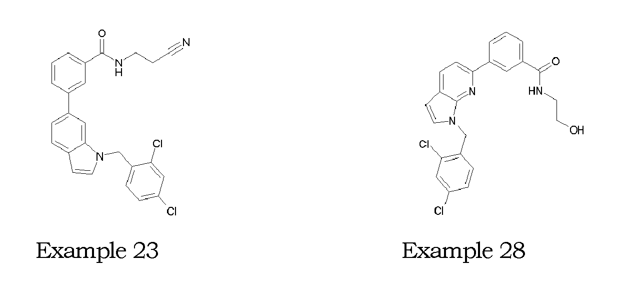

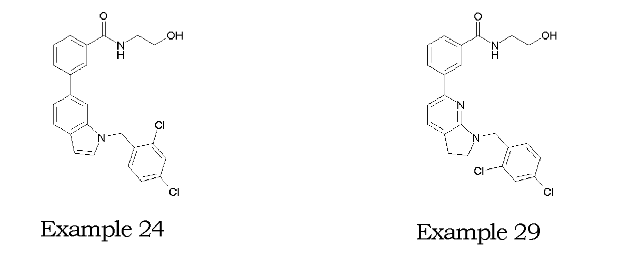

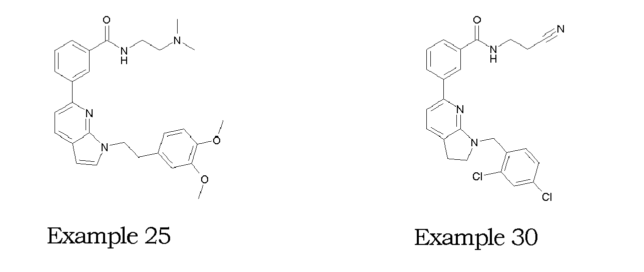

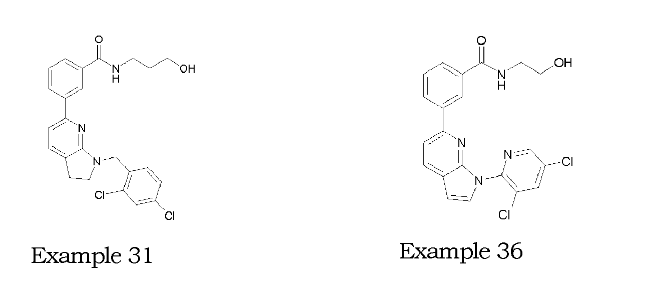

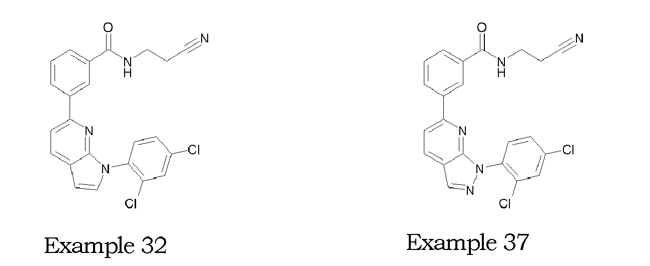

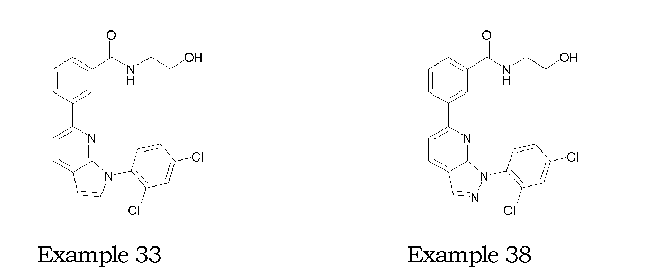

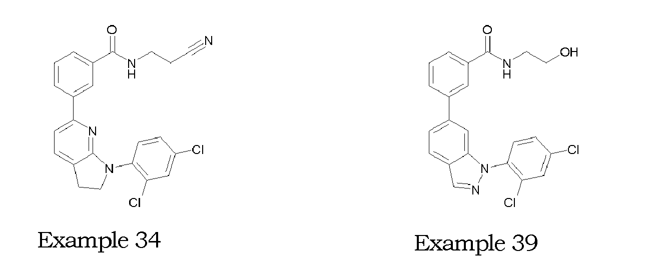

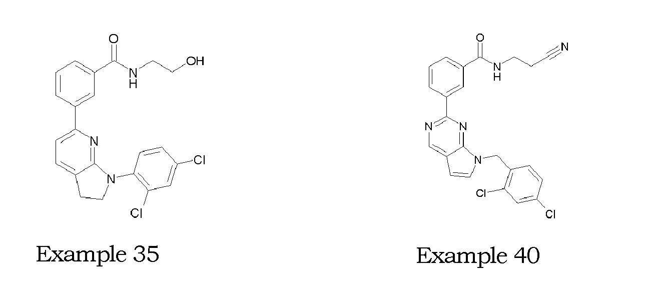

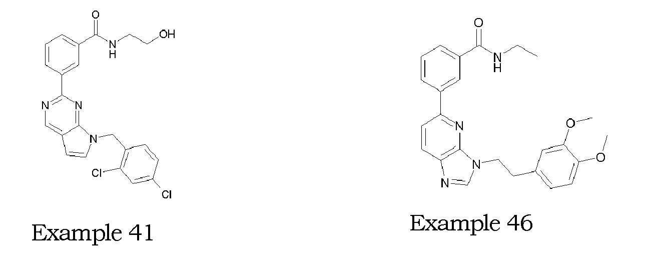

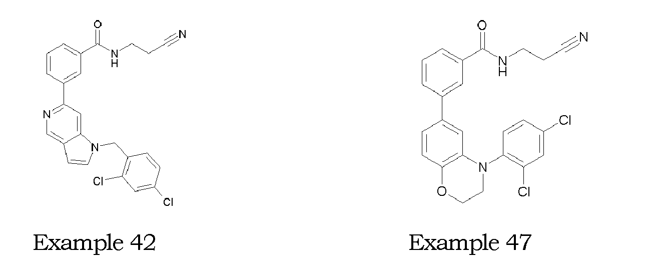

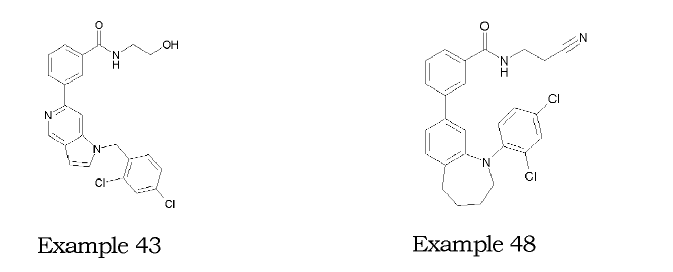

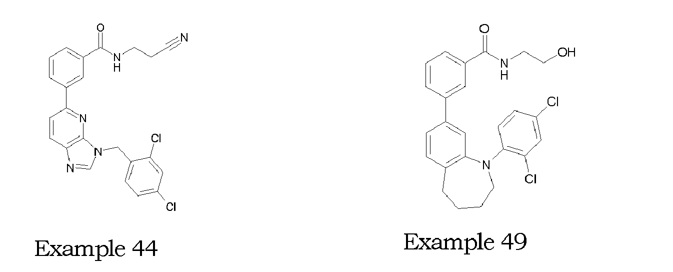

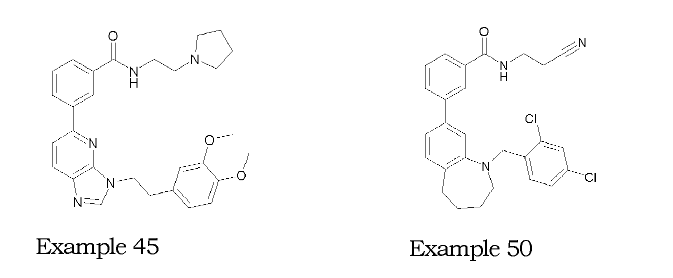

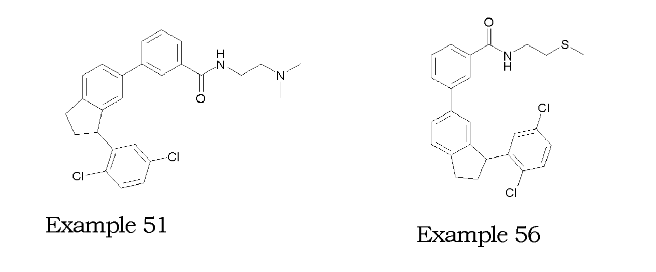

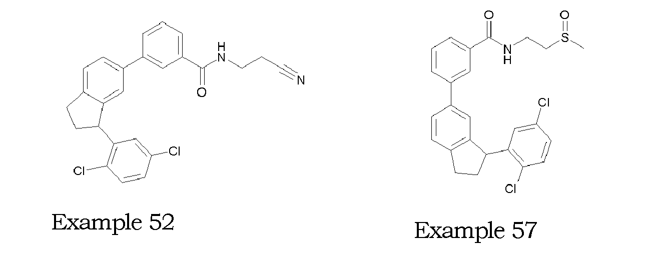

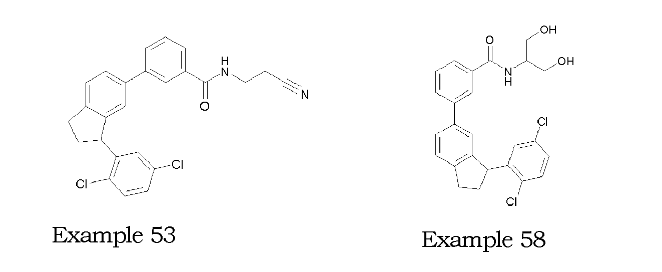

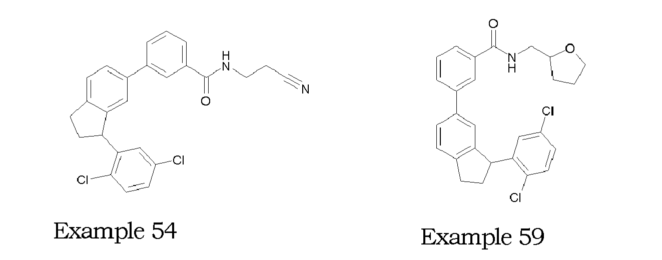

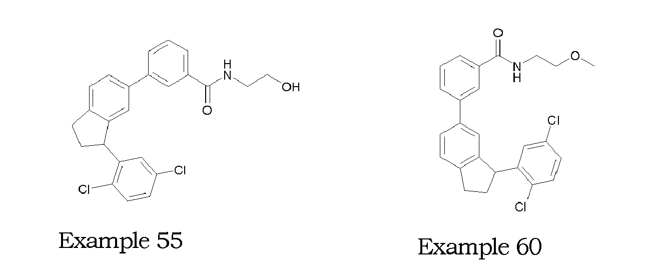

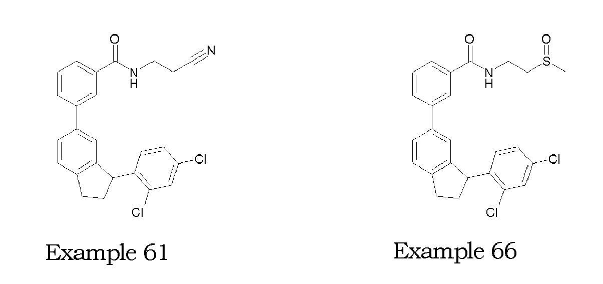

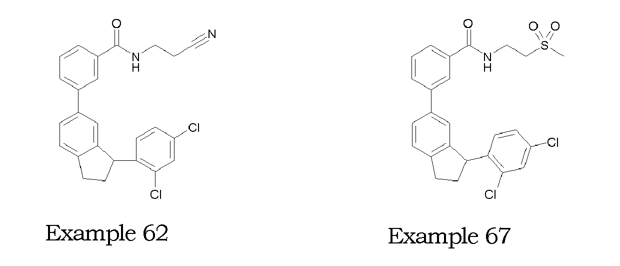

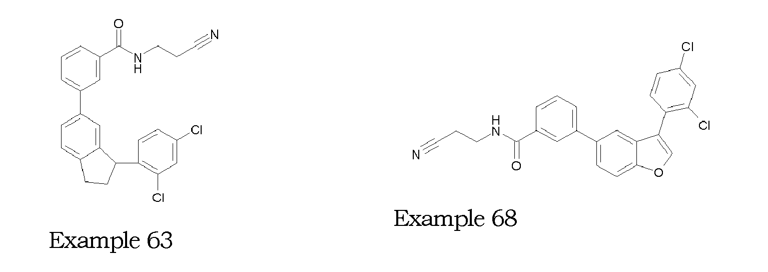

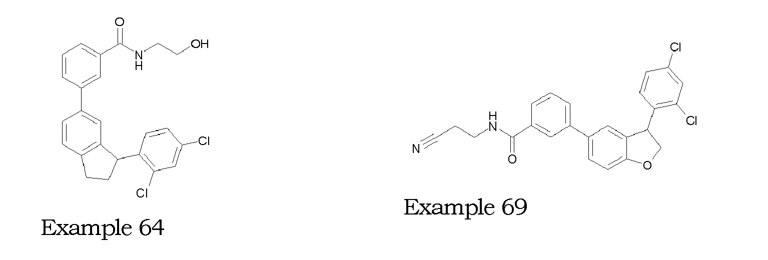

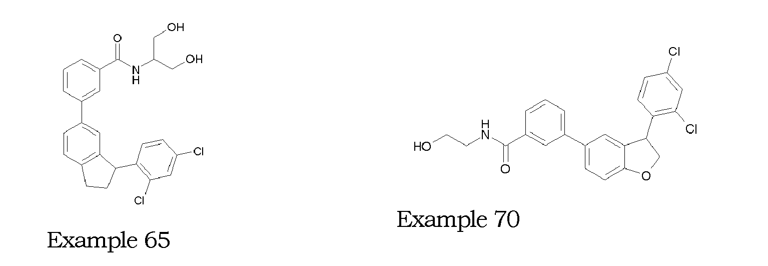

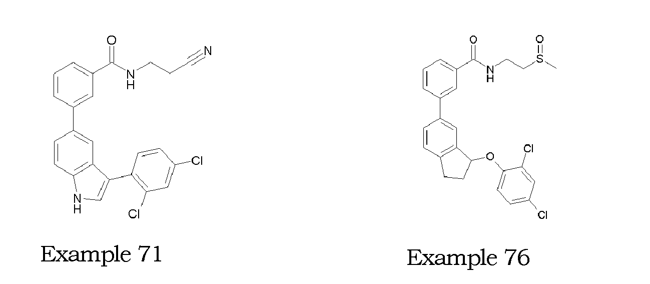

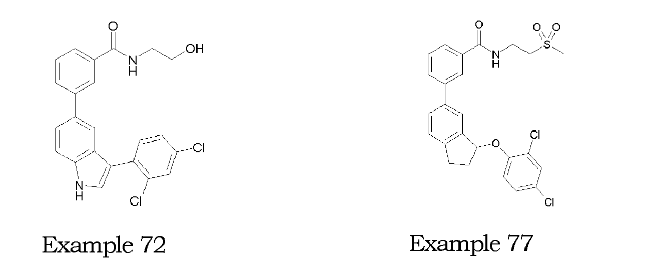

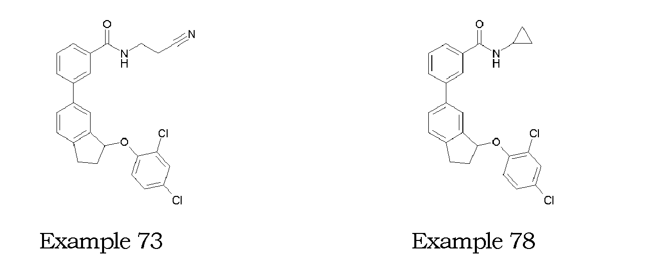

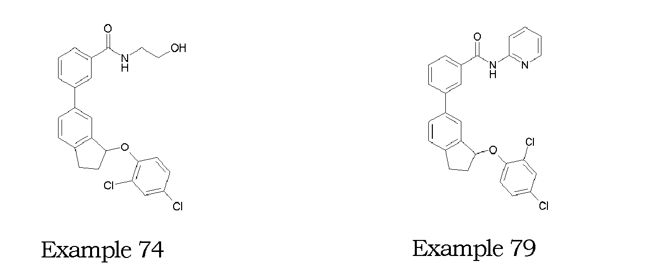

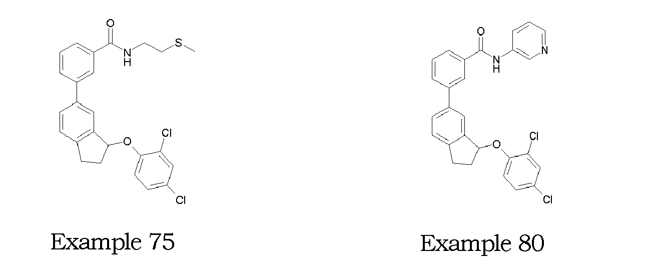

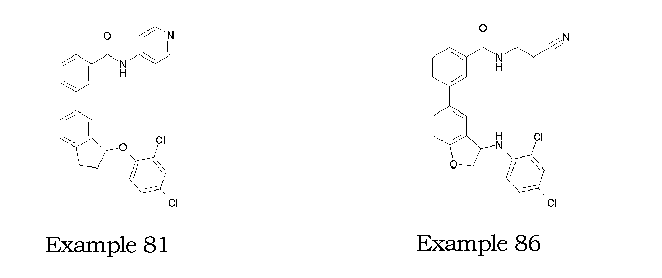

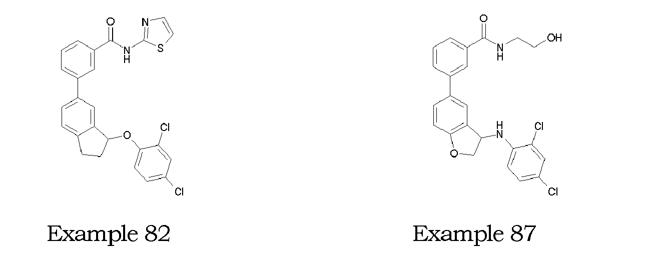

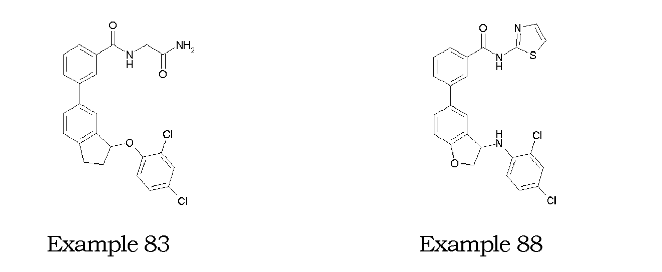

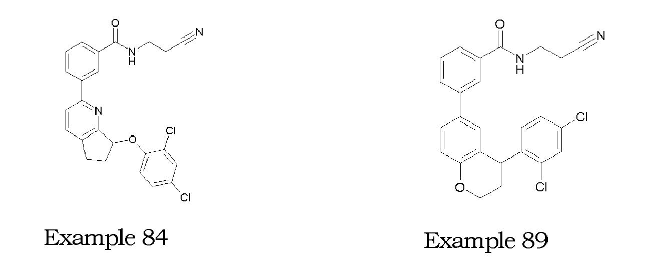

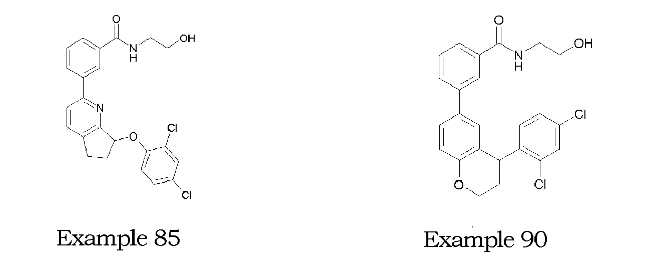

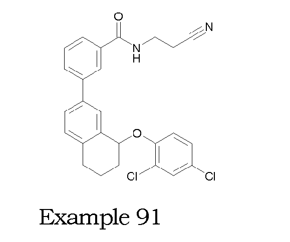

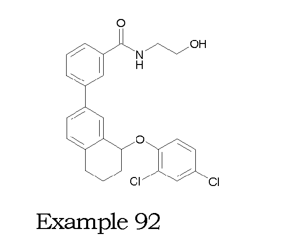

### Example 93

### N-cyclopropyl-3-[1-(2,4-dichlorophenyl)-2,3-dihydro-1H-indol-6-yl] benzamide

To a solution of 3-[1-(2,4-dichlorophenyl)-2,3-dihydro-1H-indol-6-yl]benzoic acid (300 mg, 0.78 mmol) obtained in Reference Example 4, WSC (180 mg, 0.94 mmol) and HOBt (127 mg, 0.94 mmol) in DMF (3 ml) was added cyclopropylamine (65.1 µL, 0.94 mmol), and the mixture was stirred at room temperature for 20 hr. To the reaction mixture was added saturated aqueous sodium hydrogen carbonate solution and the mixture was extracted with ethyl acetate. The combined organic layer was washed with water, dried over anhydrous magnesium sulfate, and concentrated under reduced pressure. The residue was purified by silica gel column chromatography (hexane-ethyl acetate 100:0→70:30) and HPLC, and recrystallized from ethyl acetate-hexane to give the title compound (175 mg, yield 53%) as crystals. Melting point 215 - 218°C.
¹H-NMR (CDCl₃) δ: 0.54 - 0.71 (2H, m), 0.80 - 0.95 (2H, m), 2.81 - 3.00 (1H, m), 3.22 (2H, t, J = 8.3 Hz), 3.94 (2H, brs), 6.22 (1H, brs), 6.98 (1H, dd, 7.6, 1.5 Hz), 7.21 - 7.30 (2H, m), 7.36 - 7.45 (2H, m), 7.50 (1H, d, J = 2.3 Hz), 7.56 - 7.65 (2H, m), 7.85 (1H, s).

### Example 94

### N-cyclopropyl-3-[1-(2,4-dichlorophenyl)-1H-indol-6-yl]benzamide

To a solution of 3-[1-(2,4-dichlorophenyl)-2,3-dihydro-1H-indol-6-yl]benzoic acid (300 mg, 0.78 mmol) obtained in Reference Example 4, WSC (180 mg, 0.94 mmol) and HOBt (127 mg, 0.94 mmol) in DMF (3 ml) was added cyclopropylamine (65.1 µL, 0.94 mmol), and the mixture was stirred at room temperature for 20 hr. To the reaction mixture was added saturated aqueous sodium hydrogen carbonate solution and the mixture was extracted with ethyl acetate. The combined organic layer was washed with water, dried over anhydrous magnesium sulfate, and concentrated under reduced pressure. The residue was purified by silica gel column chromatography (hexane-ethyl acetate 100:0→70:30). A small amount component was collected by HPLC and recrystallized from ethyl acetate-hexane to give the title compound (20 mg, yield 6%) as crystals. Melting point 273 - 274°C.
¹H-NMR (CDCl₃) δ: 0.55 - 0.71 (2H, m), 0.79 - 0.98 (2H, m), 2.84 - 3.02 (1H, m), 6.25 (1H, brs), 6.73 (1H, d, 3.0 Hz), 7.24 (1H, d, J = 3.0 Hz), 7.29 (1H, s), 7.40 - 7.48 (4H, m), 7.60 (1H, d, J = 7.6 Hz), 7.64 (1H, s), 7.71 (1H, d, J = 8.0 Hz), 7.75 (1H, d, J = 8.0 Hz), 7.96 (1H, s).

### Example 95

### N-(2-amino-2-oxoethyl)-3-[1-(2,4-dichlorophenyl)-2,3-dihydro-1H-indol-6-yl]benzamide

In the same manner as in Example 73 and using 3-[1-(2,4-dichlorophenyl)-2,3-dihydro-1H-indol-6-yl]benzoic acid obtained in Reference Example 4 and glycinamide hydrochloride, the title compound was obtained. Yield 90%, melting point 184 - 186°C (THF-hexane).
¹H-NMR (CDCl₃) δ: 3.22 (2H, t, J = 8.3 Hz), 3.93 (1H, brs), 4.17 (2H, d, J = 5.1 Hz), 5.47 (1H, brs), 6.05 (1H, brs), 6.60 (1H, d, 1.5 Hz), 6.96 (1H, brs), 6.99 (1H, dd, J = 7.6, 1.6 Hz), 7.22 - 7.29 (2H, m), 7.39 (1H, d, J = 8.7 Hz), 7.45 (1H, t, 7.7 Hz), 7.50 (1H, d, 2.3 Hz), 7.62 - 7.68 (1H, m), 7.69 - 7.75 (1H, m), 7.94 (1H, t, J = 1.7 Hz).

### Example 96

### 3-[1-(2,4-dichlorophenyl)-2,3-dihydro-1H-indol-6-yl]-N-(2-methoxyethyl)benzamide

In the same manner as in Example 3 and using 3-[1-(2,4-dichlorophenyl)-2,3-dihydro-1H-indol-6-yl]benzoic acid obtained in Reference Example 4 and 2-methoxyethanamine, the title compound was obtained. Yield 57%, melting point 119 - 120°C
¹H NMR (CDCl₃) δ: 3.23 (2H, t, J = 8.4 Hz), 3.39 (3H, s), 3.57 (2H, t, J = 4.8 Hz), 3.63 - 3.70 (2H, m), 3.94 (2H, br. s.), 6.51 (1H, br. s.), 6.61 (1H, d, J = 1.4 Hz), 7.00 (1H, dd, J = 7.4, 1.6 Hz), 7.21 - 7.30 (2H, m), 7.36 - 7.47 (2H, m), 7.50 (1H, d, J = 2.5 Hz), 7.59 - 7.71 (2H, m), 7.89 (1H, t, J = 1.8 Hz)

### Example 97

### N-(2-amino-2-oxoethyl)-3-[1-(2,3-dichlorophenyl)-2,3-dihydro-1H-indol-6-yl]benzamide

To a solution of 3-[1-(2,3-dichlorophenyl)-2,3-dihydro-1H-indol-6-yl]benzoic acid (384 mg, 1.00 mmol) obtained in Reference Example 14, DMTMM (375 mg, 1.20 mmol) and N-ethyldiisopropylamine (207 µL, 1.20 mmol) in DMF (5 ml) was added glycinamide hydrochloride (133 mg, 1.20 mmol), and the mixture was stirred at room temperature for 66 hr. Water was added to the reaction mixture and the mixture was extracted with ethyl acetate. The combined organic layer was washed with water, dried over anhydrous magnesium sulfate, and concentrated under reduced pressure. The residue was recrystallized from ethyl acetate to give the title compound (266 mg, yield 60%) as crystals. Melting point 203 - 204°C
¹H NMR (CDCl₃) δ:3.23 (2H, t, J = 8.5 Hz), 3.91 (2H, br. s.), 4.18 (2H, d, J = 5.2 Hz), 5.44 (1H, br. s.), 6.02 (1H, br. s.), 6.62 (1H, d, J = 1.1 Hz), 6.92 (1H, br. s.), 7.00 (1H, dd, J = 7.6, 1.5 Hz), 7.17 - 7.25 (2H, m), 7.36 (2H, ddd, J = 12.7, 8.0, 1.5 Hz), 7.45 (1H, t, J = 7.7 Hz), 7.65 (1H, dt, J = 7.7, 1. 5 Hz), 7.71 (1H, dd, J = 8.1, 1.5 Hz), 7.93 (1H,t,J= 1.6Hz)

### Example 98

### 3-[1-(2,3-dichlorophenyl)-2,3-dihydro-1H-indol-6-yl]-N-(2-methoxyethyl)benzamide

In the same manner as in Example 3 and using 3-[1-(2,3-dichlorophenyl)-2,3-dihydro-1H-indol-6-yl]benzoic acid obtained in Reference Example 14 and 2-methoxyethanamine, the title compound was obtained. Yield 50%, melting point 99 - 100°C
¹H NMR (CDCl₃) δ: 3.24 (2H, t, J = 8.2 Hz), 3.39 (3H, s), 3.56 (2H, t, J = 4.7 Hz), 3.66 (2H, t, J = 4.9 Hz), 3.97 (2H, br. s.), 6.52 (1H, br. s.), 6.62 (1H, s), 7.00 (1H, d, J = 7.4 Hz), 7.15 - 7.26 (2H, m), 7.31 - 7.50 (3H, m), 7.65 (2H, dd, J = 19.0, 7.1 Hz), 7.89 (1H, S)

### Example 99

### N-(2-cyanoethyl)-3-[1-[2-(2,4-dichlorophenyl)ethyl]-2,3-dihydro-1H-indol-6-yl]benzamide

To a solution of 3-[1-[2-(2,4-dichlorophenyl)ethyl]-2,3-dihydro-1H-indol-6-yl]benzoic acid (60 mg, 0.15 mmol) obtained in Reference Example 132 and DMTMM (53.0 mg, 0.18 mmol) in DMF (1ml) was added 3-aminopropanenitrile (13.3 µL, 0.18 mmol), and the mixture was stirred at room temperature for 15 hr. To the reaction mixture was added saturated aqueous sodium hydrogen carbonate solution and the mixture was extracted with ethyl acetate. The combined organic layer was washed with water, dried over anhydrous magnesium sulfate, and concentrated under reduced pressure. The residue was purified by silica gel column chromatography (hexane-ethyl acetate 90:10→50:50) and HPLC, and converted to hydrochloride with 4 N hydrochloric acid/ethyl acetate. Recrystallization from methanol-diethyl ether gave the title compound (15 mg, yield 20%) as crystals. Melting point 147 - 149°C.
¹H-NMR (DMSO-d₆) δ: 2.80 (2H, t, J = 6.4 Hz), 2.90 - 3.04 (4H, m), 3.35 - 3.56 (6H, m), 6.78 (1H, s), 6.90 (1H, d, J = 7.6 Hz), 7.13 (1H, d, J = 7.6 Hz), 7.38 (1H, dd, J = 8.1, 2.1 Hz), 7.49 - 7.57 (2H, m), 7.60 (1H, d, J = 1.9 Hz), 7.74 (1H, d, J = 7.6 Hz), 7.79 (1H, d, J = 8.0 Hz), 8.05 (1H, s), 8.95 (1H, t, J = 5.3 Hz).

### Example 100

### N-(2-cyanoethyl)-3-(1-[2-[3-(trifluoromethyl)phenyl]ethyl]-2,3-dihydro-1H-indol-6-yl)benzamide

In the same manner as in Example 99 and using 3-(1-[2-[3-(trifluoromethyl)phenyl]ethyl]-2,3-dihydro-1H-indol-6-yl)benzoic acid obtained in Reference Example 133 and 3-aminopropanenitrile, the title compound was obtained. Yield 50%, melting point 197 - 198°C (methanol-diethyl ether).
¹H-NMR (DMSO-d₆) δ : 2.81 (2H, t, J = 6.6 Hz), 2.89 - 3.09 (4H, m), 3.38 - 3.61 (6H, m), 6.87 - 7.03 (2H, m), 7.16 (1H, d, J = 7.6 Hz), 7.48 - 7.60 (3H, m), 7.68 (1H, d, J = 6.1 Hz), 7.73 (1H, s), 7.79 (2H, t, J = 8.9 Hz), 8.07 (1H, s), 8.97 (1H, t, J = 5.1 Hz).

### Example 101

### N-(2-amino-2-oxoethyl)-3-[1-(2, 4-dichlorobenzyl)-2,3-dihydro-1H-pyrrolo[2,3-b]pyridin-6-yl]benzamide

To a solution of 3-[1-(2,4-dichlorobenzyl)-2,3-dihydro-1H-pyrrolo[2,3-b]pyridin-6-yl]benzoic acid (280 mg, 0.70 mmol) obtained in Reference Example 37, DMTMM (263 mg, 0.84 mmol) and N-ethyldiisopropylamine (145 µL, 0.84 mmol) in methanol (7 ml) was added glycinamide hydrochloride (92.9 mg, 0.84 mmol), and the mixture was stirred at room temperature for 2 days. The reaction mixture was filtered and the residue was washed with ethyl acetate and recrystallized from ethanol to give the title compound (163 mg, yield 36%) as crystals. Melting point 183 - 184°C.
¹H NMR (DMSO-d₆) δ: 3.01 (2H, t, J = 8.2 Hz), 3.50 (2H, t, J = 8.2 Hz), 3.82 (2H, d, J = 6.0 Hz), 4.67 (2H, s), 7.02 (1H, br. s.), 7.16 (1H, d, J = 7.4 Hz), 7.33 - 7.44 (3H, m), 7.45 - 7.56 (2H, m), 7.63 (1H, d, J = 1.9 Hz), 7.82 (1H, d, J = 7.7 Hz), 8.13 (1H, d, J = 8.0 Hz), 8.44 (1H, s), 8.74 (1H, t, J = 5.8 Hz)

### Example 102

### 3-[1-(2,4-dichlorobenzyl)-2,3-dihydro-1H-pyrrolo[2,3-b]pyridin-6-yl]-N-(2-methoxyethyl)benzamide

To a solution of 3-[1-(2,4-dichlorobenzyl)-2,3-dihydro-1H-pyrrolo[2,3-b]pyridin-6-yl]benzoic acid (280 mg, 0.70 mmol) obtained in Reference Example 37 and DMTMM (263 mg, 0.84 mmol) in methanol (7 ml) was added 2-methoxyethanamine (73.0 µL, 0.84 mmol), and the mixture was stirred at room temperature for 2 days. Water was added to the reaction mixture and the mixture was extracted with ethyl acetate. The combined organic layer was washed with water, dried over anhydrous magnesium sulfate, and concentrated under reduced pressure. The residue was washed with hexane-ethyl acetate, and recrystallized from hexane-ethyl acetate to give the title compound (137 mg, yield 30%) as crystals. Melting point 140 - 141°C.
¹H NMR (CDCl₃ δ:3.04 (2H, t, J = 7.8 Hz), 3.37 (3H, s), 3.48 - 3.61 (4H, m), 3.67 (2H, t, J = 5.1 Hz), 4.76 (2H, s), 6.60 (1H, br. s.), 7.02 (1H, d, J = 7.1 Hz), 7.22 (1H, dd, J = 8.2, 1.9 Hz), 7.29 (1H, dt, J = 7.4, 1.3 Hz), 7.41 (1H, d, J = 2.2 Hz), 7.43 - 7.52 (2H, m), 7.75 (1H,dt,J= 7.7, 1.4 Hz), 8.11 (1H,dt,J=7.9, 1.4Hz), 8.36(1H,t,J= 1.6 Hz)

### Example 103

### N-(2-cyanoethyl)-3-[3-[3-(triffuoromethyl)phenoxy]-2,3-dihydro-1H-inden-5-yl]benzamide

In the same manner as in Example 1 and using 3-[3-[3-(trifluoromethyl)phenoxy]-2,3-dihydro-1H-inden-5-yl]benzoic acid obtained in Reference Example 134 and 3-aminopropanenitrile, the title compound was obtained. Yield 80%, melting point 120- 121°C (ethyl acetate-hexane).
¹H-NMR (CDCl₃) δ : 2.20 - 2.34 (1H, m), 2.59 - 2.74 (1H, m), 2.77 (2H, t, J = 6.3 Hz), 2.93 - 3.09 (1H, m), 3.13 - 3.31 (1H, m), 3.74 (2H, q, J = 6.2 Hz), 5.85 (1H, dd, J = 6.6, 4.3 Hz), 6.62 (1H, brs), 7.16 - 7.31 (3H, m), 7.38 - 7.47 (2H, m), 7.51 (1H, t, J = 7.7 Hz), 7. 59 (1H, dd, J = 7.9, 1.7 Hz), 7.66 (1H, s), 7.69 - 7.77 (2H, m), 7.99 (1H, t, J = 1.7 Hz).

### Example 104

### N-(2-hydroxyethyl)-3-[3-[3-(trifluoromethyl)phenoxy]-2,3-dihydro-1H-inden-5-yl]benzamide

In the same manner as in Example 2 and using 3-[3-[3-(trifluoromethyl)phenoxy]-2,3-dihydro-1H-inden-5-yl]benzoic acid obtained in Reference Example 134 and 2-aminoethanol, the title compound was obtained.
Yield 80%, melting point 157- 158°C (ethyl acetate-hexane).
¹H-NMR (CDCl₃) δ: 2.18 - 2.35 (1H, m), 2.52 (1H, t, J = 5.1 Hz), 2.59 - 2.73 (1H, m), 2.92 - 3.08 (1H, m), 3.14 - 3.27 (1H, m), 3.60 - 3.72 (2H, m), 3.86 (2H, q, J = 4.9 Hz), 5.85 (1H, dd, J = 6.4, 4.2 Hz), 6.65 (1H, brs), 7.15 - 7.30 (3H, m), 7.38 - 7.52 (3H, m), 7.58 (1H, dd, J = 7.8, 1.7 Hz), 7.66 (1H, s), 7.69 - 7.75 (2H, m), 7.99 (1H, s).

### Example 105

### N-(2-cyanoethyl)-3-[3-[(2,4-dichlorobenzyl)amino]-2,3-dihydro-1H-inden-5-yl]benzamide

In the same manner as in Reference Example 1 and using 3-[3-[(2,4-dichlorobenzyl)amino]-2,3-dihydro-1H-inden-5-yl]benzoic acid obtained in Reference Example 135 and 3-aminopropanenitrile, the title compound was obtained. Yield 80%, melting point 126 - 130°C (ethyl acetate-hexane).
¹H-NMR (CDCl₃) δ: 1.86 - 2.03 (1H, m), 2.42 - 2.58 (1H, m), 2.78 (2H, t, J = 6.2 Hz), 2.81 - 2.94 (1H, m), 2.98 - 3.15 (1H, m), 3.75 (2H, q, J = 6.1 Hz), 3.90 - 4.08 (2H, m), 4.35 (1H, t, J = 6.8 Hz), 6.68 (1H, brs), 7.22 - 7.26 (1H, m), 7.32 (1H, d, J = 8.0 Hz), 7.38 (1H, d, J = 2.3 Hz), 7.44 - 7.55 (3H, m), 7.60 (1H, s), 7.73 (2H, t, J = 8.1 Hz), 8.00 (1H, s).

### Example 106

### 3-[3-[(2,4-dichlorobenzyl)amino]-2,3-dihydro-1H-inden-5-yl]-N-(2-hydroxyethyl)benzamide

In the same manner as in Example 2 and using 3-[3-[(2,4-dichlorobenzyl)amino]-2,3-dihydro-1H-inden-5-yl]benzoic acid obtained in Reference Example 135 and 2-aminoethanol, the title compound was obtained as an amorphous solid. Yield 33%.
¹H-NMR (CDCl₃) δ: 1.86 - 2.01 (1H, m), 2.12 (2H, brs), 2.41 - 2.56 (1H, m), 2.78 - 2.94 (1H, m), 2.99 - 3.12 (1H, m), 3.59 - 3.66 (2H, m), 3.83 (2H, t, J = 5.1 Hz), 3.91 - 4.08 (2H, m), 4.35 (1H, t, J = 6.6 Hz), 6.75 (1H, brs), 7.23 (1H, dd, J = 8.1, 2.1 Hz), 7.30 (1H, d, J = 7.6 Hz), 7.37 (1H, d, J = 1.9 Hz), 7.41 - 7.52 (3H, m), 7.58 (1H, s), 7.70 (2H, dd, J = 7.8, 1.7 Hz), 7.98 (1H, s).

### Example 107

### 2-[3-(2,4-dichlorophenyl)-2,3-dihydro-1H-inden-5-yl]-N-(2-hydroxyethyl)pyridine-4-carboxamide

In the same manner as in Example 2 and using 2-[3-(2,4-dichlorophenyl)-2,3-dihydro-1H-inden-5-yl]pyridine-4-carboxylic acid obtained in Reference Example 136 and 2-aminoethanol, the title compound was obtained as an amorphous solid. Yield 59%.
¹H-NMR (CDCl₃) δ: 1.97 (1H, dq, J = 12.7, 8.1 Hz), 2.62 - 2.80 (1H, m), 2.94 - 3.15 (2H, m), 3.61 - 3.71 (2H, m), 3.83 - 3.89 (2H, m), 4.90 (1H, t, J = 8.0 Hz), 6.73 (1H, brs), 6.91 (1H, d, J = 8.7 Hz), 7.12 (1H, dd, J = 8.3, 1.9 Hz), 7.39 - 7.49 (3H, m), 7.65 (1H, s), 7.92 (1H, d, J = 8.0 Hz), 8.00 (1H, s), 8.73 (1H, d, J = 4.9 Hz).

### Example 108

### 2-[3-(2,4-dichlorophenyl)-2,3-dihydro-1H-inden-5-yl]-N-(2-methoxyethyl)pyridine-4-carboxamide

In the same manner as in Example 1 and using 2-[3-(2,4-dichlorophenyl)-2,3-dihydro-1H-inden-5-yl]pyridine-4-carboxylic acid obtained in Reference Example 136 and 2-methoxyethanamine, the title compound was obtained. Yield 59%, melting point 154 - 155°C (ethyl acetate-hexane).
¹H-NMR (CDCl₃) δ: 1.88 - 2.06 (1H, m), 2.63 - 2.80 (1H, m), 2.93 - 3.17 (2H, m), 3.39 (3H, s), 3.53 - 3.61 (2H, m), 3.63 - 3.73 (2H, m), 4.91 (1H, t, J = 8.0 Hz), 6.61 (1H, brs), 6.92 (1H, d, J = 8.3 Hz), 7.12 (1H, dd, J = 8.5, 2.1 Hz), 7.38 - 7.50 (3H, m), 7.65 (1H, s), 7.92 (1H, d, J = 9.1 Hz), 7.99 (1H, s), 8.74 (1H, d, J = 4.9 Hz).

### Example 109

### N-(2-amino-2-oxoethyl)-2-[3-(2,4-dichlorophenyl)-2,3-dihydro-1H-inden-5-yl]pyridine-4-carboxamide

In the same manner as in Example 73 and using 2-[3-(2,4-dichlorophenyl)-2,3-dihydro-1H-inden-5-yl]pyridine-4-carboxylic acid obtained in Reference Example 136 and glycinamide hydrochloride, the title compound was obtained. Yield 61 %, melting point 168 - 170°C (ethyl acetate-hexane).
¹H-NMR (CDCl₃) δ: 1.86 - 2.08 (1H, m), 2.63 - 2.80 (1H, m), 2.92 - 3.17 (2H, m), 4.18 (2H, d, J = 4.5 Hz), 4.90 (1H, t, J = 8.0 Hz), 5.52 (1H, brs), 5.90 (1H, brs), 6.91 (1H, d, J = 8.3 Hz), 7.12 (2H, dd, J = 8.3, 2.3 Hz), 7.37 - 7.47 (2H, m), 7.50 (1H, dd, J = 5.3, 1.5 Hz), 7.66 (1H, s), 7.92 (1H, d, J = 8.7 Hz), 8.02 (1H, s), 8.75 (1H, d, J = 4.9 Hz).

### Example 110

### N-[3-[3-(2,4-dichlorophenyl)-2,3-dihydro-1H-inden-5-yl]phenyl]-3-hydroxypropanamide

In the same manner as in Example 2 and using 3-[3-(2,4-dichlorophenyl)-2,3-dihydro-1H-inden-5-yl]aniline obtained in Reference Example 137 and 3-hydroxypropanoic acid, the title compound was obtained. Yield 21 %, melting point 202 - 203°C (THF-hexane).
¹H-NMR (CDCl₃) δ: 1.88 - 2.04 (1H, m), 2.57 - 2.67 (3H, m), 2.67 - 2.75 (1H, m), 2.96 - 3.07 (2H, m), 4.00 (2H, d, J = 4.2 Hz), 4.87 (1H, t, J = 8.0 Hz), 6.93 (1H, d, J = 8.7 Hz),7.12(1H,dd,J=8.3,1.9Hz),7.19(1H,s),7.30(1H,d,J= 10.6 Hz), 7.36 (2H, d, J = 7.6 Hz), 7.41 - 7.51 (3H, m), 7.64 (1H, s), 7.66 (1H, brs).

### Example 111

### N-[3-[3-(2,4-dichlorophenyl)-2,3-dihydro-1H-inden-5-yl]phenyl]-3-methoxypropanamide

In the same manner as in Example 1 and using 3-[3-(2,4-dichlorophenyl)-2,3-dihydro-1H-inden-5-yl]aniline obtained in Reference Example 137 and 3-methoxypropanoic acid, the title compound was obtained as an amorphous solid.
Yield 57%.
¹H-NMR (CDCl₃) δ: 1.86 - 2.05 (1H, m), 2.54 - 2.78 (3H, m), 2.90 - 3.13 (2H, m), 3.44 (3H, s), 3.73 (2H, t, J = 5.3 Hz), 4.87 (1H, t, J = 7.6 Hz), 6.93 (1H, d, J = 8.3 Hz), 7.12 (1H, d, J = 8.0 Hz), 7.17 - 7.28 (2H, m), 7.28 - 7.39 (2H, m), 7.41 - 7.53 (3H, m), 7.65 (1H, brs), 8.21 (1H, brs).

The structures of the compounds of Examples 93 to 111 are shown below.

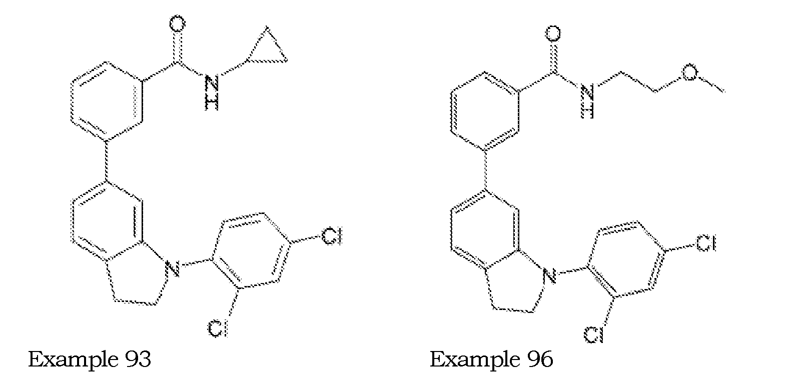

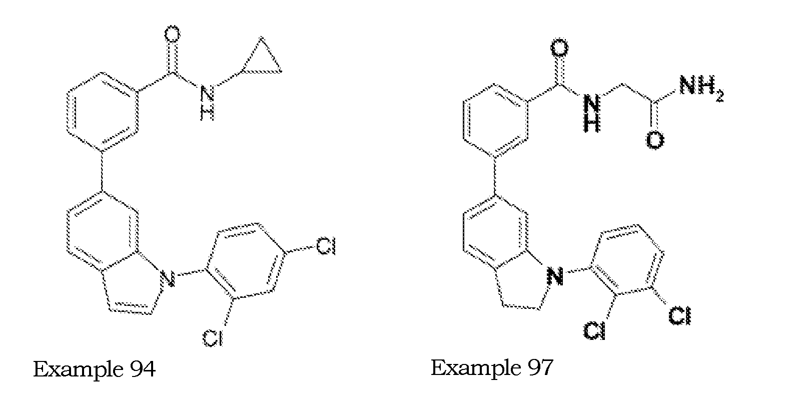

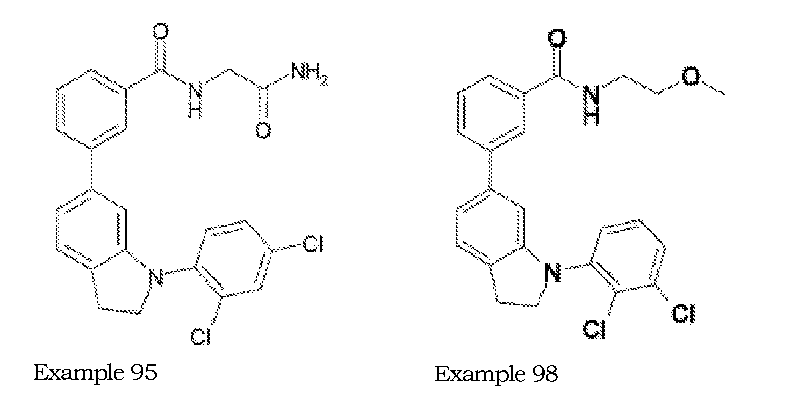

The structures of the compounds of Examples 93 to 111 are shown below.

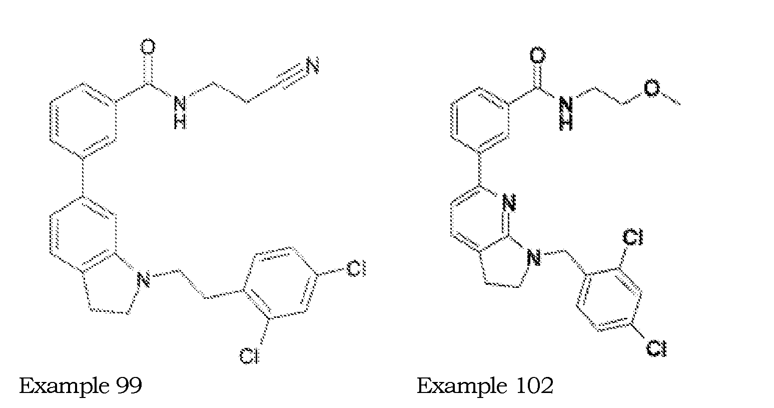

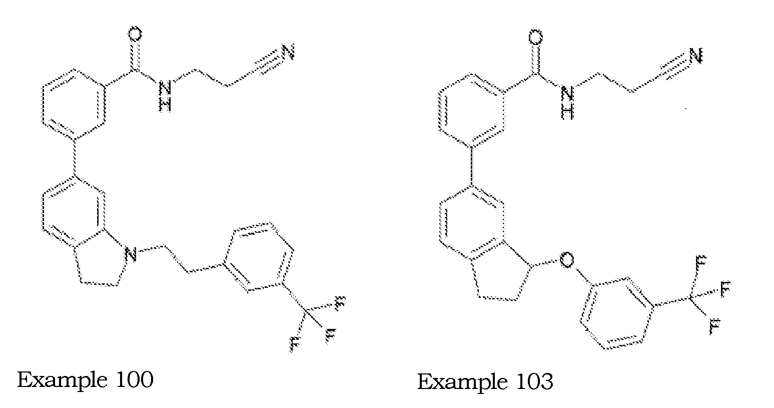

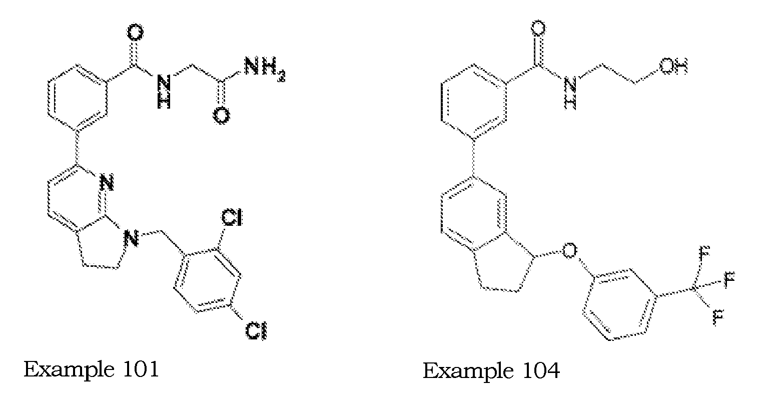

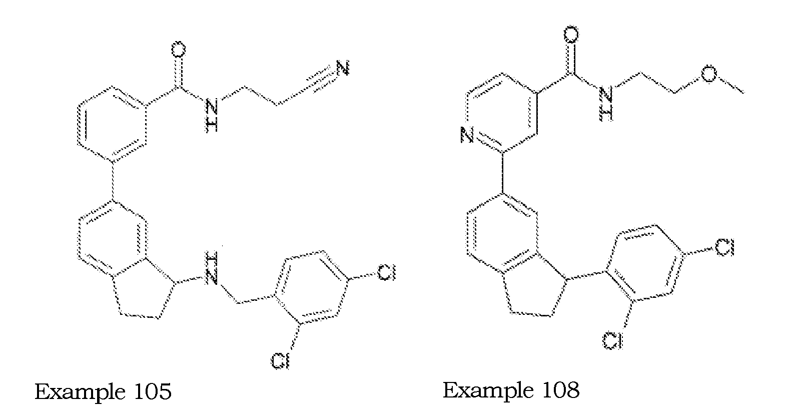

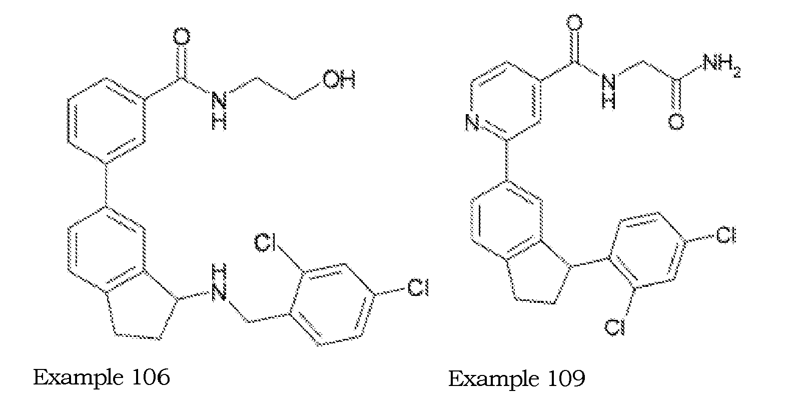

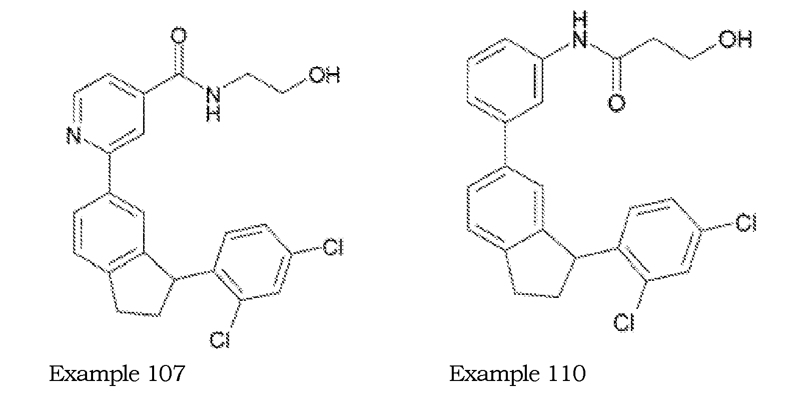

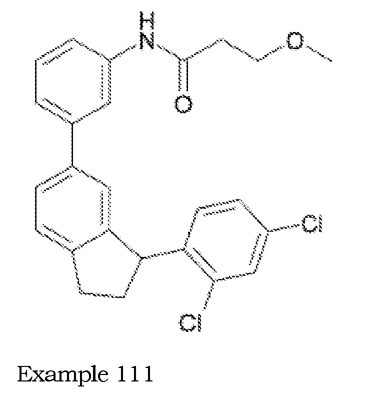

### Experimental Example

### Increase of intracellular cAMP concentration in human GPR52-expressing CHO cell

Using OptiPlate-384 (PerkinElmer Inc.), 1×10⁴ human GPR52-expressing CHO(dhfr-) cells were incubated together with 1 µM of a test compound in an assay buffer (30 µL, HBSS (containing Ca²⁺ and Mg²⁺), 0.5% BSA, 100 µm IBMX, 100 µm Ro20-1724, 5 mM HEPES (pH 7.55)) at 37°C for 30 min. Thereafter, according to the protocol of AlphaScreen cAMP Assay Kit (PerkinElmer Inc.), the intracellular cAMP concentration was measured by EnVision (PerkinElmer Inc.). The GPR52 agonist activity was calculated, assuming the intracellular cAMP concentration in the presence of 1 µM 3-(6-(2-(3,4-dimethoxyphenyl)ethoxy)pyridin-2-yl)-N-(2-pyrrolidin-1-ylethyl)benzamide (Reference Example 124) to be 100% and assuming the intracellular cAMP concentration when DMSO was added instead of the test compound to be 0%. The results are shown in Table 1.

**Table 1: GPR52 agonist activity**

| Example No. | GPR52 agonist activity (%) |
|---|---|
| 1 | 87 |
| 2 | 69 |
| 3 | 71 |
| 4 | 75 |
| 5 | 75 |
| 6 | 59 |
| 7 | 67 |
| 9 | 59 |
| 10 | 65 |
| 11 | 74 |
| 12 | 89 |
| 13 | 60 |
| 15 | 77 |
| 16 | 84 |
| 25 | 80 |
| 26 | 62 |
| 28 | 64 |
| 29 | 76 |
| 30 | 72 |
| 31 | 62 |
| 33 | 61 |
| 34 | 74 |
| 35 | 81 |
| 40 | 52 |
| 45 | 79 |
| 47 | 63 |
| 51 | 89 |
| 62 | 81 |
| 63 | 66 |
| 64 | 84 |
| 65 | 91 |
| 66 | 88 |
| 67 | 73 |
| 73 | 73 |
| 74 | 76 |
| 76 | 80 |
| 77 | 73 |
| 78 | 59 |
| 80 | 70 |
| 82 | 67 |
| 83 | 70 |
| 87 | 59 |
| 90 | 65 |

### Formulation Example 1

| | |
|---|---|
| (1) Compound of Example 1 | 10.0 g |
| (2) Lactose | 70.0 g |
| (3) Cornstarch | 50.0 g |
| (4) Soluble starch | 7.0 g |
| (5) Magnesium stearate | 3.0 g |

The compound of Example 1 (10.0 g) and magnesium stearate (3.0 g) are granulated with an aqueous solution (70 ml) of soluble starch (7.0 g as soluble starch), dried, and mixed with lactose (70.0 g) and cornstarch (50.0 g) (lactose, cornstarch, soluble starch and magnesium stearate are all products on the Japanese Pharmacopoeia 14th ed.). The mixture is compressed to give tablets.

### Industrial Applicability

Since the compound of the present invention has an agonist action on GPR52, it is useful as a medicament for the prophylaxis or treatment of mental diseases such as schizophrenia and the like, and the like.

## Claims

1. A compound represented by the formula (I) 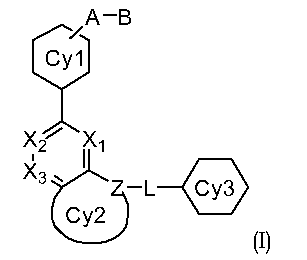 wherein
A is -CONRₐ- or -NRaCO-,
Ra is a hydrogen atom or a substituent,
B is a hydrogen atom or a substituent,
ring Cy1 is (1) a benzene ring or (2) a 6-membered nitrogen-containing aromatic heterocycle, each may have substituent(s) in addition to a group represented by -A-B, X¹, X² and X³ are each independently -CRx= or -N=,
Rx is independently a hydrogen atom, a halogen atom or a lower alkyl group which may be halogenated in each occurrence,
ring Cy2 is (1) a carbon ring having a carbon number of 5 to 7 or (2) a 5- to 7-membered heterocycle having 1 or 2 heteroatoms selected from a nitrogen atom, an oxygen atom and a sulfur atom, each may have substituent(s) (excluding oxo group, C₆₋₁₄ aryl group and carboxyl group which may be esterified),
Z is a carbon atom or a nitrogen atom,
L is a bond, -(CH₂)n-, -L'-, -L'-CH₂- or -CH₂-L'-,
n is 1 or 2,
L' is -O-, -NR^{b}- or -S(O)ₘ-,
R^{b} is a hydrogen atom or a substituent,
m is an integer of 0 to 2, and
ring Cy3 is (1) a benzene ring or (2) a 6-membered nitrogen-containing aromatic heterocycle, each may have substituent(s),
provided that a moiety represented by 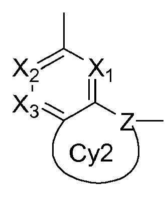 is not 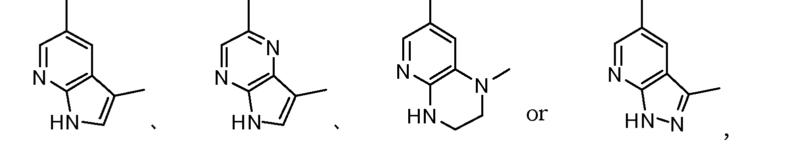 or a salt thereof.

2. The compound according to claim 1, wherein the ring Cy1 is a benzene ring or a pyridine ring.

3. The compound according to claim 1, wherein X¹ and X² are each independently - CH= or -N=.

4. The compound according to claim 1, wherein the ring Cy2 is (1) a carbon ring having a carbon number of 5 or 6 or (2) a 5- or 6-membered heterocycle having 1 or 2 heteroatoms selected from a nitrogen atom, an oxygen atom and a sulfur atom, each may have substituent(s) (excluding oxo group, C₆₋₁₄ aryl group and carboxyl group which may be esterified).

5. The compound according to claim 1, wherein the moiety represented by 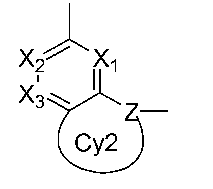 is

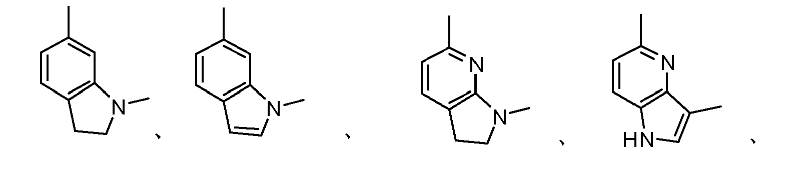

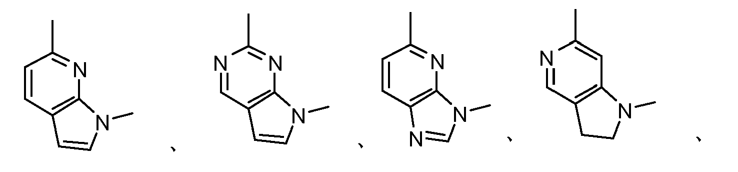

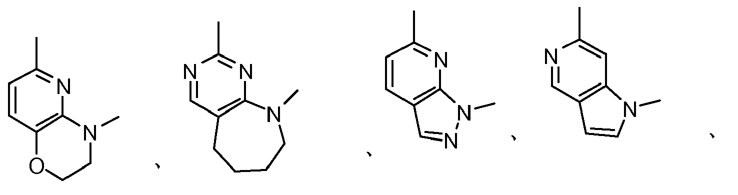

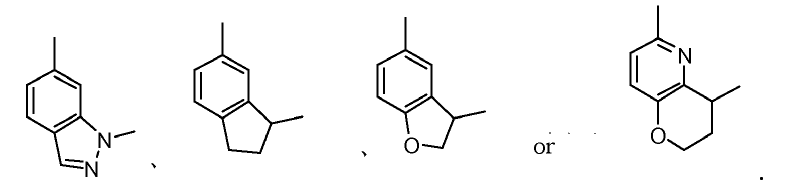

6. The compound according to claim 1, wherein the moiety represented by 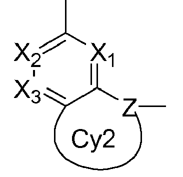 is

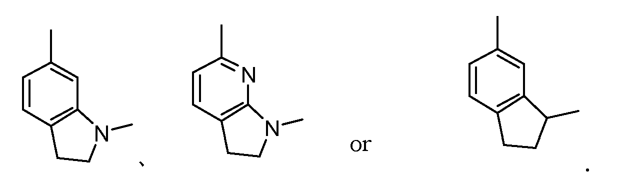

7. The compound according to claim 1, wherein L is a bond, -CH₂-, -O-, -NR^{b}- or - S(O)ₘ-.

8. The compound according to claim 1, wherein Cy3 is a dichlorobenzene ring.

9. The compound according to claim 1, wherein the ring Cy1 is a benzene ring or a pyridine ring,
X¹ and X² are each independently -CH= or -N=,
the ring Cy2 is (1) a carbon ring having a carbon number of 5 or 6 or (2) a 5- or 6-membered heterocycle having 1 or 2 heteroatoms selected from a nitrogen atom, an oxygen atom and a sulfur atom, each may have substituent(s) (excluding carboxyl group which may be esterified),
L is a bond, -CH₂-, -O-, -NR^{b}- or -S(O)ₘ-, and
Cy3 is a dichlorobenzene ring.

10. The compound according to claim 1, wherein
A is -CONH- or -CONH-,
B is
(1) a C₁₋₆ alkyl group which may have one or more substituents selected from
(a) a cyano group,
(b) a hydroxy group,
(c) C₁₋₆ alkoxy,
(d) a di-C₁₋₆ alkyl-amino group,
(e) a carbamoyl group,
(f) a C₁₋₆ alkyl-sulfanyl group,
(g) a C₁₋₆ alkyl-sulfinyl group,
(h) a C₁₋₆ alkyl-sulfonyl group, and
(i) a 5- to 7-membered heterocyclic group having one or more heteroatoms selected from a nitrogen atom, an oxygen atom and a sulfur atom,
(2) a C₃₋₁₀ cycloalkyl group, or
(3) a 5- to 7-membered heterocyclic group,
the ring Cy1 is (1) a benzene ring or (2) a 6-membered nitrogen-containing heterocycle,
the moiety represented by 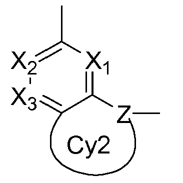 is 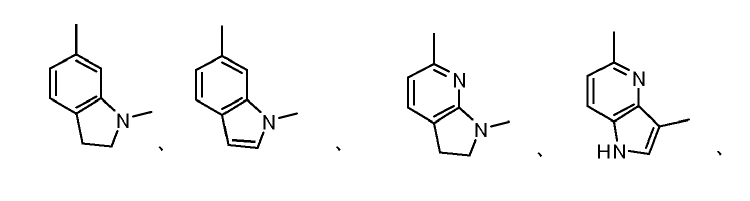 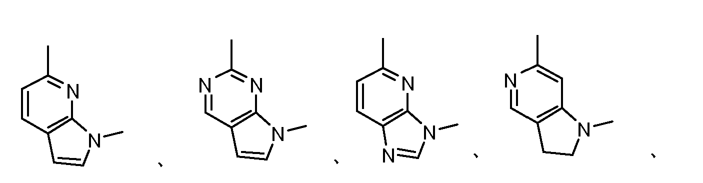 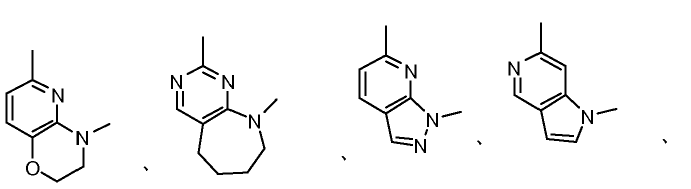 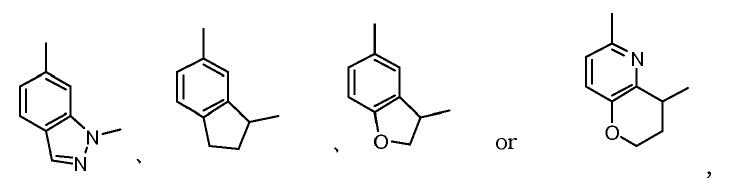 L is a bond, -CH₂-, -NH- or -O-, and
the ring Cy3 is a benzene ring or a pyridine ring, each may have one or more substituents selected from a halogen atom, a C₁₋₆ alkyl group which may be halogenated and a C₁₋₆ alkoxy group which may be halogenated.

11. The compound according to claim 1, which is N-(2-cyanoethyl)-3-[1-(2,4-dichlorophenyl)-2,3-dihydro-1H-indol-6-yl]benzamide or a salt thereof.

12. The compound according to claim 1, which is 3-[1-(2,4-dichlorobenzyl)-1H-pyrrolo[2,3-b]pyridin-6-yl]-N-(2-hydroxyethyl)benzamide or a salt thereof.

13. The compound according to claim 1, which is 3-[1-(2,4-dichlorobenzyl)-2,3-dihydro-1H-pyrrolo[2,3-b]pyridin-6-yl]-N-(2-hydroxyethyl)benzamide or a salt thereof.

14. The compound according to claim 1, which is 3-[3-[2-(3,4-dimethoxyphenyl)ethyl]-3H-imidazo[4,5-b]pyridin-5-yl]-N-(2-pyrrolidin-1-ylethyl)benzamide or a salt thereof.

15. The compound according to claim 1, which is N-(2-cyanoethyl)-3-[4-(2,4-dichlorophenyl)-3,4-dihydro-2H-1,4-benzoxazin-6-yl]benzamide or a salt thereof.

16. The compound according to claim 1, which is 3-[3-(2,4-dichlorophenyl)-2,3-dihydro-1H-inden-5-yl]-N-[2-(methylsulfinyl)ethyl]benzamide or a salt thereof.

17. The compound according to claim 1, which is N-(2-cyanoethyl)-3-[3-(2,4-dichlorophenoxy)-2,3-dihydro-1H-inden-5-yl]benzamide or a salt thereof.

18. The compound according to claim 1, which is 3-[3-[(2,4-dichlorophenyl)amino]-2,3-dihydro-1-benzofuran-5-yl]-N-(2-hydroxyethyl)benzamide or a salt thereof.

19. A prodrug of the compound according to claim 1.

20. A medicament comprising the compound according to claim 1 or the prodrug according to claim 19.

21. A GPR52 activating agent comprising a compound represented by the formula (I₀) 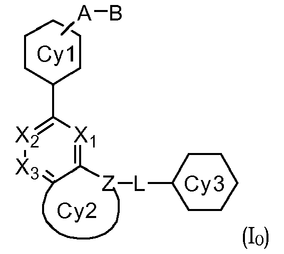 wherein
A is -CONRa- or -NRaCO-,
R^{a} is a hydrogen atom or a substituent,
B is a hydrogen atom or a substituent,
ring Cy1 is (1) a benzene ring or (2) a 6-membered nitrogen-containing aromatic heterocycle, each may have substituent(s) in addition to a group represented by -A-B,
X¹, X² and X³ are each independently -CR^{x}= or -N=,
R^{x} is independently a hydrogen atom, a halogen atom or a lower alkyl group which may be halogenated in each occurrence,
ring Cy2 is (1) a carbon ring having a carbon number of 5 to 7 or (2) a 5- to 7-membered heterocycle having 1 or 2 heteroatoms selected from a nitrogen atom, an oxygen atom and a sulfur atom, each may have substituent(s) (excluding oxo group),
Z is a carbon atom or a nitrogen atom,
L is a bond, -(CH₂)n-, -L'-, -L'-CH₂- or -CH₂-L'-,
n is 1 or 2,
L' is -O-, -NR^{b}- or -S(O)ₘ-,
R^{b} is a hydrogen atom or a substituent,
m is an integer of 0 to 2, and
ring Cy3 is (1) a benzene ring or (2) a 6-membered nitrogen-containing aromatic heterocycle, each may have substituent(s),
provided that a moiety represented by 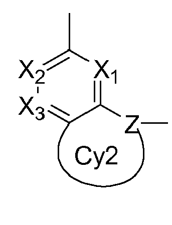 is not 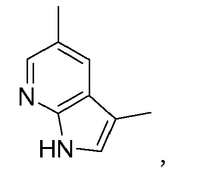 or a salt thereof or a prodrug thereof.

22. A prophylactic or therapeutic agent for schizophrenia comprising a compound represented by the formula (I₀) 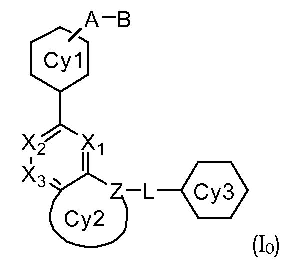 wherein
A is -CONR^{a}- or -NR^{a}CO-,
R^{a} is a hydrogen atom or a substituent,
B is a hydrogen atom or a substituent,
ring Cy1 is (1) a benzene ring or (2) a 6-membered nitrogen-containing aromatic heterocycle, each may have substituent(s) in addition to a group represented by -A-B,
X¹, X² and X³ are each independently -CR^{x}= or -N=,
R^{x} is independently a hydrogen atom, a halogen atom or a lower alkyl group which may be halogenated in each occurrence,
ring Cy2 is (1) a carbon ring having a carbon number of 5 to 7 or (2) a 5- to 7-membered heterocycle having 1 or 2 heteroatoms selected from a nitrogen atom, an oxygen atom and a sulfur atom, each may have substituent(s) (excluding oxo group),
Z is a carbon atom or a nitrogen atom,
L is a bond, -(CH₂)n-, -L'-, -L'-CH₂- or -CH₂-L'-,
n is 1 or 2,
L' is -O-, -NR^{b}- or -S(O)ₘ-,
R^{b} is a hydrogen atom or a substituent,
m is an integer of 0 to 2, and
ring Cy3 is (1) a benzene ring or (2) a 6-membered nitrogen-containing aromatic heterocycle, each may have substituent(s),
provided that a moiety represented by 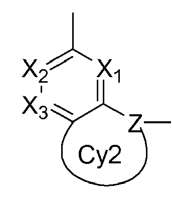 is not 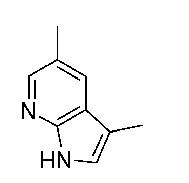 or a salt thereof or a prodrug thereof.

23. A method of activating GPR52, comprising administering, to a subject, an effective amount of a compound represented by the formula (I₀) 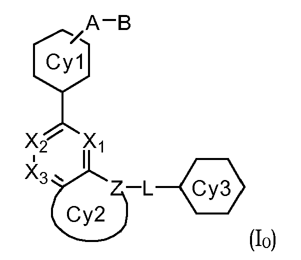 wherein
A is -CONR^{a}- or -NR^{a}CO-,
R^{a} is a hydrogen atom or a substituent,
B is a hydrogen atom or a substituent,
ring Cy1 is (1) a benzene ring or (2) a 6-membered nitrogen-containing aromatic heterocycle, each may have substituent(s) in addition to a group represented by -A-B,
X¹, X² and X³ are each independently -CR^{x}= or -N=,
R^{x} is independently a hydrogen atom, a halogen atom or a lower alkyl group which may be halogenated in each occurrence,
ring Cy2 is (1) a carbon ring having a carbon number of 5 to 7 or (2) a 5- to 7-membered heterocycle having 1 or 2 heteroatoms selected from a nitrogen atom, an oxygen atom and a sulfur atom, each may have substituent(s) (excluding oxo group),
Z is a carbon atom or a nitrogen atom,
L is a bond, -(CH₂)n-, -L'-, -L'-CH₂- or -CH₂-L'-,
n is 1 or 2,
L' is -O-, -NR^{b}- or -S(O)ₘ-,
R^{b} is a hydrogen atom or a substituent,
m is an integer of 0 to 2, and
ring Cy3 is (1) a benzene ring or (2) a 6-membered nitrogen-containing aromatic heterocycle, each may have substituent(s),
provided that a moiety represented by 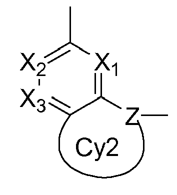 is not 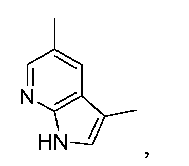 or a salt thereof or a prodrug thereof.

24. A method of preventing or treating schizophrenia, comprising administering, to a subject, an effective amount of a compound represented by the formula (I₀) 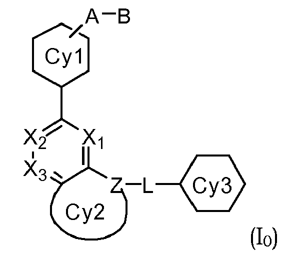 wherein
A is -CONR^{a}- or -NR^{a}CO-,
R^{a} is a hydrogen atom or a substituent,
B is a hydrogen atom or a substituent,
ring Cy1 is (1) a benzene ring or (2) a 6-membered nitrogen-containing aromatic heterocycle, each may have substituent(s) in addition to a group represented by -A-B,
X¹, X² and X³ are each independently -CR^{x}= or -N=,
R^{x} is independently a hydrogen atom, a halogen atom or a lower alkyl group which may be halogenated in each occurrence,
ring Cy2 is (1) a carbon ring having a carbon number of 5 to 7 or (2) a 5- to 7-membered heterocycle having 1 or 2 heteroatoms selected from a nitrogen atom, an oxygen atom and a sulfur atom, each may have substituent(s) (excluding oxo group),
Z is a carbon atom or a nitrogen atom,
L is a bond, -(CH₂)n-, -L'-, -L'-CH₂- or -CH₂-L'-,
n is 1 or 2,
L' is -O-, -NR^{b}- or -S(O)ₘ-,
R^{b} is a hydrogen atom or a substituent,
m is an integer of 0 to 2, and
ring Cy3 is (1) a benzene ring or (2) a 6-membered nitrogen-containing aromatic heterocycle, each may have substituent(s),
provided that a moiety represented by 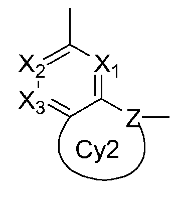 is not 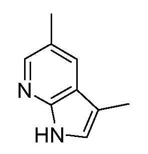 or a salt thereof or a prodrug thereof.

25. Use of a compound represented by the formula (I₀) 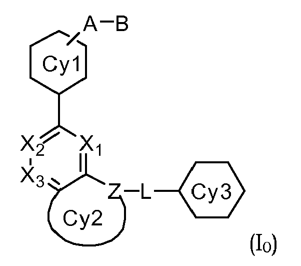 wherein
A is -CONR^{a}- or -NR^{a}CO-,
R^{a} is a hydrogen atom or a substituent,
B is a hydrogen atom or a substituent,
ring Cy1 is (1) a benzene ring or (2) a 6-membered nitrogen-containing aromatic heterocycle, each may have substituent(s) in addition to a group represented by -A-B,
X¹, X² and X³ are each independently -CR^{x}= or -N=,
R^{x} is independently a hydrogen atom, a halogen atom or a lower alkyl group which may be halogenated in each occurrence,
ring Cy2 is (1) a carbon ring having a carbon number of 5 to 7 or (2) a 5- to 7-membered heterocycle having 1 or 2 heteroatoms selected from a nitrogen atom, an oxygen atom and a sulfur atom, each may have substituent(s) (excluding oxo group),
Z is a carbon atom or a nitrogen atom,
L is a bond, -(CH₂)n-, -L'-, -L'-CH₂- or -CH₂-L'-,
n is 1 or 2,
L' is -O-, -NR^{b}- or -S(O)ₘ-,
R^{b} is a hydrogen atom or a substituent,
m is an integer of 0 to 2, and
ring Cy3 is (1) a benzene ring or (2) a 6-membered nitrogen-containing aromatic heterocycle, each may have substituent(s),
provided that a moiety represented by 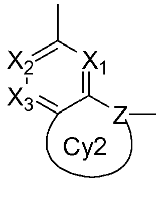 is not 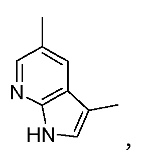 or a salt thereof or a prodrug thereof, for the manufacture of a GPR52 activating agent.

26. Use of a compound represented by the formula (I₀) 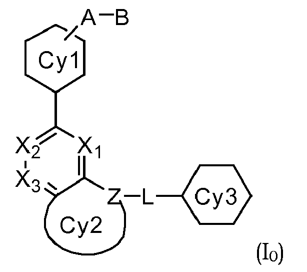 wherein
A is -CONR^{a}- or -NR^{a}CO-,
R^{a} is a hydrogen atom or a substituent,
B is a hydrogen atom or a substituent,
ring Cy1 is (1) a benzene ring or (2) a 6-membered nitrogen-containing aromatic heterocycle, each may have substituent(s) in addition to a group represented by -A-B,
X¹, X² and X³ are each independently -CR^{x}= or -N=,
R^{x} is independently a hydrogen atom, a halogen atom or a lower alkyl group which may be halogenated in each occurrence,
ring Cy2 is (1) a carbon ring having a carbon number of 5 to 7 or (2) a 5- to 7-membered heterocycle having 1 or 2 heteroatoms selected from a nitrogen atom, an oxygen atom and a sulfur atom, each may have substituent(s) (excluding oxo group),
Z is a carbon atom or a nitrogen atom,
L is a bond, -(CH₂)n-, -L'-, -L'-CH₂- or -CH₂-L'-, n is 1 or 2,
L' is -O-, -NR^{b}- or -S(O)ₘ-,
R^{b} is a hydrogen atom or a substituent,
m is an integer of 0 to 2, and
ring Cy3 is (1) a benzene ring or (2) a 6-membered nitrogen-containing aromatic heterocycle, each may have substituent(s),
provided that a moiety represented by 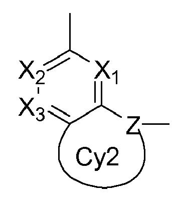 is not 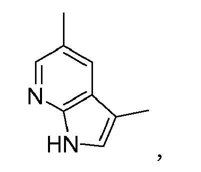 or a salt thereof or a prodrug thereof, for the manufacture of a prophylactic or therapeutic agent for schizophrenia.
